# EUROPEAN PATENT APPLICATION

(11) **EP 1 389 460 A1**
(43) Date of publication of application: **18.02.2004**
(21) Application number: 02771732.1
(22) Date of filing: 20.05.2002
(51) Int. Cl.: A61K 31/40, A61K 31/4164, A61K 31/4178, A61K 31/4402, A61K 31/4439, A61K 31/444, A61K 31/4709, A61K 31/506, A61P 29/00, A61P 35/04, A61P 43/00, C07D 207/337, C07D 213/36, C07D 215/40, C07D 233/64, C07D 233/88, C07D 401/12, C07D 401/14, C07D 403/12, C07D 409/14

(54) **CXCR4-ANTAGONISTIC DRUGS COMPRISING NITROGEN-CONTAINING COMPOUND**

(30) Priority: 24.05.2001 JP 2001154904
(71) Applicant: Kureha Chemical Industry Co., Ltd., Tokyo 103-8552 (JP)
(72) Inventor: YANAKA, Mikiro, Matsudo-shi, Chiba 271-0092 (JP); YAMAZAKI, Toru, Katsushika-ku, Tokyo 124-0022 (JP); BANNAI, Kenji, Nerima-ku, Tokyo 176-0001 (JP); HIROSE, Kunitaka, Nerima-ku, Tokyo 179-0074 (JP)
(74) Representative: Forstmeyer, Dietmar, Dr. rer. nat., Dipl.-Chem.
(86) International application number: PCT/JP2002/004846
(87) International publication number: WO 2002/094261

(57) **Abstract**

To provide novel nitrogen-containing compounds having antagonism to CXCR4 and remedies for disease, such as rheumatism, cancer metastasis, etc., based on the CXCR4 antagonism.

Nitrogen-containing compounds represented by the following general formula and CXCR4 antagonists containing these compounds as an active ingredient can be provided.
The above compounds are typified by nitrogen-containing compounds represented by the following general formula (I) 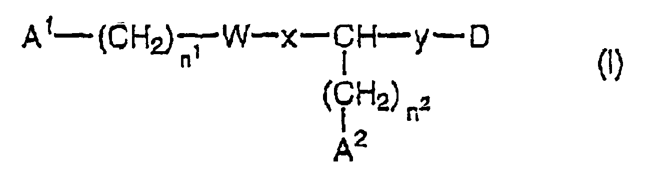 wherein A¹ and A² represent each a guanidino group or a group represented by the following general formula (ia) 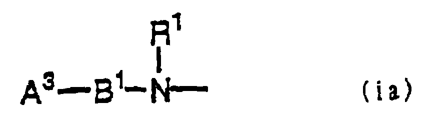 (wherein A³ represents a monocyclic or polycyclic heterocyclic aromatic ring group having 1 or 2 hetero atoms; B¹ represents a single bond or alkylene group; and R¹ represents hydrogen or alkyl group; W represents alkylene group having 2 to 3 carbon atoms, cyclic alkylene group having 5 to 10 carbon atoms, aromatic ring having 6 to 10 carbon atoms or heterocyclic aromatic ring having 5 to 10 carbon atoms; y is - (C=O) -; x is -C (=O) -NH-; n¹ is an integer of 1 or 2; n² is an integer of 2 or 3; and D is selected from among various substituents:

## Description

### Technical Field

The present invention relates to a pharmaceutical agent for associated diseases such as rheumatism and cancer metastasis, which is composed of a nitrogen-containing compound or a pharmaceutically-acceptable salt thereof as an active ingredient and is particularly based on antagonism to chemokine receptor CXCR4.

### Background Art

Among cytokines, those indicating chemotaxis to leucocyte are referred to as chemokines. Chemokines are secreting proteins classified into CXC-chemokines, CC-chemokines, C-chemokines, and CX3C-kemokines on the basis of cysteine (Cys) sequences on their respective N-terminals. The number of thereof is said to approximately 30. In those chemokine receptors, there are several subtypes. Of those, CXCR4 has proved to be relevant to various diseases using CXC-chemokine SDF-1 as ligands. For instance, rheumatism (WO 00/06086, The Journal of Immunology, 165, 659 0 (2000)) and cancer metastasis (Nature, 410, 50 (2001)) have been reported. As a therapeutic drug for those diseases, there is a strong need for a novel low-molecular pharmaceutical agent having CXCR4 antagonism and also having little toxicity and side effect to allow its employment for a long period of time.
Heretofore, AMD 3100, or the like is known as such a low molecular compound (WO 00/02870), although not being sufficient enough to meet the above need.

### Disclosure of the Invention

An object of the present invention is to provide a pharmaceutical agent having excellent CXCR4 antagonism and high safety.

The inventors of the present invention have concentrated on the study of development of a compound useful as a novel CXCR4 antagonist. As a result, they have discovered a group of nitrogen-containing compounds that are given as those exhibiting strong CXCR4 antagonism and thus having potential therapeutic abilities for rheumatism,cancer metastases,andso on. Therefore, the present invention offers pharmaceutical agents for the therapy of patients suffering from rheumatism, cancer metastases, and so on, composed of compounds having CXCR4 antagonism and being represented by the general formula (1) as defined bellow. 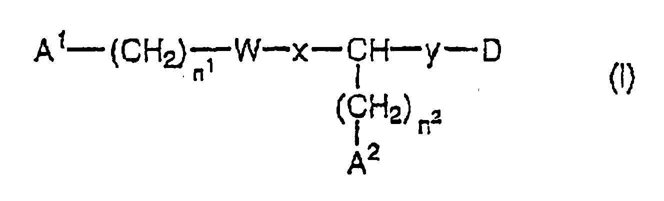 where n¹ represents an integer of 0 to 3, and n² represents an integer of 0 to 4, and
each of A¹ and A² independently represents (a) a guanidino group, which may be substituted with a nitro group, a cyano group, an alkyl group having 1 to 6 carbon atoms, or an alkylene group having 2 or 3 carbon atoms, (b) an amidino group, which may be substituted with an nitro group, a cyano group, an alkyl group having 1 to 6 carbon atoms, or an alkylene group having 2 or 3 carbon atoms, or (c) a group represented by the following formula (i) : 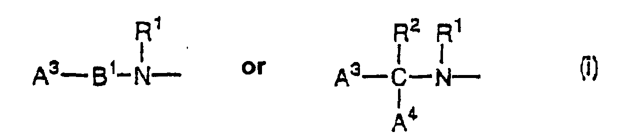 where each of A³ and A⁴ independently represents a 5 to 12-membered, preferably 5 to 10-membered, monocyclic or polycyclic heterocyclic aromatic ring, which contains 1 to 4 nitrogen atoms and may contain 1 or 2 other hetero atoms, wherein a hydrogen atom on said nitrogen atoms may be substituted, or a 5 to 12-membered, preferably 5 to 10-membered, monocyclic or polycyclic heterocyclic aromatic ring, which contains 1 to 3 nitrogen atoms and may contain 1 or 2 other hetero atoms, wherein a hydrogen atom on said nitrogen atoms may be substituted and said heterocyclic aromatic ring may be partially saturated, and
B¹ represents a single bond or a group represented by the following formula (ii): 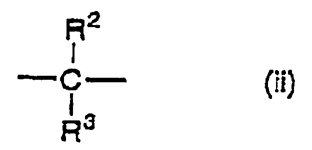 where each of R¹, R², and R³ independently represents a hydrogen atom, an alkyl group having 1 to 6 carbon atoms, which may be substituted, an alkenyl group having 2 to 6 carbon atoms, which may be substituted, and an alkynyl group having 2 to 6 carbon atoms, which may be substituted, and R² may be bonded to R¹ or R³ to form a ring,
W represents an alkylene group having 1 to 7 carbon atoms, preferably 2 to 5 carbon atoms, which may be substituted, an alkenylene group having 2 to 7 carbon atoms, preferably 2 to 5 carbon atoms, which may be substituted, an alkynylene group having 2 to 7 carbon atoms, preferably 2 to 5 carbon atoms, which may be substituted, or a monocyclic or polycyclic cyclic alkylene group having 3 to 10 carbon atoms, preferably 5 to 10 carbon atoms, which may be substituted, or a 6 to 15-membered, preferably 6 to 10-membered, monocyclic or polycyclic aromatic ring which may be substituted, or a partially-saturated 6 to 15-membered, preferably 6 to 10-membered, monocyclic or polycyclic aromatic ring which may be substituted, or a 5 to 15-membered, preferably 5 to 10-membered, monocyclic or polycyclic heterocyclic aromatic ring, which may contain 1 to 3 oxygen atoms, 1 to 3 sulfur atoms, and 1 to 3 nitrogen atoms and may be substituted, or a partially-saturated 5 to 15-membered, preferably 5 to 10-membered, monocyclic or polycyclic heterocyclic aromatic ring, which may contain 1 to 3 oxygen atoms, 1 to 3 sulfur atoms, and 1 to 3 nitrogen atoms and may be substituted, or a 3 to 15-membered, preferably 5 to 10-membered, monocyclic or polycyclic saturated heterocyclic ring, which may contain 1 to 3 oxygen atoms, 1 to 3 sulfur atoms, and 1 to 3 nitrogen atoms and may be substituted, and
D represents a functional group represented by the following formula (iii):

**―W**^{**1**}**-G**^{**1**}**-G**^{**2**}**-W**^{**2**}**-G**^{**3**} **(iii)**

where W¹ represents an oxygen atom, a sulfur atom, or a functional group represented by the following formula (iv): 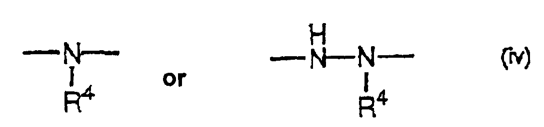 where R⁴ represents a hydrogen atom, or -G^{1'}-G^{2'}-W^{2'}-G^{3'}-, and
each of G¹ and G^{1'} independently represents a single bond, or a straight- or branched-chain alkylene group having 1 to 10 carbon atoms, preferably 1 to 5 carbon atoms, which may be substituted, a straight- or branched-chain alkenylene group having 2 to 10 carbon atoms and 1 or 2 double bounds, which may be substituted, a straight- or branched-chain alkynylene group having 2 to 10 carbon atoms, preferably 2 to 5 carbon atoms and 1 or 2 triple bonds, which may be substituted, or a functional group represented by the following formula (v): 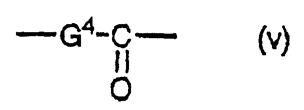 where G⁴ represents an alkylene group having 1 to 3 carbon atoms, which may be substituted,
each of G² and G^{2'} independently represents a single bond, or a monocyclic or polycyclic cyclic alkylene group having 3 to 10 carbon atoms, which may be substituted, a 6 to 15-membered, preferably 6 to 10-membered, monocyclic or polycyclic aromatic ring, which may be substituted, or a partially-saturated 6 to 15-membered, monocyclic or polycyclic aromatic ring, which may be substituted, or a 5 to 15-membered, preferably 5 to 10-membered, monocyclic or polycyclic heterocyclic aromatic ring having 1 to 3 oxygen atoms, 1 to 3 sulfur atoms, and 1 to 3 nitrogen atoms, which may be substituted, or a partially-saturated 5 to 15-membered monocyclic or polycyclic heterocyclic aromatic ring having 1 to 3 oxygen atoms, 1 to 3 sulfur atoms, and 1 to 3 nitrogen atoms, which may be substituted, or a 3 to 15-memered, preferably 5 to 10-membered, saturated heterocyclic ring which may contain 1 to 3 oxygen atoms, 1 to 3 sulfur atoms, and 1 to 3 nitrogen atoms and may be substituted, and
each of W² and W^{2'} independently represents a single bond, or an oxygen atom, a sulfur atom, or a functional group represented by the following formula (vi): 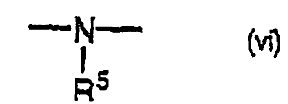 where R⁵ represents a hydrogen atom, a straight- or branched-chain alkyl group having 1 to 10 carbon atoms, which may be substituted, or G^{3''}, which may form a ring with G¹ or G² when R⁵ represents the alkyl group,
each of G³, G^{3'}, and G^{3''} independently represents a hydrogen atom, a straight- or branched-chain alkyl group having 1 to 6 carbon atoms, which may be substituted, a straight- or branched alkenyl group having 2 to 6 carbon atoms and 1 or 2 double bonds, which may be substituted, a straight- or branched-chain alkynyl group having 2 to 6 carbon atoms and 1 or 2 triple bonds, which may be substituted, or a monocyclic or polycyclic cyclic alkylene group having 3 to 10 carbon atoms, which may be substituted, or an aralkyl group having 7 to 15 carbon atoms, which may be substituted, or a 6 to 15-membered, preferably 6 to 10-membered, monocyclic or polycyclic aromatic ring, which may be substituted, or a partially-saturated 6 to 15-membered monocyclic or polycyclic aromatic ring which may be substituted, or a 5 to 15-membered, preferably 5 to 10-membered, monocyclic or polycyclic heterocyclic aromatic ring, which may contain 1 to 3 oxygen atoms, 1 to 3 sulfur atoms, and 1 to 3 nitrogen atoms and may be substituted, or a partially-saturated 5 to 15-membered, preferably 5 to 10-membered, monocyclic or polycyclic heterocyclic aromatic ring, which may contain 1 to 3 oxygen atoms, 1 to 3 sulfur atoms, and 1 to 3 nitrogen atoms and may be substituted, or a 3 to 15-membered, preferably 5 to 10-membered, saturated heterocyclic ring, which may contain 1 to 3 oxygen atoms, 1 to 3 sulfur atoms, and 1 to 3 nitrogen atoms and may be substituted, and
x represents a functional group represented by the following general formula (vii): 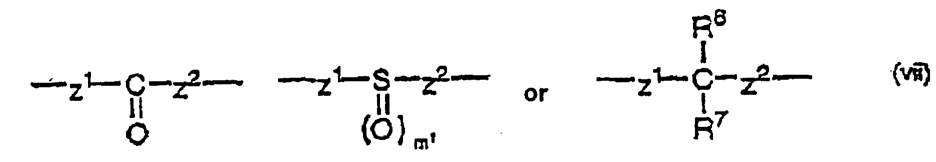 where each of z¹ and z² independently represents a single bond, a methylene group, an oxygen atom, a sulfur atom, or a substituent represented by the following formula (viii),: 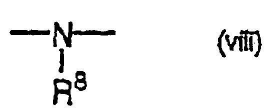 where each of R⁶, R⁷, and R⁸ is a hydrogen atom, or an alkyl group having 1 to 3 carbon atoms, which may be substituted, and m¹ represents an integer of 0 to 2, and
y represents a functional group represented by the following formula (ix): 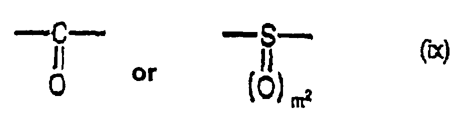 where m² represents an integer of 0 to 2,
wherein when the compounds include asymmetric points, each of absolute configurations thereof may include R, S, or a mixture thereof.

Further, in the nitrogen-containing compound using in the present invention, preferably, n¹ represents an integer of 1 or 2 and n² represents an integer of 2 or 3 in the general formula (I). Further, preferably, z¹ represents a single bond and z² represents the following formula (viii'): 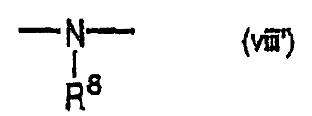 (where R⁸ represents a hydrogen atom or an alkyl group having 1 to 3 carbon atoms, which may be substituted).

Further, y preferably represents the following general formula (ix'):

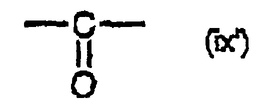

Further, W¹ preferably represents the following formula (iv'): 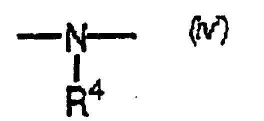 (R⁴ is based on the same definition as descried above.)

Further, a preferable compound as the nitrogen-containing compound according to the present invention includes a compound or its salt in which,
in the general formula (I), each of A¹ and A² represents a guanidino group or is represented by the following formula (ia): 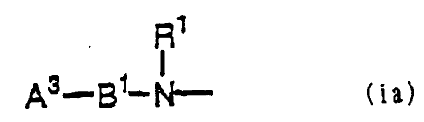 where A³ represents a monocyclic heterocyclic aromatic ring having 1 or 2 hetero atoms wherein a hydrogen atom on said nitrogen atoms may be substituted, or a bicyclic heterocyclic aromatic ring having 1 or 2 hetero atoms, wherein a hydrogen atom on said nitrogen atoms may be substituted and said heterocyclic aromatic ring may be partially saturated, B¹ and R¹ are each based on the same definition as descried above, W represents an alkylene group having 2 to 3 carbon atoms, a cyclic alkylene group having 5 to 10 carbon atoms, a monocyclic or bicyclic aromatic ring having 6 to 10 carbon atoms, or a heterocyclic aromatic ring having 5 to 10 carbon atoms, y represents -C(=O)-, X represents - (CH₂)ₙ₃-(C=O) -NH-, (where n³ represents 0 or 1), and n¹, n², and D are the same definition as defined above.

Further, a preferable compound as the nitrogen-containing compound according to the present invention is a compound or its salt in which, in the general formulas (I) and (iii), A¹, A², W, x, y, n¹, and n² are based on the same definition as described above, W¹ represents -NR⁴-, R⁴ represents a hydrogen atom or a straight- or branched-chain alkyl group having 1 to 5 carbon atoms, G¹ represents a straight- or branched-chain alkylene group having 1 to 5 carbon atoms, G² represents a single bond, W² represents a single bond, or an oxygen atom or a sulfur atom, G³ represents a monocyclic or polycyclic aromatic ring having 6 to 15 carbon atoms, which may be substituted, or a 3 to 15-membered monocyclic or polycyclic heterocyclic aromatic ring, which may contain 1 to 3 oxygen atoms, 1 to 3 sulfur atoms, and 1 to 3 nitrogen atoms and may be substituted.

Here, the substituent D which is a combination of W¹, G¹, G², W², and G³ is preferably a substituent represented by the following formulas.

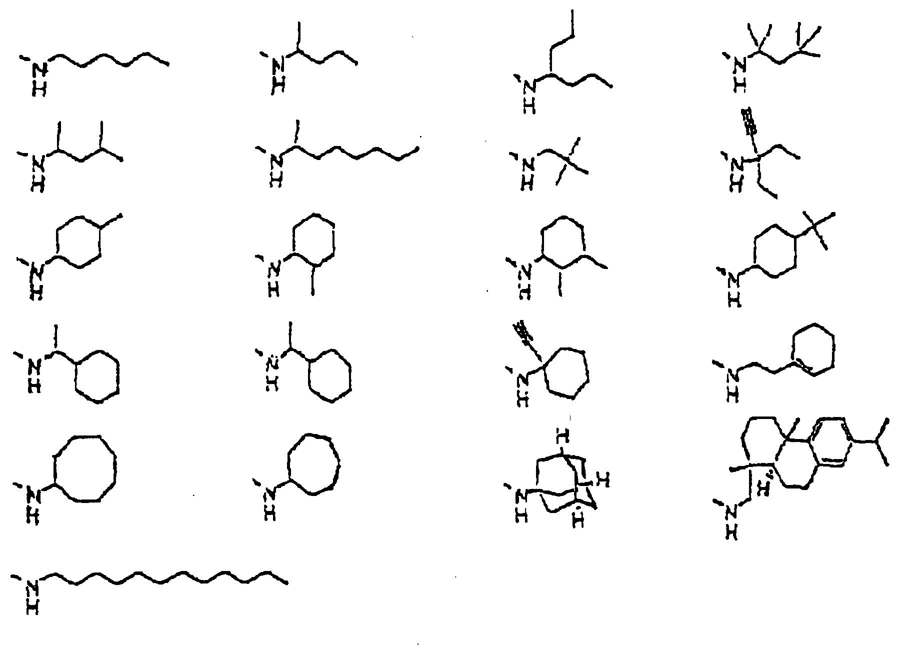

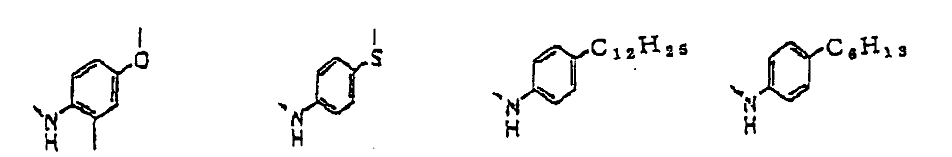

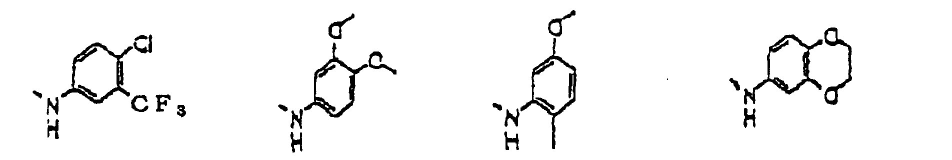

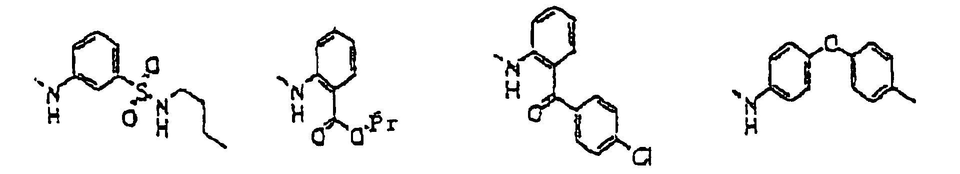

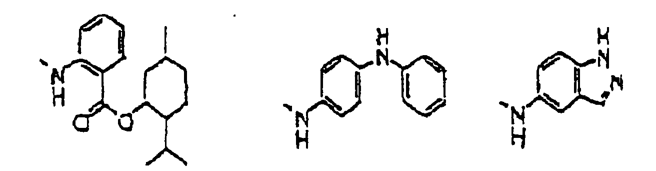

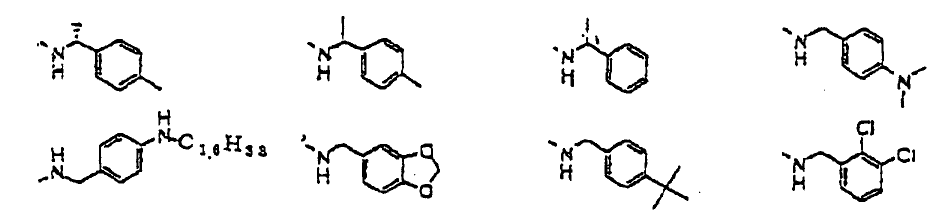

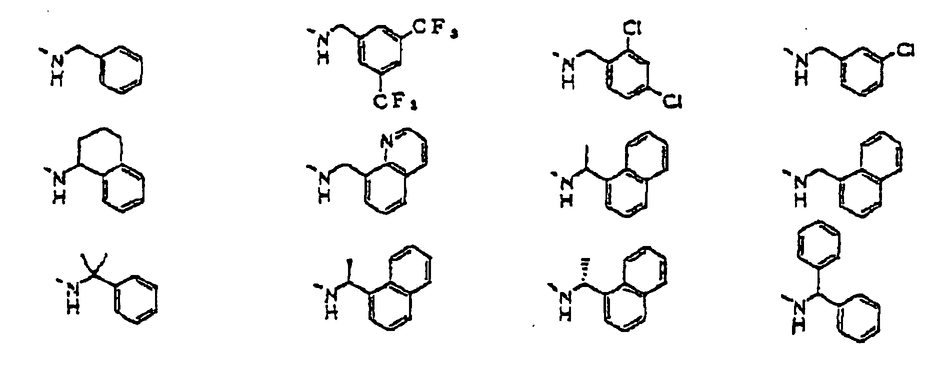

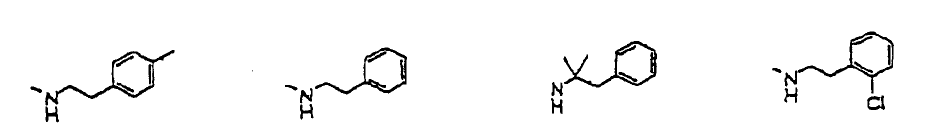

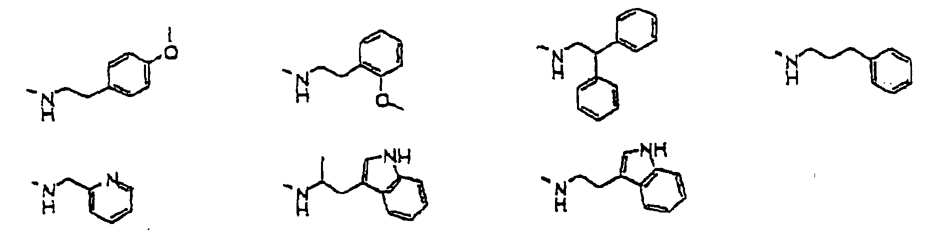

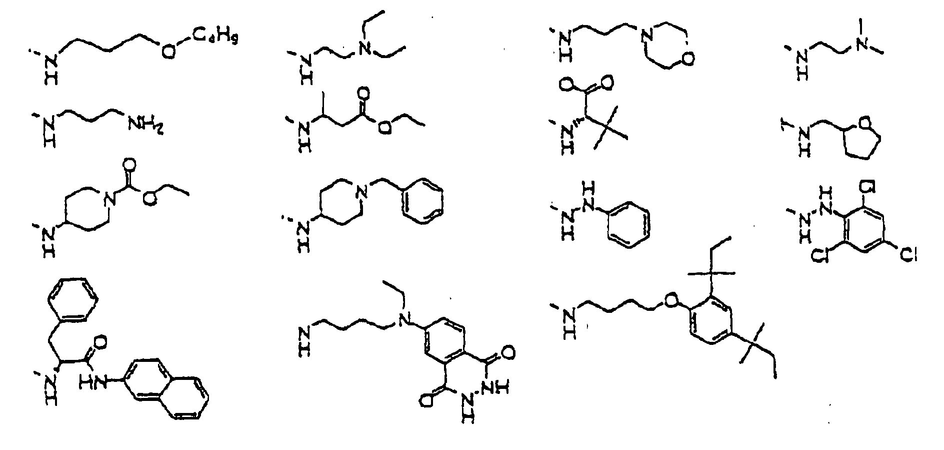

Furthermore, those salts include trifluoro acetate, hydrochloride, acetate, sulfate, nitrate, lactate, maleate, methane sulfonate, oxalate, malonate, succinate, fumarate, propionate, and butyrate.

Generic terms used in the present specification will be defined as follows and used solely or in combination.

In the explanations of A³ and A⁴ in the text, the term "monocyclic heterocyclic aromatic ring that may contain 1 to 4 nitrogen atoms and 1 to 2 other hetero atoms" includes a pyrrole ring, an imidazole ring, a pyrazole ring, an oxazole ring, an isoxazole ring, a thiazole ring, an isothiazole ring, a triazole ring, a thiadiazole ring, an oxadiazole ring, a pyridine ring, a pyrazine ring, a pyrimidine ring, a pyridazine ring, and a triazine ring. Further, the term "polycyclic heterocyclic aromatic ring that may contain 1 to 4 nitrogen atoms and 1 to 2 other hetero atoms" includes a quinoline ring, an isoquinoline ring, a benzimidazole ring, an imidazopyridyl ring, an imidazopyrimidyl ring, an imidazopyradinyl ring, a benzothiazolyl ring, an indole ring, an isoindole ring, a thiazolyl ring, a purine ring, a phenanthroline ring, an acridine ring, and a carbazole ring. Furthermore, the term "heterocyclic aromatic ring that contains 1 to 3 nitrogen atoms and may contain 1 to 2 other hetero atoms, and may be partially saturated" includes a tetrahydroquinolyl ring, a cyclopentenopyridylring, cycloheptenopyridyl ring, a cyclohexenoimidazolyl ring, and a tetrahydroindolyl ring.

In addition, any carbon atom position in such a heterocycle can be a position for coupling with the heterocycle.

In the explanations of "W" represented in the text, a functional group is a bivalent functional group bonded to groups existing on both ends thereof. The term "cyclic alkylene group" includes a cyclopropylene group, a cyclobutylene group, a cyclropentylene group, a cyclohexylene group, and a 2-cyclohexenylene group. The term "monocyclic or polycyclic aromatic ring" includes a benzene ring, a naphthalene ring, an anthracene ring, a phenanthrene ring, an indene ring, and a fluorene ring. The term "partially saturated aromatic ring" includes a tetralin ring, an indan ring, and a dihydroanthracene ring.

The term "monocyclic or polycyclic heterocyclic aromatic ring or partially saturated heterocyclic ring or saturated heterocyclic ring that may contain 1 to 3 oxygen atoms, 1 to 3 sulfur atoms, and 1 to 3 nitrogen atoms" includes a thiophene ring, a furan ring, a pyridine ring, a pyrimidine ring, a pyridazine ring, a pyrazine ring, an indole ring, an isoindole ring, a pyrrole ring, an isoquinoline ring, an isobenzthiophene ring, a quinoline ring, and a benzthiophene ring.

In addition, if W is a cyclic compound, any position thereof may be a coupling position. For example, the first or fourth position is preferable if W is a phenyl group or a naphthyl group, and the second or fifth position is preferable if W is a pyridyl group, but not limited thereto.

In the explanations of R¹, R², R³, R⁶, R⁷, R⁸, G³, G^{3'}, and G^{3"} in the text, alkyl groups are defined as monovalent straight-chain, branched-chain, or cyclic saturated hydrocarbon groups. For example, the alkyl groups may be a methyl group, an ethyl group, a propyl group, an isopropyl group, a butyl group, an isobutyl group, a tert-butyl group, a pentyl group, a hexyl group, a cyclopropyl group, a cyclobutyl group, a cyclopentyl group, a cyclohexyl group, a 2-methylcyclohexyl group, and a decalinyl group. Similarly, alkenyl groups are defined as monovalent straight-chain, branched-chain, or cyclic hydrocarbon groups having at least an ethylenic group. For example, the alkenyl groups may be a vinyl group, an allyl group, a 2-butylenyl group, a 1,3-butadienyl group, an isoprenyl group, a 3-pentenyl group, a cyclohexa-2-en group, a cyclohexadienyl group, and a tetralinyl group. Alkynyl groups are defined as of monovalent straight-chain, branched-chain, or cyclic hydrocarbon groups having at least an acethylenic group. For example, the alkynyl groups may be an ethynyl group, a 2-propynyl group, and a 3-penthynyl group.

In the explanations of W, G¹, G^{1'}, and R⁵ in the text, alkylene groups are defined as bivalent straight-chain, branched-chain, and cyclic saturated hydrocarbon groups, for example, an methylene group, an ethylene group, a propylene group, an isopropylene group, a butylene group, an isobutylene group, a tert-butylene group, a hexylene group, a heptylene group, a cyclopropylene group, a cyclobutylene group, a cyclopenthylene group, a cyclohexylene group, and a decalinylene group can be given. Alkenylene groups are defined as bivalent straight-chain, branched-chain, and cyclic hydrocarbon groups each containing at least an ethylenic group. For example, the alkenylene groups may be an ethylenyl group, a propenylene group, a 2-butenylene group, a 2-methyl-2-butenylene group, a 2-ethyl-2-butenylene group, a butadienylene group, a cyclopentenylene group, a cyclohexenylene group, and a cyclohexadienylene group. Alkynylene groups are defined as bivalent straight-chain, branched-chain, and cyclic hydrocarbon groups each containing at least an acetylenic group. For example, the alkynylene groups may be an acetynylene group, a propynylene group, a 2-butynylene group, and a 1-methyl-2-butynylene group.

In the explanations of G³, G^{3'}, and G^{3''} in the text, aralkyl groups are groups each constructed of alkyl groups and aromatic rings described above. For example, the aralkyl groups may be a benzyl group, a 1-phenetyl group, a 2-phenetyl group, a 1-phenyl propyl group, a 2-phenyl butyl group, a 1-naphthyl methyl group, a 2-naphthyl methyl group, a 1-(1-naphthyl)ethyl group, and a 2-(1-naphthyl)ethyl group. The term "monocyclic or polycyclic aromatic rings" includes a benzene ring, a naphthalene ring, and an anthracene ring. The term "monocyclic or polycyclic heterocyclic aromatic ring that may contain 1 to 3 oxygen atoms, 1 to 3 sulfur atoms, and 1 to 3 nitrogen atoms" includes an imidazole ring, a furan ring, a thiophene ring, a pyridine ring, a pyrimidine ring, a pyrazine ring, an indole ring, an indazole ring, a benzimidazole ring, and a pyrizinopyrrole ring. The term "partially-saturated monocyclic or polycyclic heterocyclic aromatic ring that may contain 1 to 3 oxygen atoms, 1 to 3 sulfur atoms, and 1 to 3 nitrogen atoms" includes a tetrahydroquinoline ring, and a cyclopenta pyridine ring. The term "saturated monocyclic or polycyclic heterocyclic ring that may contain 1 to 3 oxygen atoms, 1 to 3 sulfur atoms, and 1 to 3 nitrogen atoms" includes a tetrahydrofuran ring, a pyrrolidine ring, and an imidazolidine ring.

Furthermore, the term "group which may be substituted" in the explanations of the respective substituents includes halogen groups, nitro groups, hydroxyl groups, thiol groups, carbonyl groups, carboxyl groups, sulphenyl groups, sulfone groups, amino groups, amide groups, cyano groups, carvamoile groups, alkoxy groups, alkoxycarbonyl group, alkyl amino groups, dialkyl amino groups, aminocarbonyl groups, alkyl aminocarbonyl groups, dialkyl aminocarbononyl groups, alkanoil amino groups, alkanoil alkylamino groups, alkylthio groups, alkylsulphenyl groups, alkyl sulfone groups, and phenyl groups.

In the above explanation of the substitutes, the term "alkyl" is defined as the same alkyl group as that described above. The term "alkoxy" means '"alkyl" and "oxy", and thus meaning that the alkoxy group comprises the alkyl group described above and oxygen atoms bonded to the end thereof. The term "alkanoyl" means a substituent formed by the alkyl group described above through a carbonyl group.

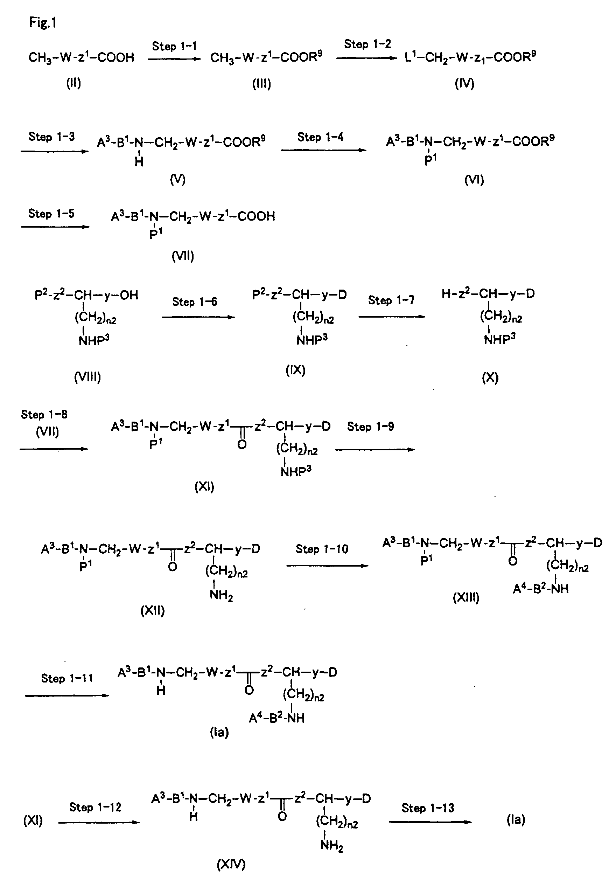

Nitrogen-containing compounds to be used in the present invention can be prepared by organic chemical reactions generally used in the art. In the following description, production methods thereof will be exemplified with reference to Figs. 1 to 5. However, the synthetic methods for the compounds of the present invention are not limited to these methods.

### Production Method Example 1

Description will be made of a method for producing a part of the compounds represented by the general formula (I) by the steps of a reaction process flow of the production method shown in Fig. 1 using a compound CH₃-W-z¹-COOH (the general formula (II), wherein W and z¹ are defined as described above) as a starting material.

### Step 1-1

A target compound (III) can be obtained by: dissolving a known and easily obtainable compound (II) CH₃-W-z¹-COOH (wherein W and z¹ are defined as described above) in any alcohol solvent R⁹-OH (wherein R⁹ is a methyl group, an ethyl group, a benzyl group, or the like); and reacting the resultant mixture for 0.5 to 24 hours at -20°C to 100°C while introducing chlorine gas therein.

### Step 1-2

A target compound (IV) (wherein L¹ is a halogen atom such as chlorine or bromine) can be obtained by: dissolving the compound (III) in an organic solvent, such as carbon tetrachloride, chloroform, or benzene; adding, to the obtained solution, a halogenation agent such as N-bromosuccineimide, N-chlorosuccineimide, and optionally, a radical-generating agent such as azoisobutylonitrile; and reacting the resultant mixture at 0°C to 100°C.

### Step 1-3

A target compound (V) can be obtained by: dissolving the compound (IV) in an organic solvent such as tetrahydrofuran (hereinafter, THF) ordimethylformamide (hereinafter, DMF) ; adding, to the obtained solution, a primary amine compound A³-B¹-NH₂ (wherein A³ and B¹ are defined as described above) ; and reacting the resultant mixture at room temperature to 100°C together with a base such as potassium carbonate or triethylamine.

### Step 1-4

A target compound (VI) can be obtained by: dissolving the compound (V) in an organic solvent such as THF or DMF; adding, to the obtained solution, a protecting agent represented as P¹-L¹ or P¹₂O (wherein P¹ is a protecting group represented as butoxycarbonyl, benzyloxycarbonyl, or the like) together with a base such as triethylamine or a sodium hydroxide aqueous solution; and reacting the resultant mixture at -10°C to 100°C.

Alternatively, the step 1-3 may be omitted and the compound (VI) may be obtained by reacting the compound (IV) with A³-B¹-NHP¹ (wherein A³, B¹, and P¹ are defined as described above) together with a base such as sodium hydroxide or powdery potassium hydroxide in an organic solvent such as DMF or THF at room temperature to 120°C.

### Step 1-5

A target compound (VII) can be obtained by: dissolving the compound (VI) in one or two organic solvents selected from DMF, THF, methanol, ethanol, and the like; adding, to the obtained solution, a basic aqueous solution such as a sodiumhydroxide aqueous solution; and reacting the resultant mixture at 0°C to 100°C.

### Step 1-6

A target compound (IX) can be obtained by: dissolving a known and easily obtainable compound (VIII) (wherein z², y, and n² are defined as described above, each of P² and P³ independently represents a protecting group such as 9-fluorenylmethylcarbonyl (hereinafter, Fmoc), t-butoxycarbonyl (hereinafterBoc), orbenzyl oxycarbonyl (hereinafter, Cbz)) in an organic solvent such as DMF; adding, to the obtained solution, a compound represented as H-D (wherein D is defined as described above) ; further adding, to the obtained solution as appropriate, a condensing agent such as N-ethyl-N-(3-dimethylaminopropyl)carbodiimide (hereinafter, WSC I) hydrochloride, benzotriazol-1-yloxy-tris(dimethylamino)phosphonium hexafluorophosphate (hereinafter, BOP), or 2-ethoxy-1-ethoxycarbonyl-1,2-dihydroquinoline (hereinafter, EEDQ), optionally together with a catalyst such as 1-hydroxybenzotriazole (hereinafter, HOBt) or 4-dimethylaminopyridine (hereinafter, DMAP); and reacting the resultant mixture at -20°C to 80°C.

### Step 1-7

A target compound (X) can be obtained by selectively removing P² from the compound (IX). For example, if P² is Fmoc, the target compound (X) can be obtained by: dissolving the compound (IX) in an organic solvent such as DMF; and reacting the obtained solution at room temperature to 100°C together with an organic base such as diethylamine or morpholine.

### Step 1-8

A target compound (XI) can be obtained by: dissolving the compound (X) in an organic solvent such as DMF; adding the compound (VII) thereto; further adding, to the obtained solution, a condensing agent such as WSCI hydrochloride, BOP, or EEDQ, optionally together with a catalyst such as HOBt or DMAP, and reacting the resultant mixture at -20°C to 80°C.

### Step 1-9

A target compound (XII) can be obtained by selectively removing P³, a protecting group of the compound (XI). For example, if P³ is Cbz, the target compound (XII) can be obtained by: dissolving the compound (XI) in ethanol, methanol, water-containing dioxane, or the like; and reacting the obtained solution in a hydrogen gas atmosphere together with a hydrogenating catalyst such as palladium carbon at room temperature.

### Step 1-10

A target compound (XIII) can be obtained by: adding the compound (XII) in an organic solvent such as material, ethanol, or acetonitrile together with A⁴CHO or A⁴=O (wherein A⁴ is defined as described above, A⁴=0 represents a compound in which carbon at any position in A⁴ is ketone, for example, 2-acetyl pyridine or tetrahydroquinolin-8-one) and a reducing agent such as sodium borohydride or sodium cyanoborohydride; adjusting, if required, a pH of the obtained solution; and reacting the resultant mixture at -20°C to 60°C.

### Step 1-11

A target compound (Ia) (Ia represents a part of the compounds included in the formula (I) described above) can be obtained by removing P¹, a protecting group of the compound (XIII).

For example, if P¹ is Boc, the target compound (Ia) can be obtained by: dissolving the compound (XIII) in a solvent such as methanol or dioxane; and adding, to the obtained solution, a mineral acid such as hydrochloric acid or an organic strong acid such as trifluoroacetic acid.

### Step 1-12

A target compound (XIV) can be obtained by removing simultaneously both the protecting groups P¹ and P² of the compound (XI). For example, if P¹ and P² are a combination of Boc and Cbz, the target compound (XIV) can be obtainedby: dissolving the compound (XI) in an organic solvent such as chloroform; adding, to the obtained solution, cresol thioanisole trifluoroacetic acid; and reacting the resultant mixture at room temperature to 80°C.

### Step 1-13

A target compound (Ia) (Ia represents a part of the compounds included in the formula (I) described above) can be obtained by: adding the compound (XIV) in an organic solvent such as methanol, ethanol, or acetonitrile together with A⁴CHO or A⁴=O (A⁴ is defined as described above, A⁴=0 represents a compound in which carbon at any position in A⁴ is ketone, for example 2-acetyl pyridine or tetrahydroquinolin-8-one) and a reducing agent such as sodium borohydride or sodium cyanoborohydride adjusting, if required, a pH of the obtained solution; and reacting the resultant mixture at -20°C to 60°C.

### Production Method Example 2

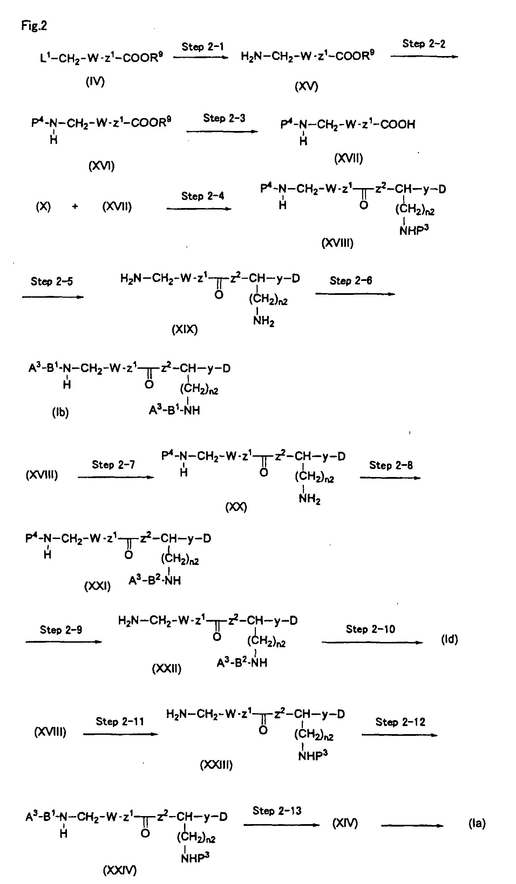

Description will be made of a method for producing a part of the compounds represented by the general formula (I) from the intermediate compound (IV) L¹-CH₂-W-z¹-COOR⁹ (wherein W, z¹, and R⁹ are defined as described above), which is produced at the midpoint in Production Method Example 1 by the steps of a reaction process flow of the production method shown in Fig. 2.

### Step 2-1

A target compound (XV) can be obtained by: dissolving the compound (IV) described in Production Method Example 1 in an organic solvent such as DMF; reacting the obtained solution with potassium phthalimide to obtain an intermediate; and reacting the intermediate with hydrated hydrazine in an organic solvent such as ethanol or methanol.

### Step 2-2

A target compound (XVI) can be obtained by: dissolving the compound (XV) in an organic solvent such as DMF or THF; and reacting the obtained solution with a protecting agent represented as P⁴-L¹ or P⁴₂O (wherein P⁴ is a protecting group such as Boc or Cbz) and a base such as triethylamine or a sodium hydroxide aqueous solution at -20°C to 80°C.

### Step 2-3

A target compound (XVII) can be obtained by: the compound (XVI) in an organic solvent comprising one or two selected from DMF, THF, methanol, ethanol, and so on; adding, to the obtained solution, a basic aqueous solution such as a sodium hydroxide aqueous solution; and reacting the resultant mixture at 0°C to 100°C.

### Step 2-4

A target compound (XVIII) can be obtained by: dissolving the compound (X) in an organic solvent such as DMF: adding the compound (XVII) described above thereto; adding, to the obtained solution, a condensing agent such as WSCI hydrochloride, BOP, or EEDQ together with, if required, a catalyst such as HOBt or DMAP; and reacting the resultant mixture at -20°C to 80°C.

### Step 2-5

A target compound (XIX) can be obtained by removing P³ and P⁴, protecting groups of the compound (XVIII). For example, if both P³ and P⁴ are Boc, the target compound (XIX) can be obtained by: dissolving the compound (XVIII) in an organic solvent such as methanol or dioxane; and adding, to the obtained solution, a mineral acid such as hydrochloric acid or an organic strong acid such as trifluoroacetic acid.

### Step 2-6

A target compound (Ib) (Ib represents part of the compounds included in the formula (I) described above) can be obtained by: adding the compound (XIX) in an organic solvent such as methanol, ethanol, or acetonitrile together with A³CHO or A³=O (wherein A³ is defined as described above, A³=0 represents a compound in which carbon at any position in A³ is ketone, for example, 2-acetyl pyridine or tetrahydroquinolin-8-one) and a reducing agent such as sodium borohydride or sodium cyanoborohydride; adjusting, if required, a pH of the obtained solution; and reacting the resultant mixture at -20°C to 60°C.

### Step 2-7

A target compound (XX) can be obtained by selectively removing the protecting group P⁴ of the compound (XVIII). For example, if P³ is Cbz, and P⁴ is Boc, the target compound (XX) can be obtained by: dissolving the compound (XVIII) in ethanol, methanol, water-containing dioxane, or the like; and reacting the obtained solution in a hydrogen gas atmosphere together with a hydrogenating catalyst such as palladium carbon at room temperature.

### Step 2-8

A target compound (XXI) can be obtained by: adding the compound (XIV) in an organic solvent such as methanol, ethanol, or acetonitrile together with A³CHO or A³=O (wherein A³ is defined as described above, A³=0 represents a compound in which carbon at any position in A³ is ketone, for example, 2-acetyl pyridine or tetrahydroquinolin-8-one) and a reducing agent such as sodium borohydride or sodium cyanoborohydride; adjusting, if required, a pH of the solution; and reacting the resultant mixture at -20°C to 60°C.

### Step 2-9

A target compound (XXII) can be obtained by removing the protecting group P³ of the compound (XXI). For example, if P³ is Boc, the target compound (XXII) can be obtained by: dissolving the compound (XXI) in an organic solvent such as methanol or dioxane; and adding, to the obtained solution, a mineral acid such as hydrochloric acid or an organic strong acid such as trifluoroacetic acid.

### Step 2-10

A target compound (Id) (Id represents a part of the compounds included in the formula (I) described above) can be obtained by: adding the compound (XXII) in an organic solvent such as methanol, ethanol, or acetonitrile together with A³CHO or A³=O (wherein A³ is defined as described above, A³=0 represents a compound in which carbon at any position in A³ is ketone, for example, 2-acetyl pyridine or tetrahydroquinolin-8-one) and a reducing agent such as sodium borohydride or sodium cyanoborohydride; adjusting, if required, a pH of the obtained solution; and reacting the resultant mixture at-20°C to 60°C, or reacting the resultant mixture with a guanidino forming agent such as pyrazole carboxamidine together with an appropriate base in an organic solvent such as methanol or DMF at 0°C to 100°C.

### Step 2-11

A target compound (XXIII) can be obtained by selectively removing the protecting group P⁴ of the compound (XVIII). For example, if P³ is Cbz and P⁴ is Boc, the target compound (XXIII) can be obtained by allowing the compound (XVIII) to exist together with hydrochloric acid in a trifluoroacetic acid in chloroform or in a mixture solvent of dioxane and methanol.

### Step 2-12

A target compound (XXIV) can be obtained by: adding the compound (XXIII) in an organic solvent such as methanol, ethanol, or acetonitrile together with A³CHO or A³=O (wherein A³ is defined as described above, A³=0 represents a compound in which carbon at any position in A³ is ketone, for example, 2-acetyl pyridine or tetrahydroquinolin-8-one) and a reducing agent such as sodium borohydride or sodium cyanoborohydride; adjusting, if required, a pH of the solution; and reacting the resultant mixture at -20°C to 60°C.

### Step 2-13

A target compound (XXV) can be obtained by removing the protecting group P³ of the compound (XXIV). For example, if P³ is Cbz, the target compound (XIV) can be obtained by: dissolving the compound (XXIV) in ethanol, methanol, dioxane hydrate, or the like; and reacting the obtained solution in a hydrogen gas atmosphere together with a hydrogenating catalyst such as palladium carbon at room temperature. The compound (XIV) can be used to obtain the compound (Ia) by the same method as in the step 1-13 of Production Method Example 1.

### Production Method Example 3

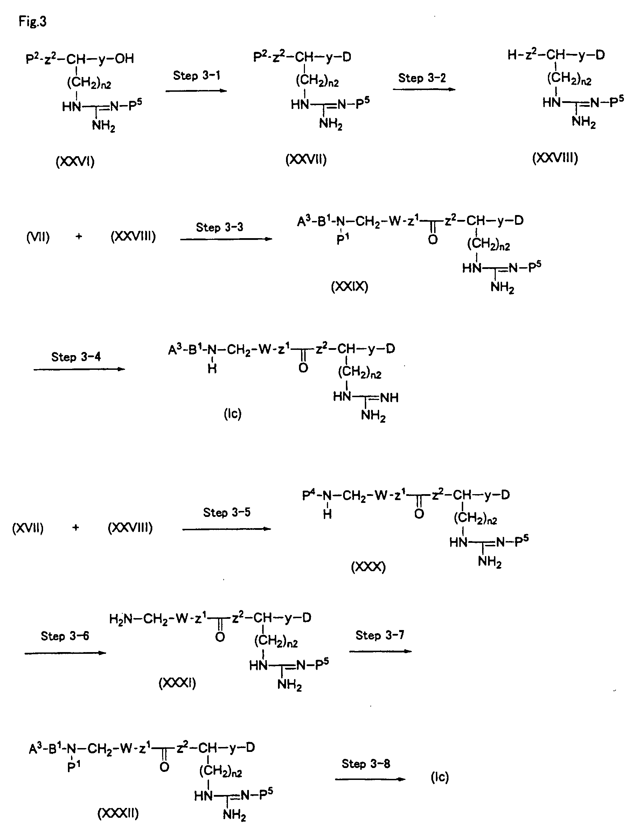

Description will be made of a method for producing a part of the compounds represented by the general formula (I) from the compound of the following general formula (XXVI), which is produced at the midpoint in Production Method Example 2 by the steps of a reaction process flow of the production method shown in Fig. 3.

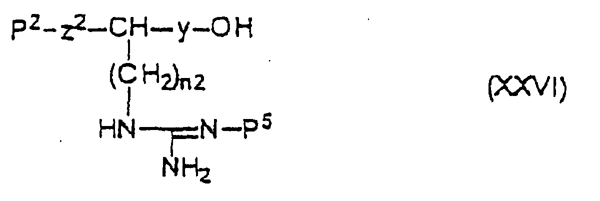

### Step 3-1

A target compound (XXVII) can be obtained by: dissolving a known and easily obtainable compound (XXVI) (wherein z², y, and n² are defined as described above, P² represents a protecting group such as Fmoc, Boc, or Cbz, and P⁵ represents a protecting group of guanidine such as a pentamethyl chroman sulphonyl group, or a toluenesulfonyl group) in an organic solvent such as DMF; adding, to the obtained solution, a compound represented as H-D (wherein D is defined as described above) ; adding, to the obtained solution, a condensing agent such as WSCI hydrochloride, BOP, or EEDQ, if required, together with a catalyst such as HOBt or DMAP; and reacting the resultant mixture at -20°C to 80°C.

### Step 3-2

A target compound (XXVIII) can be obtained by selectively removing P² of the compound (XXVII). For example, if P² is Fmoc, the target compound (XXVIII) can be obtained by: dissolving the compound (XXVII) in an organic solvent such as DMF; and reacting the obtained solution at room temperature to 100°C together with an organic base such as diethylamine or morpholine.

### Step 3-3

A target compound (XXIX) can be obtained by: dissolving the compound (XXVIII) in an organic solvent such as DMF; adding the compound (VII) thereto; further adding, to the obtained solution, a condensing agent such as WSCI hydrochloride, BOP, or EEDQ, if required, together with a catalyst such as HOBt or DMAP; and reacting the resultant mixture at -20°C to 80°C.

### Step 3-4

A target compound (Ic) (Ic represents a part of the compounds included in the formula (I) described above) can be obtained by dissolving the compound (XXIV) in an organic solvent such as chloroform or methylene chloride, followed by adding trifluoroacetic acid to the obtained solution and stirring the resultant mixture at -20°C to 60°C.

### Step 3-5

A target compound (XXX) can be obtained by: dissolving the compound (XXVIII) in an organic solvent such as DMF; adding the compound (XVII) described above thereto; further adding, to the obtained solution, a condensing agent such as WSCI hydrochloride, BOP, or EEDQ, if required together with a catalyst such as HOBt or DMAP; and reacting the resultant mixture at -20°C to 80°C.

### Step 3-6

A target compound (XXXI) can be obtained by selectively removing P⁴, a protecting group of the compound (XXX). For example, if P⁴ is Cbz, the target compound (XXXI) canbeobtainedby: dissolving the compound (XXX) in ethanol, methanol, water-containing dioxane, or the like; and reacting the obtained solution in a hydrogen gas atmosphere together with a hydrogenating catalyst such as palladium-carbon at room temperature.

### Step 3-7

A target compound (XXXII) can be obtained by: adding the compound (XXXI) in an organic solvent such as methanol, ethanol, or acetonitrile together with A⁴CHO or A⁴=O (wherein A⁴ is defined as described above, A⁴=0 represents a compound in which carbon at any position in A⁴ is ketone, for example 2-acetyl pyridine or tetrahydroquinolin-8-one) and a reducing agent such as sodium borohydride or sodium cyanoborohydride; adjusting, if required, a pH of the obtained solution; and reacting the resultant mixture at -20°C to 60°C.

### Step 3-8

A target compound (Ic) (Ic represents a part of the compounds included in the formula (I) described above) can be obtained by dissolving the compound (XXXII) in an organic solvent such as chloroform or methylene chloride, followed by adding trifluoroacetic acid to the obtained solution and stirring the resultant mixture at -20°C to 60°C.

### Production Method Example 4

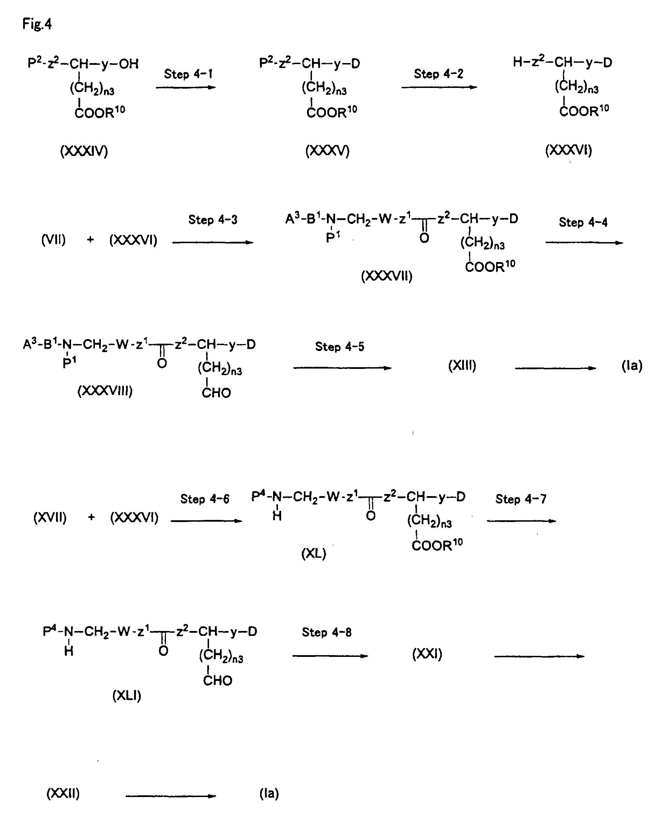

Description will be made of a method for producing a part of the compounds represented by the general formula (I) by the steps of a reaction process flow of the production method shown in Fig. 4.

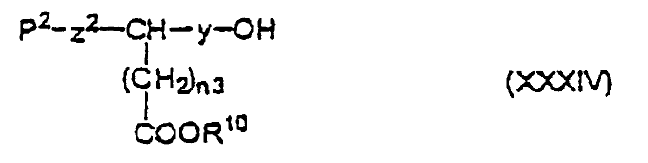

### Step 4-1

A target compound (XXXV) can be obtained by: dissolving a known and easily obtainable compound (XXXIV) (wherein Z² is defined as described above, n³ is an integer of 1 to 3, R¹⁰ is a methyl group, an ethyl group, a benzyl group, or the like, and P² is a protecting group such as Fmoc, Boc, or Cbz in an organic solvent such as DMF ; adding, to the obtained solution, a compound represented as H-D (wherein D is defined as described above) ; further adding, to the obtained solution, a condensing agent such as WSCI hydrochloride, BOP, or EEDQ, if required, together with a catalyst such as HOBt or DMAP; and reacting the resultant mixture at -20°C to 80°C.

### Step 4-2

A target compound (XXXVI) can be obtained by selectively removing the protecting group P² of the compound (XXXV). For example, if P² is Boc, the compound (XXXVI) can be obtained by dissolving the compound (XXXV) in an organic solvent such as methanol or ethanol, and introducing hydrogen chloride gas therein.

### Step 4-3

A target compound (XXXVII) can be obtained by: dissolving the compound (XXXVI) in an organic solvent such as DMF: adding the above compound (VII) thereto; further adding a condensing agent such as WSCI hydrochloride, BOP, or EEDQ, if required, together with a catalyst such as HOBt or DMAP; and reacting the resultant mixture at -20°C to 80°C.

### Step 4-4

A target compound (XXXVIII) can be obtained by converting COOR¹⁰ in the compound (XXXVII) into CHO. For example, the target compound (XXXVIII) can be obtained by synthesizing an alcohol compound as an intermediate from the compound (XXXVII) using a reducing agent such as lithiumaluminumhydride in an organic solvent such as THF, and then oxidizing the alcohol with dimethyl sulfoxide-oxalyl chloride or pyridinium dichlorochromate.

### Step 4-5

A target compound (XIII) can be obtained by: adding the compound (XXXVIII) in an organic solvent such as methanol, ethanol, or acetonitrile together with A⁴-B²-NH₂ (wherein A⁴ and B² are defined as described above) and a reducing agent such as sodium borohydride or sodium cyanoborohydride; adjusting, if required, a pH of the obtained solution; and reacting the resultant mixture at -20°C to 60°C. The compound (XIII) can derive the compound (Ia) by the same method as in the step 1-11 of Production Method Example 1.

### Step 4-6

A target compound (XL) can be obtained by: dissolving the compound (XXXVI) in an organic solvent such as DMF; adding the compound (XVII) thereto; adding, to the obtained solution, a condensing agent such as WSCI hydrochloride, BOP, or EEDQ, if required, together with a catalyst such as HOBt or DMAP; and reacting them at -20°C to 80°C.

### Step 4-7

A target compound (XLI) can be obtained by converting COOR¹⁰ in the compound (XL) into CHO. For example, the target compound (XLI) can be obtained by synthesizing an alcohol compound as an intermediate from the compound (XL) using a reducing agent such as lithium aluminum hydride, and oxidizing the alcohol compound with dimethyl sulfoxide-oxalyl chloride or pyridinium dichlorochromate.

### Step 4-8

A target compound (XXI) can be obtained by: adding the compound (XLI) in an organic solvent such as methanol, ethanol, or acetonitrile together with A⁴-B²-NH₂ (wherein A⁴ and B² are defined as described above) and a reducing agent such as sodium borohydride or sodium cyanoborohydride; adjusting, if required, a pH of the obtained solution; and reacting the resultant mixture at -20°C to 60°C. The compound (XXI) can derive the compound (Ib) by the same method as in the step 2-9 and the step 2-10 of Production Method Example 2.

### Production Method Example 5

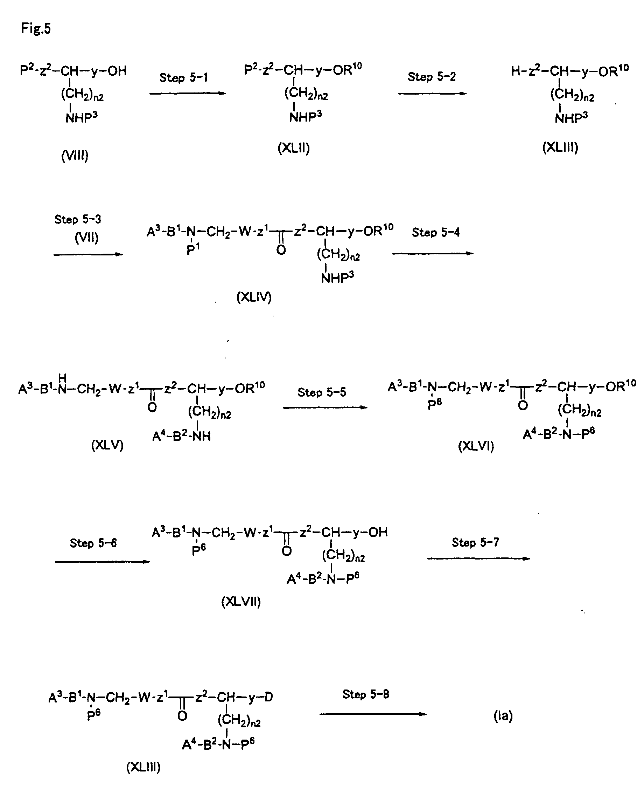

Description will be made of a method for producing a part of the compounds represented by the general formula (I) from the compound (VIII) of the following general formula, which is generated at the midpoint in Production Method Example 4 by the steps of the reaction process flow of the production method shown in Fig. 5.

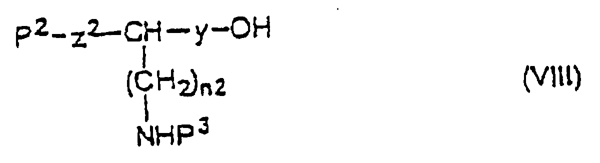

### Step 5-1

A target compound (XLII) can be obtained by: dissolving a known and easily obtainable compound (VIII) (wherein each of substituents is defined as described above) in any alcohol solvent R⁹-OH (wherein R⁹ is a methyl group, an ethyl group, a benzyl group, or the like) ; adding, to the obtained solution, a condensing agent such as WSCI hydrochloride, BOP, or EEDQ, if required, together with a catalyst such as HOBt or DMAP; and reacting the resultant mixture at -20°C to 80°C.

### Step 5-2

A target compound (XLIII) can be obtained by selectively removing P² of the compound (XLII). For example, if P² is Fmoc, the target compound (XLIII) can be obtained by: dissolving the compound (XLII) in an organic solvent such as DMF; and reacting the obtained solution with an organic base such as dimethylamine or morpholine at room temperature to 100°C.

### Step 5-3

A target compound (XLIV) can be obtained by: dissolving the compound (XLIII) in an organic solvent such as DMF: adding the compound (VII) thereto; adding, to the obtained solution, a condensing agent such as WSCI hydrochloride, BOP, or EEDQ, if required, together with a catalyst such as HOBt or DMAP; and reacting the resultant mixture at -20°C to 80°C.

### Step 5-4

A target compound (XLV) can be obtained by: removing P¹ and P³, protecting groups of the compound (XLIV) (for example, if P¹ and P³ are Boc, P¹ and P³ can be removed by: dissolving the compound (XLIV) in a solvent such as methanol or dioxane; and adding, to the obtained solution, a mineral acid such as hydrochloric acid or an organic strong acid such as trifluoroacetic acid); adding A⁴CHO or A⁴=O (wherein A⁴ is defined as described above, A⁴=0 represents a compound in which carbon at any position in A⁴ is ketone, for example, 2-acetyl pyridine or tetrahydroquinolin-8-one) and a reducing agent such as sodium borohydride or sodium cyanoborohydride to the compound (XLIV) in an organic solvent such as methanol, ethanol, or acetonitrile; adjusting, if required, a pH of the obtained solution; and reacting the resultant mixture at -20°C to 60°C.

### Step 5-5

A target compound (XLVI) can be obtained by: dissolving the compound (XLV) in an organic solvent such as THF or DMF; adding, to the obtained solution, a protecting agent represented as P⁶-L¹ or P⁶₂O (wherein P¹ is a protecting group represented as butoxycarbonyl, benzyloxycarbonyl, or the like) together with a base such as triethylamine or a sodium hydroxide aqueous solution; and reacting the resultant mixture at -10°C to 100°C.

### Step 5-6

A target compound (XLVII) can be obtained by: dissolving the compound (XLVI) in an organic solvent comprising one or two selected from DMF, THF, methanol, ethanol, and the like; adding, to the obtained solution, a basic aqueous solution such as a sodium hydroxide aqueous solution; and reacting the resultant mixture at 0°C to 100°C.

### Step 5-7

A target compound (XLVIII) can be obtained by: dissolving the compound (XLVII) in an organic solvent such as DMF; adding, to the obtained solution, a compound represented as H-D (wherein D is defined as described above) ; further adding, to the obtained solution, a condensing agent such as WSCI hydrochloride, BOP, or EEDQ, if required, together with a catalyst such as HOBt or DMAP; and reacting the resultant mixture at -20°C to 80°C.

### Step 5-8

A target compound (Ia) can be obtained by removing P⁶, a protecting group of the compound (XLVIII) . For example, if P⁶ is Boc, the compound (Ia) can be obtained by: dissolving the compound (XLVIII) in a solvent such as methanol or dioxane; and adding, to the obtained solution, a mineral acid such as hydrochloric acid or an organic strong acid such as trifluoroacetic acid.

### Detailed Description of the Drawings

Fig. 1 is a diagram showing a reaction process of Production Method Example 1 for producing a part of the compounds represented by the general formula (I) using the compound CH₃-W-z¹-COOH (the general formula compound (II), wherein W and z¹ are defined as described above) as a starting material.

Fig. 2 is a diagram showing a reaction process of Production Method Example 2 for producing a part of the compounds represented by the general formula (I) from the intermediate compound (IV), L¹-CH₂-W-z¹-COOR⁹ (wherein W, z¹, and R⁹ are defined as described above), generated in the middle of Production Method Example 1 by the reaction process different from the Production Method Example 1.

Fig. 3 is a diagram showing a reaction process of Production Method Example 3 for producing a part of the compounds represented by the general formula (I) from the general formula compound (XXIV) generated in the middle of Production Method Example 2 by the reaction process different from the one used in Production Method Example 2.

Fig. 4 is a diagram showing a reaction process of Production Method Example 4 for producing a part of the compounds represented by the general formula (I) from known compounds (general formula compound (XXXIV)).

Fig. 5 is a diagram showing a reaction process of Production Method Example 5 for producing a part of the compounds represented by the general formula (I) from the general formula compound (XXXVI) generated in the middle of Production Method Example 4 by the reaction process different from the one used in Production Method Example 4.

Fig. 6 illustrates effects of the compound No. 86 of the present invention to appendicular edemas (arthritis score) in the type-II collagen-induced arthritis DBI/1 mouse.

Fig. 7 illustrates effects of the compound No. 86 of the present invention to appendicular edemas (the number of edematous fingers) in the type-II collagen-induced arthritis DBI/1 mouse.

### Best Mode for carrying out the Invention

Nitrogen-containing compounds to be used in the present invention can be exemplified by the following compounds:
(S)-2-(4-(N-2-picolylaminomethyl)benzoylamino)-5-((imid azol-2-ylmethyl)amino) valerate 1-naphthalenemethylamide [Compound No. 1];
(S)-2-(4-(N-2-picolylaminomethyl)benzoylamino)-5-((pyrr ol-2-ylmethyl) amino) valerate 1-naphthalenemethylamide [Compound No. 2];
(S)-2-(4-(N-2-picolylaminomethyl)benzoylamino)-5-((1-me thylimidazol-2-ylmethyl)amino) valerate 1-naphthalenemethyl amide [Compound No. 3];
(S)-2-(4-(N-2-picolylaminomethyl) benzoylamino)-5-((imidazol-4-ylmethyl)amino) valerate 1-naphthalenemethylamide [Compound No. 4];
(S)-2-(4-(N-2-picolylaminomethyl)benzoylamino)-5-((1-me thylpyrrol-2-ylmethyl) amino) valerate 1-naphthalenemethylamide [Compound No. 5];
(S)-2-(4-(N-2-picolylaminomethyl) benzoylamino)-5-((1-methylbenzimidazol-2-ylmethyl) amino) valerate 1-naphthalenemethylamide [Compound No. 6];
(S)-2-(4-(N-2-picolylaminomethyl) benzoylamino)-5-((quinolin-2-ylmethyl) amino) valerate 1-naphthalenemethylamide [Compound No. 7];
(2S)-2-((4-(N-2-picolylaminomethyl) phenylacetyl) amino) - 5-(5, 6, 7, 8-tetrahydroquinolin-8-yl) amino valerate 1-naphthalenemethylamide [Compound No. 8];
(2S)-2-(4-(2-(N-2-picolylamino) ethyl) benzoylamino)-5-(5,6,7,8-tetrahydroquinolin-8-yl) amino valerate 1-naphthalene methylamide [Compound No. 9];
(S)-2-(4-(2-(N-2-picolylamino) ethyl) benzoylamino)-5-(N-2-picolylamino) valerate 1-naphthalenemethylamide [Compound No. 10];
(S)-2-(5-(N-2-picolylamino methyl) furan-2-ylcarbonyl) amino-5-(5,6,7,8-tetrahydroquinolin-8-yl) amino valerate 1-naphthalenemethylamide [Compound No. 11];
(2S)-2-(2-(N-2-picolyl aminomethyl) pyridin-5-yl carobonyl) amino-5-((5,6,7,8-tetrahydroquinolin-8-yl) amino) valerate 1-naphthalenemethylamide [Compound No. 12];
(2S)-2-(5-(N-2-picolylaminomethyl) pyrazine-2-carbonyl amino)-5-(5,6,7,8-tetrahydroquinolin-8-yl amino) valerate 1-naphthalenemethylamide [Compound No. 13];
(2S)-2-(5-(N-picolylaminomethyl) thiophene-2-carbonyl amino)-5-(5,6,7,8-tetrahydroquinolin-8-ylamino) valerate 1-naphthalenemethylamide [Compound No. 14];
(2S)-2-(5-(N-(imidazol-2-ylmethyl) aminomethyl) thiophene - 2-carbonylamino)-5-picolylamino valerate 1-naphthalenemethyl amide [Compound No. 15];
(2S)-2-(5-(N-(imidazol-2-ylmethyl) aminomethyl thiophene - 2-carbonylamino)-5-(5,6,7,8-tetrahydroquinolin-8-yl) amino valerate 1-naphthalenemethylamide[Compound No. 16];
(S)-2-(4-(8-quinolylaminomethyl)benzoylamino)-5-(2-pico lylamino) valerate 1-naphthalenemethylamide [Compound No. 17];
(2S)-2-(4-((N-imidazol-2-ylmethyl) aminomethyl) naphthoyl amino)-5-(2-picolylamino) 2-(3-indolyl) ethylamide valerate [Compound No. 18];
(2S)-2-(4-((N-imidazol-2-ylmethyl) aminomethyl) naphthoyl amino)-5-(5,6,7,8-tetrahydroquinolin-8-ylamino) 2-(3-indolyl) ethylamide valerate [Compound No. 19];
(S)-2-(4-((imidazol-4-ylmethyl) aminomethyl)benzoyl amino)-5-((imidazol-4-ylmethyl)amino) valerate 1-naphtalene methylamide [Compound No. 20];
(S)-2-(4-((imidazol-2-ylmethyl) aminomethyl) benzoyl amino)-5-((imidazol-2-ylmethyl) amino) valerate 1-naphthalene methylamide [Compound No. 21];
(S)-2-(4-((1-methylpyrrol-2-ylmethyl) aminomethyl) benzoylamino)-5-((1-methylpyrrol-2-ylmethyl) amino) valerate 1-naphthalenemethylamide [Compound No. 22];
(S)-2-(4-((1-methylimidazol-2-ylmethyl) aminomethyl) benzoylamino)-5-((1-methylimidazol-2-ylmethyl) amino) valerate 1-naphthalenemethylamide [Compound No. 23];
(S)-2-(4-(N-2-picolylamino) butyrylamino)-5-(2-picolyl amino) valerate 1-naphthalenemethylamide [Compound No. 24];
(2S)-2-(trans-(4-(5,6,7,8-tetrahydroquinolin-8-yl) aminomethyl) cyclohexylcarbonyl) amino-5-(5,6,7,8-tetrahydro quinolin-8-ylamino) valerate 1-naphthalenemethylamide [Compound No. 25];
(2S)-2-(4-(5,6,7,8-tetrahydroquinolin-8-ylaminomethyl) naphthoyl) amino-5-(5,6,7,8-tetrahydroquinolin-8-ylamino) valerate 1-naphthalenemethylamide [Compound No. 26];
(S)-2-(4-(N-2-picolylaminomethyl) naphthoylamino)-5-(2-picolylamino) valerate 1-naphthalenemethyl amide [Compound No. 27];
(S)-2-(4-((imidazol-2-ylmethyl) aminomethyl) naphthoyl amino)-5-((imidazol-2-ylmethyl) amino) valerate 1-naphthalene methylamide [Compound No. 28];
(S)-2-(4-((1-methylimidazol-2-ylmethyl) aminomethyl) naphthoylamino)-5-((1-methylimidazol-2-ylmethyl) amino) valerate 1-naphthalenemethylamide [Compound No. 29];
(S)-2-(4-((1-methylimidazol-2-ylmethyl) aminomethyl) benzoylamino)-5-((1-methylpyrrol-2-ylmethyl) amino) valerate 1-naphthalenemethylamide [Compound No. 30];
(S)-2-(4-((imidazol-2-ylmethyl) aminomethyl) benzoyl amino)-5-((1-methylpyrrol-2-ylmethyl) amino) valerate 1-naphthalenemethylamide [Compound No. 31];
(S)-2-(4-((pyrazol-3-ylmethyl) aminomethyl) benzoylamino) - 5-((1-methylpyrrol-2-ylmethyl) amino) valerate 1-naphthalene methylamide [Compound No. 32];
(S)-2-(4-((1-methylbenzimidazol-2-ylmethyl) aminomethyl) benzoylamino)-5-((1-methylbenzimidazol-2-yl)methylamino) valerate 1-naphthalenemethylamide [Compound No. 33];
(S)-2-(4-((1-methylbenzimidazol-2-ylmethyl) aminomethyl) benzoylamino)-5-((1-methylpyrrol-2-ylmethyl) amino) valerate 1-naphthalenemethylamide [Compound No. 34];
(S)-2-(4-((thiazol-2-ylmethyl) aminomethyl) benzoylamino) - 5-((1-methylpyrrol-2-ylmethyl) amino) valerate 1-naphthalene methylamide [Compound No. 35];
(S)-2-(4-((1-methylimidazol-2-ylmethyl) aminomethyl) benzoylamino)-5-((imidazol-2-ylmethyl) amino) valerate 1-naphthalenemethylamide [Compound No. 36];
(S)-2-(4-((imidazol-2-ylmethyl) aminomethyl) benzoyl amino)-5-((1-methylimidazol-2-ylmethyl) amino) valerate 1-naphthalenemethylamide [Compound No. 37];
(2S)-2-(4-(N-2-picolylaminomethyl)naphthoylamino)-5-(5, 6,7,8-tetrahydroquinolin-8-ylamino) valerate 1-naphthalene methylamide [Compound No. 38];
(2S)-2-(4-((N-imidazol-2-ylmethyl) aminomethyl) benzoyl amino)-5-(5,6,7,8-tetrahydroquinolin-8-ylamino) valerate 1-naphthalenemethylamide [Compound No. 39];
(S)-2-(4-((N-imidazol-2-ylmethyl) aminomethyl) naphthoylamino)-5-(2-picolylamino) valerate 1-naphthalenemethyl amide [Compound No. 40];
(2S) -2- (4- ((N-imidazol-2-ylmethyl) aminomethyl) naphthoyl amino)-5-(5,6,7,8-tetrahydroquinolin-8-ylamino) valerate 1-naphthalenemethylamide [Compound No. 41];
(S)-2-((4-guanidinomethylbenzoyl) amino)-5-(N-2-picolyl amino) valerate 1-naphthalenemethylamide [Compound No. 42];
N^{α}-(4-(N-2-picolylaminomethyl) benzoyl)-L-arginine 1-naphthalenemethylamide [Compound No. 43];
N^{α}-(4-(N-2-picolylaminomethyl) naphthoyl)-L-arginine 1-naphthalenemethylamide [Compound No. 44];
N^{α}-(4-((N-imidazol-2-ylmethyl) aminomethyl) naphthoyl) -L-arginine 1-naphthalenemethylamide [Compound No. 45];
N^{α}-(2-(N-2-picolylaminomethyl) pyridin-5-ylcarbonyl)-L-arginine 1-naphthalenemethylamide [Compound No. 46];
N^{α}-(5-(N-2-picolylaminomethyl) thiophen-2-ylcarbonyl)-L-arginine 1-naphthalenemethylamide [Compound No. 47];
N^{α}-(4-(imidazol-2-ylmethyl) aminomethylnaphthoyl)-L-arginine 2-(3-indolyl) ethylamide [Compound No. 48];
N^{α}-(4-(imidazol-2-ylmethyl) aminomethylnaphthoyl)-L-arginine (1'S)-(1'-(1-naphthyl) ethyl) amide [Compound No. 49];
N^{α}-(4-(imidazol-2-ylmethyl) aminomethylnaphthoyl)-L-arginine (1'R)-(1'-(1-naphthyl) ethyl) amide [Compound No. 50];
N^{α}-(4-(imidazol-2-ylmethyl) aminomethylnaphthoyl)-L-arginine 4-hexadecylaminobenzylamide [Compound No. 51];
N^{α}-(4-(5,6,7,8-tetrahydroquinolin-8-ylaminomethyl) benzoyl)-L-arginine 1-naphthalenemethylamide [Compound No. 52];
N^{α}-(4-((imidazol-2-ylmethyl) aminomethyl) benzoyl)-L-arginine 1-naphthalenemethylamide [Compound No. 53];
(S)-2-(4-(N-2-picolylaminomethyl) benzoylamino)-5-(quinolin-8-ylamino) valerate 1-naphthalenemethylamide [Compound No. 54];
(2S)-2-(4-(N-2-picolylaminomethyl) naphthoylamino)-5-((8R)-5,6,7,8-tetrahydroquinolin-8-ylamino) valerate 1-naphthalenemethylamide [Compound No. 55];
(S)-2-(4-(imidazol-2-ylmethyl) aminomethyl) naphthoyl amino)-5-(2-picolylamino) valerate (1'S)-(1'-(1-naphthyl) ethyl) amide [Compound No. 56];
(S)-2-(4-(imidazol-2-ylmethyl) aminomethyl) naphthoyl amino)-5-(5,6,7,8-tetrahydroquinolin-8-ylamino) valerate (1'S)-(1'-(1-naphthyl) ethyl) amide [Compound No. 57];
(S)-2-(4-(imidazol-2-ylmethyl) aminomethyl) naphthoyl amino)-5-(N-2-picolylamino) valerate (1'R)-(1'-(1-naphthyl) ethyl) amide [Compound No. 58];
(S)-2-(4-(imidazol-2-ylmethyl) aminomethyl) naphthoyl amino)-5-(5,6,7,8-tetrahydroquinolin-8-ylamino) valerate (1'R)-(1'-(1-naphthyl) ethyl) amide [Compound No. 59];
(S)-2-(4-(N-2-picolylaminomethyl) benzoylamino)-5-(N-2-picolylamino) valerate 1-naphthalenemethylamide [Compound No. 60];
(S)-2-(4-(N-2-picolylamino) methylbenzoylamino)-4-(N-2-picolylamino)lactate 1-naphthalenemethylamide [Compound No. 61];
(S)-2-(4-(N-2-picolylamino) methylbenzoylamino)-3-(N-2-picolylamino) propionate 1-naphthalenemethylamide [Compound No. 62];
(S)-2-(4-(N-2-picolylaminomethyl) benzoylamino)-5-(N-2-picolylamino) caprate 1-naphthalenemethylamide [Compound No. 63];
(2S)-2-(4-((5,6,7,8-tetrahydroquinolin-8-yl)aminomethyl ) benzoylamino)-5-((5,6,7,8-tetrahydroquinolin-8-yl) amino) valerate 1-naphthalenemethylamide [Compound No. 64];
(2S)-2-(4-(N-2-picolylaminomethyl)benzoylamino)-5-((5,6 ,7,8-tetrahydroquinolin-8-yl) amino) valerate 1-naphthalene methylamide [Compound No. 65];
(S)-2-(4-(3-picolylaminomethyl) benzoylamino)-5-(3-picolylamino) valerate 1-naphthalenemethylamide [Compound No. 66] ;
(S)-2-(4-(N-2-picolylaminomethyl) benzoylamino)-5-(N-2-picolylamino)valerate3-(n-butoxy) propylamide [Compound No. 67] ;
(S)-2-(4-(N-2-picolylaminomethyl) benzoylamino)-5-(N-2-picolylamino) valerate tetrahydrofurfurylamide [Compound No. 68];
(S)-2-(4-(N-2-picolylaminomethyl) benzoylamino)-5-(N-2-picolylamino) valerate phenylhydrazide [Compound No. 69];
(S)-2-(4-(N-2-picolylaminomethyl) benzoylamino)-5-(N-2-picolylamino) valerate 2-(3-indolyl)ethylamide [Compound No. 70] ;
(S)-2-(4-(N-2-picolylaminomethyl) benzoylamino)-5-(N-2-picolylamino) valerate (1-benzylpiperazine-4-yl)amide [Compound No. 71];
(2S)-2-(4-(N-2-picolylaminomethyl) benzoylamino)-5-(N-2-picolylamino) valerate (1'S)-1'-(2-naphthyl)aminocarbonyl phenethylamide [Compound No. 72];
(S)-2-(4-(N-2-picolylaminomethyl) benzoylamino)-5-(N-2-picolylamino) valerate 4-hexadecylaminobenzylamide [Compound No. 73];
(S)-2-(4-(N-2-picolylaminomethyl) benzoylamino)-5-(N-2-picolylamino)valerate4-(N-(1,2,3,4-tetrahydro-1,4-dicarbonylphthalazine-6-yl)-N-ethylamino)butylamide [Compound No. 74];
(S)-2-(4-(N-2-picolylaminomethyl) benzoylamino)-5-(N-2-picolylamino) valerate 2,4,6-trichlorophenylhydrazide [Compound No. 75];
(S)-2-(4-(N-2-picolylaminomethyl) benzoylamino)-5-(N-2-picolylamino) valerate 2-picolyamide [Compound No. 76];
(S)-2-(4-(N-2-picolylaminomethyl) benzoylamino)-5-(N-2-picolylamino) valerate 2-(N,N-diethylamino)ethylamide [Compound No. 77];
(S)-2-(4-(N-2-picolylaminomethyl) benzoylamino)-5-(N-2-picolylamino) valerate 3-(morpholin-1-yl)propylamide [Compound No. 78];
(S)-2-(4-(N-2-picolylaminomethyl) benzoylamino)-5-(N-2-picolylamino) valerate 2-(N,N-methylamino)ethylamide [Compound No. 79];
(S)-2-(4-(N-2-picolylaminomethyl) benzoylamino)-5-(N-2-picolylamino) valerate 4-(2,4-di-t-amylphenoxy)butylamide [Compound No. 80];
(S)-2-(4-(N-2-picolylaminomethyl) benzoylamino)-5-(N-2-picolylamino) valerate 3-aminopropylamide [Compound No. 81];
(S)-2-(4-(N-2-picolylaminomethyl) benzoylamino)-5-(N-2-picolylamino) valerate 5-indazolamide [Compound No. 82];
(2S)-2-(2-(N-2-picolylaminomethyl) pyridin-5-ylcarbonyl) amino-S-(5,6,7,8-tetrahydroquinolin-8-yl) amino valerate (1'S) -1'-(1-naphthyl) ethylamide [Compound No. 83];
(2S)-2-(2-(N-2-picolylaminomethyl) pyridin-5-ylcarbonyl) amino-5-((1-methyl-imidazol-2-yl) methylamino) amino valerate (1'S)-1'-(1-naphthyl)-ethylamide [Compound No. 84];
(2S)-2-(4-(N-2-picolylaminomethyl) naphthoyl) amino-5-(5,6,7,8-tetrahydroquinolin-8-yl) amino valerate (1'S)-1'-(1-naphthyl) -ethylamide [Compound No. 85];
N^{α}-(4-(N-2-picolylaminomethyl) benzoyl)-L-arginine (1'S)-1'-(1-naphthyl) ethylamide [Compound No. 86];
(2S)-2-(4-((1-methylimidazol-2-ylmethyl) aminomethyl) naphthoylamino-5-((1-methylimidazol-2-ylmethyl) amino) valerate (1'S)-1'-(1-naphthyl)-ethylamide [Compound No. 87];
(2S)-2-(2-(N-2-picolylaminomethyl) pyridin-5-ylcarbonyl) amino-5-(N-methylpyrrol-2-ylmethyl) amino valerate (1'S)-1'-(1-naphthyl)-ethylamide [Compound No. 88];
N^{α}-(4-(N-(1-methylimidazol-2-yl) methylaminomethyl)-1-naphthalenecarbonyl)-L-arginine 2-(1-naphthyl) isopropylamide [Compound No. 89];
N^{α}-(2-(N-2-picolylaminomethyl) pyridin-5-ylcarbonyl)-L-arginine (1'S)-1'-(1-naphthyl) ethylamide [Compound No. 90];
(2S)-2-(4-(N-2-picolylaminomethyl) benzoyl) amino-5-(5,6,7,8-tetrahydroquinolin-8-yl) amino valerate (1'S)-1'-(1-naphthyl) ethylamide [Compound No. 91];
(2S)-2-(4-(N-2-picolylaminomethyl) naphthoyl) amino-5-(5,6,7,8-tetrahydroquinolin-8-yl) amino valerate 2-(3-indolyl) ethylamide [Compound No. 92];
N^{α}-(4-(N-2-picolylaminomethyl) benzoyl-N^{G}-nitroarginine (1'S)-1'-(1-naphthyl) ethylamide [Compound No. 93];
(2R)-2-(4-(N-(imidazol-2-ylmethyl) aminomethyl) benzoyl) amino-5-(5,6,7,8-tetrahydroquinolin-8-yl) amino valerate (1'S)-1'-(1-naphthyl) ethylamide [Compound No. 94];
N^{α}-(4-(N-2-(imidazol-2-ylmethyl) aminomethyl) benzoyl-L-arginine (1'S)-1'-(1-naphthyl) ethylamide [Compound No. 95];
(2S)-2-((1-methylimidazol-2-ylmethyl) aminomethyl) benzoylamino-5-(5,6,7,8-tetrahydroquinolyl-8-yl) amino valerate 1-naphthalene methyleneamide [Compound No. 96];
N^{α}-(4-((N-(1-methylimidazol-2-ylmethyl) amino) methyl) naphthalene-1-carbonyl)-L-arginine (1'S)-1'-(1-naphthyl) ethyl amide [Compound No. 97];
N^{α}-(4-((imidazol-2-ylmethyl) amino) methyl) naphthalene-1-carbonyl)-L-arginine (1'S)-N-methyl-N-(1'-(1-naphthyl) ethyl) amide [Compound No. 98];
N^{α}-(4-(N-2-picolylaminomethyl) naphthoyl)-L-arginine (1'S)-1'-(1-naphthyl) ethylamide [Compound No. 99];
N^{α}-(4-(N-2-picolylaminomethyl) naphthalene-1-carbonyl) - L-arginine-D-3-(1-naphthyl) alaninemethylester [Compound No. 100];
N^{α} -(4-((N-2-picolylaminomethyl) naphthalene-1-carbonyl) - L-arginine-D-3-(1-naphthyl) alanine [Compound No. 101];
(2S)-2-(8-2-picolylaminomethylquinoline-5-carbonyl) amino-5-(5,6,7,8-tetrahydroquinolin-8-yl) amino valerate (1'S)-1'-(1-naphthyl) ethylamide [Compound No. 102];
N^{α}-(4- ((imidazol-2-ylmethyl) amino) methyl) naphthalene-1-carbonyl)-L-arginine N-methyl-1-naphthylmethylamide [Compound No. 103];
(2S)-2-(4-(2-pyridyl) aminomethylnaphthalene-1-carbonyl) anmino-5-(5,6,7,8-tetrahydroquinolin-8-yl) amino valerate 1-naphthylmethylamide [Compound No. 104];
(2S)-2-(4-(N-2-picolylaminomethyl) naphthalene-1-carbonyl) amino-5-((8S)-5,6,7,8-tetrahydroquinolin-8-yl) amino valerate 1-naphthylmethylamide [Compound No. 105];
(2S)-2-(4-((N-imidazol-2-ylmethyl) aminomethyl) benzoyl amino-5-(5,6,7,8-tetrahydroquinolin-8-yl) amino valerate (1'S) -1'-(1-naphthyl) ethylamide [Compound No. 106];
(2S)-2-(4-((N-1-methylimidazol-2-ylmethyl) aminomethyl) benzoylamino-5-(5,6,7,8-tetrahydroquinolin-8-yl) amino valerate (1'S)-1'-(1-naphthyl) ethylamide [Compound No. 107];
(2S)-2-(4-(2-picolylaminomethyl) enzoyl-5-(imidazol-2-1) amino valerate (1'S)-1'-(1-naphthyl) ethylamide [Compound No. 108];
(2S)-2-(4-2-picolylaminomethyl) benzoyl-5-(pyridin-2-yl) amino valerate (1'S)-1'-(1-naphthyl) ethylamide [Compound No. 109];
(S)-2-(4-((N-imidazol-2-ylmethyl) aminomethyl) benzoyl) amino-5-(4,5-dihydroimidazol-2-yl) amino valerate (1'S)-1'-(1-naphthyl) ethylamide [Compound No. 110];
(S)-2-(4-((N-imidazol-2-ylmethyl) aminomethyl) benzoyl) amino-5-(pyrimidin-2-yl) amino valerate (1'S)-1'-(1-naphthyl) ethylamide [Compound No. 111];
(S)-2-(4-((N-imidazol-2-ylmethyl) aminomethyl) benzoyl) amino-5-(3,4,5,6-tetrahydropyrimidin-2-yl) amino valerate (1'S)-1'-(1-naphthyl) ethylamide [Compound No. 112];
(S)-2-(4-(N-2-picolylaminomethyl) benzoyl) amino-5-(4,5-dihydroimidazol-2-yl) amino valerate (1'S)-1'-(1-naphthyl) ethylamide [Compound No. 113];
(S)-2-(4-(N-2-picolyl) aminomethyl) benzoyl) amino-5-(pyrimidin-2-yl) aminovalerate (1'S)-1'-(1-naphthyl) ethylamide [Compound No. 114];
(S)-2-(4-(N-2-picolylaminomethyl) benzoyl) amino-5-3,4,5,6-tetrahydropyrimidin-2-yl) amino valerate (1'S)-1'-1-naphthyl) ethylamide [Compound No. 115];
(S)-2-(4-(2-picolylaminomethyl)benzoyl)-5-(2-picolylami no) valerate 1'-(1-naphthyl) ethylamide [Compound No. 116];
(S)-2-(4-(2-picolylaminomethyl)benzoyl)-5-(2-picolylami no) valerate n-dodecylamide [Compound No. 117];
(S)-2-(4-(2-picolylaminomethyl)benzoyl)-5-(2-picolylami no) valerate 3,5-ditrifluoromethylbenzylamide [Compound No. 118];
(S)-2-(4-(2-picolylaminomethyl)benzoyl)-5-(2-picolylami no) valerate (+)-dehydroabietylamide [Compound No. 119];
(S)-2-(4-(2-picolylaminomethyl)benzoyl)-5-(2-picolylami no) valerate 2,3-dichlorobenzylamide [Compound No. 120];
(S)-2-(4-(2-picolylaminomethyl)benzoyl)-5-(2-picolylami no) valerate 2-octylamide [Compound No. 121];
(S)-2-(4-(2-picolylaminomethyl)benzoyl)-5-(2-picolylami no) valerate 3-(3-indolyl)-2-propylamide [Compound No. 122];
(S)-2-(4-(2-picolylaminomethyl)benzoyl)-5-(2-picolylami no) valerate 2,2-diphenylethylamide [Compound No. 123];
(S)-2-(4-(2-picolylaminomethyl)benzoyl)-5-(2-picolylami no) valerate 4-t-butylcyclohexylamide [Compound No. 124];
(S)-2-(4-(2-picolylaminomethyl)benzoyl)-5-(2-picolylami no) valerate 2,4-dichlorobenzylamide [Compound No. 125];
(S)-2-(4-(2-picolylaminomethyl)benzoyl)-5-(2-picolylami no) valerate benzhydrylamide [Compound No. 126];
(S)-2-(4-(2-picolylaminomethyl)benzoyl)-5-(2-picolylami no) valerate 3-chlorobenzylamide [Compound No. 127];
(S)-2-(4-(2-picolylaminomethyl)benzoyl)-5-(2-picolylami no) valerate 2-(4-methoxyphenyl)ethylamide [Compound No. 128];
(S)-2-(4-(2-picolylaminomethyl)benzoyl)-5-(2-picolylami no) valerate (4-(4-methylphenyl)oxy)phenylamide [Compound No. 129]; and
(S)-2-(4-(2-picolylaminomethyl)benzoyl)-5-(2-picolylami no) valerate 1-(1,2,3,4-tetrahydronaphthyl)amide [Compound No. 130].

The present invention relates to a CXCR4-antagonist containing the compounds described above or pharmaceutically-acceptable salt thereof as its active ingredient.

The CXCR4-antagonist or the salt thereof according to the present invention is used for the treatment or prevention of an associated disease such as rheumatism or cancer metastasis on the basis of their CXCR4-antagonisms.

The CXCR4-antagonist or the salt thereof of the present invention is intraperitoneally inj ected into an ICRmouse (50 mg/ml) twice a day for 4 consecutive days, and no lethal case was found after 5 days. Therefore, the conclusion can be drawn that there is no acute toxicity.

The administration dosage per day, 0.1 to 500 mg/kg, preferably 1 to 100 mg/kg of body weight/day may be one dosage ormaybe divided into several dosages. Apreferable administration route is, but not limited to, oral administration. Alternatively, parenteral administration such as injection, percutaneous administration, and intestinal administration may be suitably selected. Furthermore, the administration dosage may be suitably modified depending on the conditions of a patient.

A dosage form for oral administration may be powder, tablet, granule, capsule, suppository, injection, peroral liquid agent, or the like, in which one or more additives pharmaceutically-acceptable added to CXCR4 antagonists or salts thereof of the present invention is included. Examples of the additive may include magnesium stearate, talc, lactose, dextrin, starches, methyl cellulose, fatty acid glycerides, water, propylene glycol, macrogols, alcohol, crystalline cellulose, hydroxypropyl cellulose, low substituted hydroxypropyl cellulose, carmelloses, popidone, polyvinyl alcohol, and calcium stearate. Furthermore, as required, any additive such as a coloring agent, a stabilizing agent, an antiseptic, a pH regulator, an isotonizing agent, a solubilizing agent, and/or a soothing agent may be added. The granule, the tablet, or the capsule may be coated with a coating base material such as hydroxypropyl methylcellulose or hydroxypropyl methylcellulose phthalate. Furthermore, in a single dosage, it is preferable to include 0.1 to 500 mg, preferably 1 to 100 mg of the CXCR4 antagonist of the present invention.

Next, the method for producing the CXCR4 antagonist of the present invention will be concretely explained by representing Synthesis Examples.

### Synthesis Example 1: Production of (S)-2-(4-(N-2-picolylamino methyl)benzoylamino)-5-((imidazol-2-ylmethyl)amino) valerate 1-naphthalenemethylamide [Compound No. 1]

### Synthesis Example 1-1: Synthesis of methyl 4-(N-Boc-N-2-picolyl aminomethyl) benzoate (Compound VI-1)

108 g of commercially available 2-picolylamine was dissolved in 22.5 ml of DMF, and then 1.55 ml of triethylamine was added thereto and the obtained solution was cooled down to 0°C. In this solution, a solution prepared by dissolving 2.52 ml of di-t-butyldicarbonate in 7.5 ml of DMF was dropped during 10 minutes. The solution was warmed up to room temperature and stirred for 2 hours, and then the solvent was distilled off under reduced pressure. The residue was purified by means of silica gel chromatography (30 g, chloroform), resulting in obtaining 1.71 g of a light-yellow liquid.

1.199 g of the light-yellow liquid was dissolved in 6ml of THF, followed by suspending in 46.1 mg of sodium hydroxide (60% paraffin mixture). After the suspension was stirred for 15 minutes at room temperature, 241 mg of commercially available methyl 4-bromomethylbenzoate was added thereto and the obtained solution was stirred for further 2 days at room temperature. On completion of the reaction, the pH of the solution was adjusted to 5 to 7 using a 1 mol/l hydrochloric acid and the solution was concentrated. Then, 40 ml of chloroform was added to the solution, followed by washing with water and the solution was then concentrated. The residue was purified by means of silica gel column chromatography (6 g, chloroform/ethyl acetate = 10/1), and 2.21 g of the above-mentioned compound was obtained as a light-yellow liquid.
MS(FAB,Pos.) :m/z=357[M+1]⁺
¹H-NMR(500MHz,CDCl₃):δ=1.45(9H,s),3.91(3H,s),4.47 (1H,brs),4.52 (1H,brs),4.60(2H,s),7.17(1H,dd,J=7.6,4.1Hz),7.2-7.4(3H,m),7.6 5(1H,t,J=7.6Hz),8.52(1H,d,J=4.1Hz).

### Synthesis Example 1-2: Synthesis of 4-(N-Boc-N-2-picolylamino ethyl) benzoic acid (Compound VII-1)

To 200.6 mg of the compound obtained in Synthesis Example 1-1, 2 ml of methanol, 2ml of THF, and 2 ml of a 1 mol/l sodium hydroxide aqueous solution were added and the resultant mixture was stirred for 1 day at room temperature. On completion of the reaction, the solvent was distilled off, and then 5 ml of water was added to the residue. In this solution, a 1 mol/l hydrochloric acid was dropped to adjust the pH thereof to pH = 3. A precipitated crystal was collected by filtration and was then dried, 123.2 mg of the above-mentioned compound was obtained as a colorless crystal.
MS(FAB,Pos.):m/z=343[M+1]⁺
¹H-NMR(500MHz,DMSO-d₆) : δ=1.35 and 1.54 (9H,brs), 4.41 (1H,brs), 4.51 (2H,s), 4.58 (1H,brs), 7.2-7.4 (4H,m), 7.77 (1H,td,J=7.6,1.8Hz), 8.5 2 (1H,dd,J=4.9,1.7Hz), 12.9 (1H,s).

### Synthesis Example 1-3: Synthesis of N^{α}-4-(N-Boc-N-2-picolylamino ethyl) benzoyl-N^{δ}- Cbz-L-ornithine 1-naphthalenemethylamide (Compound XI-1)

3.00 g of commercially available N^{α}-Fmoc-N^{δ}-Cbz-L-ornithine was dissolved in 60 ml of DMF, and then 1.24 g of HOBt, 1.77g of WSCI hydrochloride, and 1.48 ml of 1-naphthalenemethylamine were sequentially added to the solution and the resultant mixture was stirred for 13 hours at room temperature. On completion of the reaction, the solvent was distilled off. Then, the residue was dissolved in chloroform, and the extraction was performed by the addition of a 1 mol/l hydrochloric acid, followed by washing an organic layer with a saturated aqueous solution of sodium hydrogencarbonate. The solvent was distilled off, 4.44 g of crude product as a light-yellowish white solid product was obtained. The crude product was dissolved in 150 ml of DMF and then 10 ml of diethylamine was added to the solution and the resultant mixture was stirred for 6 hours at room temperature. On completion of the reaction, the solvent was distilled off and dried with a vacuum pump. As a result, 4.21 g of the crude product was obtained as a light-yellowish white solid. This product was dissolved in 140 ml of DMF, and then
1.77 g of WSCI hydrochloride and 2.10 g of the compound obtained in Synthesis Example 1-2 were added to the solution, followed by stirring the resultant mixture for 21.5 hours at room temperature. On completion of the reaction, the solvent was distilled off. The residue was purified by means of silica gel column chromatography (500 g, chloroform/methanol = 40/1 to 15/1), 4.25 g of the above-mentioned compound was obtained as a light-orange color semisolid material.
MS(FAB,Pos.):m/z=730[M+1)⁺
¹H-NMR(500MHz,DMSO-d₆):δ=1.31 and 1.38(9H,2s),1.44-1.54(2H,m), 1.73-1.77(2H,m),2.89-3.03(2H,m),4.40(1H,brs),4.47-4.51(3H,m), 4.56(1H,brs),4.75(2H,d,J=5.7Hz),4.98(2H,s),7.20-7.37(9H,m),7. 45-7.47(2H,m),7.51-7.54 (2H,m),7.76-7.80(1H,m),7.83-7.85(1H,m) ,7.86(2H,d,J=8.3Hz),7.94-7.95(2H,m),8.05-8.06(1H,m),8.46(1H,d ,J=8.1Hz),8.50-8.52(2H,m).

### Synthesis Example 1-4: Synthesis of N^{α}-4-(N-Boc-N-2-picolylamino ethyl) benzoyl-L-ornithine 1-naphthalenemethylamide (Compound XII-1)

4.25 g of the compound obtained in Synthesis Example 1-3 was dissolved in 100 ml of methanol and then 4.25 g of 10%Pd-C was added to the solution, followed by stirring the resultantmixture for 4.5 hours in a hydrogen atmosphere at normal pressures. On completion of the reaction, the residue, which was obtained by removing the catalyst by means of celite filtration, was purified by means of silica gel column chromatography (200 g, chloroform/methanol = 12/1 to 4/1), 2.22g (64.0%) of the above-mentioned compound was obtained as a white crystal.
MS(FAB,Pos.):m/z=596[M+1]⁺
¹H-NMR(500MHz,DMSO-d₆):δ=1.31 and 1.38(9H,2s),1.59-1.64(2H,m), 1.76-1.88(2H,-m),2.75-2.79(2H,m),4.40(1H,s),4.49(2H,brs),4.52-4.57(2H,m),4.77(2H,d,J=5.4Hz),7.21-7.23(1H,m),7.26-7.36(3H,m) ,7.46-7.47(2H,m),7.49-7.56(2H,m),7.77-7.80(1H,m),7.84-7.86(1H ,m),7.90(2H,d,J=8.3Hz),7.94-7.96(1H,m),8.05-8.07(1H,m),8.52(1 H,m),8.56(1H,m),8.60(1H,t,J=5.6Hz).

### Synthesis Example 1-5: Synthesis of (S)-2-(4-(N-Boc-N-2-picolyl aminomethyl) benzoyl)-5-((imidazol-2-ylmethyl)amino) valerate 1-naphthalenemethylamide (Compound XIII-1)

60.0 mg of the compound obtained in Synthesis Example 1-4 was dissolved in 2.2 ml of anhydrous methanol, and then 10.2 mg of 2-imidazole carboxyaldehyde was added to the solution and the resultant mixture was stirred for 1 hour at room temperature. After the solvent was distilled off, 1.2 ml of anhydrous methanol was added to the solution and the resultant solution was cooled down to 0°C. Subsequently, 7.6 mg of sodium borohydride was added to the solution and the resultant mixture was stirred for 1.5 hours at room temperature. The residue obtained by concentrating the reaction solution was purified by means of silica gel column chromatography (6 g, chloroform/methanol = 20/1 to 9/1), and 46.7 mg of the above-mentioned compound was obtained as a white crystal.
MS(FAB,Pos.) :m/z= 676[M+1]⁺
¹H-NMR(500MHz,CDCl₃) :δ=1.45 and 1.47(9H,2s),1.92-2.22(4H,m), 2.66-2.74(2H,m),3.71-3.81(2H,m),4.46-4.55(2H,m),4.57(2H,brs), 4.68(1H,d,J=4.5Hz),4.72-4.75(1H,m),4.82-4.86(1H,m),4.99-5.03 (1H,m),6.88(2H,s),6.99(2H,s),7.18-7.26(3H,m),7.29-7.31(1H,m),7 .40-7.53(4H,m),7.63-7.69(3H,m),7.80-7.81(1H,m),7.85-7.86(1H,m ),7.86-7.88(1H,m),8.00-8.02(1H,m),8.12(1H,brs),8.52(1H,brs).

### Synthesis Example 1-6: Synthesis of (S)-2-(4-(N-2-picolylamino ethyl) benzoylamino)-5-((imidazol-2-ylmethyl)amino) valerate 1-naphthalenemethylamide [Compound No.1]

45.0 mg of the compound obtained in Synthesis Example 1-5 was dissolved in 0.9 ml of methanol, and then 0.9 ml of a 4 mol/l hydrochloric acid/dioxane solution was added to the solution and the resultant mixture was stirred for 4.5 hours at room temperature. The residue obtained by concentrating the reaction solution was purified by means of silica gel column chromatography (3.4 g, chloroform/methanol = 1/l). Subsequently, the resultant compound was dissolved in 1.5 ml of a 1 mol/l hydrochloric acid and then water was distilled off, and 35.3 mg of hydrochloride of the above-mentioned compound was obtained as a white solid product.
MS(FAB,Pos.):m/z= 576[M+1]⁺
¹H-NMR(500MHz,DMSO-d₆):δ=1.75-1.91(4H,m),3.07-3.17(2H,m),4.30 (4H,brs),4.48(2H,brs),4.55-4.59(1H,m),4.59(2H,d,J=4.9Hz),7.44-7.47(3H,m),7.52-7.57(3H,m),7.66(2H,d,J=8.6Hz),7.72(2H,brs),7. 83-7.96(3H,m),8.00(2H,d,J=8.5Hz),8.05-8.07(1H,m),8.65-8.73(3H ,m),9.84(2H,brs),10.10(1H,brs).

### Synthesis Example 2: Synthesis of (S)-2-(4-(N-2-picolylamino ethyl) benzoylamino)-5-((pyrrol -2-ylmethyl) amino) valerate 1-naphthalenemethylamide [Compound No. 2]

### Synthesis Example 2-1: Production of (S)-2-(-4-(N-Boc-N-2-picolylaminomethyl) benzoylamino)-5-((pyrrol -2-ylmethyl) amino) valerate 1-naphthalenemethylamide (Compound XIII-2)

82.4 mg of the compound obtained in Synthesis Example 1-4 was dissolved in 1.6 ml of anhydrous methanol, and then 14.5 mg of pyrrol -2- carboxyaldehyde was added to the solution and the resultant mixture was stirred for 15 hours at room temperature. After the solvent was distilled off, 1.6 ml of anhydrous methanol was added thereto the resultant and the resultant mixture was cooled to 0°C. Subsequently, 10.5 mg of sodium borohydride was added thereto and the resultant mixture was stirred for 2 hours at room temperature. The residue obtained by concentrating the reaction solution was purified by means of silica gel column chromatography (10g, chloroform/methanol = 8/1), and 55.6mg of the above-mentioned compound was obtained as a white crystal.
MS(FAB,Pos.):m/z= 675[M+1]⁺
¹H-NMR(500MHz, CDCl₃) :δ=1.44 and 1.47(9H,2s) 1.57-2.00(4H,m), 2.70-2.78(2H,m),3.66-3.81(2H,m),4.45-4.49(2H,m),4.57(2H,brs), 4.74-4.75(1H,m),4.83-4.87(1H,m),4.99-5.02(1H,m),4.99-5.02(1H, m),6.00(1H,brs),6.05-6.07(1H,m),6.67(1H,brs),7.17-7.24(3H,m), 7.28-7.36(3H,m),7.41-7.54(5H,m),7.64-7.68(3H,m),7.80-8.82(1H, m),7.87-7.88(1H,m),7.98(1H,m),8.01-8.03(1H,m),8.52-8.53(1H,m).

### Synthesis Example 2-2: Synthesis of (S)-2-(4-(N-2-picolylamino methyl) benzoylamino)-5-((pyrrol-2-ylmethyl) amino) valerate 1-naphthalenemethylamide [Compound No. 2]

55.6 mg of the compound obtained in Synthesis Example 2-1 was dissolved in 1.1 ml of methanol, and then 1.1 ml of a 4 mol/l hydrochloric acid/dioxane solution was added to the solution and the resultant mixture stirred for 3.5 hours at room temperature. A crude product obtained by concentrating the reaction solution was purified by means of silica gel column chromatography (8 g, chloroform/methanol=1/1). Subsequently, the resultant compound was dissolved in 1.1 ml of a 1 mol/l hydrochloric acid and then water was distilled off, and 49.8 mg of hydrochloride of the target compound was obtained as a light-orange color solid product.
MS(FAB,Pos.) :m/z=575[M+1]⁺
¹H-NMR(500MHz,DMSO-d₆) : δ=1.70-1.91(4H,m),2.83-2.86(2H,m),4.04-4.06 (2H,m), 4.29 (4H,m), 4.52-4.56 (1H,m), 4.75-4.77 (2H,d,J=4.9Hz) , 6.01-6.02 (1H,m), 6.17-6.18 (1H,m), 6.81-6.83 (1H,m), 7.44-7.49 (3H , m), 7.52-5.58 (3H,m), 7.66-7.68 (2H,m), 7.84-8.07 (6H,m), 8.65-8.73 (3H,m), 9.20 (1H,brs), 9.91 (1H,brs), 11.08 (1H,s).

### Synthesis Example 3: Production of (S)-2-(4-(N-2-picolylamino methyl) benzoylamino)-5-((1-methylimidazol-2-ylmethyl)amino) valerate 1-naphthalenemethylamide [Compound No.3]

### Synthesis Example 3-1: Synthesis of (S)-2-(4-(N-Boc-N-2-picolyl aminomethyl) benzoyl)-5-((1-methylimidazol-2-ylmethyl) amino) valerate 1-naphthalenemethylamide (Compound XIII-3)

100 mg of the compound obtained in Synthesis Example 1-4 was dissolved in 2 ml of anhydrous methanol, and then 20.3 mg of 1-methyl-2-imidazole carboxyaldehyde was added to the solution and the resultant mixture stirred for 5 hours at room temperature. After the solvent was distilled off, 2 ml of anhydrous methanol was added to the resultant and the resultant mixture was cooled to 0°C. Subsequently, 12.7 mg of sodium borohydride was added thereto and the resultant mixture stirred for 3 hours at room temperature. The residue obtained by concentrating the reaction solution was purified by means of silica gel column chromatography (10 g, chloroform/methanol = 12/1), and 77.4 mg of the above-mentioned compound was obtained as a white solid product.
MS(FAB,Pos.):m/z= 69.0[M+1]⁺
¹H-NMR(500MHz,DMSO-d₆) : (=1.31 and 1.38(9H,2s),1.43-1.52(2H,m),1. 74-1.81(2H,m),3.38(3H,s),4.40(1H,m),4.45-4.56(6H,m),4.74-4.47 (2H,m),5.23(1H,m),6.71-6.75(2H,m),7.01-7.07(2H,m),7.22-7.46(4 H,m),7.51-7.55(2H,m),7.76-7.80(1H,m),7.83-7.88(3H,m),7.93-7.9 5(1H,m),8.05-8.07(1H,m),8.50-8.56(3H,m).

### Synthesis Example 3-2: Synthesis of (S)-2-(4-(N-2-picolylamino methyl) benzoylamino)-5-((1-methylimidazol-2-ylmethyl)amino) valerate 1-naphthalenemethylamide [Compound No. 3]

75.9 mg of the compound obtained in Synthesis Example 3-1 was dissolved in 1.5 ml of methanol, and then 1.5 ml of a 4 mol/l hydrochloric acid/dioxane solution was added to the solution and the resultant mixture was stirred for 9 hours at room temperature. The residue obtained by concentrating the reaction solution was purified by means of silica gel column chromatography (10 g, chloroform/methanol =1/1). Subsequently, the resultant compound was dissolved in 1.5 ml of a 1 mol/l hydrochloric acid and then water was distilled off, and 19.6 mg of hydrochloride of the target compound was obtained as a white solid product.
MS(FAB,Pos.):m/z= 590[M+1]⁺
¹H-NMR(500MHz,DMSO-d₆) : (=1.77-1.91 (4H,m),3.09-3.17 (2H,m),3.95 (3H,s),4.29(4H,br),4.52-4.59(3H,m),4.76-4.77(2H,m),7.44-7.48(3 H,m),7.52-7.57(3H,m),7.65-7.67(2H,m),7.71-7.74(2H,m),7.83-8.0 8(6H,m),8.65-8.66(1H,m),8.70-8.75(2H,m),9.88(1H,br),10.09 (1H,br).

### Synthesis Example 4: Production of (S)-2-(4-(N-2-picolylamino methyl) benzoylamino)-5-((imidazol-4-ylmethyl)amino) valerate 1-naphthalenemethylamide [Compound No.4]

### Synthesis Example 4-1: Synthesis of 2-(4-(N-Boc-N-2-picolyl aminomethyl) benzoyl)-5-((imidazol-4-ylmethyl) amino) valerate 1-naphthalenemethylamide (Compound XIII-4)

100 mg of the compound obtained in Synthesis Example 1-4 was dissolved in 2 ml of anhydrous methanol, and then 17.7 mg of 4 (5)-imidazole carboxyaldehyde was added to the solution and the resultant mixture was stirred for 5 hours at room temperature. After the solvent was distilled off, 2 ml of anhydrous methanol was added to the resultant and the resultant mixture was cooled to 0°C. Subsequently, 12.7 mg of sodium borohydride was added thereto and the resultant mixture was stirred for 3 hours at room temperature. The residue obtained by concentrating the reaction solution was purified by means of silica gel column chromatography (10g, chloroform/methanol=3/1), and 86.6mg of the above-mentioned compound was obtained as a white solid product.
MS (FAB,Pos.):m/z= 676 [M+1]⁺
¹H-NMR(500MHz,DMSO-d₆) : (=1.31 and 1.38(9H,2s),1.51-1.55(2H,m),1. 77-1.81(2H,m),2.64-2.65(2H,m),3.32(2H,m),3.65(2H,br),4.40-4.5 6(SH,m),4.75-4.76(2H,m),7.20-7.35(4H,m),7.44-7.55(5H,m),7.76-7.80(1H,m),7.83-7.88(3H,m),7.91-7.95(1H,m),8.05-8.07(1H,m),8. 52(1H,d,J=4.1Hz),8.56(2H,br).

### Synthesis Example 4-2: Synthesis of (S)-2-(4-(N-2-picolylamino methyl) benzoylamino)-5-((imidazol-4-ylmethyl)amino) valerate 1-naphthalenemethylamide [Compound No. 4]

84.6 mg of the compound obtained in Synthesis Example 4-1 was dissolved in 1.7 ml of methanol, and then 1.7 ml of a 4 mol/l hydrochloric acid/dioxane solution was added to the solution and the resultant mixture was stirred for 9 hours at room temperature. A crude product obtained by concentrating the reaction solution was purified by means of silica gel column chromatography (7 g, chloroform/methanol=1/1). Subsequently, the resultant compound was dissolved in 1.7 ml of a 1 mol/l hydrochloric acid and then water was distilled off, and 52.8 mg of hydrochloride of the above compound was obtained as a white solid product.
MS(FAB,Pos.):m/z= 576[M+1]⁺
¹H-NMR(500MHz,DMSO-d₆):δ=1.74-1.91 (5H,m),2.95 (2H,br),4.25-4.30 (6H,m),4.54-4.19(3H,m),4.76-4.77(2H,d,J=5.6Hz),7.45-7.48(3H,m), 7.49-7.60(4H,m),7.66-7.68(2H,m),7.82-8.08(7H,m),8.65-8.76(3 H,m),9.14(1H,m),9.82-9.92(2H,m).

### Synthesis Example 5: Production of (S)-2-(4-(N-2-picolylamino methyl) benzoylamino)-5-((1-methylpyrrol-2-ylmethyl) amino) valerate 1-naphthalenemethylamide [Compound No. 5]

### Synthesis Example 5-1: Synthesis of (S)-2-(4-(N-Boc-N-2-picolyl aminomethyl) benzoylamino)-5-((1-methylpyrrol-2-ylmethyl) amino) valerate 1-naphthalenemethylamide (Compound XIII-5)

100 mg of the compound synthesized in Synthesis Example 1-4 was dissolved in 2 ml of anhydrous methanol, and then 20.4 (1 of 1-methyl-2-pyrrol carboxyaldehyde was added to the solution and the resultant mixture was stirred for 13.5 hour at room temperature. After the solvent was distilled off, 2 ml of anhydrous methanol was added to the resultant and the resultant mixture was cooled to 0°C. Subsequently, 12.7 mg of sodium borohydride was added thereto and the resultant mixture was stirred for 3 hours at room temperature. The residue obtained by concentrating the reaction solution was purified by means of silica gel column chromatography (10 g, chloroform/methanol = 8/1), and 38.3 mg (33.1 %) of the above-mentioned compound was obtained as a white solid product.
MS(FAB,Pos.):m/z= 576[M+1]⁺
¹H-NMR(500MHz,DMSO-d₆):δ=1.31 and 1.38(9H,2s),1.49-1.55(2H,m), 1.73-1.91(2H,m),3.54(3H,s),3.64(2H,br),4.36-4.56(4H,m),4.71-4 .80(2H,m),5.86-5.89(2H,m),6.62(1H,br),7.29-7.35(4H,m),7.44-7. 55(4H,m),7.76-7.80(1H,m),7.83-7.88(4H,m),7.94-7.96(1H,m),8.06 - 8.07(1H,m),8.50-8,54(3H,m).

### Synthesis Example 5-2: Synthesis of (S)-2-(4-(N-2-picolylamino methyl) benzoylamino)-5-((1-methylpyrrol-2-ylmethyl) amino) valerate 1-naphthalenemethylamide [Compound No. 5]

36.3 mg of the compound obtained in Synthesis Example 5-1 was dissolved in 1 ml of methanol, and then 1 ml of a 4 mol/l hydrochloric acid/dioxane solution was added to the solution and the resultant mixture was stirred for 2 hours at room temperature. The residue obtained by concentrating the reaction solution was purified by means of silica gel column chromatography (5 g, chloroform/methanol = 3/1). Subsequently, the resultant compound was dissolved in 1 ml of a 1 mol/l hydrochloric acid and then water was distilled off, and 41.1 mg of hydrochloride of the above target compound was obtained as a white solid product.
MS(FAB,Pos.):m/z=589[M+1]⁺
¹H-NMR(500MHz,DMSO-d₆) : δ=1.73-1.88 (4H,m), 2.91 (2H,m), 3.65 (3H,s) , 4.07-4.09 (2H,m), 4.29 (4H,br), 4.53-4.57 (1H,m), 4.76 (2H,d,J=5.8H z), 5.97-5.99 (1H,m), 6.23-6.24 (1H,m), 6.78 (1H,m), 7.44-7.45 (3H,m) , 7.46-7.59 (5H,m), 7.67 (2H,d,J=8.3Hz), 7.84-8.08 (6H,m), 8.65-8.75 (3H,m),9.08(1H,m),9.93(1H,br).

### Synthesis Example 6: Synthesis of (S)-2-(4-(N-2-picolylamino methyl) benzoylamino)-5-((1-methylbenzimidazol-2-ylmethyl) amino) valerate 1-naphthalenemethylamide [Compound No. 6]

### Synthesis Example 6-1: Synthesis of 2-(4-(N-Boc-N-2-picolylamino methyl) benzoyl)-5-((1-methylbenzimidazol-2-ylmethyl) amino) valerate 1-naphthalenemethylamide (Compound XIII-6)

50.3 mg of the compound synthesized in Synthesis Example 1-4 was dissolved in 1 ml of anhydrous methanol, and then 16.1 mg of 1-methyl-2-formylbenzimidazole was added to the solution and the resultant mixture was stirred for 1.5 hours at room temperature. After the solvent was distilled off, 1 ml of anhydrous methanol and a drop of acetic acid were added to the resultant and the resultant mixture was cooled to 0°C. Subsequently, 14.2 mg of sodium borohydride was added thereto and the resultant mixture was stirred for 0.5 hour at room temperature. The residue obtained by concentrating the reaction solution was purified by means of silica gel column chromatography (2.5 g, chloroform/methanol = 15/1), and 31.3 mg of the above-mentioned compound was obtained as a white solid product.
MS(FAB,Pos.):m/z=740[M+1]⁺
¹H-NMR(500MHz,CDCl₃):δ=1.43 and 1.44(9H,2s),1.52-1.63(1H,m), 1.65-1.78(1H,m),1.89-2.01(2H,m),2.68-2.83(2H,m),3.58(3H,s),3. 75(1H,d,J=14.6Hz),3.81(1H,d,J=14.6Hz),4.41and4.44(2H,2s),4.52 and 4.55(2H,2s)4.69-4.75(1H,m),4.85(1H,dd,J=14.6,5.1Hz),4.98 (1H,dd,J=14.6,5.6Hz),7.10-7.35(7H),7.36(1H,d,J=6.6Hz),7.43-7. 51(2H,m),7.52-7.60(1H,m),7.69-7.78(7H,m),7.80-7.83(1H,m),7.99 -8.01 (1H,m), 8.53 (1H,ddd,J=4.9,2.0,1.0Hz).

### Synthesis Example 6-2: Synthesis of (S)-2-(4-(N-2-picolylamino methyl) benzoylamino)-5-((1-methylbenzimidazol-2-ylmethyl) amino) valerate 1-naphthalenemethylamide [Compound No. 6]

28.7 mg of the compound obtained in Synthesis Example 6-1 was dissolved in 0.3 ml of methanol, and then 0.5 ml of a 4 mol/l hydrochloric acid/dioxane solution was added to the solution and the resultant mixture was stirred for 2.5 hours at room temperature. The residue obtained by concentrating the reaction solution was reprecipitated from chloroform/methanol, and 19.0 mg of hydrochloride of the above-mentioned compound was obtained as a light-yellow solid product.
MS(FAB,Pos.):m/z=640[M+1]⁺
¹H-NMR(500MHz, DMSO-d₆) : δ=1.7-1.96 (4H,m),3.12-3.22 (2H,m),3.99 (3H,s),4.32(4H,brs),4.53-4.62(1H,m),4.67(2H,s),4.77(1H,d,J=5.6 Hz),7.41-7.62(8H,m),7.68(2H,d,J=7.8Hz),7.69(1H,d,J=7.6Hz),7.7 8(1H,d,J=7.8Hz),7.80-7.89(2H,m),7.94-7.97(2H,m),8.00(2H,d,J=7 .8Hz),8.07(1H,d,J=7.8Hz),8.68(1H,d,J=4.2Hz),8.74(1H,d,J=8.5Hz ),8.75-8.78(1H,m),10.10(4H,brs).

### Synthesis Example 7: Synthesis of (S)-2-(4-(N-2-picolylamino methyl) benzoylamino)-5-((quinolin-2-ylmethyl) amino) valerate 1-naphthalenemethylamide [Compound No. 7]

### Synthesis Example 7-1: Synthesis of 2-(4-(N-Boc-N-2-picolylamino methyl) benzoyl)-5-((quinolin-2-ylmethyl) amino) valerate 1-naphthalenemethylamide (Compound XIII-7)

50.3 mg of the compound synthesized in Synthesis Example 1-4 was dissolved in 1 ml of anhydrous methanol, and then 15.8 mg of 2-quinoline aldehyde was added to the solution and the resultant mixture was stirred for 1.5 hours at room temperature. After the solvent was distilled off, 1 ml of anhydrous methanol and a drop of acetic acid were added to the resultant and the resultant mixture was cooled to 0°C. Subsequently, 14.2 mg of sodium borohydride was added thereto and the resultant mixture was stirred for 0.5 hour at room temperature. The residue obtained by concentrating a reaction solution was purified by means of silica gel column chromatography (2.5 g, chloroform/methanol = 15/1), and 12.9 mg of the above-mentioned compound was obtained as a light-yellow frothy product.
MS(FAB,Pos.):m/z= 737[M+1]⁺
¹H-NMR(500MHz,CDCl₃): δ=1.42(9H,s),1.61-1.73(1H,m),1.73-1.83(1H , m), 1.79-1.89 (1H,m), 2.16-2.22 (1H,m), 2.68-2.77 (1H,m), 2.78-2.84 (1H,m), 3.74 (1H,d,J=15.1Hz), 3.79 (1H,d,J=15.1Hz), 4.37 and 4.38 (2H,2s), 4.46 and 4.50 (2H,2s), 4.77-4.81 (1H,m), 4.88 (1H,dd,J=14.5, 5.2Hz), 4.94 (1H,dd,J=14.5,5.4Hz), 7.00-7.20 (4H,m), 7.21-7.28 (?H, m), 7.30 (1H,d,J=6.6Hz), 7.43-7.49 (3H,m), 7.57-7.66 (4H,m), 7.69 (1H ,d,J=8.3Hz), 7.73 (1H,d,J=7.8Hz), 7.77-7.82 (1H,m), 7.86 (1H,d,J=8. 3Hz), 7.97 (1H,d,J=8.1Hz), 8.00-8.02 (1H,m), 8.38-8.44 (1H,m), 8.53 (1H,ddd,J=4.9,2.0,1.0Hz).

### Synthesis Example 7-2: Synthesis of (S)-2-(4-(N-2-picolylamino methyl) benzoylamino)-5-((quinolin-2-ylmethyl) amino) valerate 1-naphthalenemethylamide [Compound No.7]

10.6 mg of the compound obtained in Synthesis Example 7-1 was dissolved in 0.2 ml of methanol, and then 0.2 ml of a 4 mol/l hydrochloric acid/dioxane solution was added to the solution and the resultant mixture was stirred 1.5 hours at room temperature. The residue obtained by concentrating the reaction solution was subjected to azeotropic distillation with methanol to remove excess hydrochloric acid, and 10.7 mg of hydrochloride of the above-mentioned compound was obtained a light-yellow solid product.
MS(FAB,Pos.):m/z=637[M+1]⁺
¹H-NMR(500MHz,DMSO-d₆):δ=1.79-1.97(4H,m) 3.07-3.15(2H,m), 4.31 (4H,brs),4.49-4.56(2H,m),4.57-4.65(1H,m),4.77(1H,d,J=5.6Hz),7. 42-7.58(5H,m),7.64-7.75(5H,m),7.82-7.89(2H,m),7.93-7.98(2H,m) ,8.01(2H,d,J=8.1Hz),8.03-8.10(3H,m),8.48(1H,d,J=8.5Hz),8.68(1 H,d,J=4.9Hz),8.74-8.76(2H,m),9.54(2H,brs),10.05(2H,brs).

### Synthesis Example 8: Production of (2S)-2-((4-(N-2-picolylamino methyl) phenylacetyl) amino)-5-(5, 6, 7, 8-tetrahydroquinolin-8-yl) amino valerate 1-naphthalenemethylamide [Compound No. 8]

### Synthesis Example 8-1: Synthesis of methyl 4-bromomethylphenyl acetate (Compound IV-1)

505.0 mg of commercially available 4-bromomethylphenyl acetate was dissolved in 5 ml of methanol and 5 ml of tetrahydrofuran. In this solution, 1.31 ml of a 2 mol/l trimethylsilyl diazomethane-hexane solution was dropped. After the dropping was completed, the resultant solution was stirred for 1 hour and acetic acid was dropped therein until a light-yellow color disappeared from the solution. The solvent was distilled off and the residue was then dissolved in chloroform, followed by washing with a 1 mol/l hydrochloric acid, a 1 mol/l sodium hydroxide aqueous solution, and a saturated salt solution and drying with anhydrous sodium sulfate. The solvent was distilled off and then the residue was purified by means of silica gel column chromatography (25 g, hexane/ethyl acetate = 5/1), and 447.7 mg of the above-mentioned compound was obtained as a colorless solid product.
MS(FAB,Pos.):m/z=243,245[M+1]⁺
¹H-NMR(500MHz,CDCl₃):δ=3.63(2H,s),3.70(3H,s),4.49(2H,s),7.25-7 .28(2H,m),7.34-7.38(2H,m).

### Synthesis Example 8-2: Synthesis of methyl 4-(N-2-picolylamino methyl) phenyl acetate (Compound V-1)

92.9 mg of commercially available 2-picolylamine was dissolved in 2 ml of DMF, and then 56.5 mg of potassium carbonate was added to the solution. To this solution, 100. 8 mg of the compound obtained in Synthesis Example 8-1 was added and the resultant mixture was stirred for 1 hour at room temperature. On completion of the reaction, the solution was concentrated and then the residue was dissolved in chloroform. The resultant was washed with distilled water and was dried with anhydrous sodium sulfate. The solvent was distilled off under reduced pressure and then the residue was purified by means of silica gel column chromatography (5.5 g, chloroform/methanol = 15/1), and 73.2 mg of the above-mentioned compound was obtained as a colorless oily product.
MS(FAB,Pos.):m/z=271[M+1]⁺
¹H-NMR(500MHz,CDCl₃) :δ=1.85(2H,brs),3.62(2H,s),3.69(3H,s),3.83 (2H,s),3.92(2H,s),7.15-7.18(1H,m),7.23-7.27(3H,m),7.30-7.36(3 H,m),7.64(1H,td,J=7.6,1.7Hz),8.56(1H,ddd,J=5.1,1.7,1.0Hz).

### Synthesis Example 8-3: Synthesis of methyl 4-(N-Boc-N-2-picolyl aminomethyl) phenyl acetate (Compound VI-2)

66.5 mg of the compound obtained in Synthesis Example 8-2 was dissolved in 1.3 ml of DMF, and then 41.5 µl of triethylamine and 84.8 µl of di-t-butyldicarbonate were added to the solution and the resultant mixture was stirred for 1 hour at room temperature. On completion of the reaction, the solvent was distilled off and then the residue was purified by means of silica gel column chromatography (2.5 g, chloroform), and 86.2 mg of the above-mentioned compound was obtained as a colorless liquid.
MS(FAB,Pos.):m/z=371[M+1]⁺
¹H-NMR(500MHz,CDCl₃):δ=1.42 and 1.49(9H,2s),3.61(2H,s),3.70(3H, s),4.38,4.47,4.48 and 4.57(4H,4s),7.16-7.39(6H,m),7.65(1H,td, J=7.6,1.7Hz),8.53(1H,d,J=4.4Hz).

### Synthesis Example 8-4: Synthesis of 4-(N-Boc-N-2-picolylamino methyl) phenyl acetic acid (Compound VII-2)

83.2 mg of the compound obtained in Synthesis Example 8-3 was dissolved in 0.8 ml of THF and 0.8 ml of methanol. Then, 0.8 ml of a 1N sodium hydroxide aqueous solution was added to the solution and the resultant mixture was stirred for 35 minutes at room temperature. On completion of the reaction, the solvent was distilled off and the residue was dissolved in distilled water. A 1 mol/l hydrochloric acid was added to the solution to adjust the pH to 3 to 4. A precipitated oily product was extracted using chloroform and washed with a saturated salt solution, followed by drying with anhydrous sodium sulfate. The solvent was distilled off and then vacuum drying was performed, and 78.7 mg of the above-mentioned compound was obtained as a colorless oily product.
MS(FAB,Pos.):m/z=357[M+1]⁺
¹H-NMR(500MHz,CDCl₃):δ=1.42 and 1.49(9H,2s),3.63(2H,s),4.45, 4.53 and 4.55 (4H,4s), 7.14-7.28 (6H,m), 7.71 (1H,td,J=7.6,1.7Hz), 8.58 (1H,d,J=4.4Hz).

### Synthesis Example 8-5: Synthesis of N^{α}-Fmoc-N^{δ}-Cbz-L-ornithine 1-naphthalenemethylamide (Compound IX-1)

501.8 mg of commercially available N^{α}-Fmoc-N^{δ}-Cbz-L-ornithine was dissolved in 1 0 ml of DMF, and then 177.8 mg of HOBt, 311.4 g of WSCI hydrochloride and 0.165 ml of 1-naphthalenemethylamine were added to the solution and the resultant mixture was stirred for 12 hours at room temperature. On completion of the reaction, the solvent was distilled off. Then, the residue was dissolved in chloroform, and the extraction was performed by the addition of a 1 mol/l hydrochloric acid, followed by washing an organic layer with a 1 mol/l sodium hydroxide. The solventwas distilled off, and 620 mg of the above-mentioned compound was obtained as a white solid product.
¹H-NMR(500MHz,CDCl₃) : δ=1.35-1.57 (2H,m), 1.39 (9H,s), 1.68-1.95 (2H ,m), 2.96-3.07 (2H,m), 4.17 (2H,d,J=6.1Hz), 4.48 (1H,td,J=8.3,5.1Hz), 4.75 (2H,d,J=5.6Hz), 4.98(2H,s), 7.23-7.39 (8H,m), 7.40-7.51 (3H, m), 7.51-7.59 (2H,m), 7.80-7.92 (3H,m), 7.92-7.98 (1H,m), 8.01-8.06 (1H,m),8.43(1H,d,J=7.8Hz),8.51(1H,t,J=5.6Hz).

### Synthesis Example 8-6: Synthesis of N^{δ}-Cbz-L-ornithine 1-naphthalenemethylamide (Compound X-1)

122.8 mg of the compound obtained in Synthesis Example 8-5 was dissolved in 2.6 ml of DMF, and then 0.26 ml of diethylamine was added to the solution and the resultant mixture was stirred for 2 hours at room temperature. On completion of the reaction, thesolvent wasdistilledoffunderreduced pressure. Subsequently, vacuum drying was performed, and 133.5 mg of the above-mentioned compound was obtained.
MS(FAB,Pos.):m/z=406[M+1]⁺

### Synthesis Example 8-7: Synthesis of N^{α}-4-(N-Boc-N-2-picolyl aminomethyl) phenylacetyl-N ^{δ}- Cbz-L-ornithine 1-naphthalene methylamide (Compound XI-2)

133.5 mg of the compound obtained in Synthesis Example 8-6 was dissolved in 1. 36ml of DMF, and then 54.6mg of WSCI hydrochloride, 28.2 mg of HOBt, and 68.0 mg of the compound obtained by Synthesis Example 8-4 were added to the solution and the resultant mixture was stirred for 17 hours at room temperature. On completion of the reaction, the solvent was distilled off. Then, the residue was dissolved in chloroform and was then washed with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purifiedbymeans of silica gel column chromatography (3.5 g, chloroform/methanol = 25/1), and 112.3 mg of the above-mentioned compound was obtained as a white solid product.
MS(FAB,Pos.):m/z=744[M+1]⁺
¹H-NMR (500MHz,DMSO-d₆):δ=1.2-1.5(2H,m),1.30 and 1.40(9H,2s), 1.50-1.57(1H,m),1.63-1.70(1H,m),2.94-2.98(2H,m),3.47(2H,s),4. 28-4.32(1H,m),4.35,4.38,4.42and4.46(4H,4s),4.71(1H,dd,J=15.2, 5.9Hz),4.76(1H,dd,J=15.2,5.9Hz),4.99(2H,s),7.13-7.21(5H,m),7. 25-7.39(7H,m),7.40-7.44(2H,m),7.50-7.55(2H,m),7.77(1H,td,J=7. 8,2.0Hz),7.82-7.86(1H,m),7.91-7.96(1H,m),8.01-8.04(1H,m),8.30 - 8.33(1H,m),8.49-8.55(2H,m).

### Synthesis Example 8-8: Synthesis of (2S)-2-(4-(N-Boc-N-2-picolyl aminomethyl) phenylacetyl) amino-5-(5,6,7,8-tetrahydroquinolin-8-yl) amino valerate 1-naphthalenemethylamide (Compound XIII-8)

34.9 mg of the compound obtained in Synthesis Example 8-7 was dissolved in 1.75 ml of a dioxane/water (=8/2) solution, and 37.9 mg of 10% palladium-carbon was then added to the solution and the resultant mixture was stirred for 2.5 hours at room temperature in a hydrogen atmosphere. On completion of the reaction, the catalyst was removed by means of celite filtration and then the solvent was distilled off under reduced pressure, followed by dissolving the resultant in 0.6 ml of methanol. Subsequently, 13.6 mg of 5,6,7,8-tetrahydroquinolin-8-one synthesized by the method described in Journal of Medicinal Chemistry, vol. 20, No. 10, pp 1351-1354 (1977) and 6.6 mg of sodium cyanoborohydride were added to the solution and then the pH thereof was adjusted to 4 to 5 with acetic acid. The resultant solution was stirred for 2 days at room temperature. On completion of the reaction, the solvent was distilled off and the residue was then purified by means of silica gel column chromatography (3g, chloroform/methanol = 10/1), and 21.4 mg of the above-mentioned compound was obtained as a white frothy product.
MS(FAB,Pos.) :m/z=741[M+1]⁺
¹H-NMR(500MHz,DMSO-d₆) :δ=1.30 and 1.40 (9H,2s),1.48-1.80 (6H,m), 1.90-2.00(1H,m),2.05-2.15(1H,m),2.70-3.00(4H,m),3.48(2H,s),4. 0-4.2 (1H,br), 4.30-4.52 (5H,m), 4.69-4.80 (2H,m), 7.14-7.24 (5H,m), 7.27-7.30 (2H,m), 7.41-7.45 (2H,m), 7.51-7.55 (2H,m), 7.58-7.63 (2H, m), 7.77 (1H,td,J=7.6,1.7Hz), 7.83-7.86 (1H,m), 7.92-7.96 (1H,m), 8. 02-8.05 (1H,m), 8.38 (1H,d,J=6.1Hz), 8.44 (1H,brs), 8.51 (1H,d,J=4.9 Hz), 8.57 (1H,t,J=5.6Hz).

### Synthesis Example 8-9: Synthesis of (2S)-2-((4-(N-2-picolyl aminomethyl) phenylacetyl) amino)-5-(5,6,7,8-tetrahydroquinolin -8-yl) amino valerate 1-naphthalenemethylamide [Compound No. 8]

18.0 mg of the compound obtained in Synthesis Example 8-8 was dissolved in 0.2 ml of methanol, and then 4 mol/l hydrochloric acid/dioxane was added to the solution and the resultant mixture was stirred for 1.5 hours at room temperature. On completion of the reaction, the solvent was distilled off and the residue was then purified by means of silica gel column chromatography (1.0 g, chloroform/methanol/water = 7/3/0.5). The residue was concentrated by the addition of a hydrochloric acid, and then the concentrated residue was azeotropically distilled with water and the solid product was then washed with ether, and 14.4 mg of hydrochloride of the above-mentioned compound was obtained as a white solid product.
MS(FAB,Pos.):m/z=641[M+1]⁺
¹H-NMR(500MHz,DMSO-d₆):δ=1.55-1.82(5H,m),1.82-1.93 (1H,m),1.97-2.02(1H,m),2.24-2.32(1H,m),2.78-2.83(2H,m),2.85-2.97(1H,m),3. 01-3.13(1H,m),3.55(H,d,J=14.7Hz),3.58(1H,d,J=14.7Hz),4.20(2H, s),4.29(2H,s),4.29-4.40(2H,m),4.68-4.78(2H,m),7.32(2H,d,J=8.1 Hz),7.37-7.40(4H,m),7.52-7.57(3H,m),7.68(1H,d,J=7.8Hz),7.84(1 H,d,J=7.6Hz),7.90(1H,td,J=7.6,1.7Hz),7.92(1H,d,J=8.1Hz),8.03-8.07(1H,m),8.47-8.53(2H,m),8.64-8.69(2H,m),9.12(2H,brs),9.72 (2H,brs).

### Synthesis Example 9: Production of (2S)-2-(4-(2-(N-2-picolyl amino) ethyl) benzoyl) amino-5-(5,6,7,8-tetrahydroquinolin-8-yl) amino valerate 1-naphthalenemethylamide [Compound No. 9]

### Synthesis Example 9-1: Synthesis of methyl 4-bromoethyl benzoate (Compound IV-2)

997.9 mg of commercially available 4-bromoethyl benzoic acid was dissolved in 30 ml of methanol. To this solution, 1.2527 g of WSCI hydrochloride and 598.5 mg of HOBt were added and the resultant mixture was stirred for 24 hours at 60°C. On completion of the reaction, the solvent was distilled off and was then the residue was dissolved in chloroform, followed by washing with a 1 mol/l hydrochloric acid, a 1 mol/l sodium hydroxide aqueous solution, and a saturated salt solution and drying with anhydrous sodium sulfate. The solvent was distilled off and the residue was then purified by means of silica gel column chromatography, and 829.5 mg of the above-mentioned compound was obtained as a colorless solid.
MS(FAB,Pos.):m/z=243,245[M+1]⁺
¹H-NMR(60MHz,CDCl₃) : δ=3.0-3.5 (2H,m), 3.5-3.9 (2H,m), 3.91 (3H,s), 7 .1-7.4 (2H,m), 7.8-8.1 (2H,m).

### Synthesis Example 9-2: Synthesis of methyl 4-(2-(2-picolylamino) ethyl) benzoate (Compound V-2)

0.625 ml of commercially available picolyiamine was dissolved in 15 ml of toluene and then 0.715 ml of diisopropyl ethylamine was added to the solution. To this solution, 499.7 mg of the compound obtained in Synthesis Example 9-1 was added and the resultant mixture was stirred for 2 days at 80°C. On completion of the reaction, toluene was added to the solution and the resultant solution was then washed with distilled water and a saturated salt solution, followed by drying with anhydrous sodium sulfate. The solvent was distilled off under reduced pressure, and then the residue was purified by means of silica gel column chromatography (25 g, chloroform/methanol = 25/1), and 400.1 mg of the above-mentioned compound was obtained as a light-brown oily product.
MS(FAB,Pos.):m/z=271[M+1]⁺
¹H-NMR(500MHz,CDCl₃):δ=2.88-2.97(4H,m),3.91(3H,s),3.93(2H,s),7 .16(1H,ddd,J=7.6,4.9,1.2Hz),7.24-7.30(3H,m),7.63(1H,td,J=7.6, 1.7Hz),8.54(1H,ddd,J=4.9,1.7,1.0Hz).

### Synthesis Example 9-3: Synthesis of methyl 4-(2-(N-Boc-N-2-picolylamino) ethyl) benzoate (Compound VI-3)

59.5 mg of the compound obtained in Synthesis Example 9-2 was dissolved in 1.2 ml of DMF, and then 30.9 µl of triethylamine and 50.5 µl of di-t-butyldicarbonate were added and the resultant mixture was stirred for 100 minutes at room temperature. On completion of the reaction, the solvent was distilled off and then the residue was purifiedbymeans of silica gel column chromatography (3g, chloroform/ethyl acetate=1/1), and 60.6 mg of the above-mentioned compound was obtained as a colorless oily product.
MS(FAB,Pos.):m/z=371[M+1]⁺
¹H-NMR (500MHz,CDCl₃): δ=1.40 and 1.46(9H,2s), 2.85 and 2.93 (2H,2t, J=7.4Hz),3.48 and 3.56(2H,2t,J=7.4Hz),3.90(3H,s),4.43 and 4.54 (2H,2s),7.16-7.24(2H,m),7.26(2H,brs),7.64(1H,td,J=7.8,1.7Hz), 7.94(2H,d,8.5Hz),8.53(1H,ddd,J=4.9,1.7,1.0Hz).

### Synthesis Example 9-4: Synthesis of 4-(2-(N-Boc-2-picolylamino) ethyl) benzoic acid (Compound VII-3)

446.1 mg of the compound obtained in Synthesis Example 9-3 was dissolved in 4.5 ml of THF and 4.5 ml of methanol. Then, 4.5 ml of a 1mol/l sodium hydroxide was added to the solution and the resultant mixture was stirred for 5 hours at room temperature. On completion of the reaction, the solvent was distilled off and the resultant was dissolved in distilled water. A 1 mol/l hydrochloric acid was added to the solution to adjust the pH thereof to 3 to 4. A precipitated oily product was extracted using chloroform and washed with saturated salt solution, followed by drying with anhydrous sodium sulfate. The solvent was distilled off and then vacuum drying was performed, and 432.2 mg of the above-mentioned compound was obtained as a white solid product.
MS(FAB,Pos.):m/z=357[M+1]⁺
¹H-NMR(500MHz,CDCl₃) : δ=1.26 and 1.32 (9H,2s), 2.82-2.90 (2H,m), 3.43-3.53 (2H,m), 4.39 and 4.44 (2H,2s), 7.20 (1H,d,J=7.9Hz), 7.24-7.35 (3H,m), 7.76 (2H,td,J=7.6,1.8Hz), 7.86 (2H,d,J=8.1Hz), 8.51 (1H ,brs), 12.85 (1H,brs).

### Synthesis Example 9-5: Synthesis of N^{α}-4-(2-(N-Boc-N-2-picolyl amino) ethyl) benzoyl-N^{δ}-Cbz-L-ornithine 1-naphthalenemethyl amide (Compound XI-3)

200 mg of the compound obtained in Synthesis Example 8-6 was dissolved in 4 ml of DMF, and then 92.4 mg of WSCI hydrochloride, 48. 3 mg of HOBt, and 114.9 mg of the compound prepared in Synthesis Example 9-4 were added to the solution and the resultant mixture was stirred for 17 hours at room temperature. On completion of the reaction, the solvent was distilled off. Then, the residue was dissolved in chloroform and was then washed with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purifiedbymeans of silica gel column chromatography (15 g, chloroform/methanol = 30/1), and 187.6 mg of the above-mentioned compound was obtained as a white solid product.
MS(FAB,Pos.):m/z=744[M+1]⁺
¹H-NMR(500MHz,DMSO-d₆):δ=1.26 and 1.36(9H,2s),1.39-1.60(2H,m), 1.68-1.85(2H,m),2.80-2.90(2H,m),2.92-3.06(2H,m),3.38-3.53(2H, m),4.38 and 4.42(2H,2s),4.47-4.52(1H,m),4.75(2H,d,J-5.9Hz), 4.98(2H,s),7.19(1H,d,J=8.1Hz),7.23-7.38(9H,m),7.43-7.49(1H,m) ,7.50-7.56(1H,m),7.77(1H,t,J=7.8Hz),7.84(2H,d,J=8.1Hz),7.92-7 .95 (1H,m), 8.03-8.06 (1H,m), 8.41 (1H,br), 8.51 (2H,brs).

### Synthesis Example 9-6: Synthesis of N^{α}-4-(2-(N-Boc-N-2-picolyl amino) ethyl) benzoyl-N^{δ}-L-ornithine 1-naphthalenemethylamide (Compound XII-2)

76.0 mg of the compound obtained in Synthesis Example 9-5 was dissolved in 5 ml of a dioxane/water (=8/2) solution, and 78.9 mg of 10% palladium carbon was then added to the solution and the resultant mixture was stirred for 4.5 hours at room temperature in a hydrogen atmosphere. On completion of the reaction, the catalyst was removed by means of celite filtration and then the solvent was distilled off under reduced pressure, and 63.7 mg of the above-mentioned compound was obtained as a colorless oily product.

### Synthesis Example 9-7: Synthesis of (2S)-2-(4-(2-(N- 2-picolyl amino) ethyl) benzoylamino)-5(5,6,7,8-tetrahydroquinolin-8-yl) amino valerate 1-naphthalenemethylamide [Compound No. 9]

28.9 mg of the compound obtained in Synthesis Example 9-6 was dissolved in 0.6 ml of methanol, and then 14.0 mg of 5,6,7,8-tetrahydroquinolin-8-one and 7.1 mg of sodium cyanoborohydride were added to the solution and then the pH thereof was adjusted to approximately 4 with acetic acid. The resultant solution was stirred for 2 hours at room temperature. On completion of the reaction, the solvent was distilled off and the residue was then purified by means of silica gel column chromatography (3g, chloroform/methanol = 10/1), and 21.2 mg of a white frothy product was obtained.

8.4 mg of the compound was dissolved in 0.2 ml of methanol, and then 0.2 ml of 4 mol/l hydrochloric acid/dioxane was added to the solution and the resultant mixture was stirred at 75 minutes at room temperature. On completion of the reaction, the solvent was distilled off and the residue was purified by means of silica gel column chromatography (0.5 g, chloroform/methanol/water = 7/3/0.5). The purified residue was concentrated by the addition of a hydrochloric acid, and then the resultant was azeotropically distilled with water and the solid product was then washed with ether, and 5.6 mg of hydrochloride of the above-mentioned compound was obtained as a white solid product.
MS(FAB,Pos.) :m/z=641[M+1]⁺
¹H-NMR(500MHz,DMSO-d₆) :δ=1.62-1.94 (6H,m) 1.95-2.01(1H,m),2.25-2.35(1H,m),2.78-2.82(2H,m),2.95-3.02(1H,m),3.05-3.18(3H,m),3. 44(2H,t,J=7.0Hz),4.38(2H,t,J=5.4Hz),4.42(1H,m),4.51-4.61(1H,m ),4.76(2H,d,J=5.6Hz),7.35-7.38(2H,m),7.44-7.47(2H,m),7.48-7.6 0(3H,m),7.66(1H,d,J=7.6Hz),7.80-7.84(1H,m),7.85-7.99(4H,m),8. 02-8.05(1H,m),8.41-8.44(1H,m),8.59(1H,d,J=8.1Hz),8.62-8.73(2H ,m),9.10(2H,br),9.53(2H,br).

### Synthesis Example 10: Production of (S)-2-(4-(2-(N- 2-picolyl amino) ethyl) benzoylamino)-5-(N-2-picolylamino) valerate 1-naphthalenemethylamide [Compound No. 10]

### Synthesis Example 10-1: Synthesis of (S)-2-(4-(N-Boc-2-picolyl aminoethyl) benzoyl) amino-5-(2-picolylamino) valerate 1-naphthalenemethylamide (Compound XIII-9)

25.0 mg of the compound obtained in Synthesis Example 9-6 was dissolved in 0.6 ml of methanol and then 4.6 µl of 2-pyridine aldehyde was added and the resultant mixture was stirred for 2 days at room temperature. On completion of the reaction, the solvent was distilled off and then vacuum drying was performed, followed by the addition of 0.6 ml of anhydrous methanol. The resultant was cooled to 0°C and then 9.2 mg of sodium cyanoborohydride was added thereto, followed by stirring for 1.5 hours at room temperature. On completion of the reaction, the solvent was distilled off and then the residue was purified by means of silica gel column chromatography (3 g, chloroform/methanol = 10/1), and 15.4 mg of a white frothy product was obtained.
¹H-NMR(500MHz,DMSO-d₆) :δ=1.26 and 1.36 (9H,2s) 1.40-1.60(2H,m), 1.71-1.84(2H,m),2.40-2.60(2H,m),2.89-2.91(2H,m),3.18-3.51(2H, m),3.73(2H,s),4.39 and 4.42(2H,2s),4.48(1H,dd,J=14.2,8.5Hz), 4.73(1H,dd,J=15.4,5.4Hz),4.76(1H,dd,J=15.4,5.4Hz),7.18-7.32(5 H,m),7.37(1H,d,J=7.8Hz),7.43-7.47(2H,m),7.50-7.55(2H,m),7.70 (1H,td,7.5,1.7Hz),7.76(1H,t,J=7.5Hz),7.83(2H,d,J=8.1Hz),7.82-7 .85(1H,m),7.92-7.95(1H.m),8.04-8.07(1H,m),8.45(1H,ddd,J=4.9,1 .7,1.0Hz),8.51(3H,brs).

### Synthesis Example 10-2: Synthesis of (S)-2-(4-(2-(N-2-picolyl amino) ethyl) benzoylamino)-5-(N-2-picolylamino) valerate 1-naphthalenemethylamide [Compound No. 10]

13.9 mg of the compound obtained in Synthesis Example 10-1 was dissolved in 0.28 ml of methanol, and then 0.28ml of a 4 mol/l hydrochloric acid/dioxane was added to the solution and the resultant mixture was stirred for 75 minutes 6 hours at room temperature. On completion of the reaction, the solvent was distilled off and the residue was then purified by means of silica gel column chromatography (0.5 g, chloroform/methanol/water = 7/3/0.5). The residue was concentrated by the addition of a hydrochloric acid, and then the resultant solution was azeotropically distilled with water and the solid product was then washed with ether, and 13.8 mg of hydrochloride of the above-mentioned compound was obtained as a white solid product.
MS(FAB,Pos.):m/z=601[M+1]⁺
¹H-NMR(500MHz,DMSO-d₆) :δ=1.68-1.92(4H,m),2.96-3.03(2H,m),3.07-3.15(2H,m),3.20-3.28(2H,m),4.29(2H,t,J=5.6Hz),4.38(2H,t,J=5.6 Hz),4.52-4.58(1H,m),4.76(2H,J=5.6Hz),7.37(1H,d,J=8.3Hz),7.43-7.49(4H,m),7.52-7.57(3H,m),7.59(1H,d,J=8.1Hz),7.83-7.86(1H,m) ,7.88-7.96(5H,m),8.05-8.08(1H,m),8.59-8.63(1H,m),8.65-8.70(1H ,m),9.33(2H,brs),9.59(2H,brs).

### Synthesis Example 11: Production of (S)-2- (5-(N-2-picolylamino methyl) furan-2-ylcarbonyl) amino-5-(5,6,7,8-tetrahydroquinolin-8-yl) amino valerate 1-naphthalenemethylamide [Compound No. 11]

### Synthesis Example 11-1: Synthesis of ethyl 5-(N-2-picolylamino methyl furan)-2-carboxylate (Compound V-3)

100.6 mg of commercially available 5-chloromethyl-2-furan ethyl carboxylate was dissolved in 2.0 ml of DMF, and then 75.1 mg of potassium carbonate and 0.167 ml of 2-picolylamine were added and the resultant mixture was stirred for 3 hours at room temperature. On completion of the reaction, the solvent was concentrated and dissolved in chloroform, followed by washing with distilled water. The resultant was dried with anhydrous sodium sulfate and a solvent was then distilled off. The residue was purified by means of silica gel column chromatography (4.5 g, chloroform/methanol = 25/1), and 103.2 mg of the above-mentioned compound was obtained as a light-yellow oily product.
MS(FAB,Pos.):m/z=261[M+1]⁺
¹H-NMR(500MHz,CDCl₃):δ=1.37(3H,t,J=7.1Hz) 3.91(2H,s) 3.94(2H,s ),4.35(2H,q,J=7.1Hz),6.36(d,J=3.4Hz),7.17(1H,ddt,J=7.5,4.9,1. 0Hz),7.31(1H,dt,J=7.5,1.0Hz),7.65(1H,td,J=7.5,1.7Hz),8.47(1H, ddd,J=4.9,1.7,1.0Hz).

### Synthesis Example 11-2: Synthesis of ethyl 5-(N-Boc-N-2-picolyl aminomethylfuran)-2-carboxylate (Compound VI-4)

96.1 mg of the compound obtained in Synthesis Example 11-1 was dissolved in 2.0 ml of DMF, and then 62.3 µl of triethylamine and 84.8 µl of di-t-butyldicarbonate were added to the solution and the resultant mixture was stirred for 4 hours at room temperature. On completion of the reaction, the solvent was distilled off and then the residue was purified by means of silica gel column chromatography (chloroform / ethyl acetate = 1/1), and 131.0 mg of the above-mentioned compound was obtained as a light-yellow oily product.
MS(FAB,Pos.):m/z=361[M+1]⁺
¹H-NMR(500MHz,CDCl₃) : δ=1.36 (3H,t,J=7.1Hz), 1.41 and 1.51 (9H,2s), 4.34 (2H,q,J=7.1Hz), 4.49,4.58,4.61 and 4.64(4H,4s),6.21 and 6.35 (1H,2brs), 7.07 (1H,brs), 7.17 (1H,dd,J=7.5,4.9Hz), 7.23-7.27 (1H,m ), 7.64 (1H,td,J=7.5,1.7Hz), 8.53 (1H,dd,J=4.9,1.7Hz).

### Synthesis Example 11-3: Synthesis of 5-(N-Boc-N-2-picolylamino methylfuran)-2-carboxylic acid (Compound VII-4)

122.8 mg of the compound obtained in Synthesis Example 11-2 was dissolved in 1.2 ml of methanol and 1.2 ml of THF, and then 1.2 ml of a 1 mol/l sodium hydroxide aqueous solution was added to the solution and the resultant mixture was stirred for 5 hours at room temperature. On completion of the reaction, the solvent was distilled off and the resultant was dissolved in 1.2 ml of distilled water, followed by adjusting the pH of the resultant solution to 4 with a 1 mol/l hydrochloric acid. The resultant solution was extracted with chloroform and was dried with anhydrous sodium sulfate. The solvent was distilled off, and 113.3 mg of the target compound as a light-yellow oily product.
MS(FAB,Pos.):m/z=333[M+1]⁺
¹H-NMR (500MHz,CDCl₃) : δ=1.27 and 1.42(9H,2s),4.44,4.47,4.50 and 4.57(4H,4s),6.37 and 6.45 (1H,2brs), 7.09 (1H,brs), 7.18 (1H,m), 7.27 (1H,d,J=7.1Hz), 7.75 (1H,t,J=7.1Hz), 8.50 (1H,d,J=4.4Hz).

### Synthesis Example 11-4: Synthesis of N^{α}-(5-(N-Boc-N-2-picolylaminomethyl) furan-2-ylcarbonyl)-N^{d}-Cbz-L-ornithine 1-naphthalenemethylamide (Compound XI-4)

152.6 mg of the compound obtained in Synthesis Example 8-6 was dissolved in 3 ml of DMF, and then 73.3 mg of WSCI hydrochloride, 30.8 mg of HOBt, and 85.5 mg of the compound obtained in Synthesis Example 11-3 were added to the solution and the resultant mixture was stirred for 19 hours at room temperature. On completion of the reaction, the solvent was distilled off. Then, the residue was dissolved in chloroform and was then washed with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purified by means of silica gel column chromatography (7 g, chloroform/methanol = 25/1), and 137.3 mg of the above-mentioned compound was obtained as a white frothy product.
MS(FAB,Pos.):m/z=720[M+1]⁺

### Synthesis Example 11-5: Synthesis of (2S)-2-(5-(N-Boc-N-2-picolyl aminomethyl)furan-2-ylcarbonyl) amino-5-(5,6,7,8-tetrahydro quinoline-8-yl) amino valerate 1-naphthalenemethylamide (Compound XIII-10)

135.9 mg of the compound obtained in Synthesis Example 11-4 was dissolved in 6.5 ml of a dioxane/water (=8/2) solution, and then 138.5 mg of 10% palladium-carbon was added to the solution and the resultant mixture was stirred for 4.5 hours at room temperature in a hydrogen atmosphere. On completion of the reaction, the catalyst was removed by means of celite filtration, and then the solvent was distilled off under reduced pressure, and 107.6 mg of a crude product was obtained. Then, 75.1 mg of the crude product was dissolved in 2.25 ml of methanol, and 27.2 mg of 5,6,7,8-tetrahydroquinolin-8-one, and 10.4 mg (0.165 mml) of sodium cyanoborohydride were added to the solution, followed by dropping 40 drops of acetic acid in the solution to adjust the pH to approximately 4. The resultant solution was stirred for 14 hours at room temperature. On completion of the reaction, the solvent was distilled off and then the residue was purified by means of silica gel column chromatography (5 g, chloroform/methanol =10/1), and 38.7 mg of a white frothy product was obtained.
MS(FAB,Pos.):m/z=717[M+1]⁺
¹H-NMR(500MHz,DMSO-d₆) : δ=1.28 and 1.42 (9H,2s), 1.49-1.92 (6H,m), 2.00-2.20 (1H,m), 2.65-2.95 (5H,m), 4.40-4.60 (6H,m), 4.76 (2H,d,J=5 .6Hz),6.38,6.42 and 6.44 (1H,brs), 7.12 and 7.14 (1H,brs), 7.18-7.22 (3H,m), 7.40-7.48 (2H,m), 7.49-7.62 (3H,m), 7.77 (1H,td,J=7.8,1 .0Hz), 7.80-7.85 (1H,m), 7.87-7.91 (1H,m), 8.02-8.08 (1H,m), 8.30-8. 49(1H,m),8.50(1H,d,J=4.9Hz),8.60-8.75(1H,br).

### Synthesis Example 11-6: Synthesis of (2S)-2-(5-(N-2-picolyl aminomethyl) furan-2-ylcarobnyl) amino-5-(5,6,7,8-tetrahydro quinolin-8-yl) amino valerate 1-naphthalenemethylamide [Compound No. 11]

30.6 mg of the compound obtained in Synthesis Example 11-5 was dissolved in 0.7 ml of methanol, and then 0.7 ml of a 4 mol/l hydrochloric acid/dioxane was added to the solution and the resultant mixture was stirred for 3 hours at room temperature. On completion of the reaction, the solvent was distilled off and the residue was then purified by means of silica gel column chromatography (1 g, chloroform/methanol/water = 7/3/0.5). The residue was concentrated by the addition of a hydrochloric acid aqueous solution, and then the resultant solution was azeotropically co-distilled with water and the solid product was then washed with ether, and 22.7 mg of hydrochloride of the above-mentioned compound was obtained as a white solid product.
MS(FAB,Pos.):m/z=617[M+1]⁺
¹H-NMR(500MHz,DMSO-d₆):δ=1.68-2.02(7H,m),2.23-2.35(1H,m),2.77-3.05(2H,m),2.88-3.01(1H,m),3.02-3.18(1H,m),4.36(2H,s),4.39(2H ,s),4.37-4.43(1H,m),4.51-4.59(1H,m),4.76(1H,d,J=5.6Hz),6.79(1 H,d,J=3.4Hz),7.29(1H,d,J=3.4Hz),7.38(1H,dd,J=7.5,4.9Hz),7.43-7.49(3H,m),7.51-7.57(2H,m),7.59(1H,brs),7.67(1H,d,J=7.6Hz),7. 83-7.87(1H,m),7.88-7.92(1H,m),7.93-7.96(1H,m),8.05-8.08(1H,m) ,8.46(2H,brs),8.64(1H,d,J=4.9Hz),8.51(1H,brs),9.19(2H,brs),10 .09 (2H,brs).

### Synthesis Example 12: Production of (2S)-2-(2-(N-2-picolyl aminomethyl) pyridin-5-ylcarobnyl) amino-5-((5,6,7,8-tetra hydroquinolin-8-yl) amino) valerate 1-naphthalenemethylamide [Compound No. 12]

### Synthesis Example 12-1: Synthesis of methyl 6-(N-2-picolylamino methyl) nicotinic acid (Compound V-4)

2.5116 g of commercially available methyl 6-methyl nicotinic acid was dissolved in 50 ml of chloroform, and then 3.5682 g of N-bromosuccinimide and 290.4 mg of azoisobutyronitril were added to the solution and the resultant mixture was stirred for 22 hours at 70°C. On completion of the reaction, the solvent was distilled off and the residue was purified by means of silica gel column chromatography (100 g, hexane/ethyl acetate = 4/1), and 1.0883 g of a crude product was obtained as a light-yellow solid product. 280.7 mg of the crude product was dissolved in 5.6 ml of DMF, and then 168. 5 mg of potassium carbonate and 0.370 ml of 2-picolylamine were added to the solution and the resultant mixture was stirred for 12 hours at room temperature. On completion of the reaction, the solvent was concentrated and dissolved in chloroform and washed with distilled water. The resultant solution was dried with anhydrous sodium sulfate, and then the solvent was distilled off. The residue was purifiedbymeans of silica gel column chromatography (25 g, chloroform/methanol = 25/1), and 298.3 mg of the above-mentioned compound was obtained as a light-yellow oily product.
MS(FAB,Pos.):m/z=258[M+1]⁺
¹H-NMR(500MHz,CDCl₃):δ=3.97(3H,s),3.99(2H,s),4.03(2H,s),7.18(1 H,dd,J=7.5,4.9Hz),7.34(1H,d,J=7.5Hz),7.47(1H,d,J=7.5Hz),7.65 (1H,t,J=7.5Hz),8.28(1H,d,J=7.5Hz),8.56(1H,d,J=4.9Hz),9.17(1H,s).

### Synthesis Example 12-2: Synthesis of methyl 6-(N-Boc-N-2-picolyl aminomethyl) nicotinic acid (Compound VI-5)

292.7 mg of the compound obtained in Synthesis Example 12-1 was dissolved in 6 ml of DMF, and then 0.192 ml of triethylamine and 0.288 ml of di-t-butyldicarbonate were added to the solution and the resultant mixture was stirred for 1.5 hours at room temperature. On completion of the reaction, the solvent was distilled off and then the residue was purified by means of silica gel column chromatography (chloroform/ethyl acetate = 1/1), and 352.2 mg of the above-mentioned compound was obtained as a light-yellow oily product.
MS(FAB,Pos.):m/z=358[M+1]⁺
¹H-NMR(500MHz,CDCl₃) : δ=1.39 and 1.44 (9H,s), 3.95 (3H,s), 4.61, 4.64, 4.71 and 4.74 (4H,4s), 7.17 (1H,ddd,J=4.9,1.7.1.0Hz), 7.20-7.26 (1H, m), 7.31-7.411H,m), 7.67 (1H,t,J=7.6Hz), 8.25(1H,d,J=8.1Hz), 8.51 (1H,m), 9.11 (1H,s).

### Synthesis Example 12-3: Synthesis of 6-(N-Boc-N-2-picolylamino methyl) nicotinic acid (Compound VII-5)

351.3 mg of the compound obtained in Synthesis Example 12-2 was dissolved in 3.5 ml of methanol and 3.5 ml of THF, and then 3.5 ml of a 1 mol/l sodium hydroxide aqueous solution was added to the solution and the resultant mixture was stirred for 2 hours at room temperature. On completion of the reaction, the solvent was distilled off and the resultant was dissolved in 1.2 ml of distilled water, followed by adjusting the pH of the resultant solution to 4 with a 1 mol/l hydrochloric acid. The resultant solution was extracted with chloroform and dried with anhydrous sodium sulfate. The solvent was distilled off, and 277.9 mg of the target compound was obtained as a white solid product.
MS(FAB,Pos.):m/z=344[M+1]⁺
¹H-NMR(500MHz,CDCl₃):δ=1.46 and 1.55(9H,s),4.54,4.63,4.77 and 4.84 (4H,4s), 7.20-7.30 (1H,m), 7.30-7.40 (1H,m), 7.42-7.63 (1H,m), 7 .87 (1H,td,J=7.6,1.7Hz), 8.16-19 (1H,m), 8.51 (1H,dd,J=4.1,1.7Hz), 8.86 (1H,s).

### Synthesis Example 12-4: Synthesis of N^{α}-(2-(N-Boc-N-2-picolyl aminomethyl) pyridin-5-ylcarbonyl)-N^{δ}-Cbz-L-ornithine 1-naphthalenemethylamide (Compound XI-5)

165.3 mg of the compound obtained in Synthesis Example 8-6 was dissolved in 3 ml of DMF, and then 78.5 mg of WSCI hydrochloride, 40.1 mg of HOBt, and 90.4 mg of the compound obtained in Synthesis Example 12-3 were added to the solution and the resultant mixture was stirred for 23 hours at room temperature. On completion of the reaction, the solvent was distilled off. Then, the residue was dissolved in chloroform and was then washed with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purifiedbymeans of silica gel column chromatography (10 g, chloroform/methanol = 20/1), and 167.6 mg of the above-mentioned compound was obtained as a white frothy product.
MS(FAB,Pos.):m/z=731[M+1]⁺
¹H-NMR(500MHz,DMSO-d₆):δ=1.41 and 1.43(9H,2s),1.45-1.58(1H,m), 1.60-1.73(1H,m),1.77-1.81(1H,m),1.82-1.94(1H,m),3.03-3.17(1H, m),3.49-3.62(1H,m),4.40-4.49(1H,m),4.55-4.80(7H,m),4.85-5.02 (3H,m),7.10-7.20(4H,m),7.21-7.40(7H,m),7.41-7.55(3H,m),7.66(1H ,t,J=7.6Hz),7.75(1H,d,J=8.1Hz),7.83(1H,d,J=8.1Hz),7.98(1H,d,J =8.5Hz),8.05(1H,dd,J=8.1,2.0Hz),8.52(1H,brs),8.95(1H,brs).

### Synthesis Example 12-5: Synthesis of (2S)-2-(2-(N-Boc-N-2-picolyl aminomethyl) pyridin-5-ylcarbonyl) amino-5-(5,6,7,8-tetrahydro quinolin-8-yl) amino valerate 1-naphthalenemethylamide (Compound XIII-11)

149.9 mg of the compound obtained in Synthesis Example 12-4 was dissolved in 7.5 ml of a dioxane/water (=8/2) solution, and then 154.6 mg of 10% palladium -carbon was added to the solution and the resultant mixture was stirred for 4.5 hours at room temperature in a hydrogen atmosphere. On completion of the reaction, the catalyst was removed by means of celite filtration, and then the solvent was distilled off under reduced pressure, and 100.2 mg of a crude product was obtained. Then, 75.4 mg of the crude product was dissolved in 1.5 ml of methanol, and 53.6 mg of 5,6,7,8-tetrahydroquinolin-8-one, and 43.2 mg of sodium cyanoborohydride were added, followed by dropping 25 drops of acetic acid to adjust the pH of the resultant solution to approximately 5. The resultant solution was stirred for 22 hours at room temperature. On completion of the reaction, the solvent was distilled off and then the residue was purified by means of silica gel column chromatography (5 g, chloroform/methanol = 10/1), and 30.6 mg of a white frothy product was obtained.
MS(FAB,Pos.):m/z=728[M+1]⁺
¹H-NMR(500MHz,DMSO-d₆) : δ=1.31(9H,s), 1.60-2.00(5H,m), 2.15-2.35 (1H,m),2.70-2.85(2H,m),2.86-3.05(2H,m),4.50-4.62(2H,m),4.52,4. 56,4.60 and 4.65(4H,4s),4.77(2H,d,J=5.9Hz),7.20-7.41(4H,m), 7.47(2H,d,J=4.1Hz),7.52-7.55(2H,m),7.64(1H,d,J=6.4Hz),7.79(1H ,td,J=7.8,1.7Hz), 7.84-7.86 (1H,m), 7.93-7.96 (1H,m), 8.03-8.08 (1H ,m), 8.22 (1H,d,J=8.1Hz), 8.42-8.48 (1H,m), 8.51 (1H,dd,J=5.6,1.5Hz ), 8.61 (1H,brs),8.75-8.88 (1H,m), 9.00 (1H,s).

### Synthesis Example 12-6: Synthesis of (2S)-2-(2-(N-2-picolylamino methyl) pyridin-5-ylcarbonyl) amino-5-((5,6,7,8-tetrahydro quinolin-8-yl) amino) valerate 1-naphthalenemethylamide [Compound No. 12]

26.8 mg of the compound obtained in Synthesis Example 12-5 was dissolved in 0.5 ml of methanol, and then 0.5 ml of a 4 mol/l hydrochloric acid/dioxane was added to the solution and the resultant mixture was stirred for 4.5 hours at room temperature. On completion of the reaction, the solvent was distilled off and the residue was then purified by means of silica gel column chromatography (1 g, chloroform/methanol/water = 7/3/0.5). The purified residue was concentrated by the addition of a hydrochloric acid, and then the resultant was azeotropically distilled with water and the solid product was then washed with ether, and 25.8 mg of hydrochloride of the above-mentioned compound was obtained as a white solid product.
MS(FAB,Pos.):m/z=628[M+1]⁺
¹H-NMR(500MHz,DMSO-d₆):δ=1.63-2.05(7H,m),2.25-2.38(1H,m),2.77-2.82(2H,m),2.88-3.01(1H,m),3.01-3.17(1H,m),4.40-4.43(1H,m),4. 44(2H,s),4.49(2H,s),4.50-4.61(1H,m),4.77(2H,d,J=5.9Hz),7.37(1 H,dd,J=7.5,4.9Hz),7.44-7.52(3H,m),7.52-7.59(2H,m),7.60(1H,d,J =7.8Hz),7.66-7.69(1H,m),7.67(1H,d,J=5.9Hz),7.83-7.87(1H,m),7. 88-7.97(2H,m),8.06-8.09(1H,m),8.39(1H,dt,8.1,2.0Hz),8.46(1H,t d,J=4.6,1.5Hz),8.67(1H,ddd,J=4.9,1.7,1.0Hz),8.79(1H,t,J=5.9Hz ), 9.24 (1H,d,J=8.1H), 9.14 (1H,d,J=2.0Hz).

### Synthesis Example 13: Production of (2S)-2-(5-(N-2-picolylamino methyl) pyrazine-2-carbonylamino)-5-(5,6,7,8-tetrahydroquinolin-8-yl amino) valerate 1-naphthalenemethylamide [Compound No. 13]

### Synthesis Example 13-1: Synthesis of 2-methoxycarbonyl-5-methyl pyrazine (Compound III-1)

2.05 g of commercially available 5-methylpyradzine 2-carboxylate was dissolved in 0.6 ml of methanol, and then a hydrochloric acid gas was brown into the solution for 5 minutes. After 4 hours, the reaction solution was concentrated, and then a.1 mol/l sodium hydroxide aqueous solution was added thereto, followedby extractionwith chloroform. An organic layer was washed with a saturated salt solution and was then dried with anhydrous sodium sulfate and concentrated, followed by re-crystallizing the resulting solid product from hexane/ethyl acetate. The precipitated crystal was filtrated and dried under reduced pressure, and 1.44 g of the above-mentioned compound was obtained as a light-brown squamous crystal.
MS(EI,Pos.):m/z=152[M+1]⁺
¹H-NMR(500MHz,DMSO-d₆) : δ=2.68 (3H,s), 4.04 (3H,s), 8.59 (1H,d,J=1.0 Hz), 9.20 (1H,1.0Hz).

### Synthesis Example 13-2: Synthesis of 5-bromomethyl-2-methoxy carbonyl pyrazine (Compound IV-3)

500 mg of the compound obtained in Synthesis Example 13-1 was dissolved in 10 ml of carbon tetrachloride, and then 585 mg of N-bromosuccinimide and 54 mg of azobisisobutyronitrile were added to the solution. After the solution was stirred in oil bath for 20 hours at 70°C, the reaction solution was filtrated, and then the filtrate was then concentrated. The resulting residue was purified by means of silica gel column chromatography (14 g, chloroform/ethyl acetate= 2/1), and 328.7mg of the above-mentioned compound was obtained as light-yellow syrup.
MS(EI,Pos.):m/z=229,231[M+1]⁺
¹H-NMR(500MHz,DMSO-d₆) : δ=4.06 (3H,s), 4.62 (2H,s), 8.83 (1H,d,J=1. 5Hz), 9.26 (1H,d,J=1.5Hz).

### Synthesis Example 13-3: Synthesis of 5-(N-Boc-N-2-picolylamino methyl)pyrazine-2-carboxylic acid (Compound VII-6)

320 mg of the compound obtained in Synthesis Example 13-2 was dissolved in 6.4 ml of DMF, and then 383 mg of potassium carbonate and 286 µl of 2-picolylamine were added to the solution. After the 17-hour reaction, the reaction solution was concentrated and then water was added thereto, followed by extracting with chloroform. An organic layer was washed with a saturated salt solution. Subsequently, the organic layer was dried with anhydrous sodium sulfate and concentrated, and 444.1 mg of a crude product of the above-mentioned compound was obtained as brown syrup. The crude product was dissolved in 4 ml of dioxane, and then 0.35 ml of di-t-butyldicarbonate and 4 ml of a 1 mol/l sodium hydroxide aqueous solution were added to the solution. After 2 hours, the reaction solution was concentrated and diluted hydrochloric acid was added to the reaction solution, followed by extraction with chloroform. An organic layer was washed with a saturated salt solution and then dried with anhydroussodiumsulfate,followed by concentration, and 158.8 g of the above-mentioned compound was obtained as a light-brown solid product.
MS(FAB,Pos.):m/z=345[M+1]⁺

### Synthesis Example 13-4: Synthesis of N^{α}-(S-(N-Boc-N-2-picolyl aminomethyl) pyrazine-2-carbonyl)-N^{δ}-Cbz-L-ornithine 1-naphthalenemethylamide (Compound XI-6)

160 mg of the compound obtained in Synthesis Example 8-6 was dissolved in 3.2 ml of DMF, followed by the addition of 0.32 ml of diethylamine. After 1 hour, the residue obtained by concentrating the reaction solution was dissolved in 1 ml of DMF, and then 73 mg of WSCI hydrochloride, 31 mg of DMAP, and a 1 ml DMF solution of the compound obtained in Synthesis Example 13-3 were added to the solution one after another. After 15 hours, the reaction solution was concentrated and then chloroform and 1 mol/l hydrochloric acid were added to the resulting residue, followed by extraction with chloroform. An organic layer was washed with a saturated salt solution and was then dried with anhydrous sodium sulfate. The resulting residue was roughly purified by means of silica gel column chromatography (4 g, chloroform/ethyl acetate =1/2). Consequently, 95.2 mg of the above-mentioned compound was obtained as colorless syrup.
MS(FAB,Pos.):m/z=732[M+1]⁺

### Synthesis Example 13-5: Synthesis of (2S)-2-(5-(N-Boc-N-2-picolyl aminomethyl) pyrazine-2-carbonylamino)-5-(5,6,7,8-tetrahydro quinolin-8-ylamino) valerate 1-naphthalenemethylamide (Compound XIII-12)

95.2 mg of the compound obtained in Synthesis Example 13-4 was dissolved in 4ml of dioxane and 1 ml of water, followed by the addition of 5% Pd-C. The reaction solution was subjected to hydrogen substitution, after 16 hours, the reaction solution was filtrated through a glass filter G4 and the f iltrate was concentrated. The resulting residue was dissolved in 2.4 ml of methanol, and then 0.24 ml of acetic acid, 57 mg of 5,6,7,8-tetrahydroquinolin -8-one, and 24 mg of sodium cyanoborohydride were added to the solution. After 21 hours, the reaction solution was concentrated, and the resulting residue was purified by means of silica gel column chromatography (10 g, chlorof orm/methanol = 10/1). Consequently, 58.4 mg of the above-mentioned compound was obtained as colorless syrup.
MS(FAB,Pos.):m/z=729[M+1]⁺
¹H-NMR(500MHz,CDCl3):δ=1.41,1.42,1.43 and 1,44(9H,4s),1.70-2.97 (10H,m),3.03-3.11(0.5H,m),3.28-3.34(0.5H,m),3.90-4.00(0.5H,m) ,4.19-4.26(0.5H,m),4.60-5.02(8H,m),7.06-7.54(10H,m),7.67(1H,t ,J=7.1Hz),7.72-7.79(1H,m),7.84(1H,t,J=9.5Hz),8.03(1H,t,J=9.8H z),8.28(1H,d,J=3.2Hz),8.38-8.57(2H,m),9.19(1H,d,J=3.4Hz).

### Synthesis Example 13-6: Synthesis of (2S)-2-(5-(N-2-picolylamino methyl) pyrazine-2-carbonylamino)-5-(5,6,7,8-tetrahydro quinolin-8-ylamino) valerate 1-naphthalenemethylamide (Compound No. 13)

57.2 mg of the compound obtained in Synthesis Example 13-5 was dissolved in 0.6 ml of methanol, followed by the addition of 0.6 ml of 4 mol/l hydrochloric acid/dioxane. After 4 hours, the reaction solution was concentrated. The resulting residue was purified by means of silica gel column chromatography (1 g, chloroform/methanol = 5/1). A 1 mol/l hydrochloric acid was added to the resulting residue, followed by concentration, and drying under reduced pressure. Consequently, 29.3 mg of hydrochloride of the above-mentioned compound was obtained as a light-brown solid product.
MS(FAB,Pos.):m/z=629[M+1]⁺
¹H-NMR(500MHz,DMSO-d₆) : δ=1.68-2.02 (7H,m), 2.25-2.31 (1H,m), 2.79 (2H,t,J=6.3Hz), 2.90-3.00 (1H,m), 3.00-3.14 (1H,m), 4.38-4.42 (1H,m) , 4.47 (2H,s), 4.61 (2H,s), 4.60-4.67 (1H,m), 4.78 (2H,d,J=5.6Hz), 7.3 5-7.39 (1H,m), 7.43-7.58 (8H,m), 7.67 (1H,d,J=7.8Hz), 7.93-7.97 (3H, m), 8.03-8.07 (1H,m), 8.45 (1H,d,J=3.9Hz), 8.63-8.67 (1H,m), 8.81 (1H ,s), 8.89 (1H,d,J=8.5Hz), 8.95 (1H,s), 8.97-9.17 (2H,br), 10.00-10.1 1(2H,br).

### Synthesis Example 14: Production of (2S)-2-(5-(N-2-picolylamino methyl) thiophene-2-carbonylamino)-5-(5,6,7,8-tetrahydro quinolin-8-ylamino) valerate 1-naphthalenemethylamide [Compound No. 14]

### Synthesis Example 14-1: Synthesis of 2-bromomethyl-5-methoxy carbonylthiophene (Compound IV-4)

2.05 g of commercially available 5-methylthiophene carboxylic acid was dissolved in 60 ml of methanol, and then a hydrochloric acid gas was brown into the solution for 5 minutes. After 23 hours, the reaction solution was concentrated, and then a 1 mol/l sodium hydroxide aqueous solution was added thereto, followedbyextractionwithchloroform. An organic layer was washed with a saturated salt solution and was then dried with anhydrous sodium sulfate and concentrated. 500 mg of the resulting residue was dissolved in 10 ml of carbon tetrachloride, followed by the addition of 570 mg of N-bromosuccinimide and 53 mg of azobisisobutyronitrile. After the resultant solution was stirred in oil bath for 20 hours at 70°C, the reaction solution was filtrated, and then the filtrate thereof was then concentrated. The resulting residue was purified by means of silica gel column chromatography (15 g, hexane/ethyl acetate = 8/1). Consequently, 516.1 mg of the above-mentioned compound was obtained as light-yellow syrup.
MS(EI,Pos.) :m/z=233,235[M+1]⁺
¹H-NMR(500MHz,CDCl3):δ=3.89(3H,s),4.68(2H,s),7.10(1H,d,J=3.7Hz ),7.64(1H,d,J=3.7Hz).

### Synthesis Example 14-2: Synthesis of 5-(N-Boc-N-2-picolylamino methyl) thiophene-2-carboxylic acid (Compound VII-7)

513 mg of the compound obtained in Synthesis Example 14-1 was dissolved in 10 ml of DMF, and then 603 mg of potassium carbonate and 0.45 ml of 2-picolylamine were added to the solution. After 17 hours, the reaction solution was concentrated and then water was added thereto, followed by extraction with chloroform. An organic layer was washed with a saturated salt solution and was then dried with anhydrous sodium sulfate. The residue obtained by concentrating the layer was dissolved in 6 ml of dioxane, and then 0.55 ml of di-t-butyldicarbonate and 6 ml of a 1 mol/l sodium hydroxide aqueous solution were added to the solution. After 6 hours, about 2 ml of a 1 mol/l sodium hydroxide aqueous solution was added to the reaction solution. Then, the solution was further stirred for 4 hours. The reaction solution was concentrated and water and 1 mol/l hydrochloric acid were added to the solution to be slightly acidic. Then, the solution was extracted with chloroform. An organic layer was dried with anhydrous sodium hydroxide and concentrated. The resulting residue was dissolved in 12 ml of methanol, to which 12 ml of a 1 mol/l sodium hydroxide aqueous solution was added. After 16 hours, the reaction solution was concentrated and dissolved in water, followed by gradually adding 1 mol/l hydrochloric acid to adjust to pH 4 to 5. After the solution was left to stand for overnight, a precipitated solid was filtrated and dried under reduced pressure. Consequently, 523.3 mg of the above-mentioned material was obtained as a white solid product.
MS(FAB,Pos.):m/z=349[M+1]⁺
¹H-NMR(500MHz,CDCl₃) : δ=1.50 and 1.55 (9H,2s), 4.52, 4.60, 4.64 and 4.69 (4H,4s),6.93 and 6.97 (1H,2d,J=3.7Hz), 7.33 (1H,dd,J=5.6, 7.1Hz), 7.37 and 7.50 (1H,2d,J=7.6Hz), 7.67 (1H,d,J=3.7Hz), 7.80 (1H,t,J=7.3Hz), 8.69 (1H,brs).

### Synthesis Example 14-3: Synthesis of N^{α}-(5-(N-Boc-N-2-picolyl aminomethyl) thiophene-2-carbonyl)-N^{δ}-Cbz-L-ornithine 1-naphthalenemethylamide (Compound XI-7)

283 mg of the compound obtained in Synthesis Example 8-6 was dissolved in 5.6 ml of DMF, followed by the addition of 0.56 ml of diethylamine. After 1 hour, the reaction solution was concentrated. 146 mg of the resulting residue was dissolved in DMF, and then 104 mg of WSCI hydrochloride, 66 mg of DMAP, and 138 mg of the compound obtained in Synthesis Example 14-2 were added to the solution. After 14 hours, chloroform and 1 mol/l hydrochloric acid were added to the reaction solution, followed by extracting with chloroform. An organic layer was washed with a saturated salt solution and concentrated. The resulting residue was purified by means of silica gel column chromatography (10 g, chloroform/ethyl acetate = 2/1). Consequently, 208.6 mg of the above-mentioned compound was obtained as colorless syrup.
MS(FAB,Pos.):m/z=736[M+1]⁺
¹H-NMR(500MHz,CDCl₃) : δ=1.43 and 1.54 (9H,2s),1.40-1.55 (2H,m), 1.69-1.78 (1H,m), 1.82-1.90 (1H,m), 3.05-3.12 (1H,m), 3.47-3.56 (1H, m), 4.44-4.90 (6H,m), 4.99 (1H,dd,J=6.1,14.6Hz), 6.92-7.54 (13H,m), 7.64 (1H,t,J=7.8Hz), 7.75 (1H,d,J=8.3Hz), 7.83 (1H,d,J=7.8Hz), 7.97 (1H,d,J=8.5Hz), 8.54 (1H,d,J=4.2Hz).

### Synthesis Example 14-4: Synthesis of N^{α}-(5-(N-2-picolylamino methyl) thiophene-2-carbonyl)-L-ornithine 1-naphthalenemethyl amide (Compound XIV-1)

Added to 56.2 mg of the compound obtained in Synthesis Example 14-3 was a mixture solution of 1.4 ml of trifluoroacetic acid, 0.36 ml of thioanisole, and 0.32 ml of metacresol. After 1.5 hours, the reaction solution was concentrated and then methanol was added to the solution, followed by washing with hexane. A methanol layer was concentrated. The resulting residue was roughly purified by means of silica gel column chromatography (3 g, chloroform/methanol /water = 7/3/0.5). Consequently, 18.2 mg of roughly purified product of the above-mentioned compound was obtained as colorless syrup.
MS (FAB,Pos.):m/z=502[M+1]⁺

### Synthesis Example 14-5: Synthesis of (2S)-2-(5-(N-2-picolylamino methyl) thiophene-2-carbonylamino)-5-(5,6,7,8 tetrahydro quinoline-8-ylamino) valerate 1-naphthalenemethylamide [Compound No. 14]

18.2 mg of the compound obtained in Synthesis Example 14-4 was dissolved in 0.6 ml of methanol, followed by the addition of 0.06 ml of acetic acid, 16 mg of 5,6,7, 8-tetrahydroquinolin-8-one and 7 mg of sodium cyanoborohydride. After 3 days, the reaction solution was concentrated. The resulting residue was purified by means of silica gel column chromatography (1 g, chloroform/methanol = 5/1). A 1 mol/l hydrochloric acid was added to the resulting colorless syrup, followed by concentration, and drying under reduced pressure. Consequently, 6.7 mg of the above-mentioned compound was obtained as a white solid product.
MS(FAB,Pos.):m/z=633[M+1]⁺
¹H-NMR(500MHz,DMSO-d₆) : δ=1.65-2.07 (7H,m), 2.24-2.33 (1H,m), 2.79 (2H,t,J=6.3Hz), 2.89-2.99 (1H,m), 3.03-3.13 (1H,m), 4.30 (2H,s), 4.38 - 4.55 (4H,m), 4.76 (2H,d,J=5.6Hz), 7.35-7.40 (2H,m), 7.42-7.49 (3H,m), 7.50-7.572H,m), 7.61 (1H,d,J=7.8Hz), 7.67 (1H,d,J=7.8Hz), 7.81-7 .88 (1H,m), 7.89-7.97 (3H,m), 8.02-8.07 (1H,m), 8.46 (1H,dt,J=1.5,4. 6Hz), 8.66 (1H,d,J=4.9Hz), 8.77 (1H,dd,J=3.9,5.9Hz), 8.87 (1H,d,J=8 .3Hz),9.00-9.38(2H,br),9.98-10.09 (2H,br)

### Synthesis Example 15: Production of (2S)-2-(5-(N-(imidazol-2-yl methyl) aminomethyl) thiophene-2-carbonylamino)-5-picolyl amino valerate 1-naphthalenemethylamide [Compound No. 15] and (2S)-2-(5-(N-(imidazol-2-ylmethyl) aminomethyl thiophene-2-carbonylamino)-5-(5,6,7,8-tetrahydroquinolin-8-yl) amino valerate 1-naphthalenemethylamide [Compound No. 16]

### Synthesis Example 15-1: Synthesis of 2-methoxycarbonyl-5-(N-(imidazol-2-ylmethyl) aminomethyl) thiophene (Compound V-5)

2.02 g of the compound obtained in Synthesis Example 14-1 was dissolved in 30 ml of DMF, and then 1.92 gof potassiumphthalimide was added to the solution. After 3.5 hours, the reaction solution was concentrated and then water was added thereto, followed by extraction with chloroform. An organic layer was dried with anhydrous sodium sulfate and concentrated. The resulting residue was re-crystallized frommethanol, and 1.52 gof the above-mentioned compound was obtained as a yellow solid product.

714 mg of the compound was dissolved in 3.6 ml of ethanol, and then 0.57 ml of hydrazine monohydrate was added to the solution. After the 20-hours reaction, the reaction solution was concentrated. The resulting solid product was washed with chloroform. Filtrate was concentrated and then the resulting residue was dissolved in 14 ml of methanol. Subsequently, 232 mg of 2-imidazole carboxyaldehyde and 0.34 ml of triethylamine were added to the solution and the resultant mixture was stirred for 20 hours. Then, 183 mg of sodium borohydride was added to the reaction mixture. After additional 2 hours, a small amount of silica gel was added to the reaction solution and the solution was concentrated. The resulting residue was purified by means of silica gel column chromatography (15 g, chloroform/methanol = 20/1). Consequently, 105 mg of the above-mentioned compound was obtained as yellow syrup.

### Synthesis Example 15-2: Synthesis of 2-methoxycarbonyl-5-(N-(imidazol-2-ylmethyl)-N-Boc aminomethyl) thiophene (Compound VI-6)

102 mg of the compound obtained in Synthesis Example 15-1 was dissolved in 3 ml of DMF, and then 84 µl of triethylamine and 102 µl of di-t-butyl dicarbonate were added to the solution. After 18 hours, the reaction solution was concentrated and then water was added thereto, followed by extraction with chloroform. An organic layer was dried with anhydrous sodium sulfate and concentrated. Theresultingresiduewaspurifiedbymeansof silica gel column chromatography (4 g, hexane/ethyl acetate = 2/1). Consequently, 84 mg of the above-mentioned compound was obtained as yellow syrup.
MS(FAB,Pos.):m/z=352[M+1]⁺
¹H-NMR(500MHz,CDCl₃):δ=1.57(9H,s),3.88(3H,s),4.73(2H,s),4.86(2 H,s),6.87(1H,d,=3.7Hz),6.91(1H,d.J=1.7Hz),7.29(1H,s),7.61(1H, d,J=3.7Hz).

### Synthesis Example 15-3: Synthesis of 5-(N-(imidazol-2-ylmethyl) -N-Boc aminomethyl) thiophene-2-carboxylic acid (Compound VII-8)

84 mg of the compound obtained in Synthesis Example 15-2 was dissolved in 0.8 ml of methanol, and then 0.8 ml of a 1 mol/l sodium hydroxide aqueous solution and 0.8 ml of THF were added to the solution. After 1.5 hours, the reaction solution was concentrated and then water and 1 mol/l hydrochloric acid were added to thereto, followed by distilling the solvent off. Consequently, 121 mg of the above-mentioned compound (sodium chloride mixture) was obtained as a light-yellow solid product.
MS(FAB,Pos.):m/z=338[M+1]⁺

### Synthesis Example 15-4: Synthesis of (2S)-2-(5-(N-(imidazol-2-ylmethyl) aminomethyl) thiophene-2-carbonylamino)-5-picolyl amino valerate 1-naphthalenemethylamide [Compound No. 15] and (2S)-2-(5-(N-(imidazol-2-ylmethyl) aminomethyl) thiophene-2-carbonylamino)-5-(5,6,7,8-tetrahydroquinolin-8-yl) amino valerate 1-naphthalenemethylamide [Compound No. 16]

180 mg of the compound obtained in Synthesis Example 8-6 was suspended in 3.6 ml of DMF and then 0.36 ml of diethylamine was added to the suspension. After 30 minutes, the reaction solution was concentrated. The residue obtained by drying under reduced pressure was dissolved in 0.8 ml of DMF and was then added to a 1. 6 ml DMF solution of 81 mg of the compound obtained in Synthesis Example 15-3. Furthermore, 69 mg of the WSCI hydrochloride and 44 mg of DMAP were added thereto. After 5 hours, the reaction solution was concentrated and then 1-mol/l hydrochloric acid was added, followed by extraction with chloroform. An organic layer was washed with a saturated salt solution and dried with anhydrous sodium sulfate and concentrated. The resulting residue was purified by means of silica gel column chromatography (4 g, chloroform/methanol = 10/1). Consequently, 105 mg of a crude product as yellow syrup was obtained. A mixture solution of 2.5 ml of trifluoroacetic acid, 0.68 ml of thioanisole, and 0.61 ml of m-cresol was added to the crude product. After 30 minutes, the reaction solution was concentrated. Then, 1 mol/l hydrochloric acid was added to the resulting residue and the resultant mixture was washed with chloroform, followed by concentrating the water layer. The resulting residue was dissolved in 1.6 ml of methanol to use in the following reactions (1) and (2).
(1) 3 µl of pyridine-2-aldehyde and 13 µl of triethylamine were added to 0.8 ml of the above solution. After 15 hours, 3.6 mg of sodium borohydride was added to the reaction solution. After 10 minutes, a small amount of silica gel was added to the reaction solution and the reaction solution was then concentrated. The resulting residue was purified by means of silica gel column chromatography (0.5 g, chloroform/methanol/water = 7/3/0.5). 1 mol/l hydrochloric acid was added to the resulting residue and the mixture was then concentrated and azeotropically distilled with methanol. Consequently, 10.0 mg of hydrochloride of the above-mentioned compound was obtained as a white solid product.
   MS(FAB,Pos.):m/z=582[M+1]⁺
   ¹H-NMR(500MHz,DMSO-d₆) : δ=1.68-1.90 (4H,m), 3.00 (2H,t,J-7.3Hz), 4. 28 (2H,s), 4.30 (2H,s), 4.36 (2H,s), 4.47-4.52 (1H,m), 4.77 (2H,d,J=4. 9Hz), 7.26 (1H,d,J=3.4Hz), 7.43-7.50 (4H,m), 7.54-7.60 (4H,m), 7.85-7.92 (3H,m), 7.92-7,99 (1H,m), 8.03-8.08 (1H,m), 8.27 (1H,s), 8.62 (1H ,d,J=4.9Hz), 8.69 (1H,t,J=5.7Hz).
(2) 21 mg of 5,6,7,8-tetrahydroxyquinolin-8-one, 0.16 ml of acetic acid, and 12 mg of sodium cyanoborohydride were added to 0.8 ml of the above solution and the resultant mixture was reacted for 30 hours. The reaction solution was condensed and then the resulting residue was purified by means of silica gel column chromatography (0.5 g, chloroform/methanol/water = 7/3/0.5). Then, the resulting residue was added with 1 mol/l hydrochloric acid, then concentrated and azeotropically distilled with methanol, and 5.4 mg of hydrochloride of the above-mentioned compound was obtained as a white solid product.
   MS(FAB,Pos.):m/z=622[M+1]⁺
   ¹H-NMR(500MHz,DMSO-d₆) : δ=1.70-2.03 (7H,m), 2.28-2-35 (1H,m), 2.78-2.83 (2H,m), 2.88-3.00 (1H,m), 4.39-4.46 (1H,m), 4.51 (2H,s), 4.55 (2H ,s), 4.76 (2H,d,J=5.6Hz), 37 (1H,dd,J=4.8,7.7Hz), 7.40 (1H,d,J=3.4H z), 7.46 (2H,d,J=4.6Hz), 7.51-7.58 (2H,m), 67 (1H,d,J=7.8Hz), 7.73(2 H,s), 7.85 (1H,t,J=4.7Hz), 7.93-7.98 (2H,m), 8.04-8.09 (1H,m), 46(1H ,t,J=3.2Hz), 8.75(1H,brt), 8,85(1H,d,J=8,3Hz), 9.13 (2H,brs), 10.0 5(2H,brs).

### Synthesis Example 16: Production of (S)-2-(4-(8-quinolylamino methyl)benzoylamino)-5-(2-picolylamino) valerate 1-naphthalene methylamide [Compound No. 17]

### Synthesis Example 16-1: Preparation of 4-(8-quinolyl aminomethyl) methylbenzoate (Compound VI-7)

1.26 g of 8-aminoquinoline was dissolved in 20 ml of DMF, and then 1.2 g of potassium carbonate and 1.0 g of methyl 4-bromomethyl benzoate were added to the solution and the resultant mixture was stirred for 20 hours. The reaction solution was concentrated. The resulting residue was diluted with chloroform and then water was added thereto. Extraction with chloroform was performed and then an organic layer was washed with a saturated salt solution. The organic layer was dried with anhydrous sodium sulfate and concentrated, and 2.20 g of the above-mentioned compound was obtained as yellow syrup.

### Synthesis Example 16-2: Synthesis of 4-(8-quinolylaminomethyl) benzoic acid (Compound VII-9)

1 g of the compound obtained in Synthesis Example 16-1 was dissolved in 10 ml of methanol, and then 10 ml of a 1 mol/l sodium hydroxide aqueous solution was added to the solution. In the reaction solution, 10 ml of THF was added and dissolved. After 7-hour reaction, the reaction solution was concentrated. The resulting residue was dissolved in water. 1 mol/l hydrochloric acid was gradually added to the solution while stirring to cause acidic precipitation. The resulting precipitated product was filtrated through a glass filter G4, followed by washing with water. Consequently, 560.1 mg of the above-mentioned compound was obtained as a light-yellow solid product.

### Synthesis Example 16-3: Synthesis of N^{α}-(4-(8-quinolylamino methyl) benzoyle)-N^{δ}-Boc-L-ornithine 1-naphthalenemethylamide (Compound XI-8)

228.1 mg of the compound obtained in Synthesis Example 23-1 was dissolved in 4.5 ml of DMF, followed by the addition of 0.45 ml of diethylamine. After 1-hour reaction, the reaction solution was concentrated. The resulting residue was dissolved in 4 ml of DMF, and then 110 mg of WSCI hydrochloride, 70 mg of DMAP, and 128 mg of the compound obtained in Synthesis Example 16-2 were added to the solution. After 15 hours, the reaction solution was concentrated, and then 0.2 N hydrochloric acid was added thereto. Extraction with chloroform was performed and then an organic layer was washed with a saturated salt solution. The organic layer was dried with anhydrous sodium sulfate and concentrated. The resulting residue was purified by means of silica gel column chromatography (15 g, chloroform/ethyl acetate = 4/1). Consequently, 210 mg of the above-mentioned compound was obtained as light-yellow syrup.
MS(FAB,Pos.):m/z=632[M+1]⁺
¹H-NMR(500MHz,CDCl₃) : δ=1.27 (9H,s), 1.45-1.63 (2H,m), 1.70-1.80 (1H ,m), 1.89-1.97 (1H,m), 3.00-3.17 (1H,m), 3.35-3.42 (1H,m), 4.62 (2H,d , J=5.6Hz), 4.65-4.70 (1H,brt), 4.80-4.95 (3H,m), 6.55 (1H,d,J=7.8Hz ), 6.70 (1H,t,J=6.0Hz), 7.08 (2H,d,J=8.3Hz), 7.17 (1H.d.J=7.8Hz), 7. 23-7.50 (8H,m), 7.70-7.80 (3H,m), 7.84 (1H,d,J=7.6Hz), 7.97 (1H,d,J= 8.1Hz), 8.08 (1H,dd,J=8.3Hz,1.7Hz), 8.75 (1H,dd,J=4.1Hz,1.7Hz).

### Synthesis Example 16-4: Synthesis of (S)-2-(4-(8-quinolylamino methyl) benzoylamino)-5-(2-picolylamino) valerate 1-naphthalene methylamide [Compound No. 17]

122. 6 mg of the compound obtained in Synthesis Example 16-3 was dissolved in 1.2 ml of methanol, followed by the addition of 1.2 ml of a 4 mol/l hydrochloric acid/dioxane solution. After 1-hour reaction, the reaction solution was concentrated and dried under reduced pressure. The resulting residue was dissolved in 2 ml of methanol and then 18 µl of pyridine-2-aldehyde and 27 µl of triethylamine were added to the solution. After 15 hours, the reaction solution was concentrated and dried under reduced pressure. The resulting residue was dissolved again in 2 ml of methanol and then 22 mg of sodium borohydride was added thereto. After 1-hour reaction, the reaction solution was concentrated. The resulting reside was purified by means of silica gel column chromatography (4 g, chloroform/ methanol /water = 7/3/0.5). Consequently, 114.0 mg of the above-mentioned compound was obtained as an orange-colored solid product.
MS(FAB,Pos.):m/z=623(M+1]⁺
¹H-NMR(500MHz,DMSO-d₆):δ=1.69-1.90(4H,m),2.90-3.00(2H,br),4.4 4-4.50(1H,m),3(2H,s),4.74(2H,d,J=5.9Hz),4.91(2H,s),6.61(1H,d, J=7.6Hz),7.13(1H,d,J=7.8Hz),7.331H,t,J=7.9Hz),7.40-7.63(7H,m), 7.80-7.97 (5H,m), 8.00-8.09 (2H,m), 8.37 (1H,d,=8.1Hz),51-8.62 (3H,m), 8.79 (1H,d,J=4.9Hz), 8.85 (1H,dd,J=1.7Hz,4.1Hz), 9.28 (2H,b r).

### Synthesis Example 17: Production of (2S)-2-(4-((N-imidazole -2-ylmethyl) aminomethyl) naphthoylamino)-5-(2-picolylamino) 2-(3-indolyl) ethylamide valerate [Compound No. 18]

### Synthesis Example 17-1: Synthesis of methyl 4-methyl-1-naphthalene carboxylate (Compound III-2)

250.6 mg of commercially available 4-methyl-1-naphtalene carboxylate was dissolved in 7. 5 ml ofmethanol, followedby aerating with hydrogen chloride gas under ice-cold condition. After that, the mixture was stirred for 19 hours at room temperature and then the solvent was distilled off. The residue was dissolved in chloroform and was then washed with a 1 mol/l sodium hydroxide aqueous solution and dried with anhydrous sodium sulfate. Subsequently, the solvent was distilled off and dried under reduced pressure, and 269.9 mg of the above-mentioned compound was obtained as a colorless oily product.
MS(FAB,Pos.):m/z=200[M⁺],201[M+1]⁺
¹H-NMR(500MHz,CDCl3):δ=2.73(3H,s),3.98(3H,s),7.33(1H,d,J=7.3H z),7.61(1H,d,J=8.5,6.8Hz),7.56(1H,dd,J=8.3,6.8Hz),8.04(1H,d,J =8.3Hz),8.08(1H,d,J=7.3Hz),8.97(1H,d,J=8.5Hz).

### Synthesis Example 17-2: Synthesis of methyl 4-bromomethyl-1-naphthalene carboxylate (Compound IV-5)

269.9 mg of the compound obtained in Synthesis Example 17-1 was dissolved in 8 ml of carbon tetrachloride, and then 252.6 mg of N-bromosuccinimide and 22.1 mg of azobisisobutyronitrile were added to the solution and the whole was stirred for 6 hours at 70°C. On completion of the reaction, a solid product was removed by a glass filter and concentrated. The residue was dissolved in chloroform, followed by washing with a 1 mol/l sodium hydroxide aqueous solution and a saturated salt solution. The solution was dried with anhydrous sodium sulfate and the solvent was distilled off, followed by drying under reduced pressure. Consequently, 363.3 mg of the above-mentioned compound was obtained as a light-yellow oily product.
MS(FAB,Pos.):m/z=279,281[M+1]⁺
¹H-NMR(60MHz,CDCl₃):δ=3.94(3H,s),4.86(3H,s),7.35-7.68(3H,m),7. 88-8.21(2H,m),8.66-8.89(1H,m).

### Synthesis Example 17-3: Synthesis of methyl 4-aminomethyl-1-naphthalene carboxylate (Compound XV-1)

328.1 mg of the compound obtained in Synthesis Example 17-2 was dissolved in 7.2ml of DMF, and then 359.4 mg of potassiumphthalimide was added to the solution and the whole was stirred for 12 hours at room temperature. On completion of the reaction, the solvent was distilled off. Then, the residue was dissolved in chloroform and was then washed with distilled water, a 1 mol/l sodium hydroxide aqueous solution and a saturated salt solution. After the resultant was dried with anhydrous sodium sulfate and the solvent was distilled off, the residue was purified by means of silica gel column chromatography (15 g, hexane/ethyl acetate = 2/1), and 281.2 mg of a white solid product was obtained. 1.50 g of this solid product was dissolved in 30 ml of methanol and then 7.5 ml of hydrazine monohydrate was added to the solution and the whole was heated at 60°C. Additionally, 30 ml of methanol was added thereto and the whole was continuously stirred for 1 hour at 60°C. On completion of the reaction, the solvent was distilled off and then the residue was dissolved in chloroform. Subsequently, the resultant solution was washed with distilled water and a saturated salt solution, followedby drying with anhydrous sodium sulfate. After the solvent was distilled off, the residue was dried under vacuum. Consequently, 789.1 mg of the above-mentioned compound was obtained as a light-yellow solid product.
MS(FAB,Pos.):m/z=216[M+1]⁺
¹H-NMR(500MHz,CDCl₃) : δ=4.00 (3H,s), 4.39 (2H,s), 7.55 (1H,d,J=7.6Hz ), 7.57-7.65 (2H,m), 8.11 (1H,d,J=8.3Hz), 8.15 (1H,d,J=7.3Hz), 8.97 (1H,d,J= 8.5Hz).

### Synthesis Example 17-4: Synthesis of 4-(N-Boc-N-(imidazole-2-ylmethyl) aminomethyl) naphthalene carboxylic acid (Compound VII-10)

120.9 mg of the compound obtained in Synthesis Example 17-3 was dissolved in 4.8 ml of methanol, and then 51.5 µl of triethylamine and 65.0 mg of imidazole-2-carboaldehyde were added to the solution and the whole was stirred for 8 hours at room temperature. On completion of the reaction, the mixture was concentrated and dried under a reduced pressure. Subsequently, it was dissolved in 8 ml of anhydrous methanol and the whole was cooled to 0°C. Then, 40.7 mg of sodium borohydride was added to the solution and the whole was stirred for 0.5 hour at room temperature. On completion of the reaction, the solution was concentrated and dissolved in chloroform, followedbywashingwith distilled water and a saturated salt solution. Then, it was dried with anhydrous sodium sulfate and the solvent was then distilled off, and 203.0 mg of a crude product was obtained as a light-yellow oily product. The product was dissolved in 4 ml of DMF and then 94.8 µl of triethylamine and 142 µl of di-t-butyldicarbonate were added to the solution and the whole was stirred for 15 hours at room temperature. On completion of the reaction, the resultant solution was concentrated and dried under reduced pressure, and 314.5 mg of a crude product was thus obtained as a yellow viscous oily product. Subsequently, 314.5 mg of the product was dissolved in 3 ml of THF and 3 ml of methanol, and then 3 ml of a 1 mol/l sodium hydroxide aqueous solution was added to the solution and the whole was stirred for 2 hours at room temperature. On completion of the reaction, the solvent was distilled off and then neutralization with a 1 mol/l hydrochloric acid aqueous solution was performed. Water was added to the solution and extraction with chloroform was performed, and 59.9 mg of the above-mentioned compound was obtained as a colorless oily product.
MS(FAB,Pos.):m/z=382[M+1]⁺
¹H-NMR(500MHz,DMSO-d₆+D₂O): δ=1.39(9H,s),4.26 and 4.36(2H,2s), 4.92 and 4.95(2H,2s),6.80-7.28(3H,m),7.48-7.52(2H,m),7.58(1H, d,J=7.3Hz),8.02(1H,br),8.70(1H,br).

### Synthesis Example 17-5: Synthesis of N^{α}-Fmoc-N^{δ}-Cbz-L-ornithine 2-(3-indolyl) ethylamide (Compound IX-2)

528.0 mg of commercially available N^{α}-Fmoc-N^{δ}-Cbz-L-ornithine was dissolved in 10.6 ml of DMF, and then 235 mg of HOBt, 334 mg of WSCI hydrochloride, and 0.165 ml of tryptamine were added to the solution and the whole was stirred for 16 hours at room temperature. On completion of the reaction, the solvent was distilled off and then the residue was dissolved in chloroform. After extraction with a 1 mol/l hydrochloric acid was performed, an organic layer was washed with a saturated sodium bicarbonate solution. The solvent was distilled off and then the residue was purified by means of silica gel column chromatography (50 g, chloroform/ethyl acetate = 1/1), and 690 mg of the above-mentioned compound was obtained as a white solid product.
MS(FAB,Pos.):m/z=597 [M+1]⁺
¹H-NMR(500MHz,CDCl₃) : δ=1.63 (9H,s), 1.64-1.78 (1H.m), 2.90-3.04 (3H ,m), 3.20-3.28 (1H,m), 3.48-3.62 (3H,m).4.10-4.22 (2H,m), 4.28-4.40 (2H,m),4.58-4.62(1H,m),5.58-5.62(1H,m).6.28-6.38(1H,m),7.00(1 H,s), 7.10 (1H,t,J=7.3Hz), 7.17 (1H,t,J=7.3Hz), 7.28-7.38 (3H,m), 7. 40(2H,t,J=7.4Hz),7.50-7.62(3H,m),7.77(2H,d,J=7,5Hz),8.12-8.20 (1H,m).

### Synthesis Example 17-6: Synthesis of N^{α}-(4-(N-Boc-N-imidazol -2-ylmethyl) aminomethyl naphthoyl)-N^{δ}-Boc-L-ornithine-2-(3-indolyl) ethylaminde (Compound XI-9)

453.2 mg of the compound obtained in Synthesis Example 17-5 was dissolved in 9.1 ml of DMF, and then 0.91 ml of diethylamine was added to the solution and the whole was stirred for 1 hour at room temperature. On completion of the reaction, the solvent was distilled off under reduced pressure, followed by drying the residue under vacuum. Then, 289.7 mg of the compound obtained in Synthesis Example 17-4 and 153.9 mg of HOBt were added to the residue and the mixture was dissolved in 5.8 ml of anhydrous DMF. Subsequently, 218.4 mg of WSCI hydrochloride was added to the solution and the whole was stirred for 19 hours. The solvent was distilled off and then a 1 mol/l hydrochloric acid was added, followed by extracting with chloroform. An organic layer was washed with a saturated sodium bicarbonate solution, followed by drying with anhydrous sodium sulfate and distilling the solvent off. The residue was purified by means of silica gel column chromatography (60 g, chloroform/methanol = 15/1), and 233.5 mg of the above-mentioned compound was obtained as a light-yellow solid product.
MS(FAB,Pos.):m/z=738[M+1]⁺
¹H-NMR(500MHz,CDCl₃):δ=1.63(9H,s),1.90-1.98(1H,m),2.92-3.10(3H ,m),3.22-3.38(1H,m),3.60-3.72(2H,m),4.22-4.35(2H,m),4.62-4.84 (2H,m),5.00-5.10(1H,m),6.48-6.60(1H,m),6.76-6.82(1H,m),6.84-7 .60(13H,m,,J=7.3Hz),7.92-8.04(1H,m),8.12-8.18(1H,m).

### Synthesis Example 17-7: Synthesis of N^{α}-4-((imidazol-2-ylmethyl) aminomethyl) naphthoyl-L-ornithine-2-(3-indolyl) ethylamide (Compound XIV-2)

233.5 mg of the compound obtained in Synthesis Example 17-6 was dissolved in 4.67 ml of methanol, and then 4.67 ml of a 4 mol/l hydrochloric acid/dioxane to the solution and the whole was stirred for 1 hour at room temperature. On completion of the reaction, the solvent was distilled off and then the residue was dried under reduced pressure, and 216 mg of the above-mentioned compound was obtained as a white solid product.

### Synthesis Example 17-8: Synthesis of (2S)-2-(4((N-imidazol-2-ylmethyl) aminomethyl) naphthoylamino)-5-(2-picolylamino) 2-(3-indolyl) ethylamide valerate [Compound No. 18]

107 mg of the compound obtained in Synthesis Example 17-7 was dissolved in 2.55 ml of anhydrous methanol, and then 20.3 mg of pyridine-2-aldehyde and 66.2 µl of triethylamine were added to the solution and the whole was stirred for 14.5 hours. On completion of the reaction, the solvent was distilled off and then the residue was dried under reduced pressure, followed by dissolving in 2.55 ml of anhydrous methanol and adding 12.0 mg of sodium borohydride. After the resultant solution was stirred for 1 hour, the solvent was distilled off. The residue was purified by means of silica gel column chromatography (15 g, chloroform/methanol/water = 7/3/0.5). Then, 1 mol/l hydrochloric acid was added to the resulting above-mentioned compound, followed by concentrating and drying so as to be solidified. Consequently, 65.6 mg of hydrochloride of the above-mentioned compound was obtained as a light-brown solid product.
MS(FAB,Pos.):m/z=629[M+1]⁺
¹H-NMR(500MHz,DMSO-d₆):δ=1.65-1.90(4H,m),2.88(2H,t,J=7.32),2.9 0-3.04(3H,m),3.36-3.44(2H,m),4.22-4.36(2H,m),4.64-4.78(2H,m), 4.82-4.96(2H,m),6.98(1H,t,J=7.4Hz),7.06(1H,t,J=7.4Hz),7.20(1H ,s),7.34(1H,d,J=8.1Hz),7.40-7.44(1H,m),7.56(2H,t,J=8.3Hz),7.6 0-7.80(5H,m),7.84(1H,d,J=7.3Hz),7.91(1H,t,J=7.3Hz),8.24-8.36 (3H,m),8.64(1H,d,J=3.9Hz),8.77(1H,d,J=7.8Hz),9.20-9.42(2H,m).

### Synthesis Example 18: Production of (2S)-2-(4-((N-imidazol-2-ylmethyl) aminomethyl) naphthoylamino)-5-(5,6,7,8-tetrahydro quinolin-8-ylamino) 2-(3-indolyl) ethylamide valerate [Compound No. 19]

107 mg of the compound obtained in Synthesis Example 17-7 was dissolved in 2.55 ml of anhydrous methanol, and then 66.2µl of triethylamine was added to the solution. After that, 28.0 mg of 5,6,7,8-tetrahydroquinolin-8-one and 19.9 mg of sodium cyanoborohydride were added to the solution, and pH thereof was then adjusted to 4 with acetic acid and the whole was stirred for 15 hours at room temperature. On completion of the reaction, the solvent was distilled off and then the residue was purified by means of silica gel column chromatography (5 g, chloroform/methanol/water = 7/3/0.5). Subsequently, 1 mol/l hydrochloric acid was added to the resulting compound, followed by concentrating and drying the mixture so as to be solidified. Consequently, 82.4 mg of the above-mentioned compound was obtained as a white solid product.
MS(FAB,Pos.):m/z=669[M+1]⁺
¹H-NMR(500MHz,DMSO-d₆):δ-1.65-1.96(4H,m),2.78-2.92(4H,m),3.36-3.50(2H,m),4.42-4.58(2H,m),4.64-4.78(2H,m),4.84-4.96(2H,m),6. 98(1H,t,J=7.1Hz),7.06(1H,t,J=6.1Hz),7.20(1H,s),7.34-7.42(2H,m), 7.56 (2H,t,J=7.8Hz), 7.62-7.78 (4H,m), 7.85 (1H,d,J=7.3Hz), 8.24-8.36 (3H,m), 8.49 (1H,d,J=3.4Hz), 8.77 (1H,d,J=7.8Hz), 9.05-9.24 (2H ,m).

### Synthesis Example 19: Production of (S)-2-(4-((imidazol-4-yl methyl) aminomethyl) benzoylamino)-5-((imidazol-4-ylmethyl) amino) 1-naphtalenemethylamide valerate [Compound No. 20]

### Synthesis Example 19-1: Synthesis of 4-N-Boc-aminomethyl benzoic acid (XVII-1)

20.85 g of commercially available 4-aminomethyl benzoic acid was dissolved in 100 ml of dioxane, and then 100 ml of a 1 mol/l sodium hydroxide was added to the solution and the whole was cooled to 0°C. In this solution, a solution prepared by dissolving 30.71 g of di-t-butyldicarbonate in 100 ml of dioxane were dissolved was dropped over 30 minutes. The solution was heated to room temperature and stirred for 16 hours, followed by distilling the solvent off under reduced pressure. The residue was dissolved with 276 ml of a 0.5N sodium hydroxide aqueous solution and the resultant was then precipitated under acidic conditions with a 1 mol/l hydrochloric acid. The resulting solidproduct was dried, and 31.32 g of the above-mentioned compound was obtained as a white solid product.
MS(FAB,Pos.) :m/z=252[M+1]⁺
¹H-NMR(500MHz,DMSO-d₆):δ=1.48(9H,s),4.40(2H,brs),4.96(1H,brs), 7.38(2H,d,J=8.5Hz),8.07(2H,d,J=8.5Hz).

### Synthesis Example 19-2: Synthesis of N^{α}-(4-(N-Boc-aminomethyl) benzoyl-N^{δ}-Boc-L-ornithine 1-naphthalenemethylamide (Compound XVIII-1)

501.7 mg of commercially available N^{α}-Fmoc-N^{δ}-Boc-ornithine was dissolved in 6.0ml of DMF, and then 328.0mg of WSCI hydrochloride and 166.4 mg of HOBt were added and dissolved. Then, 195 ml of 1-naphthalene methylamine was added to the solution and the whole was stirred for 20 hours at room temperature. On completion of the reaction, the solvent was distilled off. Then, the residue was dissolved in chloroform and washed with a 1 mol/l hydrochloric acid and a saturated salt solution. An organic layer was dried with anhydrous sodium sulfate, followed by distilling the solvent off to obtain 631.7 mg of a crude product as a white frothy product. 499.9 mg of the product was dissolved in 10 ml of DMF, and then 1.0 ml of diethylamine was added to the solution and the whole was stirred for 180 minutes at room temperature. On completion of the reaction, the solvent was distilled off and then the product was dried by a vacuum pump. 509.2 mg of the dried product was dissolved in 10 ml of DMF, and then 241.9 mg of WSCI hydrochloride, 113.8 mg of HOBt, and 253.9 mg of the compound obtained in Synthesis Example 19-1 were added to the solution and the whole was stirred for 13.5 hours at room temperature. On completion of the reaction, the solvent was distilled off. The residue was dissolved in chloroform and washed with a 1 mol/l hydrochloric acid, a 1 mol/l sodium hydroxide aqueous solution, and a saturated salt solution. An organic layer was dried with anhydrous sodium sulfate and then the solvent was distilled off. The residue was purified by means of silica gel column chromatography (25 g, chloroform/methanol = 25/1), and 310.8 mg of the above-mentioned compound was obtained as a white solid product.
MS(FAB,Pos.):m/z=605[M+1]⁺
¹H-NMR(500MHz,DMSO-d₆): δ=1.36 (9H,s), 1.39 (9H,s), 1.35-1.45 (2H,m) , 1.60-1.80 (2H,m), 2.91 (2H,m), 4.17 (2H,d,J=7.7Hz), 4.48 (1H.m), 4.7 5 (2H,d,J=5.9Hz), 6.80 (1H,t,J=5.6Hz), 7.31 (2H,d,J=8.3Hz), 7.4-7.4 5 (3H,m), 7.5-7.6 (2H,m), 7.8-7.9 (3H,m), 7.95 (1H,m), 8.06 (1H,m), 8.4 5(1H,d,J=5.9Hz),8.51(1H,d,J=5.4Hz).

### Synthesis Example 19-3: Synthesis of N^{α}-(4-aminomethylbenzoyl) -L-ornithine 1-naphthalenemethylamide (Compound XIX-1)

106.2 mg of the compound obtained in Synthesis Example 19-2 was dissolved in 1.0 ml of methanol, and then 2.0 ml of a 4 mol/l dioxane was added to the solution and the whole was stirred for 20 hours at room temperature. On completion of the reaction, the solvent was distilled off and the residue was then dried by a vacuum pump, and 98.1 mg of the above-mentioned compound was obtained as a white solid product.
MS(FAB,Pos.):m/z=405[M+1]⁺

### Synthesis Example 19-4: Synthesis of (S)-2-(4-((imidazole-4-yl methyl) aminomethyl) benzoylamino)-5-((imidazol-4-ylmethyl) amino) valerate 1-naphthalenemethylamide [Compound No. 20]

77.2 mg of the compound obtained in Synthesis Example 19-3 was dissolved in 1.5 ml of anhydrous methanol, and then 54.1 µl of triethylamine and 32.7 mg of 1-methyl-2-imidazole carboxyaldehyde were added to the solution and the whole was stirred for 2.5 hours at room temperature. On completion of the reaction, the solvent was distilled off. Subsequently, 1.5 ml of anhydrous methanol was added to the solution and the whole was cooled to 0°C. Then, 18. 4 mg of sodium borohydride was added to the solution and the whole was stirred for 1 hour while being gradually returned to room temperature. On completion of the reaction, the solvent was distilled off. The residue was purified by means of silica gel column chromatography (6 g, chloroform/methanol/water = 7/3/0.5). The purified compound was dissolved in 2 ml of a 1 mol/l hydrochloric acid and then water was distilled off, and 96.0 mg of hydrochloride of the above-mentioned compound was obtained as a white solid product.
MS(FAB,Pos.):m/z=[M+1]⁺
¹H-NMR(500MHz,DMSO-d₆) : δ=1.47-1.94 (4H,m), 2.78-3.00 (2H,m), 4.26 (4H,s), 4.32 (2H,s), 4.51-4.60 (1H,m), 4.77 (2H,d,J=5.9Hz), 7.44-7.49 (2H,m), 7.52-7.60 (2H,m), 7.69 (2H,d,J=8.3Hz), 7.80 (1H,s), 7.81-7.8 5 (1H,m), 7.85 (1H,s), 7.93-7.95 (1H,m), 8.00 (2H,d,J=8.3Hz), 8.05 (1H ,m), 8.70 (1H,d,J=8.1Hz), 8.74 (1H,t,J=5.9Hz), 9.09 (1H,s), 9.11 (1H, s),9.77(2H,br),10.34(1H,br).

### Synthesis Example 20: Production of (S)-2-(4-((imidazole-2-yl methyl) aminomethyl) benzoylamino)-5-((imidazol-2-ylmethyl) amino) valerate 1-naphthalenemethylamide [Compound No. 21]

16.8 mg of the compound obtained in Synthesis Example 19-3 was dissolved in 0.6 ml of methanol, and then 7.1mg of triethylamine and 7.1 mg of 2-imidazole carboxyaldehyde were added to the solution and the whole was stirred for 2 hours at room temperature. After the solvent was distilled off, 0.6 ml of anhydrous methanol was added to the resultant and the whole was cooled to 0ºC. Then, 8.0 mg of sodium borohydride was added thereto and the whole was stirred for 30 minutes while being gradually returned to room temperature. On completion of the reaction, the solvent was distilled off. The residue was purified by means of silica gel column chromatography (0.8 g, chloroform/methanol/water = 7/3/0.5). The resultant compound was dissolved in a 1 mol/l hydrochloric acid and then water was distilled off, and 15.9 mg of hydrochloride of the above-mentioned compound was obtained as a white solid product.
MS(FAB,Pos.):m/z=595[M+1]⁺
¹H-NMR(500Mz,DMSO-d₆):δ=1.71-1.90(4H,m),3.05-3.17(2H,m),4.34(2H,m),4.43(2H,m),4.55(1H,m),4.77(2H,d,J=2.2Hz),7 .47(2H,m),7.56(2H,m),7.64(6H,m),7.86(1H,m),7.96(1H,m),7.98(1H,m) ,8.06(1H,m),8.70(2H,m),9.94(1H,brs).

### Synthesis Example 21: Production of (S)-2-(4-((1-methylpyrrol-2-ylmethyl) aminomethyl) benzoylamino)-5-((1-methylpyrrol-2-yl methyl) amino) valerate 1-naphthalenemethylamide [Compound No. 22]

100 mg of the compound obtained in Synthesis Example 19-2 was dissolved in 2 ml of methanol, and then 2 ml of a 4 mol/l hydrochloric acid/dioxane was added to the solution, followed by stirring for 2 hours at room temperature. On completion of the reaction,thesolvent wasdistilled off. Subsequently, the residue was dissolved in 2 ml of anhydrous methanol, and then 55.3 µl of triethylamine and 41.8 µl of 1-methyl-2-pyrrole carboxyaldehyde were added to the solution and the whole was stirred for 4 hours at room temperature. On completion of the reaction, the solvent was distilled off. Subsequently, 2 ml of anhydrous methanol was added thereto and the whole was cooled to 0°C. Then, 18.8 mg of sodium borohydride was added to the solution and the whole was stirred for 12 hours while being gradually returned to room temperature.

On completion of the reaction, the solvent was distilled off and the residue was purified by means of silica gel column chromatography (10 g, chloroform/methanol = 10/1). After the compound was dissolved in 2 ml of a 1 mol/l hydrochloric acid, water was distilled off. Consequently, 83.7 mg of hydrochloride of the above-mentioned compound was obtained as a white solid product.
MS(FAB,Pos.):m/z=591[M+1]⁺
¹H-NMR(500MHz,DMSO-d₆) : δ=1.70-1.91 (4H,m), 2.93 (2H,br), 3.63 (6H,2 s), 4.10-4.22 (6H,m), 4.53-4.57 (1H,m), 4.76-4.77 (2H,m), 5.98-6.02 (2H,m), 6.21-6.27 (2H,m), 6.79-6.82 (2H,m), 7.45-7.56 (4H,m), 7.64 (2H ,d,J=7.8Hz), 7.84-7.86 (1H,m), 7.94-7.99 (3H,m), 8.05-8.08 (1H,m), 8 .67-8.70(2H,m),8.82(1H,br),9.42(1H,br).

### Synthesis Example 22: Production of (S)-2-(4-((1-methylimidazole -2-ylmethyl) aminomethyl) benzoylamino)-5-((1-methylimidazole -2-ylmethyl) amino) valerate 1-naphthalenemethylamide [Compound No. 23]

100 mg of the compound obtained in Synthesis Example 19-2 was dissolved in 2 ml of methanol and then 2 ml of a 4 mol/l hydrochloric acid/dioxane was added to the solution, followed by stirring for 3 hours at room temperature. On completion of the reaction, the solvent was distilled off. Subsequently, it was dissolved in 2 ml of anhydrous methanol, and then 55.3 µl of triethylamine and 38.2 mg of 1-methyl-2-imidazole carboxyaldehyde were added to the solution and the whole was stirred for 3 hours at room temperature. On completion of the reaction, the solvent was distilled off. Subsequently, it was dissolved in 2 ml of anhydrous methanol and cooled to 0°C. Then, 18.8 mg of sodium borohydride was added to the solution and the whole was stirred for 25 hours while being gradually returned to room temperature. On completion of the reaction, the solvent was distilled off, and the residuewaspurifiedbymeans of silica gel column chromatography (10 g, chloroform/methanol = 4/1). After the purified compound was dissolved in 2 ml of a 1 mol/l hydrochloric acid, water was distilled off. Consequently, 89.2 mg of hydrochloride of the above-mentioned compound was obtained as a white solid product.
MS(FAB,Pos.):m/z=593[M+1]⁺
¹H-NMR(500MHz,DMSO-d₆) : δ=1.77-1.91(4H,m),3.04-3.09(3H,m),3.95 (3H,s),3.96(3H,s),4.40(2H,br),4.52(2H,br),4.55-4.58(3H,m),4.76 -4.77(2H,m),7.4-7.50(2H,m),7.52-7.57(2H,m),7.71-7.75(6H,m),7. 84-7.86(1H,m),7.93-7.96(1H,m),8.00-8.04(2H,m),8.06-8.08(1H,m) ,8.72-8.75(2H,m),10.07(1H,br).

### Synthesis Example 23: Production of (S)-2-(4-(N-2-picolylamino) butyrylamino)-5-(2-picolylamino) valerate 1-naphthalenemethyl amide [Compound No. 24]

### Synthesis Example 23-1: Synthesis of N^{α}-Fmoc-N^{δ}- Boc-L-ornithine 1-naphthalenemethylamide (Compound IX-3)

501.7 g of commercially available N^{α}-Fmoc-N^{δ}-Boc-L-ornithine was dissolved in 10 ml of DMF, and then 166.4 g of HOBt, 328.0 mg of WSCI hydrochloride and 0.195 ml of 1-naphthalenemethylamine were added to the solution and the whole was stirred for 12 hours at room temperature. On completion of the reaction, the solvent was distilled off. Then, the residue was dissolved in chloroform, and the extraction was performed by the addition of a 1 mol/l hydrochloric acid, followed by washing an organic layer with a saturated sodium hydrogencarbonate aqueous solution. The solvent was distilled off, and 631.7 mg of the above-mentioned compound was obtained as a white solid product.
MS(FAB,Pos.):m/z=594[M+1]⁺

### Synthesis Example 23-2: Synthesis of N^{δ}-Boc-L-ornithine 1-naphthalenemethylamide (Compound X-2)

250.8 mg of the compound obtained in Synthesis Example 23-1 was dissolved in 5 ml of DMF, and then 0.5 ml of diethylamine was added to the solution and the whole was stirred for 2 hours at room temperature. On completion of the reaction, the solvent was distilled off under reduced pressure. Subsequently, vacuum drying was performed, and 269.1 mg of the above-mentioned compound was obtained.
MS (FAB,Pos.):m/z=372[M+1]⁺

### Synthesis Example 23-3: Synthesis of N^{α}-(4-N-Boc-aminobutyryl) -N^{δ}-Boc-L-ornithine 1-naphthalenemethylamide (Compound XVIII-2)

216.2 mg of the compound obtained in Synthesis Example 23-2 was dissolved in 4 ml of DMF, and then 102.0 mg of WSCI hydrochloride, 50.2 mg of HOBt, and 76.5 mg of commercially available 4-N-Boc-aminovalerate were added to the solution and the whole was stirred for 16 hours at room temperature. On completion of the reaction, the solvent was distilled off. Then, the residue was dissolved in chloroform and the whole was then washed with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purified by means of silica gel column chromatography (10 g, chloroform/methanol = 30/1), and 148.9 mg of the above-mentioned compound was obtained as a white solid product.
MS(FAB,Pos.):m/z=557[M+1]⁺
¹H-NMR(500MHz,DMSO-d₆) : δ=1.36 (9H,s),1.37 (9H,s), 1.41-1.57 (1H,m) , 1.58-1.70 (3H,m), 2.13 (2H,t,J=7.4Hz), 2.84-2.97 (2H,m), 4.27 (1H,m ), 4.73 (2H,d,J=4.4Hz), 6.77 (1H,d,J=5.7Hz), 6.81 (1H,t,J=5.7Hz), 7. 41-7.49 (2H,m), 7.53-7.58 (2H,m), 7.845 (1H,d,J=8.1Hz), 7.92-7.97 (1 H,m), 8.01-8.05 (2H,m), 8.47 (1H,d,J=5.7Hz)

### Synthesis Example 23-4: Synthesis of (S)-2-(4-(N-2-picolylamino) butyrylamino)-5-(2-picolylamino) valerate 1-naphthalenemethyl amide [Compound No. 24]

102.7 mg of the compound obtained in Synthesis Example 23-3 was dissolved in 1.0 ml of methanol, and then 1.0 ml of a 4 mol/l hydrochloric acid/dioxane was added to the solution and the whole was stirred for 2 hours at room temperature. On completion of the reaction, the solvent was distilled off. After that, it was dried by vacuum pump. 50.7 mg of the dried product was dissolved in 1.0 ml of methanol, and then 39.1 µl of triethylamine and 23.1 µl of 2-picolylamine were added to the solution and the whole was stirred for 2 hours at room temperature. On completion of the reaction, the solvent was distilled off. Subsequently, 1.0 ml of anhydrous methanol was added to the residue and the whole was cooled to 0°C, and 35.3 mg of sodium borohydride was added to the solution and the whole was stirred for 1 hour. On completion of the reaction, the solvent was distilled off. The residue was purified by means of silica gel column chromatography (4 g, chloroform/methanol/water = 7/3/0.5). After the purified compound was dissolved in a 1 mol/l hydrochloric acid, water was distilled off. Consequently, 44.2 mg of hydrochloride of the above-mentioned compound was obtained as a white solid product.
MS(FAB,Pos.):m/z=539[M+1]⁺
¹H-NMR(500MHz,DMSO-d₆) : δ=1.57-1.62(1H,m),1.62-1.71(3H,m),1.85-2.00(2H,m),2.90-3.05(2H,m),4.30(2H,s),4.29-4.35(1H,m),4.34(2H ,s),4.73(2H,d,J=5.6Hz),7.40-7.60(6H,m),7.67(2H,dd,J=10.9,7.8H z),7.84(1H,d,7.8Hz),7.90-8.01(3H,m)8.05(1H,d,J=7.3Hz),8.31(1H ,d,J=8.1Hz), 8.63-8.75 (3H,m), 9.40-9.68 (4H,br).

### Synthesis Example 24: Production of (2S)-2-(trans-(4-(5,6,7,8 -tetrahydroquinolin-8-yl) aminomethyl) cycrohexylcarbonyl) amino-5-(5,6,7,8-tetrahydroquinolin-8-ylamino) valerate 1-naphthalenemethylamide [Compound No. 25]

### Synthesis Example 24-1: Synthesis of N-Boc tranexamic acid (Compound XVII-2)

3.14 g of tranexamic acid was dissolved in 63 ml of dioxane, and then 4.59 ml of di-t-butyl-di-carbonate and 20 ml of a 1 mol/l sodium hydroxide aqueous solution were added to the solution and the whole was stirred for 3.5 hours at room temperature. On completion of the reaction, the solvent was distilled off and then the residue was dissolved by the addition of 20 ml of a 1 mol/l sodium hydroxide aqueous solution and 10 ml of distilled water, followed by precipitating under acetic conditions with a 1 mol/l hydrochloric acid. A crystal was collected through filtration and was then dissolved in chloroform, followed by washing with a saturated salt solution and drying with anhydrous sodium sulfate. Subsequently, the solvent was distilled off, and 4.88 g of the above-mentioned compound was obtained as a white solid product.
MS(FAB,Pos.):m/z=258[M+1]⁺
¹H-NMR(500MHz,DMSO-d₆) : δ=0.95 (2H,qd,J=12.8,3.0Hz), 1.20-1.58 (12 H,m), 1.83 (2H,d,J=11.5Hz), 2.04 (2H,dd,J=13.9,3.0Hz), 2.25 (1H,tt, J=12.2,3.0Hz), 2.99 (2H,t,J=6.3Hz), 4.66 (1H,brs).

### Synthesis Example 24-2: Synthesis of N^{α}-(4-trans-(N-Boc amino methylcylohexyl) carbonyl)-N(-Boc-L-ornithine 1-naphthalene methylamide (Compound XVIII-3)

328.2 mg of the compound obtained in Synthesis Example 23-2 was dissolved in 6 ml of DMF, and then 146.3 mg of WSCI hydrochloride, 75. 4 mg of HOBt, and 156.3 mg of the compound obtained in Synthesis Example 24-1 were added to the solution and the whole was stirred for 5.5 hours at room temperature. On completion of the reaction, the solvent was distilled off. Then, the residue was dissolved in chloroform and the whole was then washed with a 1 mol/l hydrochloric acid aqueous solution, a 1 mol/l sodium hydroxide aqueous solution, and a saturated salt solution. An organic layer was dried with anhydrous sodium sulfate, followed by distilling the solvent off. The residue was purified by means of silica gel column chromatography (25 g, chloroform/methanol = 25/1), and 213.0 mg of the above-mentioned compound was obtained as a white solid product.
MS(FAB,Pos.):m/z=611[M+1]⁺
¹H-NMR(500MHz,DMSO-d₆) : δ=0.76-0.90 (2H,m), 1.20-1.40 (4H,m), 1.36 (9H,s), 1.37(9H,s), 1.40-1.55 (1H,m), 1.58-1.77 (6H,m), 2.13 (1H,t,J= 11.9Hz), 2.75 (2H,dd,J=12.5,6.3Hz), 2.81-2.93 (2H,m), 4.25 (1H,dd,J =14.0,8.5Hz), 4.72 (2H,d,J=5.6Hz), 6.77-6.83 (2H,m), 7.41 (1H,d,J=6 .6Hz), 7.45 (t,J=7.5Hz), 7.52-7.57 (2H,m), 7.83-7.87 (2H,m), 7.92-7. 96(1H,m),8.01-8.04(1H,m),8.38 (1H,t,J=5.6Hz).

### Synthesis Example 24-3: Synthesis of (2S)-2-(trans-(4-(5,6,7,8 -tetrahydroquinoline-8-yl) aminomethyl)cyclohexylcarbonyl) amino-5-(5,6,7,8-tetrahydroquinolin-8-ylamino) valerate 1-naphthalenemethylamide [Compound No. 25]

108.3 mg of the compound obtained in Synthesis Example 24-2 was dissolved in 1.0 ml of methanol, and then 1.0 ml of a 4 mol/l hydrochloric acid/dioxane was added to the solution and the whole was stirred for 90 minutes at room temperature. On completion of the reaction, the solvent was distilled off and the residue was then dried by a vacuum pump, and 111.5 mg of a crude product was obtained as a white solid product. 39.1 mg of the product was dissolved in 0.8 ml of methanol, and then three drops of triethylamine were added to the solution, and the pH of the solution was adjusted to 7 to 8. Subsequently, 72.4 mg of 5,6,7,8-tetrahydroquinolin-8-one was added to the solution, and furthermore 41.6 mg of sodium cyanoborohydride and 30 drops of acetic acid were added to the solution and the whole was stirred for 22 hours at room temperature. On completion of the reaction, the solvent was distilled off. The residue was purified by means of silica gel column chromatography (2 g, chloroform/methanol/water = 7/3/0.5). The purified compound was dissolved in a 1 mol/l hydrochloric acid,followed by distilling water off. Consequently, 23.3 mg of hydrochloride of the above-mentioned compound was obtained as a white solid product.
MS(FAB,Pos.):m/z=673[M+1]⁺
¹H-NMR(500MHz,DMSO-d₆):δ=0.90-1.07(2H,m),1.23-1.42(2H,m)1.55-1.95(14H,m),1.97-2.08(2H,m),2.20(1H,d,J=11.8Hz),2.27-2.36(2H, m),2.65-2.72(2H,m),2.75-2.85(4H,m),2.87-3.00(1H,m),3.01-3.11 (1H,m),4.28-4.35(1H,m),4.37-4.42(1H,m),4.42-4.52(1H,m),4.73(1H ,dd,J=15.8,5.6Hz),4.75(1H,dd,J=15.8,5.6Hz),7.36-7.45(3H,m),7. 46(1H,d,J=7.1Hz),7.53-7.56(2H,m),7.68(2H,d,J=7.8Hz),7.85(1H,d ,J=7.8Hz),7.94-7.96(1H,m),8.03-8.06(1H,m),8.49(1H,d,J=3.9Hz), 8.45-8.60(2H,brs),8.96(2H,brs), 9.04(2H,brs).

### Synthesis Example 25: Production of (2S)-2-(4-(5,6,7,8-tetra hydroquinolin-8-ylaminomethyl) naphthoyl) amino-5-(5,6,7,8-tetrahydroquinolin-8-ylamino) valerate 1-naphthalenemethylamide [Compound No. 26]

### Synthesis Example 25-1: Synthesis of methyl 4-Boc-aminomethyl-1-naphthalene carboxylate (Compound XVI-1)

209.9 mg of the compound obtained in Synthesis Example 17-3 was dissolved in 4 ml of DMF, and then 322 µl of di-t-butyldicarbonate and 262 µl of triethylamine were added to the solution and the whole was stirred for 18 hours at room temperature. On completion of the reaction, the solvent was distilled off and then the residue was purified by means of silica gel column chromatography (6 g, chloroform), and 288.9 mg of the above-mentioned compound was obtained as a light-yellow oily product.
MS(FAB,Pos.):m/z=316[M+1]⁺
¹H-NMR(500MHz,CDCl₃):δ=1.47 (9H,s),4.00(3H,s),4.82 (2H,d,J=5.6Hz ),4.89(1H,brs),7.48(1H,d,J=7.6Hz),7.58-7.66(2H,m),8.09(1H,d,J =8.3Hz),8.11(1H,d,J=7.6Hz),8.94(1H,d,J=8.1Hz).

### Synthesis Example 25-2: Synthesis of methyl 4-Boc-aminomethyl-1-naphthalene carboxylic acid (Compound XVII-3)

266.6 mg of the compound obtained in Synthesis Example 25-1 was dissolved in 2.7 ml of THF and 2.7 ml of methanol, and then 2.7 ml of a 1 mol/l sodium hydroxide aqueous solution was added to the solution and the whole was stirred for 5 hours at room temperature. On completion of the reaction, the solvent was distilled off and the residue was dissolved in distilled water, followed by precipitating under acidic conditions with a 1 mol/l hydrochloric acid aqueous solution. A precipitated solid product was collected through filtration and dried, and 233.5 mg of the above-mentioned compound was obtained as a white solid product.
MS(FAB,Pos.):m/z=302[M+1]⁺
¹H-NMR(500MHz,CDCl₃):δ=1.41(9H,s),4.64(2H,d,J=5. 8Hz),4.89(1H,b rs),7.46(1H,d,J=7.5Hz),7.57(1H,t,J=5.8Hz),7.58-7.68(2H,m),8.1 0(1H,d,J=7.5 Hz),8.19(1H,d,J=8.2Hz),8.90(1H,d,J=7.5Hz).

### Synthesis Example 25-3: Synthesis of N^{α}-4-(N-Boc-aminomethyl) naphthoyl)-N^{δ}-Boc-L-ornithine 1-naphthalenemethylamide (Compound XVIII-4)

170.3 mg of the compound obtained in Synthesis Example 23-2 was dissolved in 3 ml of DMF, and then 73.6 mg of WSCI hydrochloride, 42.9 mg of HOBt, and 78.6 mg of the compound obtained in Synthesis Example 25-2 were added to the solution and the whole was stirred for 14 hours at room temperature. On completion of the reaction, the solvent was distilled off. Then, the residue was dissolved in chloroform and was then washed with a 1 mol/l hydrochloric acid, a 1 mol/l sodium hydroxide aqueous solution, and a saturated salt solution, followed by drying with anhydrous sodium sulfate. The solvent was distilled off. The residue was purified by means of silica gel column chromatography (10 g, chloroform/methanol = 30/1), and 84.0 mg of the above-mentioned compound was obtained as a white solid product.
MS(FAB,Pos.):m/z=655[M+1]⁺

### Synthesis Example 25-4: Synthesis of (2S)-2-(4-(5,6,7,8-tetra hydroquinolin-8-ylaminomethyl) naphthoyl) amino-5-(5,6,7,8-tetrahydroquinoline-8-ylamino) valerate 1-naphthalenemethyl amide [Compound No. 26]

50.4 mg of the compound obtained in Synthesis Example 25-3 was dissolved in 0.5 ml of methanol, and then 0.5 ml of a 4 mol/l hydrochloric acid/dioxane solution was added to the solution and the whole was stirred for 2 hours at room temperature. On completion of the reaction, the solution was concentrated and dried under reduced pressure. Subsequently, 50.5 mg of the compound was dissolved in 1 ml of methanol, and then 26.9 µl of triethylamine, 28.3 mg of 5,6,7,8-tetrahydroquinolin-8-one, and 15. 0 mg of sodium cyanoborohydride were added thereto. The pH of the solution was adjusted to about 4 to 5 by the addition of 10 drops of acetic acid, followed by stirring for 19 hours at room temperature. On completion of the reaction, the solution was concentrated and the residue was then purified by means of silica gel column chromatography (3.5 g, chloroform/methanol = 10/1.5), followed by treating withal mol/l hydrochloric acid. Consequently, 52.8 mg of hydrochloride of the above-mentioned compound was obtained as a light-yellow solid product.
MS(FAB,Pos.):m/z=717[M+1]⁺
¹H-NMR(500MHz,DMSO-d₆) : δ=1.75-2.11(10H,m), 2.13-2.21 (1H,m), 2.31 - 2.40 (1H,m), 2.79-2.84 (2H,m), 2.85-2.91 (2H,m), 2.95-3.03 (1H,m), 3 .04-3.20 (1H,m), 4.41-4.48 (1H,m), 4.58-4.71 (3H,m), 4.79-4.89 (2H,m ), 4.95-5.05 (1H,m), 7.39 (1H,dd,J=7.8,4.8Hz), 7.43-7.47 (1H,m),7.4 9 (1H,d,J=8.1Hz), 7.53-7.59 (3H,m), 7.6-0-7.64 (1H,m), 7.69 (1H,d,J=8 .1Hz), 7.69-7.75 (3H,m), 7.87 (2H,t,J=7.1Hz), 7.95-7.99 (1H,m), 8.10 - 8.13 (1H,m), 8.29 (1H,d,J=8.5Hz), 8.37 (1H,d,J=6.8Hz), 8.48 (1H,d,J =3.7Hz), 8.61 (1H,d,J=3.9Hz), 8.78 (1H,t,J=5.6Hz), 8.87 (1H,d,J=8.1 Hz), 9.26 (2H,br), 9.74 (2H,br).

### Synthesis Example 26: Production of (S)-2-(4-(N-2-picolylamino methyl) naphthoylamino)-5-(2-picolylamino) valerate 1-naphthalenemethylamide [Compound No. 27]

26.9 mg of the compound obtained in Synthesis Example 25-3 was dissolved in 0.56 ml of methanol, and then 0.56 ml of a 4 mol/l hydrochloric acid/dioxane solution was added to the solution and the whole was stirred for 2 hours at room temperature. On completion of the reaction, the solution was concentrated and dried under reduced pressure. Subsequently, 50.5 mg of the compound was dissolved in 0.56 ml of methanol, and then 13.9µl of triethylamine and 8.2 mg of 2-pyridinealdehyde were added to the solution and the whole was stirred for 19 hours at room temperature. On completion of the reaction, the solution was concentrated and dried under reduced pressure, and was then dissolved in 0.56 ml of anhydrous methanol and cooled to 0°C. Furthermore, 10.3 mg of sodium borohydride was added to the solution and the whole was stirred for 7 hours at room temperature. On completion of the reaction, the solution was concentrated. The residue was purified by means of silica gel column chromatography (1.5 g, chloroform/methanol/water = 7/3/0.5), followed by treating with a 1 mol/l hydrochloric acid. Consequently, 21.8 mg of hydrochloride of the above-mentioned compound was obtained as a white solid product.
MS(FAB,Pos.):m/z=637[M+1]⁺
¹H-NMR(500MHz,DMSO-d₆) : δ=1.78-1.97 (4H,m), 2.96-3.10 (2H,m), 4.31 (2H,s), 4.47 (2H,s), 4.58-4.65 (1H,m), 4.72-4.88 (3H,m), 7.44-7.51 (3H ,m).7.54-7.64 (6H,m), 7.68-7.70 (2H,m), 7.88 (1H,d,J=8.3Hz), 7.90 (1 H,d,J=5.9Hz), 7.91-8.00 (3H,m), 8.10-8.13 (1H,m), 8.28 (1H,d,J=8.8H z),8.30 (1H,d,J=8.5Hz), 8.63 (1H,ddd,J=4.9,1.7,1.0Hz), 8.71 (1H,d, J=4.9Hz), 8.75 (1H,d,J=5.4Hz), 8.86 (1H,d,J=8.1Hz), 9.30 (2H,br), 9. 83 (2H,br).

### Synthesis Example 27: Production of (S)-2-(4-((imidazol-2-yl methyl) aminomethyl) naphthoylamino)-5-((imidazol-2-ylmethyl) amino) 1-napththalenemethylamide valerate [Compound No. 28]

100.0 mg of the compound obtained in Synthesis Example 25-3 was dissolved in 2 ml of methanol, and then 2 ml of a 4 mol/l hydrochloric acid/dioxane solution was added to the solution and the whole was stirred for 1.5 hours at room temperature. On completion of the reaction, the solution was concentrated and dried under a reduced pressure. Subsequently, 108.2 mg of a the crude product thereof was dissolved in 2 ml of methanol, and then 51.5 µl of triethylamine and 30.9 mg of 2-imidazolecarboaldehyde were added to the solution and the whole was stirred for 24 hours at room temperature. On completion of the reaction, the solution was concentrated and dried under a reduced pressure, and it was then dissolved in 2 ml of anhydrous methanol and the whole was cooled to 0°C. Furthermore, 26.3 mg of sodium borohydride was added to the solution and the whole was stirred for 0.5 hour at room temperature. On completion of the reaction,. the solution was concentrated. The residue was purified by means of silica gel column chromatography (1.5g, chloroform/methanol/water=7/3/0.5) and treated with a 1 mol/l hydrochloric acid, and 64.2 mg of the above-mentioned compound was obtained as a white solid product.
MS(FAB,Pos.):m/z=614[M+1]⁺
¹H-NMR(500MHz,DMSO-d₆+D₂O) :δ=1.70-1.96(4H,m),3.02-3.15(2H,m),4 .47(2H,s),4.60-4.65(1H,m),4.65(2H,s),4.75-4.91(3H,m),7.48-7.5 2(1H,m),7.54-7.68(8H,m),7.70-7.74(2H,m),7.77(1H,d,J=7.6Hz),7. 89(1H,d,J=8.1Hz),7.97-7.990(1H,m),8.09-8.12(1H,m),8.23-8.28(2H ,m).

### Synthesis Example 28: Synthesis of (S)-2-(4-((1-methylimidazol -2-ylmethyl) aminomethyl) naphthoylamino)-5-((1-methylimidazol -2-ylmethyl) amino) valerate 1-naphthalenemethylamide [Compound No. 29]

100.3 mg of the compound obtained in Synthesis Example 25-3 was dissolved in 2 ml of methanol, and then 2 ml of a 4 mol/l hydrochloric acid/dioxane solution was added to the solution and the whole was stirred for 4 hours at room temperature. On completion of the reaction, the solution was concentrated and dried under reduced pressure. Subsequently, it was dissolved in 2 ml of methanol, and then 51.5 µl of triethylamine and 37.4 mg of 1-methyl-2-imidazolecarboaldehyde were added to the solution and the whole was stirred for 24 hours at room temperature. On completion of the reaction, the solution was concentrated and dried under reduced pressure, and it was then dissolved in 2 ml of anhydrous methanol and the whole was cooled to 0°C. Furthermore, 33.1 mg of sodium borohydride was added to the solution and the whole was stirred for 0.5 hour at room temperature. On completion of the reaction, the solution was concentrated. The residue was purified by means of silica gel column chromatography (1.5 g, chloroform/methanol/water = 7/3/0.5) and treated with a 1 mol/l hydrochloric acid, and 75.4 mg of hydrochloride of the above-mentioned compound was obtained as a white solid product.
MS(FAB,Pos.):m/z=643(M+1]⁺
¹H-NMR(500MHz,DMSO-d₆) : δ=1.80-1.97 (4H,m), 3.02-3.17 (2H,m), 3.98 (3H,s), 4.01 (3H,s), 4.55 (2H,s), 4.60-4.66 (1H,m), 4.80 (2H,s), 4.81 (1 H,dd,J=15.6,5.6Hz), 4.85 (1H,dd,J=15.6,5.6Hz), 4.92 (2H,s), 7.47-7 .51 (1H,m), 7.54-7.60 (3H,m), 7.63 (1H,t,J=7.1Hz), 7.69 (1H,t,J=7.1H z), 7.74 (1H,d,J=7.3Hz), 7.76-7.79 (4H,m), 7.86-7.89 (2H,m), 7.96-7. 98 (1H,m), 8.10-8.12 (1H,m), 8.29 (1H,d,J=8.3Hz), 8.37 (1H,d,J,8.5Hz ),8.78(1H,t,J=5.6Hz),8.89(1H,d,J=7.8Hz),10.21(2H,brs).

### Synthesis Example 29: Production of (S)-2-(4-((1-methylimidazol -2-ylmethyl) aminomethyl)benzoylamino)-5-((1-methylpyrrol-2-yl methyl) amino) valerate 1-naphthalenemethylamide [Compound No. 30]

### Synthesis Example 29-1: Synthesis of N^{α}-4-(N-Boc-aminomethyl) benzoyl-N^{δ}-Cbz-L-ornithine 1-naphthalenemethylamide (Compound XVIII-5)

5.12 g of the compound obtained in Synthesis Example 8-6 was dissolved in 100 ml of DMF, and then 2. 94 g of WSCI hydrochloride, 1.88 g of DMAP, and 2.572 g of the compound obtained in Synthesis Example 19-1 were added to the solution and the whole was stirred for 16 hours at room temperature. On completion of the reaction, the solvent was distilled off and then chloroform was added, followed by washing with a 1 mol/l hydrochloric acid. Subsequently, the solvent was distilled off. The resulting solid product was washed with a mixture solution of hexane/ethyl acetate = 1/1, and 6.21 g of the above mentioned compound was obtained as a white solid product.
MS(FAB,Pos.):m/z=639[M+1]⁺

### Synthesis Example 29-2: Synthesis of (S)-2-(4-(N-Boc-aminomethyl) benzoylamino)-5-((1-methylpyrrole-2-ylmethy) amino) valerate 1-naphthalenemethylamide (Compound XXI-1)

209.5 mg of the compound obtained in Synthesis Example 29-1 was dissolved in 8 ml of methanol, and then 209.5 mg of 10% Pd-C was added to the solution and the whole was stirred for 140 minutes under a normal pressure in a hydrogen atmosphere. On completion of the reaction, the residue obtained by removing the catalyst and the solvent through celite filtration and distillation respectively was dissolved in 5 ml of anhydrous methanol. Then, 49.7 µl of 1-methyl-2-pyrrole carboxyaldehyde was added, followed by the addition of acetic acid to adjust the pH of the reaction solution to about 5. Subsequently, 61.8 mg of sodium cyanoborohydride was added to the solution and the whole was stirred for 25 hours at room temperature. On completion of the reaction, the solvent was distilled off and the residue was then purified by means of silica gel column chromatography (12 g, chloroform/methanol = 15/1), and 101.8 mg of the above-mentioned compound was obtained as a white solid product.
MS(FAB,Pos.):m/z=598[M+1]⁺
¹H-NMR(500MHz,DMSO-d₆) : δ=1.39 (9H,s), 1.63-1.90 (4H,m), 2.64-2.77 (2H,m), 3.57 (3H,s), 3.92 (2H,m), 4.17 (2H,d,J=6.2Hz), 4.49-4.54 (1H,m), 4.76 (2H,d,J=5.6Hz), 5.94 (1H,m), 6.06 (1H,m), 6.72 (1H,m), 7.31 (2H ,d,J=8.3Hz), 7.44-7.55 (5H,m), 7.83-7.87 (3H,m), 7.93-7.96 (1H,m), 8 .04-8.08 (1H,m), 8.49-8.51 (1H,m), 8.55-8.57 (1H,m).

### Synthesis Example 29-3: Synthesis of (S)-2-(4-((1-methylimidazol -2-ylmethyl)aminomethyl)benzoylamino)-5-((1-methylpyrrol-2-yl methyl) amino) valerate 1-naphthalenemethylamide [Compound No. 30]

100 mg of the compound obtained in Synthesis Example 29-2 was dissolved in 2 ml of methanol, and then 2 ml of a 4 mol/l hydrochloric acid/dioxane was added to the solution, followed by stirring for 2 hours at room temperature. On completion of the reaction, the solvent was distilled off. Subsequently, it was dissolved in 4ml of anhydrous methanol, and then 83.9 µl of triethylamine and 22.1 mg of 1-methyl-2-imidazole carboxyaldehyde were added to the solution and the whole was stirred for 3 hours at room temperature. After the solvent was distilled off, 2 ml of anhydrous methanol was added thereto and the whole was cooled to 0°C. Then, 12.7 mg of sodium borohydride was added to the solution and the whole was stirred for 62 hours while being gradually returned to room temperature. On completion of the reaction, the solvent was distilled off and the residue was purified by means of silica gel column chromatography (10 g, chloroform/methanol = 2/1). After the resultant compound was dissolved in 2 ml of a 1 mol/l hydrochloric acid, water was distilled off. Consequently, 71.7 mg of hydrochloride of the above-mentioned compound was obtained as a light-red solid product.
MS(FAB,Pos.):m/z=592[M+1]⁺
¹H-NMR(500MHz,DMSO-d₆): δ=1.72-1.87(4H,m),2.91(2H,m),3.95(3H,s) , 4.08-4.09 (2H,m), 4.39 (2H,m), 4.53-4.56 (3H,m), 4.76 (2H,d,J=5.7Hz ), 5.98-5.99 (1H,m),6.22-6.24 (1H,m), 6.78-6.79 (1H,m), 7.45-7.49 (2 H,m), 7.53-7.56 (2H,m), 7.71-7.73 (4H,m), 7.83-7.86 (1H,m), 7.93-7.9 5 (1H,m), 7.96-8.01 (2H,m), 8.06-8.08 (1H,m), 8.71-8.73 (2H,m), 9.00 (2H,br).

### Synthesis Example 30: Production of (S)-2-(4-((imidazol-2-yl methyl) aminomethyl) benzoylamino)-5-((1-methylpyrrol-2-yl methyl) amino) valerate 1-naphthalenemethylamide [Compound No. 31]

106.1 mg of the compound obtained in Synthesis Example 29-2 was dissolved in 2 ml of methanol, and then 2 ml of a 4 mol/l hydrochloric acid/dioxane was added to the solution, followed by stirring for 3 hours at room temperature. On completion of the reaction, the solvent was distilled off. Subsequently, it was dissolved in 8 ml of anhydrous methanol, and then 89.0 µl of triethylamine and 25.6 mg of 2-imidazole carboxyaldehyde were added to the solution and the whole was stirred for 2 hours at room temperature. After the solvent was distilled off, 4 ml of anhydrous methanol was added thereto and the whole was cooled to 0°C. Then, 13.4 mg of sodium borohydride was added to the solution and the whole was stirred for 24 hours while being gradually returned to room temperature. On completion of the reaction, the solvent was distilled off and the residue was purified by means of silica gel column chromatography (10 g, chloroform/methanol = 3/1). After the resultant compoundwas dissolved in 2 ml of a 1 mol/l hydrochloric acid, water was distilled off. Consequently, 75.0 mg of hydrochloride of the above-mentioned compound was obtained as a light-red solid product.
MS(FAB,Pos.):m/z=578[M+1]⁺
¹H-NMR(500MHz,DMSO-d₆):δ=1.72-1.91(4H,m),2.91(2H,m),4.07-4.10 (2H,m),4.39(2H,s),4.53-4.57(3H,m),4.75-4.77(2H,m),5.98-5.99(1H ,m),6.22-6.24(1H,m),6.78-6.79(1H,m),7.45-7.48(2H,m),7.53-7.56 (2H,m),7.69-7.73(4H,m),7.83-7.86(1H,m),7.93-7.99(3H,m),8.01-8 .08 (1H,m), 8.71-8.72 (2H,m), 8.98 (2H,brs).

### Synthesis Example 31: Production of (S)-2-(4-((pyrazol-3-yl methyl) aminomethyl) benzoylamino)-5-((1-methylpyrrol-2-yl methyl) amino) valerate 1-naphthalenemethylamide [Compound No. 32]

110.0 mg of the compound obtained in Synthesis Example 29-2 was dissolved in 2 ml of methanol, and then 2 ml of a 4 mol/l hydrochloric acid/dioxane was added to the solution, followed by stirring for 3 hours at room temperature. On completion of the reaction, the solvent was distilled off. Subsequently, it was dissolved in 4 ml of anhydrous methanol and then 92.2 µl of triethylamine and 21. 2 mg of pyrazole-3-carboxyaldehyde were added to the solution and the whole was stirred for 16 hours at room temperature. After the solvent was distilled off, 4 ml of anhydrous methanol was added thereto and the whole was cooled to 0°C. Then, 13.9 mg of sodium borohydride was added to the solution and the whole was stirred for 25 minutes while being gradually returned to room temperature. On completion of the reaction, the solvent was distilled off and the residue was purified by means of silica gel column chromatography (10 g, chloroform/methanol=5/1). After the compound was dissolved in 2 ml of a 1 mol/l hydrochloric acid, water was distilled off. Consequently, 55.6 mg of hydrochloride of the above-mentioned compound was obtained as a light-orange solid product.
MS(FAB,Pos.):m/z=578[M+1]⁺
¹H-NMR(500MHz,DMSO-d₆): δ=1.72-1.91(4H,m),2.91(2H,br),3.64(3H,s ), 4.07-4.22 (6H,m), 4.53-4.57 (1H,m), 4.76-4.77 (2H,m), 5.98-5.99 (1 H,m), 6.22-6.23 (1H,m), 6.45-6.51 (1H,m), 6.78-6.79 (1H,m), 7.45-7.4 9 (2H,m), 7.52-7.57 (2H,m), 7.63-7.65 (2H,m), 7.79-7.80 (1H,m), 7.83-7.86 (1H,m), 7.93-8.00 (3H,m), 8.06-8.08 (1H,m), 8.70-8.83 (2H,m), 9. 00 (2H,br), 9.76 (2H,br).

### Synthesis Example 32: Synthesis of (S)-2-(4-((1-methylbenz imidazol-2-ylmethyl) aminomethyl) benzoylamino)-5-((1-methyl benzimidazol-2-yl) methylamino) valerate 1-naphthalenemethyl amide [Compound No. 33]

100.4 mg of the compound obtained in Synthesis Example 19-2 was dissolved in 1 ml of methanol, and then 1 ml of a 4 mol/l hydrochloric acid/dioxane was added to the solution and the whole was stirred for 3 hours at room temperature. On completion of the reaction, the solvent was distilled off and the residue was then dissolved in methanol, followed by neutralizing with amberlite IRA-410 and distilling the solvent off. The residue was dissolved in 1 ml of anhydrous methanol, and 53.8 mg of 1-methyl-2-formyl benzimidazole was then added to the solution, allowing the reaction thereof for 1.5 hours at room temperature. A reaction solution was concentrated and dried under a reduced pressure, followed by dissolving in 2 ml of methanol again. Subsequently, 25.6 mg of sodium cyanoborohydride was added to the solution and the whole was reacted for 0.5 hour at room temperature. On completion of the reaction, the solvent was concentrated, and the residue was then dissolved in chloroform, followed by washing with distilled water and a saturated salt solution. After the solution was dried with anhydrous sodium sulfate, the solvent was distillated off and the resulting residue was then purified by means of silica gel column chromatography (5 g, chloroform/methanol/water = 7/3/0.5). A 1 mol/l hydrochloric acid was added to the resulting compound, and the mixture was then concentrated and azeotropically co-distilled, and 28.2 mg of hydrochloride of the above-mentioned compound was obtained as a white solid product.
MS(FAB,Pos.):m/z=693[M+H]⁺
¹H-NMR(500MHz,DMSO-d₆):δ=1.87-1.97(4H,m),3.93(3H,s),3.97(3H,s) ,4.46(2H,s),4.55-4.62(1H,m),4.64(2H,s),4.67(2H,s),4.76(2H,d,J =5.6Hz),7.36-7.58(6H,m),7.71-7.79(7H,m),7.93-7.95(1H,m),8.01 (2H,d,J=8.5Hz),8.05-8.08(1H,m),8.73-8.76(2H,m),9.94(2H,brs).

### Synthesis Example 33: Production of (S)-2-(4-((1-methylbenz imidazol-2-ylmethyl) aminomethyl) benzoylamino)-5-((1-methyl pyrrol-2-ylmethyl) amino) valerate 1-naphthalenemethylamide [Compound No. 34]

156.3 mg of the compound obtained in Synthesis Example 29-2 was dissolved in 2 ml of methanol, and then 2 ml of a 4 mol/l hydrochloric acid/dioxane was added to the solution, followed by stirring for 165 minutes at room temperature. On completion of the reaction, the solvent was distilled off. Subsequently, it was dissolved in 4 ml of anhydrous methanol, and then 131.1 µl of triethylamine and 62.8 mg of 1-methyl-2-formylbenzimidazole were added to the solution and the whole was stirred for 12 hours at room temperature. After the solvent was distilled off, subsequently, 4 ml of anhydrous methanol was added thereto and the whole was cooled to 0°C. Then, 19.8 mg of sodium borohydride was added to the solution and the whole was stirred for 30 minutes while being gradually returned to room temperature. On completion of the reaction, the solvent was distilled off and the residue was purified by means of silica gel column chromatography (10 g, chloroform/methanol = 10/1). After the resultant compound was dissolved in 2 ml of a 1 mol/l hydrochloric acid, water was distilled off. Consequently, 44.1mg of hydrochloride of the above-mentioned compound was obtained as a white-red solid product.
MS(FAB,Pos.):m/z=642[M+1]⁺
¹H-NMR(500MHz,DMSO-d₆):δ=1.73-1.91(4H,m),2.09(2H,br),3.64(3H,s ),3.85(3H,s),4.08-4.12(2H,m),4.38-4.43(2H,m),4.47-4.59(3H,m), 4.76-4.77(2H,m),5.98-5.99(1H,m),6.22-6.23(1H,m),6.79-6.80(1H, m),7.29-7.59(6H,m),7.60-7.73(2H,m),7.84-7.86(1H,m),7.94-8.08 (4H,m), 8.70-8.72 (2H,m), 8.96 (2H,br).

### Synthesis Example 34: Production of (S)-2-(4-((thiazol-2-yl methyl) aminomethyl) benzoylamino)-5-((1-methylpyrrol-2-yl methyl) amino) valerate 1-naphthalenemethylamide [Compound No. 35]

161.1 mg of the compound obtained in Synthesis Example 29-2 was dissolved in 2 ml of methanol, and then 2 ml of a 4 mol/l hydrochloric acid/dioxane was added to the solution, followed by stirring for 12 hours at room temperature. On completion of the reaction, the solvent was distilled off. Subsequently, it was dissolved in 4 ml of anhydrous methanol, and then 135.1 µl of triethylamine and 28.1 µl of 2-formylthiazole were added to the solution and the whole was stirred for 2.5 hours at room temperature. After the solvent was distilled off, 4 ml of anhydrous methanol was added thereto and the whole was cooled to 0°C. Then, 20.4 mg of sodium borohydride was added to the solution and the whole was stirred for 20 minutes while being gradually returned to room temperature. On completion of the reaction, the solvent was distilled off and the residue was purified by means of silica gel column chromatography (10 g, chloroform/methanol = 10/1). After the resultant compound was dissolved in 2 ml of a 1 mol/l hydrochloric acid, water was distilled off. Consequently, 81.4 mg of hydrochloride of the above-mentioned compound was obtained as a light-orange solid product.
MS(FAB,Pos.) :m/z=595[M+1]⁺
¹H-NMR(500MHz,DMSO-d₆) : δ=1.72-1.91 (4H,m), 2.91 (2H,br), 3.65 (3H,s ), 4.17-4.32 (2H,m), 4.53-4.57 (3H,m), 4.76-4.77 (2H,m), 5.98-5.99 (1 H,m),6.23-6.24(1H,m),6.78-6.79(1H,m),7.45-7.52(2H,m),7.53-7.5 6 (2H,m), 7.65-7.70 (2H,m), 7.83-7.86 (2H,m), 7.88-7.99 (4H,m), 8.00-8.08 (1H,m), 8.70-8.74 (2H,m), 9.02 (2H,br), 10.14 (2H,br).

### Synthesis Example 35: Production of (S)-2-(4-((1-methylimidazol -2-ylmethyl) aminomethyl) benzoylamino)-5-((imidazol-2-yl methyl) amino) valerate 1-naphthalenemethylamide [Compound No. 36]

### Synthesis Example 35-1: Synthesis of (S)-2-((N-Boc-aminomethyl) benzoylamino)-5-((imidazol-2-ylmethyl) amino)-L-ornithine 1-naphthalenemethylamide (Compound XXI-2)

233.5 mg of the compound obtained in Synthesis Example 29-1 was dissolved in 8 ml of methanol, and then 233.5 mg of 10% Pd-C was added to the solution and the whole was stirred for 140 minutes under a normal pressure in a hydrogen atmosphere. On completion of the reaction, the residue obtained by removing the catalyst and the solvent through celite filtration and distillation respectively was dissolved in 5 ml of anhydrous methanol. Then, 42.2 mg of 2-imidazolecarobxyaldehyde was added to the solution, followed by cooling to 0°C. Subsequently, 27.7 mg of sodium borohydride was added to the solution and the whole was stirred for 30 minutes at 0°C and for 1 hour at room temperature. On completion of the reaction, a solvent was distilled off and the residue was then purified by means of silica gel column chromatography (12 g, chloroform/methanol = 10/1), and 108.1 mg of the above-mentioned compound was obtained as a white solid product.
¹H-NMR(500MHz,DMSO-d₆) : δ=1.39 (9H,s), 1.42-1.56 (2H,m), 1.72-1.82 (2H,m), 3.68 (2H,s), 4.12-4.17 (2H,m), 4.43-4.51 (5H,m), 4.75-4.76 (2H ,m),5.29-5.31(2H,m),7.30-7.31(2H,m),7.44-7.55(5H,m),7.83-7.86 (3H,m),7.92-7.95(1H,m), 8.04-8.06(1H,m),8.48-8.56(2H,m).

### Synthesis Example 35-2: Synthesis of (S)-2-(4-((1-methylimidazol -2-ylmethyl) aminomethyl) benzoylamino)-5-((imidazol-2-yl methyl) amino) valerate 1-naphthalenemethylamide [Compound No. 36]

104.2 mg of the compound obtained in Synthesis Example 35-1 was dissolved in 2 ml of methanol and then 2 ml of a 4 mol/l hydrochloric acid/dioxane was added to the solution, followed by stirring for 2 hours at room temperature. On completion of the reaction, the solvent was distilled off. Subsequently, it was dissolved in 4 ml of anhydrous methanol, and then 89.3 µl of triethylamine and 29.4 mg of 1-methyl-2-imidazole carboxyaldehyde were added to the solution and the whole was stirred for 12 hours at room temperature. After the solvent was distilled off, 4 ml of anhydrous methanol was added thereto and the whole was cooled to 0°C. Then, 13. 5 mgofsodiumborohydride was added to the solution and the whole was stirred for 85 minutes while being gradually returned to room temperature. On completion of the reaction, the solvent was distilled off and the residue was purified by means of silica gel column chromatography (10 g, chloroform/methanol = 1/1). After the resultant compound was dissolved in 2 ml of a 1 mol/l hydrochloric acid, water was distilled off. Consequently, 34.7 mg of hydrochloride of the above-mentioned compound was obtained as a white solid product.
MS(FAB,Pos.):m/z=579[M+1]⁺
¹H-NMR(500MHz,DMSO-d₆):δ=1.74-1.91(4H,m),3.91(3H,s),4.37-4.59 (8H,m),4.73-4.77(2H,m),7.44-7.71(10H,m),7.84-7.86(1H,m),7.94-8 .08(4H,m),8.69-8.72(2H,m).

### Synthesis Example 36: Production of (S)-2-(4-((imidazol-2-yl methyl) aminomethyl) benzoylamino)-5-((1-methylimidazol-2-yl methyl) amino) valerate 1-naphthalenemethylamide [Compound No. 37]

### Synthesis Example 36-1: Synthesis of (S)-2-((N-Boc-aminomethyl) benzoylamino)-5-((1-methylimidazol-2-ylmethyl)amino) valerate 1-naphthalenemethylamide (Compound XXI-3)

255.2 mg of the compound obtained in Synthesis Example 29-1 was dissolved in 10 ml of methanol, and then 170 mg of 10% Pd-C was added to the solution and the whole was stirred for 120 minutes under a normal pressure in a hydrogen atmosphere. On completion of the reaction, the residue obtained by distilling off removing the catalyst and the solvent through celite filtration and distillation respectively was dissolved in 8 ml of anhydrous methanol. Then, 52.8 mg of 1-methyl-2-imidazole carobxyaldehyde was added to the solution, followed by cooling to 0°C. Subsequently, 30.2 mg of sodium borohydride was added to the solution and the whole was stirred for 5 minutes at 0°C and for 45 minutes at room temperature. On completion of the reaction, the solvent was distilled off and the residue was then purified by means of silica gel column chromatography (13 g, chloroform/methanol = 10/1), and 117.4 mg of the above-mentioned compound was obtained as a white solid product.
MS (FAB,Pos.) :m/z=598 [M+1]⁺
¹H-NMR(500MHz,DMSO-d₆) : δ=1.39 (9H,s), 1.73-1.81 (2H,m), 3.57 (3H,s) ,4.16-4.17(2H,m),4.45-4.49(3H,m),4.71-4.79(2H,m),5.22(1H,t,J= 5.6Hz), 6.70 (1H,d,J=1.0Hz), 6.75 (1H,d,J=1.0Hz), 7.00 (1H,d,J=1.0H z),7.06(1H,d,J=1.0Hz),7.29-7.31(2H,m),7.44-7.49(3H,m),7.82-7. 85(3H,m),7.92-7.96(2H,m),8.04-8.07(2H,m),8.46-8.48(1H,m),8.53 -8.56(1H,m).

### Synthesis Example 36-2: Synthesis of (S)-2-(4-((imidazol-2-yl methyl) aminomethyl) benzoylamino)-5-((1-methylimidazol-2-yl methyl) amino) valerate 1-naphthalenemethylamide [Compound No. 37]

114.4 mg of the compound obtained in Synthesis Example 36-1 was dissolved in 2 ml of methanol, and then 2 ml of a 4 mol/l hydrochloric acid/dioxane was added to the solution, followed by stirring for 2 hours at room temperature. On completion of the reaction, the solvent was distilled off. Subsequently, it was dissolved in 4 ml of anhydrous methanol, and then 95.8 µl of triethylamine and 22.0 mg of 2-imidazole carboxyaldehyde were added to the solution and the whole was stirred for 16 hours at room temperature. After the solvent was distilled of f , 4 ml of anhydrous methanol was added thereto and the whole was cooled to 0°C. Then, 14.5 mg of sodium borohydride was added to the solution and the whole was stirred for 4.5 hours while being gradually returned to room temperature. On completion of the reaction, the solvent was distilled off and the residue was purified by means of silica gel column chromatography (10g, chloroform/methanol = 2/1). After the resultant compoundwas dissolved in 2 ml of a 1 mol/l hydrochloric acid, water was distilled off. Consequently, 81.3 mg of hydrochloride of the above-mentioned compound was obtained as a light-yellow solid product.
MS(FAB,Pos.):m/z=579[M+1]⁺
¹H-NMR(500MHz,DMSO-d₆):δ=1.77-1.91(4H,m),3.93(3H,s),4.37-4.38 (2H,m),4.50-4.59(5H,m),4.73-4.77(2H,m),7.44-7.70(10H,m),7.82-7 .88(1H,m),7.92-8.01(3H,m),8.06-8.08(1H,m),8.62-8.75(2H,m),10. 05-10.15 (2H,m).

### Synthesis Example 37: Production of (2S)-2-(4-(N-2-picolylamino methyl) naphthoylamino)-5-(5,6,7,8-tetrahydroquinolin-8-yl amino) valerate 1-naphthalenemethylamide [Compound No. 38]

### Synthesis Example 37-1: Synthesis of N^{α}-4-(N-Boc-aminomethyl) naphthoyl-N(-Cbz-L-ornithine 1-naphthalenemethylamide (Compound XVIII-6)

209.1 mg of the compound obtained in Synthesis Example 8-6 was dissolved in 4 ml of DMF, and then 91.5 mg of WSCI hydrochloride, 54.0 mg of HOBt, and 85.5 mg of the compound obtained in Synthesis Example 25-2 were added to the solution and the whole was stirred for 19 hours at room temperature. On completion of the reaction, the solvent was distilled off and then the residue was dissolved in chloroform. Methanol was added to wash the residue, and then the residue was collected through filtration, and 155.1 mg of the above-mentioned compound was obtained as a white frothy product.
MS(FAB,Pos.):m/z=689[M+1]⁺
¹H-NMR(500MHz,DMSO-d₆):δ=1.41(9H,s),1.48-1.62(2H,m),1.63-1.82 (2H,m),2.99-3.10(2H,m),4.52-4.61(1H,m),4-.62(2H,d,J=5.9Hz),4.81 (2H,d,J=5.9Hz),5.00(2H,s),7.28-7.39(6H,m),7.40(1H,d,J=7.3Hz), 7.45-7.49(1H,m),7.49-7.61(7H,m),7.86.(1H,d,J=8.Hz),7.95-7.98(1 H,m),8.07(1H,d,J=7.1Hz),8.15(1H,d,J=8.1Hz),8.24(1H,d,J=8.1Hz) ,8.59(1H,br),8.67(1H,d,J=7.8Hz).

### Synthesis Example 37-2: Synthesis of N^{α}-4-(N-2-picolylamino methyl) naphthoyl-N^{δ}-Cbz-L-ornithine 1-naphthalenemethylamide (Compound XXIV-1)

149.6 mg of the compound obtained in Synthesis Example 37-1 was dissolved in 1.5 ml of methanol, and then 1.5 ml of a 4 mol/l hydrochloric acid/dioxane solution was added to the solution and the whole was stirred for 2.5 hours at room temperature. On completion of the reaction, it was concentrated and dried under reduced pressure. Then, 70.0 mg of the compound was dissolved in 2.8 ml of methanol, and furthermore 48.0 µl of triethylamine and 12.0 µl of 2-pyridinealdehyde were added to the solution and the whole was stirred for 1 hour at room temperature. On completion of the reaction, the solvent was distilled and the residue was dried under reduced pressure. Subsequently, 6.5 ml of anhydrous methanol was added thereto and the whole was cooled to 0°C. Then, 22.8 mg of sodium borohydride was added to the solution and the whole was stirred for 15 minutes at room temperature. On completion of the reaction, it was concentrated and the residue was then purified by means of silica gel column chromatography (3.5 g, chloroform/methanol 20/1), and 40.4 mg of the above-mentioned compound was obtained as a white frothy product.
MS(FAB,Pos.):m/z=680[M+1]⁺
¹H-NMR(500MHz,DMSO-d₆): δ=1.52-1.58(2H,m),1.70-1.78(2H,m),3.02 (2H,m),3.89(2H,s),4.21(2H,s),4.53-4-.59(1H,m),4.81(2H,d,J=4.9Hz ),4.99(2H,s),7.21-7.40(7H,m),7.44-7.52(3H,m),7.52-7.61(6H,m), 7.78(1H,td,J=7.6,1.7Hz),7.86(1H,d,J=8.1Hz),7.95-7.98(1H,m),8. 09(1H,d,J=7.1Hz),8.21(1H,d,J=8.3Hz),8.23(1H,d,J=8.5Hz),8.47(1 H,d,J=5.6Hz), 8.56 (1H,t,J=5.6Hz), 8.64 (1H,d,J=7.8Hz).

### Synthesis Example 37-3: Synthesis of (2S)-2-(4-(N-2-picolylamino methyl) naphthoylamino)-5-(5,6,7,8-tetrahydroquinolin-8-yl amino) valerate 1-naphthalenemethylamide [Compound No. 38]

32.7 mg of the compound obtained in Synthesis Example 37-2 was dissolved in 1.5 ml of a dioxane/water (= 8/2) solution, and then 32.4 mg of 10% palladium-carbon was added to the solution and the whole was stirred for 24 hours at room temperature in a hydrogen atmosphere. On completion of the reaction, the catalyst was removed through celite filtration, and the solvent was then distilled off under reduced pressure, and 24.9 mg of a crude product was obtained. The product was dissolved in 0.5 ml of methanol, and then 7.9 mg of 5,6,7,8-tetrahydroquinolin-8-one and 4.7 mg of sodium cyanoborohydride were added to the solution.

Subsequently, the pH of the solution was adjusted to about 4 to 5 by the addition of seven drops of acetic acid. Then, the solution was stirred for 15.5 hours at room temperature. On completion of the reaction, the solvent was distilled off and the residue was then purified by means of silica gel column chromatography (5 g, chloroform/methanol = 10/2). After that, a 1mol/l hydrochloric acid was added to the purified residue, and then the solution was concentrated and dried under reduced pressure, and 9.7 mg of the above-mentioned compound was obtained as a white solid product.
MS(FAB,Pos.):m/z=677[M+1]⁺
¹H-NMR(500MHz,DMSO-d₆): δ=1.62-2.01(7H,m),2.30-2.40(1H,m),2.78-2.88(2H,m),2.95-3.03(1H,m),3.04-3.22(1H,m),4.47(3H,m),4.61-4. 70(1H,m),4.79(2H,s),4.83(1H,d,J=15.6,5.6Hz),4.85(1H,dd,J=15.6 , 5.6Hz), 7.37-7.42 (1H,m), 7.47-7.51 (2H,m), 7.52-7.64 (5H,m), 7.67-7.73 (3H,m), 7.80 (1H,d,J=7.1Hz), 7.88 (1H,d,J=8.1Hz), 7.93 (1H,td,J =7.6,1.7Hz), 8.07-8.13 (1H,m), 8.26-8.33 (2H,m), 8.49 (1H,d,J=4.6Hz ), 8.71 (1H,d,J=4.8Hz), 8.76 (1H,brs), 8.87 (1H,d,J=7.8Hz), 9.10 (2H, br),9.80(2H,br).

### Synthesis Example 38: Production of (2S)-2-(4-((N-imidazol-2-ylmethyl) aminomethyl) benzoylamino)-5-(5,6,7,8-tetrahydro quinolin-8-ylamino) valerate 1-naphthalenemethylamide [Compound No. 39]

### Synthesis Example 38-1: Synthesis of N^{α}-4-(aminomethyl) benzoylamino-N^{δ}-Cbz-L-ornithine 1-naphthalenemethylamide (Compound XXIII-1)

351.2 mg of the compound obtained in Synthesis Example 29-1 was dissolved in 5 ml of chloroform, and 5 ml of trifluoroacetic acid was added. After 15 minutes, the reaction solution was concentrated. The residue thus obtained was purified by means of silica gel column chromatography (10 g, chloroform/methanol = 5/1), and 363.7 mg of the above-mentioned compound was obtained as a light-brown solid product.
MS(FAB,Pos.):m/z=539[M+1]⁺
¹H-NMR(500MHz,CDCl₃) : δ=1.42-1.56 (2H,m), 1.70-1.84 (2H,m),2.96-3. 06 (2H,m), 4.08-4.13 (2H,m), 4.48-4.53 (1H,m), 4.74-4.79 (2H,m), 4.98 (2H,s), 7.26-7.37 (6H,m), 7.45-7.50 (2H,m), 7.51-7.58 (4H,m), 7.84-7 .88 (1H,m), 7.92-7.98 (3H,m), 8.05-8.09 (1H,m), 8.20 (2H,br), 8.52-8. 59 (2H,m) .

### Synthesis Example 38-2: Synthesis of N^{α}-(4-(N-(imidazol-2-yl methyl) aminomethyl) benzoyl)-N^{δ}-Cbz-L-ornithine 1-naphthalene methylamide (Compound XXIV-2)

107.2 mg of the compound obtained in Synthesis Example 38-1 was dissolved in 5 ml of methanol, and then 28 µl of triethylamine and 19 mg of 2-imidazole carboxyaldehyde were added to the solution. After the mixture was stirred for 2 days, 23 mg of sodium borohydride was added thereto. After 20 minutes, a small amount of silica gel was added to the reaction solution and the whole was concentrated. The residue thus obtained was purified by means of silica gel column chromatography (5 g, chloroform/methanol = 5/1), and 72.2 mg of the above-mentioned compound was obtained as a light-yellow solid product.
MS(FAB,Pos.):m/z=619 [M+1]⁺
¹H-NMR(500MHz,DMSO-d₆) : δ=1.41-1.58 (2H,m), 1.70-1.84 (2H,m), 2.97-3.05 (2H,m), 4.48-4.53 (1H,m), 4.75 (2H,d,J=5.6Hz), 4.98 (2H,s), 6.79 (1H,s), 7.02 (1H,s), 7.26-7.38 (6H,m), 7.42-7.48 (4H,m), 7.51-7.57 (2 H,m), 7.82-7.90 (3H,m), 7.93-7.96 (1H,m), 8.04-8.08 (1H,m), 8.44 (1H, d,J=8.1Hz),8.51(1H,t, J=5.7Hz).

### Synthesis Example 38-3: Synthesis of (2S)-2-(4-((N-imidazol-2-yl methyl) aminomethyl) benzoylamino)-5-(5,6,7,8-tetrahydro quinolin-8-ylamino) valerate 1-naphthalenemethylamide [Compound No. 39]

71.1 mg of the compound obtained in Synthesis Example 38-2 was dissolved in 2.8 ml of dioxane and 0.7 ml of water, and then 35 mg of 5% Pd-C was added to the solution. The reaction solution was hydrogen-substituted, and after 22 hours, the reaction solution was filtrated through a glass filter G4, followed by concentrating filtrate. The resulting residue was dissolved in 3.2 ml of methanol, and then 21 mg of 5,6,7,8-tetrahydroquinolin-8-one and 28 µl of triethylamine were added to the solution. After 19 hours, 15 mg of sodium borohydride was added to the reaction solution. After 15 minutes, a small amount of silica gel was added to the reaction solution and the mixture was then concentrated. The residue thus obtained was purified by means of silica gel column chromatography (2.5 g, chloroform/methanol/water = 7/3/0.5), and then the resulting residue was prepared as a methanol solution, followed by the addition of a small amount of active carbon. Subsequently, the active carbon was separated by filtration and the filtrate was then concentrated. A 1 mol/l hydrochloric acid was added to the yellow syrup thus obtained for concentration, and azeotropically distilled with methanol, followed by drying under reduced pressure. Consequently, 21.7 mg of hydrochloride of the above-mentioned compound was obtained as a yellow solid product.
MS(FAB,Pos.):m/z=616[M+1]⁺
¹H-NMR(500MHz,CDCl₃):δ=1.68-2.03(5H,m),2.29-2.36(1H,m),2.50-2. 63(2H,m),2.92-3.17(2H,m),4.36-4.47(3H,m),4.48-4.60(3H,m),4.76 (2H,d,J=5.4Hz),7.34-7.40(1H,m),7.42-7.48(2H,m),7.50-7.56(3H,m ),7.65-7.75 (5H,m),7.82-7.89(1H,m),7.93-8.08(4H,m),8.46(1H,t,J = 4.6Hz),8.59-8.72 (2H,m),9.06(1H,br)

### Synthesis Example 39: Production of (S)-2-(4-((N-imidazol-2-yl methyl) aminomethyl) naphthoylamino)-5-(2-picolylamino)valerate 1-naphthalenemethylamide [Compound No. 40]

### Synthesis Example 39-1: Synthesis of N^{α}-4-(aminomethyl) naphthoyl amino-N^{δ}-Cbz-L-ornithine 1-naphthalenemethylamide (Compound XXIII-2)

291.0 mg of the compound obtained in Synthesis Example 37-1 was dissolved in 4.4 ml of chloroform, and 4.4 ml of trifluoroacetic acid was added to the solution. After 1 hour, the reaction solution was concentrated and azeotropically distilled with methanol, followed by drying under reduced pressure. The residue thus obtained was purified by means of silica gel column chromatography (10 g, chloroform/methanol = 10/1), and 306.2 mg of the above-mentioned compound was obtained as a white solid product.
MS(FAB,Pos.):m/z=589[M+1]⁺
¹H-NMR(500MHz,CDCl₃):δ=1-49-1.64 (2H,m),1.67-1.83(2H,m),3.00-3. 10(2H,m),4.57-4.63(3H,m),4.79-4.86(2H,m),5.00(2H,s),7.28-7.39 (5H,m),7.48(1H,dd,J=7.1,8.1Hz),7.52-7.71(6H,m),7.87(1H,d,J=8. 1Hz),7.95-8.00(1H,m),8.08-8.13(1H,m),8.18(1H,d,J=8.5Hz),8.27 (1H,d,J=8.5Hz),8.30-8.40(2H,br),8.62(1H,t,J=5.8Hz),8.77(1H,d,J =7.8Hz).

### Synthesis Example 39-2: Synthesis of N^{α}-4-(imidazol-2-ylmethyl aminomethyl) naphthoylamino-N^{δ}- Cbz-L-ornithine 1-naphthalene methylamide (Compound XXIV-3)

304.0 mg of the compound obtained in Synthesis Example 39-1 was dissolved in 9 ml of methanol, and then 55 mg of 2-imidazolecarboxyaldehyde was added to the solution. After the solution was stirred for 3 days, 59 mg of sodium borohydride was added thereto. After 1 hour, a small amount of silica gel was added to the reaction solution to concentrate. The residue thus obtained was roughly purified by means of silica gel column chromatography (10 g, chloroform/methanol = 10/1), and 204.4 mg of the above-mentioned compound was obtained as a light-yellow solid product.

### Synthesis Example 39-3: Synthesis of N^{α}-4-(imidazol-2-ylmethyl aminomethyl) naphthoylamino-L-ornithine 1-naphthalenemethyl amide (Compound XXV-1)

A mixture solution of 1.75 ml of trifluoroacetic acid, 0. 49 ml of thioanisole, and 0.44 ml of m-cresol was added to 70.0 mg of the compound obtained in Synthesis Example 39-2. After 30 minutes, the reaction solution was concentrated, followed by the addition of water. Then, the solution was made acidic with a small amount of hydrochloric acid. The aqueous solution was washed with chloroform and then a water layer was concentrated, and 112.5 mg of the above-mentioned compound was obtained as a white solid product.

### Synthesis Example 39-4: Synthesis of (S)-2-(4-((N-imidazol-2-yl methyl) aminomethyl) naphthoylamino)-5-(2-picolylamino) valerate 1-naphthalenemethylamide [Compound No. 40]

56 mg of the compound obtained in Synthesis Example 39-3 was dissolved in 1.4 ml of methanol, and then 7 µl of pyridine-2-aldehyde and 11 µl of triethylamine were added to the solution. After 17.5 hours, 6 mg of sodium borohydride was added to the reaction solution and the whole was stirred for 2 hours. The reaction solution was concentrated by the addition of a small amount of silica gel. The residue thus obtained was purified by means of silica gel column chromatography (1 g, chloroform/methanol /water = 7/3/0.5). Then, a 1 mol/l hydrochloric acid was added to the above-mentioned compound thus obtained, followed by concentrating and drying so as to be solidified. Consequently, 8.3 mg of hydrochloride of the above-mentioned compound was obtained as a light-brown solid product.
MS(FAB,Pos.):m/z=626[M+1]⁺
¹H-NMR(500MHz,CDCl₃): δ=1.78-1.97(4H,m),2.47-2.60(4H,m),3.00-3. 12 (2H,m), 4.31 (2H,t,J=5.6Hz),), 4.57-4.67 (3H,m), 4.78-4.90 (3H,m) , 7.44-7.78 (12H,m), 7.74-8.00 (4H,m), 8.10-8.14 (1H,m), 8.28 (1H,d,J =9.3Hz), 8.33 (1H,d,J=8.5Hz), 8.64 (1H,d,J=4.9Hz), 8.73 (1H,t,J=5.6 Hz), 8.88 (1H,d,J=8.1Hz), 9.29 (2H,br).

### Synthesis Example 40: Production of (2S)-2-(4-((N-imidazol-2-yl methyl) aminomethyl) naphthoylamino)-5-(5,6,7,8-tetrahydro quinolin-8-ylamino) valerate 1-naphthalenemethylamide [Compound No. 41]

13.7 mg of the compound obtained in Synthesis Example 39-4 was dissolved in 0.69 ml of methanol, and then 5.7 mg of 5,6,7,8-tetrahydroquinolin-8-one, 69µl of acetic acid, and 4.8 mg of sodium cyanoborohydride were added to the solution. After 2 days, the reaction solution was concentrated. The residue thus obtained was purified by means of silica gel column chromatography (0.5 g, chloroform/methanol/water = 7/3/0.5). Subsequently, a 1 mol/l hydrochloric acid was added to the resulting residue, followed by concentrating and drying so as to be solidified. Consequently, 8.2 mg of the above-mentioned compound was obtained as a white solid product.
MS(FAB,Pos.):m/z=666[M+1]⁺
¹H-NMR(500MHz,DMSO-d₆):δ=1.74-2.08(8H,m),2.79-2.88(2H,m),2.94-3.19(2H,m),4.43-4.5,0(1H,m),4.57-4.69(3H,m),4.79-4.90(4H,m),7. 37-7.77(12H,m),7.79-7.91(2H,m),7.96-8.00(1H,m),8.09-8.13(1H,m ),8.28-8.34(2H,m),8.49(1H,d,J=4.6Hz),8.70-8.77(1H,m),8.87(1H, d,J=7.1Hz), 9.08(2H,brs).

### Synthesis Example 41: Production of (S)-2-((4-quanidinomethyl benzoyl) amino)-5-(N-2-picolylamino) valerate 1-naphthalene methylamide [Compound No. 42]

### Synthesis Example 41-1: Synthesis of 4-(N-Cbz aminomethyl) benzoic acid (Compound XVII-4)

5 mg of commercially available 4-aminomethyl benzoic acid was dissolved in 33 ml of a 1 mol/l sodiumhydroxide aqueous solution. Under stirring at room temperature, 5.2 ml of benzylchloroformate, and 40 ml of a 1 mol/l sodium hydroxide aqueous solution were gradually added to the solution at the same time. After 3 hours, a 1 mol/l hydrochloric acid was added to the reaction solution to adjust the pH thereof to pH = 3. A precipitated product was filtrated through a glass filter G4. Then, filtrate was washed with water, and with hexane, followed by drying under reduced pressure. Consequently, 8.162 g of the above-mentioned compound was obtained as a white solid product.
¹H-NMR(500MHz,DMSO-d₆) :δ=4.26 (2H,d,J=6.3Hz), 5.05 (2H,s), 7.30-7. 40 (7H,m), 7.88 (2H,d,J=8.3Hz), 7.91 (1H,t,J=6.3Hz).

### Synthesis Example 41-2: Synthesis of N^{α}-(4-(N-Cbz) aminomethyl benzoyl)-L-ornithine 1-naphthalenemethylamide (Compound XX-1)

350 mg of the compound obtained in Synthesis Example 23-2 was dissolved in 7 ml of DMF, and then 265.0 g of WSCI hydrochloride, 169 mg of DMAP, and 276.0 mg of the compound obtained in Synthesis Example 41-1 were added to the solution and the whole was stirred for 24 hours at room temperature. On completion of the reaction, the solvent was distilled off and the residue was then dissolved in chloroform, followed by washing with a 1 mol/l hydrochloric acid and a saturated sodium hydrogencarbonate solution, and drying with sodium hydrogensulfate. The solvent was distilled off and the residue was then purified by means of silica gel column chromatography (20 g, chloroform/methanol = 10/1). 130 mg of the purified product was dissolved in 2.6 ml of methanol, and then 2.6 ml of a 4 mol/l hydrochloric acid/dioxane solution was added to the solution and the whole was stirred for 70 minutes at room temperature. On completion of the reaction, the solvent was distilled off and the residue was dried under vacuum, and 147 mg of hydrochloride of the above-mentioned compound was obtained as a white solid product.

### Synthesis Example 41-3: Synthesis of (S)-2-((4-(N-Cbz) amino methylbenzoyl) amino)-5-(2-picolylamino) valerate 1-naphthalene methylamide (Compound XXI-4-1)

0.15 g of the compound obtained in Synthesis Example 41-2 was dissolved in 3.0 ml of methanol, and then 0.02 g of 2-pyridinealdehide, 0.04 g of sodium cyanoborohydride, and 0.02 g of triethylamine were added to the solution. Liquidity of the reaction solution was adjusted to about pH = 5 using acetic acid, and then the solution was stirred for 48 hours at room temperature, followed by concentrating and drying the reaction solution. It was then purified by means of silica gel column chromatography (15 g, chloroform/methanol = 10/1), and 0.05 gof the above-mentioned compound was obtained as a colorless oily product.
MS(FAB,Pos.):m/z=630[M+1]⁺
¹H-NMR(500Mz,DMSO-d₆) : δ=1.52-1.62 (2H,m), 1.78-1.85 (2H,m), 2.64 (2 H,m), 3.90 (2H,brs), 4.26 (2H,d), 4.49 (1H,m), 4.75 (2H,m), 5.05 (2H,s) , 7.27 (2H,m), 7.33 (2H,d), 7.36 (4H,m), 7.38 (1H,m), 7.46 (2H,m), 7.53 (2H,m), 7.75 (1H,m), 7.84 (3H,m), 8.05 (1H,m), 8.50 (1H,m), 8.53 (2H,m).

### Synthesis Example 41-4: Synthesis of (S)-2-((4-(N-Cbz) amino methylbenzoyl) amino)-5-(N-Boc-2-picolylaminomethyl) valerate 1-naphthalenemethylamide (Compound XXI-4-2)

0.05 g of the compound obtained in Synthesis Example 41-3 was dissolved in 1.0 ml of DMF and 0.01 g of triethylamine, followed by dropping at room temperature and stirring for 1.5 hours. The reaction solution was concentrated and dried, and then chloroform was added to the residue, followed by washing with a saturated ammonium chloride aqueous solution. An organic layer was concentrated and dried, and was then purified by means of column chromatography (6 g, chloroform/methanol = 30/1), and 0.04 g of the above-mentioned compound was obtained as a colorless oily product.
MS(FAB,Pos.):m/z=730[M+1]⁺
¹H-NMR(500Mz,DMSO-d₆) : δ=1.24,1.35 (9H,2s), 1.50 (1H,m), 1.59 (1H,m) ,1.73(2H,m),3.21(1H,m),3.32(1H,brs),4.25(2H,d),4.36,4.40(2H,2 s),4.48(1H,m),4.73(2H,d),5.05(2H,s),7.15(1H,d),7.23(1H,m),7.3 3(3H,m),7.37(4H,m),7.45(2H,m),7.53(2H,m),7.73(1H,m),7.84(3H,m ),7.93(2H,m),8.47(2H,m),8.53(1H,m).

### Synthesis Example 41-5: Synthesis of (S)-2-((4-aminomethyl benzoyl) amino)-5-(N-Boc-2-picolylaminomethyl) valerate 1-naphthalenemethylamide (Compound XXII-1)

0.02 g of the compound obtained in Synthesis Example 41-4 was dissolved in 0.5 ml of methanol, and then 0.02 g of 10% Pd-C was added to the solution in a nitrogen atmosphere and the whole was stirred for 2 hours at room temperature in a hydrogen atmosphere. On completion of the reaction, the catalyst was separated by filtration, and then the solvent was concentrated and dried under vacuum, and 0.01 g of the above-mentioned compound was obtained as a colorless oily product.

### Synthesis Example 41-6: Synthesis of (S)-2-((4-quanidinomethyl benzoyl) amino)-5-(2-picolylaminomethyl) valerate 1-naphthalene methylamide [Compound No. 42]

0.01 g of the compound obtained in Synthesis Example 41-5 was dissolved in 0.3 ml of DMF, and then 0.01 g of dimethyl pyrazolecarboxyamidine nitrate was added to the solution. Then, the reaction solution was adjusted to pH = 8 by triethylamine and was stirred for 3 days. After the reaction solution was concentrated, chloroform was added thereto and then it was washed with a saturated sodium hydrogencarbonate aqueous solution. Furthermore, an organic layer was concentrated and dried under reduced pressure, and was then purified by means of column chromatography (5 g, chloroform/methanol/water = 7/3/0.5), and 0.01 g of a white frothy product was obtained. The product was dissolved in 0.3 ml of methanol, and then 0.3 ml of a 4 mol/l hydrochloric acid/dioxane was dropped to the solution, followed by stirring the solution for 3 hours. The reaction liquid was concentrated and dried under reduced pressure and 0.01 g of a crude reaction product was obtained. Then, it was purified by means of column chromatography (0.5g, chloroform/methanol/water=7/3/0.5). Subsequently, a 1 mol/l hydrochloric acid was added to the product, and then the product was concentrated, and 0.01 g of hydrochloride of the above-mentioned compound was obtained as a white frothy product.
MS(FAB,Pos.):m/z=538[M+1]⁺
¹H-NMR(500Mz,DMSO-d₆) : δ=1.75-1.87 (4H,m), 2.98 (2H,brs), 4.29 (2H,s ), 4.46 (2H,d), 4.55 (1H,m), 4.76 (2H,d), 7.40-7.45 (5H,m), 7.50-7.59 (3H,m), 7.84-7.90 (2H,m), 7.93-7.97 (3H,m), 8.06 (1H,m), 8.61 (1H,m), 8 .66(1H,brs),9.22 (2H,brs).

### Synthesis Example 42: Production of N^{α}-(4-(N-2-picolylamino methyl) benzoyl)-L-arqinine 1-naphthalenemethylamide [Compound No. 43]

### Synthesis Example 42-1: Synthesis of N^{α}-Fmoc-N^{G}-Pmc-L-arginine 1-naphthalenemethylamide (Compound XXVII-1)

301.4 mg of commercially available N^{α}-Fmoc-N^{G}-Pmc-L-arginine was dissolved in 6.0ml of DMF, and then 132.1 mg of WSCI hydrochloride and 90.3 mg of HOBt were added and dissolved in the solution. Then, 98.0 µl of 1-naphthalenemethylamine was added to the solution and the whole was stirred for 20 hours at room temperature. On completion of the reaction, the solvent was distilled off. The residue was purified by means of silica gel column chromatography (30 g, chloroform/methanol = 20/1), and 259.7 mg of the above-mentioned compound was obtained as a white frothy product.
MS (FAB,Pos.) :m/z=788[M+1]⁺
¹H-NMR(500MHz,CDCl₃) : δ=1.24 (6H,s), 1.4-1.5 (2H,m), 1.6-1.7 (3H,m), 1.75-1.84 (1H,m), 2.05 (3H,s), 2.44 (3H,s), 2.47 (3H,s), 2.50 (2H,t,J= 6.8Hz), 3.0-3.2 (2H,m), 4.00 (1H,t,J=7.1Hz), 4.21 (2H,d,J=7.1Hz), 4. 2-4.3 (1H,m), 4.69 (1H,dd,J=13.4,5.3Hz), 4.81 (1H,dd,J=13.4,5.3Hz) , 5.9-6.2 (3H,brs), 6.04 (1H,brs), 7.15-7.5 (11H,m), 7.65 (1H,d,J=8.1 Hz),7.70(2H,d,J=7.6Hz),7.91(1H,d,J=7.8Hz).

### Synthesis Example 42-2: Synthesis of N;N^{α}- (4-(N-Boc-N-2-picolyl aminomethyl) benzoyl)-N^{G}-Pmc-L-arginine 1-naphthalenemethyl amide (Compound XXIX-1)

200 mg of the compound obtained in Synthesis Example 42-1 was dissolved in 4.0 ml of DMF, and then 0.4 ml of diethylamine was added to the solution and the whole was stirred for 30 minutes at room temperature. On completion of the reaction, the solvent was distilled off and the residue was dried by a vacuum pump. It was dissolved in 4 ml of DMF, and then 86.3 mg of WSCI, 51.5 mg of HOBt, and 131.2 mg of the compound obtained in Synthesis Example 1-2 were added to the solution and the whole was stirred for 3.5 hours at room temperature. On completion of the reaction, the solvent was distilled off. The residue was purified by means of silica gel column chromatography (5g, chloroform/methanol = 25/1), and a 127.1 mg of the above-mentioned compound was obtained as white frothy product.
MS(FAB,Pos.):m/z=904[M+1]⁺
¹H-NMR(500MHz,CDCl₃): δ=1.28(6H,s),1.43 and 1.45(9H,brs),1.4-1.5 (2H,m), 1.77 (2H,t,J=6.8Hz), 1.8-1.95 (2H,m), 2.06 (3H,s), 2.46 (3H,s ), 2.47 (3H,s), 2.56 (2H,t,J=6.8Hz), 3.1-3.2 (1H,m), 3.3-3.4 (1H,m), 4 .44(1H,brs), 4.48(1H,brs), 4.56 (2H,brs), 4.70(1H,dd,J=15.4,5.8Hz ),4.88(1H,dd,J=15.4,5.8Hz),5.9-6.2(3H,brs),7.1-7.35(6H,m),7.3 5-7.45(4H,m),7.64(1H,td,J=7.6,1.7Hz),7.65-7.75(3H,m),7.8(1H,m ),7.95(1H,d,J=5.6Hz),8.52(1H,brs).

### Synthesis Example 42-3: Synthesis of N^{α}(4-(N-2-picolylamino methyl) benzoyl) L-arginine 1-naphthalenemethylamide [Compound No. 43]

90.2 mg of the compound obtained in Synthesis Example 42-2 was dissolved in 0.45 ml of chloroform, and 0.45 ml of trifluoroacetic acid was added to the solution and the whole was stirred for all overnight at room temperature. On completion of the reaction, the solvent was distilled off and the residue was then purified by means of silica gel column chromatography (10 g, chloroform/methanol/water = 7/3/0.5) . Subsequently, a 1 mol/l hydrochloric acid aqueous solution was added to the purified product and the whole was azeotropically co-distilled, and 16.4 mg of hydrochloride of the above-mentioned compound was obtained as a white solid product.
MS(FAB,Pos.):m/z=538[M+1]⁺
¹H-NMR(500MHz,DMSO-d₆) : δ=1.4-1.6 (2H,m), 1.7-1.8 (1H,m), 1.8-1.9 (1 H,m), 3.1-3.2 (2H,m), 4.31 (2H,s), 4.54 (2H,dd,J=14.0,5.6Hz), 4.77 (2 H,d,J=5.6Hz), 7.4-7.7 (11H,m), 7.8-8.1 (7H,m), 8.4-8.55 (3H,m), 9.5-9.7 (2H,brs).

### Synthesis Example 43: Production of N^{α}(4-(N-2-picolylamino methyl) naphthoyl)-L-arginine 1-naphthalenemethylamide [Compound No. 44]

### Synthesis Example 43-1: Synthesis of methyl 4-(N-Boc-N-2-picolyl aminomethyl) -1-naphthalenecarboxylate (Compound VI-8)

1.500 g of the compound obtained in Synthesis Example 17-3 was dissolved in 30 ml of DMF, and then 750.9 mg of potassium carbonate and 1.63 ml of 2-picolylamine were added to the solution and the whole was stirred for 12 hours at room temperature. On completion of the reaction, the solvent was concentrated and dissolved in chloroform, followed by washing with distilled water. It was dried with anhydrous sodium sulfate, and the solvent was then distilled off. Subsequently, it was dried under reduced pressure, and 1.87 g of a crude product was obtained as an orange oily product. This product was dissolved in 30 ml of DMF, and then 1.5 ml of triethylamine and 1.85 ml (8.06 mmol) of di-t-butyldicarbonate were added to the solution and the whole was stirred for 22 hours at room temperature. On completion of the reaction, the solution was concentrated and the residue was then purified by means of silica gel column chromatography (100 g, hexane/ethyl acetate = 3/2), and 1. 60 g of the above-mentioned compound was obtained as a yellow viscous oily product.
MS(FAB,Pos.):m/z=407[M+1]⁺
¹H-NMR(500MHz,CDCl₃) : δ=1.45(9H,s) ,4.00 (3H,s) ,4.43 and 4.61(2H, 2s),5.04 and 5.11(2H,2s),7.10-7.33(2H,m), 7.34-7.40(1H,m),7.52-7.65(3H,m),8.00-8.23(2H,m),8.51(1H,d,J=4.4Hz),8.91-8.96(1H,m).

### Synthesis Example 43-2: Synthesis of 4-(N-Boc-N-2-picolylamino methyl)-1-naphthalene carboxylic acid (Compound VII-11)

1.60 g of the compound obtained in Synthesis Example 43-1 was dissolved in 16 ml of THF and 16 ml of methanol, and then 16 ml of a 1 mol/l sodium hydroxide aqueous solution was added to the solution and the whole was stirred for 16 hours at room temperature. On completion of the reaction, the solvent was distilled off and the residue was dissolved in distilled water, followed by precipitating under acidic conditions with a 1 mol/l hydrochloric acid. A precipitated solid product was collected through filtration and dried, and 1.3537 g of the above-mentioned compound was obtained as a white solid product.
MS(FAB,Pos.):m/z=393[M+1]⁺
¹H-NMR(500MHz,DMSO-d₆): δ=1.33 and 1.37(9H,2s) 4.41 and 4.51(2H, 2s),5.02 and 5.07 (2H,2s), 7.19-7.28 (2H,m), 7.36-7.45 (1H,m), 7.62 (1H,td,J=6.9,1.4Hz), 7.67 (1H,td,J=6.9,1.4Hz), 7.75 (1H,td,J=7.6, 1.8Hz),8.09-8.23(2H,m),8.51(1H,brs),8.91(1H,d,J=7.8Hz).

### Synthesis Example 43-3: Synthesis of N^{α}-(4-(N-Boc-N-2-picolyl aminomethyl) naphthoyl)-N^{G}-Pmc-L-arginine 1-naphthalenemethyl amide (Compound XXIX-2)

253.4 mg of the compound obtained in Synthesis Example 42-1 was dissolved in 5 ml of DMF, and then 0.5 ml of diethylamine was added to the solution and the whole was stirred for 4 hours at room temperature. On completion of the reaction, the solvent was distilled off and the residue was dried under vacuum. It was dissolved in 5 ml of DMF, and then 91.8 mg of WSCI hydrochloride, 54.1 mg of HOBt, and 141.2 mg of the compound obtained in Synthesis Example 43-2 were added to the solution and the whole was stirred for 17 hours at room temperature. On completion of the reaction, the solvent was distilled off and the residue was dissolved in chloroform, followed by washing with a 1 mol/l hydrochloric acid, a 1 mol/l sodium hydroxide aqueous solution, and a saturated salt solution, and further washing with anhydroussodiumsulfate. After the solution was concentrated under reduced pressure, the residue was purified by means of silica gel column chromatography (15 g, chloroform/methanol = 30/1) , and 252.5 mg of the above-mentioned compound was obtained as a yellow viscous oily product.
MS(FAB,Pos.):m/z=954[M+1]⁺
¹H-NMR(500MHz,CDCl₃) : δ=1.28 (6H,s), 1.44 and 1.49(9H,2s),1.52-1.65(2H,m), 1.70-1.82(3H,m), 1.83-1.98(1H,m), 2.05(3H,s), 2.44 and 2.45(6H,2s), 2.54(2H,t,J=6.6Hz), 3.10-3.33(2H,m),4.37 and 4.51(2H, 2s),4.69-4.91(3H,m),4.96 and 5.00(2H,2s),6.14(3H,br),7.08-7.16 (2H,m), 7.20-7.35 (3H,m), 7.37-7.57 (6H,m), 7.58-7.61 (1H,m), 7.69 (1 H,d,J=8.1Hz), 7.77-7.82 (1H,m), 7.97 (1H,br), 8.10-8.21 (2H,m), 8.49 (1H,br).

### Synthesis Example 43-4: Synthesis of N^{α}-(4-(N-2-picolylamino methyl) naphthoyl)-L-arginine 1-naphthalenemethylamide [Compound No. 44]

119.7 mg of the compound obtained in Synthesis Example 43-3 was dissolved in 1.2 ml of chloroform and the solution was cooled to 0°C, and then 1.2 ml of trifluoroacetic acid was added to the solution and the whole was stirred 4 hours at room temperature. On completion of the reaction, the solvent was distilled off and the residue was dried by a vacuum pump. The residue was purified by means of silica gel column chromatography (5 g, chloroform/methanol/water = 7/3/0.5) and a fraction was concentrated. Then, it was dissolved in a 1 mol/l hydrochloric acid and concentrated. After the solution was azeotropically distilled with water, a solid product was washed with ether, and 25.5 mg of the above-mentioned compound was obtained as a white solid product.
MS(FAB,Pos.):m/z=588[M+1]⁺
¹H-NMR(500MHz,DMSO-d₆) : δ=1.53-1.70 (2H,m), 1.70-1.80 (1H,m), 1.80-1.92 (1H,m), 3.09-3.20 (2H,m), 4.47 (2H,s), 4.55-4.70 (1H,m), 4.78 (2H ,s), 4.80-4.95 (2H,m), 6. 8 (1H,br), 7.40 (2H,br), 7.46-7.50 (2H,m), 7. 54-7.64 (5H,m), 7.67-7.72 (2H,m), 7.80 (2H,d,J=7.3Hz), 7.87 (1H,d,J= 8.3Hz), 7.93 (1H,td,J=7.8,1.7Hz), 7.96-7.99 (1H,m), 8.11-8.13 (1H,m ), 8.26 (1H,d,J=8.5Hz), 8.30 (1H,d,J=8.5Hz), 8.71 (1H,d,J=4.9Hz), 8. 74 (1H,t,J=5.9Hz), 8.85 (1H,d,J=7.8Hz), 9.84(2H,br).

### Synthesis Example 44: Production of N^{α}(4-((N-imidazol-2-yl methyl) aminomethyl) naphthoyl)-L-arginine 1-naphthalenemethyl amide [Compound No. 45]

### Synthesis Example 44-1: Synthesis of N^{α}-(4-((N-Boc-N-imidazol -2-ylmethyl) aminomethyl) naphthoyl)-N^{G}-Pmc-L-arginine 1-naphthalenemethylamide (Compound XXIX-3)

110.0 mg of the compound obtained in Synthesis Example 42-1 was dissolved in 2.2 ml of DMF, and then 0.22 ml of diethylamine was added to the solution and the whole was stirred for 2 hours at room temperature. On completion of the reaction, the solvent was distilled off and the residue was dried under vacuum. The resultant was dissolved in 2 ml of DMF, and then 40.0 mg of WSCI hydrochloride, 22.5 mg of HOBt, and 53.3 mg of the compound obtained in Synthesis Example 17-4 were added to the solution and the whole was stirred for 17 hours at room temperature. On completion of the reaction, the solvent was distilled off and the residue was dissolved in chloroform, followed by washing with a 1 mol/l hydrochloric acid, a 1 mol/l sodium hydroxide aqueous solution, and a saturated salt solution, and further washing with anhydrous sodiumsulfate. After the resultant was concentrated under reduced pressure, the residue was purified by means of silica gel column chromatography (7 g, chloroform/methanol = 25/1), and 59.7 mg of the above-mentioned compound was obtained as a yellow viscous oily product.
MS(FAB,Pos.):m/z=943[M+1]⁺
¹H-NMR(500MHz,CDCl₃):δ=1.28(6H,s),1.49(9H,s),1.50-1.70(2H,m), 1.76(2H,t,J=6.8Hz),1.70-1.81(1H,m),1.83-1.97(1H,m),2.06(3H,s), 2.45 and 2.46(6H,2s),2.55(2H,t,J=6.6Hz),3.08-3.30(2H,m),4.25 (2H,s),4.70-4.80(2H,m),4.81-4.95(3H,m),6.17(3H,br),6.87(2H,s) ,7.18(1H,d,J=7.3Hz),7.31(1H,tJ=7.8Hz),7.36-7.51(7H,m),7.65-7. 78(1H,m),7.71(1H,d,J=8.1Hz),7.80-7.83(1H,m),7.93-8.00(1H,m), 8.13 (1H,d,J=8.5Hz).

### Synthesis Example 44-2: Synthesis of N^{α}-(4-((N-imidazol-2-yl methyl) aminomethyl) naphthoyl)-L-arginine 1-naphthalenemethyl amide [Compound No. 45]

53.7 mg of the compound obtained in Synthesis Example 44-1 was dissolved in 1 ml of chloroform, and the solution was cooled to 0°C, and then 1 ml of trifluoroacetic acid was added thereto and the whole was stirred 5 hours at room temperature. On completion of the reaction, the solvent was distilled off and the residue was dried by a vacuum pump. The residue was purified by means of silicagel column chromatography (2.5g, chloroform/methanol/water =7/3/0.5) and a fraction was concentrated. Then, it was dissolved in a 1 mol/l hydrochloric acid and concentrated. After the resultant was azeotropically distilled with water, a solid product was washed with ether, and 6.0 mg of the above-mentioned compound was obtained as a white solid product.
MS(FAB,Pos.):m/z=577[M+1]⁺
¹H-NMR(500MHz,DMSO-d₆+D₂O) : δ=1.53-1.70 (2H,m), 1.70-1.80 (1H,m), 1.80-1.90 (1H,m), 3.08-3.20 (2H,m), 4.55-4.62 (3H,m), 4.77-4.95 (4H,m ), 7.47-7.51 (1H,m), 7.54-7.65 (5H,m), 7.66 (1H,d,J=7.3Hz), 7.71 (1H, t,J=7.1Hz), 7.75 (1H,d,7.3Hz), 7.89 (1H,d,J=8.1Hz), 7.97-8.00 (1H,m ),8.09-8.12(1H,m),8.22(1H,d,J=8.5Hz),8.26(1H,d,J=8.5Hz).

### Synthesis Example 45: Production of N^{α}-(2-(N-2-picolylamino methyl) pyridin-5-ylcarbonyl)-L-arginine 1-naphthalenemethyl amide [Compound No. 46]

### Synthesis Example 45-1: Synthesis of N^{α}-(2-(N-Boc-N-2-picolyl aminomethyl) pyridin-5-ylcarbonyl)-N^{G}-Pmc-L-arginine 1-naphthalenemethylamide (Compound XXIX-4)

116.8 mg of the compound obtained in Synthesis Example 42-1 was dissolved in 2 ml of DMF, and then 0.2 ml of diethylamine was added to the solution and the whole was stirred for 2 hours at room temperature. On completion of the reaction, the solvent was distilled off and the residue was dried under vacuum. It was dissolved in 2 ml of DMF, and then 44.5 mg of WSCI hydrochloride, 24.4 mg of HOBt, and 50.1 mg of the compound obtained in Synthesis Example 12-3 were added to the solution and the whole was stirred for 17 hours at room temperature. On completion of the reaction, the solvent was distilled off and the residue was dissolved in chloroform, followed by washing with a 1 mol/l hydrochloric acid, a 1 mol/l sodium hydroxide aqueous solution, and a saturated salt solution, and further washing withanhydroussodiumsulfate. After the resultant was concentrated under reduced pressure, the residue was purified by means of silica gel column chromatography (10 g, chloroform/methanol = 15/1) , and 118.0 mg of the above-mentioned compound was obtained as a yellow viscous oily product.
MS(FAB,Pos.):m/z=905[M+1]⁺
¹H-NMR(500MHz,DMSO-d₆):δ=1.23(6H,s),1.31(9H,s),1.49-1.68(2H,m) ,1.73(2H,t,J=6.8Hz),1.69-1.79(1H,m),1.79-1.90(1H,rn),2.00(3H,s ),2.45 and 2.46(6H,2s),2.55(2H,t,J=6.6Hz),2.99-3.12(2H,m),4.47 - 4.55(1H,m),4.52,4.56, 4.60 and 4.65(4H,4s),4.73(1H,dd,J=15.2, 5.6Hz),4.78(1H,dd,J=15.2,5.6Hz),6.37(2H,br),6.67(1H,br),7.25-7.40(3H,m),7.45-7.47(2H,m),7.50-7.56(2H,m),7.78(1H,td,J=7.6,1 .7Hz),7.82-7.86(1H,m),7.92-7.96(1H,m),8.03-8.06(1H,m),8.21(1H ,dd,J=-8.3,2.0Hz),8.51(1H,d,J=4.9-Hz),8.58(1H,br),8.73(1H,d,J=7 1Hz),8.98-8.99(1H,m).

### Synthesis Example 45-2: Synthesis of N^{α}-(2-(N-2-picolylamino methyl) pyridin-5-ylcarbonyl)-L-arqinine 1-naphthalenemethyl amide [Compound No. 46]

45.0 mg of the compound obtained in Synthesis Example 45-1 was dissolved in 0.5 ml of chloroform, and the solution was cooled to 0°C, and then 0.5 ml of trifluoroacetic acid was added thereto and the whole was stirred 5 hours at room temperature. On completion of the reaction, the solvent was distilled off and the residue was dried by a vacuum pump. The residue was purified by means of silica gel column chromatography (2 g, chloroform/methanol/water =7/3/0.5) and a fraction was concentrated. Then, it was dissolved in a 1 mol/l hydrochloric acid and concentrated. After the resultant was azeotropically distilled with water, a solid product was washed with ether, and 10 .1 mg of the above-mentioned compound was obtained as a white solid product.
MS(FAB,Pos.):m/z=539[M+1]⁺
¹H-NMR (500MHz,DMSO-d₆):δ=1.48-1.64(2H,m),1.78-1.95 (2H,m),3.09-3.19(2H,m),4.43(2H,s),4.49(2H,s),4.50-4.58(1H,m),4.77(1H,d,J= 5.6Hz),6.90(2H,br),7.40(1H,br),7.45-7.50(3H,m),7.52-7.57(3H,m ),7.64(1H,d,J=8.1Hz),7.78-7.88(2H,m),7.91(1H,td,J=7.6,1.7Hz), 7.92-7.98(1H,m),8.05-8.10(1H,m),8.39(1H,d,J=7.1Hz),8.66(1H;d, J=4.9Hz), 8.98 (1H,br), 9.14 (1H,br), 9.83(2H,br).

### Synthesis Example 46: Production of N^{α}-(5-(N-2-picolylamino methyl) thiophen-2-ylcarbonyl)-L-arginine 1-naphthalenemethyl amide [Compound No. 47]

### Synthesis Example 46-1: Synthesis of N^{α}-(5-(N-Boc-N-2-picolyl) aminomethyl thiophen-2-ylcarbonyl)-N^{G}-Pmc-L-arginine 1-naphthalenemethylamide (Compound XXIX-5)

199.5 mg of the compound obtained in Synthesis Example 42-1 was dissolved in 4 ml of DMF, and then 0.4 ml of diethylamine was added to the solution and the whole was stirred for 4 hours at room temperature. On completion of the reaction, the solvent was distilled off and the residue was dried under vacuum. It was dissolved in 2 ml of DMF, and then 72.9 mg of WSCI hydrochloride, 40.4 mg of HOBt, and 93.0 mg of the compound obtained in Synthesis Example 14-2 were added to the solution and the whole was stirred for 17 hours at room temperature. On completion of the reaction, the solvent was distilled off and the residue was dissolved in chloroform, followed by washing with a 1 mol/l hydrochloric acid, a 1 mol/l sodium hydroxide aqueous solution, and a saturated salt solution, and further washingwith anhydrous sodium sulfate. After the resultant was concentrated under reduced pressure, the residue was purified by means of silica gel column chromatography (10 g, chloroform/methanol = 30/1), and 142.1 mg of the above-mentioned compound was obtained as a light-yellow frothy product.
MS(FAB,Pos.):m/z=910[M+1]⁺
¹H-NMR(500MHz,DMSO-d₆): (=1.2-1.6(2H,m) 1.23 (6H,s), 1.30 and 1.47 (9H,2s), 1.63-1.82 (2H,m), 1.73 (2H,t,J=6.8Hz), 2.00 (3H,s), 2.4 5(3H,s), 2.46 (3H,s), 2.54 (2H,t,J=6.8Hz), 2.99-3.10 (2H,m), 4.38-4. 45(3H,m),4.58 and 4.65 (2H,2s), 4.73 (1H,dd,J=15.1,5.4Hz), 4.76 (1H,dd,J=15.1,5.4Hz), 6.39 (2H,brs), 6.68 (1H,brs), 7.00 (1H,d,J=12 .9Hz), 7.18-7.24 (1H,m), 7.18-7.24 (1H,m), 7.44-7.48 (2H,m), 7.50-7. 55 (2H,m), 7.74 (1H,m), 7.77 (1H,td,J=7.6,1.7Hz), 7.83-7.86 (1H,m), 7 .92-7.96(1H,m),8.02-8.05(1H,m),8.51-8.58 (3H,m).

### Synthesis Example 46-2: Synthesis of N^{α}-(5-(N-2-picolylamino methyl) thiophen-2-ylcarbonyl)-L-arginine 1-naphthalenemethyl amide [Compound No. 47]

121.6 mg of the compound obtained in Synthesis Example 46-1 was dissolved in 1 ml of chloroform and the solution was cooled to 0°C, and then 1 ml of trifluoroacetic acid was added thereto and the whole was stirred for seven hours at room temperature. On completion of the reaction, the solvent was distilled off and the residue was dried by a vacuum pump. The residue was purified by means of silica gel column chromatography (4 g, chloroform/methanol/water = 7/3/0.5) and a fraction was concentrated. Then, it was dissolved in a 1 mol/l hydrochloric acid and concentrated. After the resultant was azeotropically distilled with water, a solid product was washed with ether, and 51.6 mg of the above-mentioned compound was obtained as a white solid product.
MS(FAB,Pos.):m/z=544[M+1]⁺
¹H-NMR(500MHz,DMSO-d₆):δ=1.42-1.61 (2H,m) ,1.74-1.90 (2H,m) ,3.05-3.19(2H,m),4.30(2H,s),4.43-4.51(3H,m),4.75(2H,d,J=5.9Hz),6.80 - 7.10(2H,brs),7.32-7.60(1H,brs),7.36(1H,d,J=3.7Hz),7.43-7.52 (4H,m),7.52-7.57(2H,m),7.58(1H,d,J=8.1Hz),7.82-7.89(2H,m),7.92 (1H,td,J=7.8,2.0Hz),7,93-7.96(1H,m),7.99(1H,d,J=3.7Hz),8.05-8 .08(1H,m),8.66(1H,dd,J=4.9,1.7Hz),8.73(1H,d,J=5.9Hz),8.81(1H, d,J=8.1Hz),9.94(2H,brs).

### Synthesis Example 47: Production of N^{α}-(4-(imidazol-2-ylmethyl) aminomethylnaphthoyl)-L-arginine 2-(3-indolyl) ethylamide [Compound No. 48]

### Synthesis Example 47-1: Synthesis of N^{α}-Fmoc-N^{G}-Pmc-L-arginine 2-(3-indolyl) ethylamide (Compound XXVII-2)

0.954 g of WSCI hydrochloride was added to a anhydrous DMF (20 ml) solution of 2.000 g of commercially available N^{α}-Fmoc-N^{G}-Pmc-L-arginine, 0.798 g of tryptamine and 0.673 g of HOBt, and the whole was stirred for 16 hours, followed by distilling the solvent off. The residue was dissolved in 20 ml of chloroform and was then washed with 30 ml of a 1 mol/l hydrochloric acid and 30 ml of a saturated sodium hydrogencarbonate aqueous solution in sequence, followed by drying with anhydrous magnesium sulfate. A mixture obtained by distilling the solvent off was purified by means of silica gel column chromatography (75 g, 2% methanol/chloroform), and 1.606 g of the above-mentioned compound was obtained as a light-yellow solid product.
¹H-NMR(500MHz,CDCl₃): δ=1.26 (6H,s), 1.24-1.29 (2H,m), 1.42-1.52 (1H ,m), 1.55-1.65 (1H,m), 1.74 (2H,t,J=6.8Hz), 2.09 (3H,s), 2.55 (3H,s), 2.55-2.58 (2H,m), 2.58 (3H,s), 2.91 (3H,m), 3.00-3.18 (2H,m), 3.42-3. 52 (1H,m), 3.60-3.70 (1H,m), 4.00-4.08 (1H,m), 4.08 (1H,t,J=6.8Hz), 4 .25-4.32(2H,m),5.8-5.9(1H,bs),6.0-6.1(2H,bs),6.6-6.7(1H,bs),6 .95(1H,s),7.01(1H,t,J=7.3Hz),7.10(1H,t,J=8.1Hz),7.21-7.32(4H, m),7.35-7.40(2H,m),7.50(2H,d,J=8.3Hz),7.52(1H,d,J=7.6Hz),7.73 (2H,d,J=7.6Hz),8.64(1H,s).

### Synthesis Example 47-2: Synthesis of N^{α}-(4-(N-Boc-N-imidazol-2-yl methyl) aminomethylnaphthoyl)-N^{G}-Pmc-L-arginine 2-(3-indolyl) ethylamide (Compound XXIX-6)

0.322 g of the compound obtained in Synthesis Example 47-1 was dissolved in 10 ml of anhydrous DMF, and then 0.059 g of diethylamine was added to the solution and the whole was stirred for 2. 5 hours, followed by distilling the solvent off. Then, 0.153 g of the compound obtained in Synthesis Example 17-4 and 0.0595 g of HOBt were added to the resultant product, followed by dissolving in 10 ml of anhydrous DMF. Subsequently, 0.084 g of WSCI hydrochloride was added to the solution and the whole was stirred for 12 hours. Then, 0.5 ml of water was added to the reaction solution, and then the solution was concentrated. 5 ml of chloroform was added to the solution, followed by washing with 4 ml of a 0.25 mol/l hydrochloric acid and 4 ml of a saturated sodium hydrogencarbonate aqueous solution. After that, it was treated with diatomaceous earth column and the solvent was then distilled off. The mixture thus obtained was purified by means of silica gel column chromatography (5 g, 0% to 6% methanol/chloroform), and 0.0579 g of the above-mentioned compound was obtained as a white frothy product.
¹H-NMR(500MHz,CDCl₃):δ=:1.23-1.38(1H,m),1.29(6H,s),1.45-1.75(2H ,m),1.52(9H,s),1.78(2H,t,J=6.6Hz),1.78-1.88(1H,m),2.09(3H,s), 2.55(3H,s),2.56(3H,s),2.60(2H,t,J=6.8Hz),2.90-3.00(2H,m),3.10 - 3.30(2H,m),3.44-3.54(1H,m),3.60-3.70(1H,m),4.27(2H,s),4.58-4 .65(1H,m),4.85(1H,m),4.93(1H,d,J=15.9Hz),6.05-6.12(1H,bs),6.1 2-6.20(1H,bs),6.80-6.96(2H,bs),6.99(1H,s),7.05(1H,dt,1.5Hz,9. 3Hz),7.13(1H,t,J=7.3Hz),7.21(1H,d,J=6.3Hz),7.34(2H,d,J=7.8Hz) ,7.44-7.52(2H,m),7.56(1H,d,J=7.3Hz),7.97(1H,d,J=7.3Hz),8.23(1 H,d,J=7.6Hz).

### Synthesis Example 47-3: Synthesis of N^{α}-(4-(imidazol-2-ylmethyl) aminomethylnaphthoyl)-L-arginine 2-(3-indolyl) ethylamide [Compound No. 48]

A 1 ml chloroform solution of the compound (0.0499g) obtained in Synthesis Example 47-2 was cooled with ice, and then 0.5 ml of trifluoroacetic acid was dropped therein. After the mixture was stirred for 5 hours at room temperature, the solvent was distilled off, and then 3 ml of a 1 mol/l hydrochloric acid and 2 ml of chloroform were added thereto. An aqueous phase was separated and washed with 2 ml of chloroform and the solvent was then distilled off. A 1 mol/l hydrochloric acid methanol solution was added to the resulting residue and the solvent was then distilled off, followed by adding 0.25 ml of water and 5 ml of acetone to collect a solid product being generated. The sold product was purified by means of silica gel column chromatography (1.5 g, chloroform/methanol/32% acetic acid aqueous solution = 7/3/0.5). The oily product thus obtained was dissolved in a 1 ml of 1 mol/l hydrochloric acid. The solvent was distilled off and the residue was then dissolved in methanol. A precipitation was obtained by the addition of ethyl acetate, followed by distilling the solvent off and drying under vacuum. Consequently, 0.0040 g of hydrochloride of the above-mentioned compound was obtained as a white solid product.
MS(FAB,Pos.):m/z=580[M+1]⁺

### Synthesis Example 48: Production of N^{α}-(4-(imidazol-2-ylmethyl) aminomethylnaphthoyl)-L-arginine (1'S)-(1'-(1-naphthyl) ethyl) amide [Compound No. 49]

### Synthesis Example 48-1: Synthesis of N^{α}-Fmoc-N^{G}-Pmc-L-arginine (1'S)-(1'-(1-naphthyl) ethyl) amide (Compound XXVII-3)

0.636 g of WSCI hydrochloride was added to anhydrous DMF (13 ml) solution of 1.334 g of commercially available N^{α}-Fmoc-N^{G}-Pmc-L-arginine, 0.568 g of (S)-1-(1-naphthyl) ethylamine and 0.449 g of HOBt, and the whole was stirred for 16 hours, followed by distilling the solvent off. The residue was dissolved in 20 ml of chloroform and was then washed with 30 ml of a 1 mol/l hydrochloric acid and 30 ml of a saturated sodium hydrogencarbonate aqueous solution in sequence, followed by drying withanhydrousmagnesiumsulfate. Amixtureobtainedbydistilling the solvent off was purified by means of silica gel column chromatography (50 g, 2% methanol/chloroform) , and 1.949 g of the above-mentioned compound was obtained as a colorless oily product.
¹H-NMR(500MHz,CDCl₃):δ=1.26[3H,s),1.27(3H,s,Hz),1.26-1.42(2H,m ),1.43-1.55(1H,m),1.58(3H,d,J=6.8Hz),1.75(2H,t,J=6.6Hz),2.07 (3H,s,),2.51(3H,s,),2.52(3H,s,),2.57(2H,t,J=6.8Hz),3.00-3.10(2 H,m),4.10(1H,t,J=7.3Hz),4.25-4.55(3H,m),5.65-5.70(1H,bs),5.70 - 5.80(1H,bs),5.91(1H,d,J=8.5Hz),7.23-7.26(2H,m),7.35-7.45(5H, m),7.28-7.45(3H,m),7.70(1H,d,J=8.3Hz),7.74(2H,d,J=7.6Hz),7.80 (1H,d,J=8.3Hz),7.88-7.98(1H,bs).

### Synthesis Example 48-2: Synthesis of N^{α}- (4-(N-Boc-N-imidazol-2-yl methyl) aminomethylnaphthoyl)-N^{G}-Pmc-L-arginine (1'S)-(1'-(1-naphthyl) ethyl) amide (Compound XXIX-7)

0.326 g of the compound obtained in Synthesis Example 48-1 was dissolved in 10 ml of anhydrous DMF, and then 0.0604 g of diethylamine was added to the solution and the whole was stirred for 2.5 hours, followed by distilling the solvent off. Then, 0.157 g of the compound obtained in Synthesis Example 17-4 and 0.0610 g of HOBt were added to the resultant product, followed by dissolving in 10 ml of anhydrous DMF. Subsequently, 0.0861 g of WSCI hydrochloride was added to the solution and the whole was stirred for 12 hours. Then, 0.5 ml of water was added to the reaction solution, and then the solution was concentrated. 5 ml of chloroform was added to the solution, followed by washing with 4 ml of a 0.25 mol/l hydrochloric acid and 4 ml of a saturated sodium hydrogencarbonate aqueous solution. After that, it was treated with diatomaceous earth column and the solvent was then distilled off. The mixture thus obtained was purified by means of silica gel column chromatography (5 g, 0% to 6% methanol/chloroform), and 0.0672 g of the above-mentioned compound was obtained as a white frothy product.
¹H-NMR(500MHz, CDCl₃) :δ=1.25-1.36(1H,m) ,1.29 (6H,s) ,1.480(9H,s), 1 .60(3H,d,J=6.8Hz),1.77(2H,t,J=6.6Hz),2.07(3H,s),2.49(6H,s),2. 58(2H,t,J=6.6Hz),3.00-3.30(2H,m),4.20-4.35(2H,m),4.72-4.82(1H ,m),4.85-4.95(2H,m),5.80-5.90(1H,m),6.05-6.15(1H,bs),6.15-6.2 5(2H,bs),6.82-7.00(2H,m),7.21(1H,d,J=7.3Hz),7.38(2H,t,J=7.8Hz ),7.30-7.60(5H,m),7.74(1H,d,J=8.3Hz),7.83(1H,d,J=6.8Hz),7.94-8.06(2H,m),8.22(1H,d,J=7.6Hz).

### Synthesis Example 48-3: Synthesis of N^{α}-(4-(imidazol-2-ylmethyl) aminomethylnaphthoyl)-L-arginine (1'S)-(1'-(1-naphthyl) ethyl) amide [Compound No. 49]

A1.7 ml chloroform solution of the compound (0.172 g) obtained in Synthesis Example 48-2 was cooled with ice, and then 1.7 ml of trifluoroacetic acid was added therein. After the mixture was stirred for 5 hours at room temperature, the solvent was distilled off, and then 5 ml of a 1 mol/l hydrochloric acid and 3 ml of chloroform were added to the concentrate. An aqueous phase was separated and washed with 3 ml of chloroform and the solvent was then distilled off. A 1 mol/l hydrochloric acid methanol solution was added thereto and the solvent was then distilled off, followed by dissolving the oily product thus obtained in 0.5 ml of water. Then, 5 ml of acetone was added to the solution and a solid product thus generated was centrifuged and then a supernatant thereof was discarded, followed by distilling the solvent off. The resulting solid product was dissolved in 1 ml of a 1 mol/l hydrochloric acid and then the solvent was distilled off. The resultant product was dissolved in methanol and a solid product was then precipitated by the addition of ethyl acetate. The solvent was distilled off. Consequently, 0.0891 g of hydrochloride of the above-mentioned compound was obtained as a white solid product.
MS(FAB,Pos.):m/z=591[M+1]⁺
¹H-NMR(500MHz,DMSO-d₆): δ=1.50-1.63(2H,m)1.56(3H,d,J=6.8Hz),1.6 4-1.76 (1H,m) 1.78-1.86 (1H,m) 3.08-3.18 (2H,m) 4.58-4.65 (3H,m), 4.8 2(2H,s), 5.78 (1H,quint,J=7.3Hz), 7.28-7.71 (9H,m) 7.74 (1H,t,J=5.4 Hz), 7.79 (1H,d,J=7.3Hz), 7.85 (1H,d,J=8.3Hz), 7.96 (1H,d,J=8.1Hz), 8.15 (1H,d,J=8.8Hz), 8.26 (1H,d,J=7.6Hz), 8.30 (1H,d,J=8.5Hz), 8.72 (1H,d,J=8.1Hz), 8.80 (1H,d,J=7.8Hz).

### Synthesis Example 49: Production of N^{α}-(4-(imidazol-2-ylmethyl) aminomethylnaphthoyl)-L-arginine (1'R)-(1'-(1-naphthyl) ethyl) amide [Compound No. 50]

### Synthesis Example 49-1: Synthesis of N^{α}-Fmoc-N^{G}-Pmc-L-arginine (1'R)-(1'-(1-naphthyl) ethyl) amide (Compound XXVII-4)

0.954 g of WSCI hydrochloride was added to a anhydrous DMF (20 ml) solution of 2.00 g of commercially available N^{α}-Fmoc-N^{G}-Pmc-L-arginine, 0.853 g of (R) -1- (1-naphthyl) ethylamine and 0.674 g of HOBt, and the whole was stirred for 16 hours, followed by distilling the solvent off. The residue was dissolved in 20 ml of chloroform and was then washed with 30 ml of a 1 mol/l hydrochloric acid and 30 ml of a saturated sodium hydrogencarbonate aqueous solution in sequence, followed by drying withanhydrousmagnesiumsulfate. Amixture obtained by distilling the solvent off was purified by means of silica gel column chromatography (50 g, 2% methanol/chloroform), and 1.122 g of the above-mentioned compound was obtained as a colorless oily product.
¹H-NMR(500MHz,CDCl₃) : δ=1.26 (3H,s), 1.26 (3H,s), 1.45-1.78 (3H,m), 1 .56 (3H,d,J=6.8Hz), 1.75 (2H,t,J=6.8Hz), 1.80-1.90 (1H,m), 2.08 (3H, s), 2.52 (3H,s), 2.55 (3H,s), 2.52-2.57 (2H,m), 3.1-3.2 (1H,m), 3.2-3. 3 (1H,m), 4.04 (1H,t,J=7.1Hz), 4.18-4.28 (3H,m), 5.81 (1H,t,J=7.3Hz) ,5.9-6.0 (1H,bs), 6.0-6.1 (2H,bs), 7.18-7.55 (10H,m), 7.67 (1H,d,J=8 .3Hz),7.72(2H,d,J=7.6Hz),7.77(1H,d,J=8.1Hz),8.04(1H,d,J=8.3Hz ).

### Synthesis Example 49-2: Synthesis of N^{α}-(4-(N-Boc-N-imidazol-2-yl methyl) aminomethylnaphthoyl)-N^{G}-Pmc-L-arginine (1'R)-(1'-(1-naphthyl) ethyl) amide (Compound XXIX-8)

0.326 g of the compound obtained in Synthesis Example 49-1 was dissolved in 10 ml of anhydrous DMF, and then 0.0604 g of diethylamine was added to the solution and the whole was stirred for 2.5 hours, followed by distilling the solvent off. Then, 0.157 g of the compound obtained in Synthesis Example 17-4 and 0.0610 g of HOBt were added to the resultant product, followed by dissolving in 10 ml of anhydrous DMF. Subsequently, 0.0861 g of WSCI hydrochloride was added to the solution and the whole was stirred for 12 hours. Then, 0.5 ml of water was added to the reaction solution, and then the solution was concentrated. 5 ml of chloroform was added to the solution, followed by washing with 4 ml of a 0.25 mol/l hydrochloric acid and 4 ml of a saturated sodium hydrogencarbonate aqueous solution. After that, the resultant was treated with a diatomaceous earth column and the solvent was then distilled off. The mixture thus obtained was purified by means of silica gel column chromatography (5 g, 0% to 6% methanol/ chloroform) , and 0. 0780 g of the above-mentioned compound was obtained as a white frothy product.
¹H-NMR(500MHz,CDCl₃) : δ=1.22-1.32(1H,m),1.29(3H,s),1.48(9H,s),1 .40-1.70(2H,m),1.60(3H,d,J=6.8Hz),1.77(2H,t,J=6.8Hz),1.85-2.0 0(1H,m),2.08(3H,s),2.50(3H,s),2.52(3H,s),2.57(2H,t,J=6.8Hz),3 .06-3.26(2H,m),4.23(2H,s),4.62-4.72(2H,m),4.80-4.95(2H,m),5.8 0-5.90(1H,m),6.08-6.18(1H,bs),6.80-6.98(2H,bs),7.12(1H,d,J=7. 1Hz),7.29(1H,t,J=7.8Hz),7.30-7.46(5H,m),7.52(1H,d,J=7.3Hz),7. 67(1H,d,J=8.3Hz),7.79(1H,d,J=7.6Hz),7.94(1H,d,J=9Hz),8.02-8.1 2(2H,m).

### Synthesis Example 49-3: Synthesis of N^{α}-(4- (imidazol-2-ylmethyl) aminomethylnaphthoyl)-L-arginine (1'R)-(1'-(1-naphthyl) ethyl) amide [Compound No. 50]

A chloroform (1.3 ml) solution of the compound (0.1328 g) obtained in Synthesis Example 49-2 was cooled with ice, and then 1.3 ml of trifluoroacetic acid was added thereto. After the mixture was stirred for 5 hours at room temperature, the solvent was distilled off, and then 5 ml of a 1 mol/l hydrochloric acid and 3 ml of chloroform were added to a concentrate. An aqueous phase was separated and washed with 3 ml of chloroform and the solvent was then distilled off. A 1 mol/l hydrochloric acid methanol solution was added thereto and the solvent was then distilled off, followed by dissolving the oily product thus obtained in 0.25 ml of water. Then, 5 ml of acetone was added to the solution and a solid product thus generated was centrifuged and then a supernatant thereof was discarded, followed by distilling the solvent off. The resulting solid product was dissolved in 1 ml of a 1 mol/l hydrochloric acid and then the solvent was distilled off. The resultant product was dissolved in methanol and a solid product was then precipitated by the addition of ethyl acetate thereto. The solvent was distilled off. Consequently, 0.0891 g of hydrochloride of the above-mentioned compound was obtained as a white solid product.
MS(FAB,Pos.):m/z=591[M+1]⁺
¹H-NMR(500MHz,DMSO-d₆) : δ=1.55 (3H,d,J=6.8Hz), 1.50-1.70 (2H,m), 1. 70-1.80 (1H,m), 1.80-1.90 (1H,m), 3.18 (2H,q,J=6.3Hz), 4.58-4.64 (3H ,m),4.83(2H,s),5.75(1H,quint.,J=7.1Hz),7.46-7.72(9H,m),7.78(2 H,d,J=7.1Hz), 7.86 (1H,d,J=8.1Hz), 7.94-7.97 (1H,m) 8.14-8.17 (1H,m ) 8.19 (1H,d,J=8.5Hz), 8.29 (1H,d,J=8.8Hz), 8.80 (2H,d,J=8.1Hz).

### Synthesis Example 50: Production of N^{α}-(4-(imidazol-2-ylmethyl) aminomethylnaphthoyl)-L-arginine 4-hexadecylaminobenzylamide [Compound No. 51]

### Synthesis Example 50-1: Synthesis of N^{α}-Pmc-NG-Pmc-L-arginine 4-hexadecylaminobenzylamide (Compound XXVII-5)

1.040 g of WSCI hydrochloride was added to a anhydrous DMF (20 ml) solution of 2.18 g of commercially available N^{α}-Fmoc-N^{G}-Pmc-L-arginine, 1. 883 g of 4-hexadecylaminobenzylamine and 0.735 g of HOBt, and the whole was stirred for 16 hours, followed by distilling the solvent off. The residue was dissolved in 20 ml of chloroform and was then washed with 30 ml of a 1 mol/l hydrochloric acid and 30 ml of a saturated sodium hydrogencarbonate aqueous solution in sequence, followed by drying with anhydrous magnesium sulfate. A mixture obtained by distilling the solvent off was purified by means of silica gel column chromatography (50 g, 2% methanol/chloroform), and 1.321 g of the above-mentioned compound was obtained as a colorless oily product.
¹H-NMR(500MHz,CDCl₃):δ=0.88(3H,t,J=6.8Hz),1.20-1.40(34H,m),1.4 5-1.60(4H,m),1.76(2H,t,J=6.8Hz),2.08(3H,s),2.53(3H,s),2.55(3H ,s),2.58(2H,t,J=6.8Hz),2.99(2H,t,J=7.1Hz),3.10-3.20(1H,bs),3. 20-3.30(1H,bs),4.10(1H,t,J=7.1Hz),4.15-4.25(3H,m),4.31(2H,d,J =7.3Hz),5.90-6.00(1H,bs),6.05-6.15(2H,bs),6.46(2H,d,J=8.3Hz), 7.04(2H,d,J=7.1Hz),7.23-7.26(2H,m),7.36(2H,t,J=7.8Hz),7.54(2H ,d,J=7.6Hz),7.55(2H,d,J=7.3Hz).

### Synthesis Example 50-2: Synthesis of N^{α}-(4-(N-Boc-N-imidazol-2-yl methyl) aminomethylnaphthoyl)-N^{G}-Pmc-L-arginine 4-hexadecyl aminobenzylamide (Compound XXIX-9)

0.397 g of the compound obtained in Synthesis Example 50-1 was dissolved in 10 ml of anhydrous DMF, and then 0.0604 g of diethylamine was added to the solution and the whole was stirred for 2.5 hours, followed by distilling the solvent off. Then, 0.157 g of the compound obtained in Synthesis Example 17-4 and 0.0610 g of HOBt were added to the resultant product, followed by dissolving in 10 ml of anhydrous DMF. Subsequently, 0.0861 g of WSCI hydrochloride was added to the solution and the whole was stirred for 12 hours. Then, 0.5 ml of water was added to the reaction solution, and then the solution was concentrated. 5 ml of chloroform was added to the solution, followed by washing with 4 ml of a 0.25 mol/l hydrochloric acid and 4 ml of a saturated sodium hydrogencarbonate aqueous solution. After that, it was treated with a diatomaceous earth column and the solvent was then distilled off. The mixture thus obtained was purified by means of silica gel column chromatography (5 g, 0% to 6% methanol/chloroform), and 0.164 g of the above-mentioned compound was obtained as a colorless viscous product.
¹H-NMR(500MHz,CDCl₃):δ=0,88(3H,t,J=6.8Hz),1.20-1.40(34H,m),1.4 9(9H,s),1.45-1.70(3H,m),1.78(H,,6.6Hz),2.08(3H,s),2.51(3H,s), 2.52(3H,s),2.59(2H,t,J=6.8Hz),3.03(2H,t,J=7.1Hz),3.10-3.25(1H ,m),3.25-3.35(1H,m),4.20-4.35(4H,m),4.65-4.75(1H,m),4.93(2H,s ),6.16-6.26(2H,bs),6.49(2H,d,J=8.5Hz),6.85-6.95(2H,bs),7.06(2 H,d,J=8.3Hz),7.20(1H,d,J=7.3Hz),7.30-7.50(3H,m),7.98(1H,d,J=7 .8Hz),8.20(1H,d,J=8.1Hz).

### Synthesis Example 50-3: Synthesis of N^{α}-(4-(imidazol-2-ylmethyl) aminomethylnaphthoyl)-L-arginine 4-hexadecylaminobenzylamide [Compound No. 51]

A chloroform (1.4 ml) solution of the compound (0.139 g) obtained in Synthesis Example 50-2 was cooled with ice, and then 1.4 ml of trifluoroacetic acid was added to the solution and the whole was stirred for 5.5 hours at room temperature, followed by distilling the solvent off. Then, 5 ml of a 1 mol/l hydrochloric acid and 3 ml of chloroform were added to a concentrate, and an aqueous phase was separated and washed with 3 ml of chloroform, followed by distilling the solvent off. The oily product thus obtained was dissolved in a 1 mol/l hydrochloric acid methanol solution and the solvent was then distilled off, followed by dissolving in 0.25 ml of water. Then, 5 ml of acetone was added to the solution and a solid product thus generated was precipitated by centrifugation and then a supernatant thereof was discarded, followed by drying under vacuum. Consequently, 0.0846 g of hydrochloride of the above-mentioned compound was obtained as a white solid product.
MS(FAB,Pos.):m/z=766[M+1]⁺
¹H-NMR(500MHz,DMSO-d₆): δ=0.85(3H,t,J=6.6Hz),1.18-1.40(26H,m),1 .52-1.70 (4H,m), 1.70-1.80 (1H,m), 1.80-1.90 (1H,m), 3.10-3.20 (4H,m ), 4.33 (2H,s), 4.54 (1H,dd,J=13.2,7.6Hz), 4.66 (2H,s), 4.86 (2H,s),6 .8-7.6 (6H,m), 7.32 (2H,bs), 7.62-7,75 (4H,m), 7.78 (1H,t,J=5.6Hz),7 .82 (1H,d,J=7.3Hz), 8.28 (1H,dd,8.6Hz,1.0Hz), 8.31 (1H,d,J=7.8Hz), 8.68(1H,bs),8.84(1H,d,J=7.6Hz).

### Synthesis Example 51: Production of N^{α}-(4-(5,6,7,8-tetrahydro quinolin-8-ylaminomethyl) benzoyl)-L-arginine 1-naphthalene methylamide [Compound No. 52]

### Synthesis Example 51-1: Synthesis of N^{α}-(4-(N-Cbz-aminomethyl) benzoyl)-N^{G}- Pmc-L-arginine 1-naphthalenemethylamide (Compound XXX-1)

2.187 g of the compound obtained in Synthesis Example 42-1, 0.95 g of WSCI and 0.67 g of HOBt were dissolved in 33 ml of DMF, and then 0.51 ml of 1-naphthalenemethylamine was added to the solution. After 18 hours, the reaction solution was concentrated and a 1 mol/l hydrochloric acid was added thereto, followed by extraction with chloroform. A saturated sodium bicarbonate solution was added to an organic layer and extraction was performed with chloroform, followed by washing the organic layer with a saturated salt solution. The organic layer was driedwith anhydrous sodium sulfate and concentrated. The residue thus obtained was dissolved in 50 ml of DMF and 5 ml of diethylamine was added to the solution. After 1 hour, the residue obtained by concentrating the reaction solution was suspended in 40 ml of DMF, followed by the addition of 0.95 g of WSCI, 0.60 g of DMAP, and 0.75 g of the compound obtained in Synthesis Example 41-1 to the suspension. After 3 days, the reaction solution was concentrated and then a 1 mol/l hydrochloric acid was added thereto. Then, it was extracted with chloroform and an organic layer was washed with a saturated salt solution. The organic layer was dried with anhydrous sodium sulfate and concentrated. The residue thus obtained was purified by means of silica gel column chromatography (80 g, chloroform/methanol = 20/1), and 2.47 g of the above-mentioned compound was obtained as a light-yellow solid product.

### Synthesis Example 51-2: Synthesis of N^{α}-(4-(aminomethyl)benzoyl) -N^{G}-Pmc-L-arginine 1-naphthalenemethylamide (Compound XXXI-1)

407.3 mg of the compound obtained in Synthesis Example 51-1 was dissolved in 20 ml of ethanol, and then 41 mg of 10% Pd-C was added to the solution, followed by hydrogen substitution. After the reaction was continued for 3 days, the reaction solution was filtrated through a glass filter. The residue obtained by concentrating the filtrate was purified by means of silica gel column chromatography (12 g, chloroform/methanol = 5/1) , and 113.4 mg of the above-mentioned compound was obtained as a white solid product.
MS(FAB,Pos.):m/z=713[M+1]⁺
¹H-NMR(500MHz,CDCl₃) : δ=1.28 (6H,s), 1.45-1.95 (2H,m), 2.05 (3H,s), 2 .44 (3H,s), 2.45 (3H,s), 2.50-2.60 (2H,m), 3.08-3.19 (1H,m), 3.23-3.3 2(1H,m),3.85(2H,s),4.60-4.73(2H,m),4.83-4.92(1H,m),6.14-6.32 (2H,br),7.20-7.47(6H,m),7.61-7.82(4H,m),7.92-7.99(1H,m).

### Synthesis Example 51-3: Synthesis of N^{α}-(4-(5,6,7,8-tetrahydro quinolin-8-ylaminomethyl) benzoyl)-N^{G}-(2,2,6,7,8-pentamethyl chroman-6-ylsulfonyl)-L-arginine 1-naphthalenemethylamide (Compound XXXII-1)

109.8 mg of the compound obtained in Synthesis Example 51-2 was dissolved in 2 ml of methanol, and then 34 mg of 5,6,7,8-tetrahydroquinolin-8-one,and 21 µl of triethylamine were added to the solution. After 4 hours, the reaction solution was concentrated and dissolved in 2 ml of methanol again, followed by the addition of 18 mg of sodium borohydride. After the reaction was continued for 2 hours, a small amount of water was added to the reaction solution. Then, the solution was concentrated and the resulting residue was purified by means of silica gel column chromatography (5 g, chloroform/methanol = 10/1). Consequently, 61.8 mg of the above-mentioned compound was obtained as a brown solid product.
MS(FAB,Pos.):m/z=844[M+1]⁺
¹H-NMR(500MHz,DMSO-d₆):δ=1.23(6H,s),1.38-2.09(9H,m),2.00(3H,s) ,2.45(3H,s),2.46(3H,s),2.50-2.56(2H,m),2.67-2.82(2H,m),2.99-3 .10 (2H,m), 3.17 (2H,d,J=4.4Hz), 3.90(2H,dd,J=14.2,23.4Hz), 4.08-4 .12 (1H,m), 4.43-4.50 (1H,m), 4.75 (2H,d,J=4.9Hz), 7.17-7.21 (1H,m), 7.40-7.55 (7H,m), 7.83 (1H,t,J=4.7Hz), 7.87 (2H,d,J=8.1Hz), 7.90-7. 95 (1H,m), 8.00-8.06 (1H,m), 8.36 (1H,dd,J=1.7,2.9Hz), 8.45 (1H,d,J= 7.8Hz), 8.53 (1H,t,J=5.7Hz).

### Synthesis Example 51-4: Synthesis of N^{α}-(4-(5,6,7,8-tetrahydro quinolin-8-ylaminomethyl) benzoyl)-L-arginine 1-naphthalene methylamide [Compound No. 52]

41.7 mg of the compound obtained in Synthesis Example 51-3 was dissolved in 0.4 ml of chloroform, and then 0.4 ml of trifluoroacetic acid was added to the solution. After the reaction was continued for 5 hours, the reaction solution was concentrated and the resulting residue was purified by means of silica gel column chromatography (1.5 g, chloroform/methanol /water = 7/3/0.5). Subsequently, active carbon was added to the above-mentioned compound thus obtained, and was then separated by filtration. An excess amount of a 1 mol/l hydrochloric acid was added to the product and concentrated. Consequently, 3.5 mg of the above-mentioned compound was obtained as hydrochloride of a light-yellow solid product.
MS(FAB,Pos.):m/z=578[M+1]⁺
¹H-NMR(500MHz,DMSO-d₆) : δ=1.45-1.63(2H,m),1.71(3H,m),1.95(2H,m) ,2.34(1H,m),2.77-2.85(2H,m),3.08-3.15(2H,m),4.28-4.43(3H,m),4 .49-4.56 (1H,m), 4.76 (2H,d,J=5.6.Hz), 7.40 (1H,dd,J=4.7,7.7Hz), 7. 46 (2H,d,J=4.9Hz), 7.51-7.57 (2H,m), 7.66-7.78 (4H,m), 7.84 (1H,t,J= 4.8Hz), 7.95 (1H,t,J=4.8Hz), 8.01 (2H,d,J=8.3Hz), 8.07 (1H,t,J=4.8H z),8.53(1H,d,J=4.2Hz),8.66(2H,d,J=7.8Hz).

### Synthesis Example 52: Production of N^{α}- (4-((imidazol-2-ylmethyl) aminomethyl) benzoyl)-L-arginine 1-naphthalene methylamide [Compound No. 53]

### Synthesis Example 52-1: Synthesis of N^{α}-(4-((imidazol-2-ylmethyl) aminomethyl) benzoyl)-N^{G}-Pmc-L-arginine 1-naphthalenemethyl amide (Compound XXXII-2)

85.5 mg of the compound obtained in Synthesis Example 51-2 was dissolved in 1.7 ml of methanol and then 12.2 mg of 2-imidazolylcarboaldehyde was added to the solution and the whole was stirred for 2 days and 15.5 hours at room temperature. On completion of the reaction, the solvent was distilled off and then vacuum drying was performed, followed by the addition of 1.7 ml of anhydrous methanol. It was cooled to 0°C and then 10.7 mg of sodium borohydride was added thereto, followed by stirring for 3 hours at room temperature. On completion of the reaction, the solvent was distilled off and then the residue was purified by means of silica gel column chromatography (5 g, chloroform/methanol = 5/1), and 75.6 mg of a white frothy product was obtained.
MS(FAB,Pos.):m/z=793[M+1]⁺
¹H-NMR(500MHz,DMSO-d₆) : δ=1.23(6H,s), 1.38-1.58(2H,m) ,1.65-1.82 (2H,m),1.73(2H,t,J=6.8Hz),2.00(3H,s),2.45(3H,s),2.46(3H,s),2.5 0-2.58(2H,t,J=6.8Hz),3.00-3.08(2H,m),3.66(2H,s),3.73(2H,s),4. 46-4.55(1H,m),4.75(2H,d,J=5.1Hz),6.37(1H,brs),6.70(1H,brs),6. 79 (1H,s), 7.02 (1H,s), 7.41-7.50 (4H,m), 7.52-7.58 (2H,m), 7.84 (1H,t ,J=4.6Hz), 7.86 (2H,d,J=8.5Hz), 7.93-7.96 (1H,m), 8.02-8.06 (1H,m), 8.44 (1H,d,J=7.8Hz), 8.53 (1H,t,J=5.6Hz).

### Synthesis Example 52-2: Synthesis of N^{α}-(4-((imidazol-2-ylmethyl) aminomethyl) benzoyl)-L-arginine 1-naphthalenemethylamide [Compound No. 53]

35.7 mg of the compound obtained in Synthesis Example 52-1 was dissolved in 0.7 ml of chloroform, and then 0.7 ml of trifluoroacetic acid was added to the solution and the whole was stirred for 5 hours at room temperature. On completion of the reaction, the solvent was distilled off and the residue was dried by a vacuum pump. The residue was purified by means of silica gel column chromatography(1.5g, chloroform/methanol/water=7/3/0.5) and a fraction was concentrated. Then, it was dissolved in a 1 mol/l hydrochloric acid and concentrated. After the resultant was azeotropically distilled with water, a solid product was washed with ether, and 9.1 mg of the above-mentioned compound was obtained as a white solid product.
MS(FAB,Pos.):m/z=527(M+1]⁺
¹H-NMR(500MHz,DMSO-d₆) : δ=1.45-1.62(2H,m),1.70-1.90(2H,m),3.02-3.15 (2H,m), 4.36 (2H,s), 4.49 (2H,s), 4.45-4.60 (1H,m), 4.76 (2H,d,J= 5.9Hz), 6.90 (2H,br), 7.39 (1H,br), 7.43-7.50 (2H,m), 7.52-7.57 (2H,m ), 7.67 (4H,d,J=8.1Hz), 7.77 (1H,brs), 7.82-7.87 (1H,m), 7.93-7.97 (1 H,m),7.99(2H,d,J=8.1Hz),8.03-8.09(1H,m),8.60-8.70(2H,m).

### Synthesis Example 53: Production of (S)-2-(4-(N-2-picolylamino methyl) benzoylamino)-5-(quinolin-8-ylamino) valerate 1-naphthalenemethylamide [Compound No. 54]

### Synthesis Example 53-1: Synthesis of N^{α}-Boc-L-gultamate 1-naphthalenemethylamide γ-benzylester (Compound XXXV-1)

3.168 g of commercially available N^{α}-Boc-L-gultamate γ-benzylester was dissolved in 64 ml of DMF, and then 2.7 g of WSCI, 1.7 g of DMAP and 2.06 ml of 1-naphthalenemethylamine were added to the solution. After 15 hours, the reaction solution was concentrated and then 1 mol/l hydrochloric acid was added thereto, followedbyextractionwithchloroform. An organic layer was washed with a saturated salt solution. An organic layer was dried with anhydrous sodium sulfate and concentrated, and 6.087 g of a rough-purified target product was obtained as a light-yellow solid product.

### Synthesis Example 53-2: Synthesis of N^{α}-4-(N-Boc-N-2-picolylaminomethylbenzoyl)-L-gultamate 1-naphthalenemethylamide γ-methylester (Compound XXXVII-1)

1.125 g of the compound obtained in Synthesis Example 53-1 was dissolved in 11 ml of methanol, and then 5.5 ml of a 4-mol/l hydrochloric acid/dioxane solution was added to the solution. After the reaction was continued for 1. 5 hours, the reaction solution was concentrated and azeotropically distilled with chloroform, followedbydryingthesolutionunderreducedpressure. Theresidue thus obtained (1.13 g) was dissolved in 23 ml of DMF, and then 0.50 g of WSCI, 0.32 g of DMAP, and 0.65 g of the compound obtained in Synthesis Example 1-2 were added to the solution. After 27 hours , the reaction solution was concentrated and a 1-mol/l hydrochloric acid was added to the solution, followed by extraction with chloroform. An organic layer was washed with a saturated salt solution, and then the organic solution was dried with anhydrous sodium sulfate and concentrated. The residue thus obtained was purified by means of silica gel column chromatography (50 g, hexane/ethyl acetate = 1/3) , and 678 mg of the target product was obtained as yellow syrup.
MS(FAB,Pos.).:m/z=625[M+1]⁺
¹H-NMR(500MHz,CDCl₃):δ=1.44 and 1.46(9H,2s),2.05-2.28(2H,m), 2.37-2.44(1H,m),2.57-2.64(1H,m),3.62(3H,s),4.46(1H,s),4.50(1H ,s),4.59(2H,s),4.63-4.70(1H,m),4.86-4.98(2H,m),6.90(1H,br),7. 45-7.20(1H,m),7.23-7.51(8H,m),7.65(1H,t,J=7.5Hz),7.70(2H,d,J= 5.6Hz),7.80(1H,d,J=8.1Hz),7.87(1H,d,J=7.1Hz),7.97(1H,d,J=6.8H z),8.01(2H,s),8.53(1H,d,J=4.4Hz).

### Synthesis Example 53-3: Synthesis of (S)-2-(4-(N-Boc-N-2-picolyl aminomethyl) benzoylamino)-5-(quinolin-8-ylamino) valerate 1-naphthalenemethylamide(XIII-13)

109 mg of aluminum lithium aluminum hydride was suspended in 13 ml of THF, and then a 3.5-ml THF (3.5 ml) solution of the compound (672.1 mg) obtained in Synthesis Example 53-2 was added to the suspension under ice-cold condition. After the reaction was continued for 1 hour, water was gradually pored added into the reaction solution and concentrated. Furthermore, water was additionally provided and then a saturated potassium sodium tartrate aqueous solution was added thereto, followed by extraction with chloroform. An organic layer was washed with a saturated salt solution. The organic layer was dried with anhydrous sodium sulfate and concentrated. Consequently, 566.4 mg of intermediate alcohol was obtained as a low-light yellow solid product. 3.3 ml of methylene chloride was added to 52 µl of DMSO, and 32 µl of oxalyl chloride at 78°C was dropped in the mixture at -78°C. After 5 minutes, a 0.6-ml methylene chloride (0.6 ml) solution of the intermediate alcohol (110.03 mg) obtained in the previous forgoing section was dropped in the mixture. After an additional 25 minutes, 0.21 ml of triethylamine was added to the mixture. Subsequently, it was gradually heated and after 1.5 hours water was added at -15°C. An organic layer was extracted with chloroform, followed by drying with anhydrous sodium sulfate and concentrating. The resulting residue was dissolved in 2.2 ml of methanol, and then 32 mg of 8-aminoquinoline and 26 µl of triethylamine were added to the solution, followed by leaving the solution as it was for all day and night. The reaction solution was concentrated and dried under a reduced pressure, and then it was dissolved in 2.2 ml of methanol again, followed by adding 0.2 ml of acetic acid and 35 mg of sodium cyano borohydride in sequence. After the reaction was continued for 7 days, the reaction solution was concentrated and water was then added thereto, followed by extraction with chloroform. An organic layer was dried with anhydrous sodium sulfate and concentrated, and the resulting residue was purified by means of silica gel column chromatography (5 g, hexane/ethyl acetate = 1/4) , and 19.9 mg of the above-mentioned compound was obtained as a yellow solid product.
MS(FAB,Pos.):m/z=723[M+1]⁺
¹H-NMR(500MHz,CDCl₃):δ=1.44 and 1.46(9H,2s) ,1.83-1.95 (2H,m), 1.95-2.02(1H,m),2.11-2.20(1H,m),3.35(2H,t,J=6.6Hz),4.41-4.72 (6H,m),4.82-4.96(2H,m),6.49(1H,br),6.63(1H,d,J=7.3Hz),6.93(1H, br),7.03(1H,d,J=7.8Hz),7.12-7.62(6H,m),7.60-7.68(3H,m),7.73-7 .97(3H,m),8.04(1H,d,J=7.1Hz),8.53(1H,d,J=4.4Hz),8.62(1H,dd,J= 4.1,1.7Hz).

### Synthesis Example 53-4: Synthesis of (S)-2-(4-(N-2-picolylamino methyl) benzoylamino)-5-(quinolin-8-ylamino) valerate 1-naphthalenemethylamide [Compound No. 54]

19.0 mg of the compound obtained in Synthesis Example 53-3 was dissolved in 0.19 ml of methanol, and then 0.19 ml of a 4-mol/l hydrochloric acid/dioxane was added to the solution. After 2.5 hours, the reaction solution was concentrated and azeotropically co-distilled with methanol. The residue thus obtained was purified by means of silica gel column chromatography (1 g, chloroform/methanol/water = 7/3/0.5), and the resulting above-mentioned compound was dissolved in methanol. Then, 4 mg of active carbon was added to the solution, and it was filtrated and concentrated. Subsequently, a 1 mol/l hydrochloric acid and a small amount of methanol were added to the resulting residue, followed by concentrating and drying under a reduced pressure. Consequently, 17.9 mg of hydrochloride of the above-mentioned compound was obtained as a yellow solid product.
MS(FAB,Pos.):m/z=623[M+1]⁺
¹H-NMR(500MHz,CDCl₃):δ=1.70-1.89(2H,m),1.90-2.00(2H,m),3.30(2H ,t,J=7.6Hz),4.30(4H,s),4.57-4.63(1H,m),4.71-4.82(2H,m),6.77(1 H,brd,J=5.9Hz),7.15(1H,d,J=8.1Hz),7.38-7.60(8H,m),7.65(2H,d,J =8.5Hz),7.84(1H,d,J=7.8Hz),7.86-7.96(2H,m),7.99(2H,d,J=8.5Hz) ,8.06(1H,m),8.34(1H,brd,J=7.3Hz),8.62-8.70(3H,m),8.77(1H,d,J= 2.9Hz) ,9.79(2H,s).

### Synthesis Example 54: Production of (2S)-2-(4-(N-2-picolylamino methyl) naphthoylamino)-5-((8R)-5,6,7,8-tetrahydroguinolin-8-ylamino) valerate 1-naphthalenemethylamide [Compound No. 55]

### Synthesis Example 54-1: Synthesis of 8-amino-5,6,7,8-tetrahydro quinoline L-tartrate

3.53 g of 5,6,7,8-tetrahydroquinoline-8-ol synthesized by the method described in Journal of Medicinal Chemistry, vol. 20, No. 10, pp 1351-1354 (1977) was dissolved in 18 ml of benzene, and then 6.74 ml of tribromophosphine was added to the solution. After the reaction was continued for 1 hour, a 1 mol/l sodium hydroxide aqueous solution was added to the solution and the pH thereof was adjusted to about pH = 10, followed by extraction with chloroform. An organic layer was washed with a saturated salt solution, and was then dried with anhydrous sodium sulfate. The residue thus obtained was dissolved in 50 ml of DMF, followed by the addition of 4.38 g of potassium phthalimide. After the reaction solution was stirred for 3 days, the reaction solution was concentrated and water was then added thereto, followed by extraction with chloroform. An organic layer was washed with a saturated salt solution, and then the organic layer was dried with anhydrous sodium sulfate and concentrated. The residue thus obtained was purified by means of silica gel column chromatography (60 g, hexane/ethyl acetate = 1/1), and an intermediate thereof was obtained. 500mg of the intermediate was suspended in 2.5 ml of ethanol, and then 0.44 ml of hydrazine monohydrate was added to the suspension. After the mixture was stirred for 5 hours, water was added to the reaction solution, followed by extraction with ethyl acetate. An organic layer was washed with a saturated salt solution and dried with anhydrous sodium sulfate and concentrated. 61.7mg of the resulting residue was dissolved in methanol, and then 62.5 mg of L-tartaric acid was added to the solution. Furthermore, chloroform was added to the solution and the whole was left standing overnight. A precipitated crystal was collected by filtration and dried, and consequently 102.1 mg of the above-mentioned compound was obtained as a white needle crystal.
MS(FAB,Pos.):m/z=149[M+1]⁺
¹H-NMR(500MHz,CDCl₃):δ=1.69-1.85(2H,m)1.87-2.03(1H,m)2.17-2. 24(1H,m),2.72-2.88(2H,m),3.97-4.03(1H,m),7.07(1H,dd,J=4.7,7.6 Hz),7.38(1H,d,J=7.6Hz),8.42(1H,d,J=4.7Hz).

### Synthesis Example 54-2: Synthesis of (R)-8-amino-5,6,7.8-tetra hydroquinoline-L-tartrate

99.8 mg of the compound obtained in Synthesis Example 54-1 was dissolved in 5 ml of methanol again, and then 5 ml of chloroform was added to the solution and the whole was allowed to stand for 3 days. A precipitated crystal was collected by filtration, and 30.1 mg of a white needle crystal was obtained. Furthermore, 18.4mg of the crystal was dissolved in 1 ml of methanol, followed by the addition of 0.18 ml of chloroform. A precipitated crystal was collected by filtration, and 9.8 mg of an optically active substance of the above-mentioned amine was obtained as a white needle crystal. The resulting crystal was determined as an R body by X-ray structural analysis.
[α]_{D}=-25.5° (H₂O, c=0.2)

### Synthesis Example 54-3: Synthesis of (2S)-2-(4-(N-2-picolyl-N-Boc aminomethyl) naphthoylamino)-5-((8R)-5,6,7,8-tetrahydro quinolin-8-ylamino) valerate 1-naphthalenemethylamide (Compound XIII-14)

669.3 mg of the compound obtained in Synthesis Example 53-1 was dissolved in 6.7 ml of methanol, and then 3.3 ml of a 4 mol/l hydrochloric acid/dioxane was added to the solution and the whole was stirred for 1 hour at room temperature. On completion of the reaction, the solvent was distilled off and the residue was dried under reduced pressure, followed by dissolving in 12 ml of DMF. Subsequently, 404 mg of WSCI hydrochloride, 257 mg of DMAP, and 606 mg of the compound obtained in Synthesis Example 43-2 were added to the solution and the whole was stirred for 11 hours at room temperature. On completion of the reaction, the solvent was distilled off and a residue was dissolved in chloroform, followed by washing with a 1 mol/l hydrochloric acid and a saturated salt solution. After the solution was dried with anhydrous sodium sulfate, the solvent was distilled off. The resulting residue was dissolved in 20 ml of THF, and then 10 ml of a THF solution of lithium hydride (160 mg) was added to the solution and the whole was stirred for 1.5 hours at room temperature. On completion of the reaction, ethyl acetate was added thereto and the whole was filtrated using celite. After the solvent was distilled off, the resultant was purified by means of silica gel column chromatography (30 g, chloroform/methanol = 10/1) , and 464.7 mg of an intermediate was obtained. A methylene chloride (0.3 ml) solution of the obtained intermediate (58.9 mg) was dropped into a methylene chloride (1.8 ml) solution of DMSO (26 µl) and oxalyl chloride (16 µl) at -78°C. After the mixture was stirred for 1 hour, 0.1 ml of triethylamine was added thereto and the whole was heated to room temperature. Then, chloroform was added and washed with water, followed by drying with anhydrous sodium sulfate and distilling the solvent off. A residue was purified by means of silica gel column chromatography (3 g, ethyl acetate). The intermediate thus obtained was dissolved in 0.79 ml of methanol, followed by the addition of 8.8 mg of the compound obtained in Synthesis Example 54-2, 0.03 ml of acetic acid and 6 mg of NaBH₃CN. The reaction proceeded for 2 days. The reaction solution was concentrated. The resulting residue was purifiedbymeans of silica gel column chromatography (0.5 g, chloroform/methanol = 10/1), and 7.9 mg of the above-mentioned compound was obtained as colorless syrup.
MS(FAB,Pos.):m/z=777[M+1]⁺
¹H-NMR(500MHz,CDCl₃) : δ=1.46 and 1.49 (9H,2s), 1.70-2.50 (6H,m), 2.80-2.92 (2H,m), 3.09-3.18 (1H,m), 3.24-3.31 (1H,m), 4.32-4.45 (2H, m), 4.52-4.59 (1H,m), 4.85-5.10 (5H,m), 7.08-7.62 (15H,m), 7.79 (1H,d ,J=7.8Hz), 7.86 (1H,d,J=8.1Hz), 8.00-8.07 (1H,m), 8.12 (1H,d,J=8.8H z),8.17-8.23(1H,m),8.40(1H,brs),8.51(1H,brs).

### Synthesis Example 54-4: Synthesis of (2S)-2-(4-(N-2-picolyl aminomethyl) naphthoylamino)-5-((8R)-5,6,7,8-tetrahydro quinolin-8-ylamino) valerate 1-naphthalenemethylamide [Compound No. 55]

7.2 mg of the compound obtained in Synthesis Example 54-3 was dissolved in 0.15 ml of methanol, and then 0.15 ml of a 4mol/l hydrochloric acid/dioxane was added to the solution. After 2 hours , the reaction solution was concentrated. The residue thus obtained was purified by means of silica gel column chromatography (chloroform/methanol/water = 7/3/0.5), and a 1 mol/l hydrochloric acid was then added to the resulting residue, followed by concentrating and azeotropically distilling with methanol. Consequently, 6.3 mg of hydrochloride of the above-mentioned compound was obtained as a white solid product.
MS(FAB,Pos.):m/z=677[M+1]⁺
¹H-NMR(500MHz,DMSO-d₆):δ=1.62-2.01(7H,m),2.30-2.40(1H,m),2.78-2.88(2H,m),2.95-3.03(1H,m),3.04-3.22(1H,m),4.47(3H,m),4.61-4. 70(1H,m),4.79(2H,s),4.83(1H,d,J=15.6,5.6Hz),4.85(1H,dd,J=15.6 ,5.6Hz),7.37-7.42(1H,m),7.47-7.51(2H,m),7.52-7.64(5H,m),7.67-7.73(3H,m),7.80(1H,d,J=7.1Hz),7.88(1H,d,J=8.1Hz),7.93(1H,td,J =7.6,1.7Hz),8.07-8.13(1H,m),8.26-8.33(2H,m),8.49(1H,d,J=4.6Hz ),8.71(1H,d,J=4.8Hz) ,8.76(1H,brs),8.87(1H,d,J=7.8Hz),9.10(2H, br), 9.80 (2H,br).

### Synthesis Example 55: Production of (S)-2-(4-(imidazol-2-yl methyl) aminomethyl) naphthoylamino)-5-(2-picolylamino)valerate (1'S)-(1'-(1-naphthyl) ethyl) amide [Compound No. 56]

### Synthesis Example 55-1: Synthesis of N(-Boc-L-glutamate (1'S)-(1'-(1-naphthyl) ethyl) amideγ-benzylester (Compound XXXV-2)

1.05 g of commercially available N(-Boc-L-glutamate γ-benzylester was dissolved in 21.0 ml of DMF, and then 0.799 g of commercially available (S)-1-(1-naphthyl) ethylamine, 0.894 g of WSCI hydrochloride, and 0.631 g of HOBt were added to the solution and the whole was left standing for 1 day at room temperature. After the termination of a reaction was confirmed using TLC. After that, the reaction system was concentrated without modification under a reduced pressure, and then a 1N hydrochloric acid aqueous solution was added to a residue the reactant, followed by separatory extraction with chloroform. The resulting organic phase was washed with a saturated sodium bicarbonate solution and was then dried with anhydrous sodium sulfate and concentrated under a reduced pressure. A residue was subjected to silica gel column chromatography (50 g, chloroform/ethyl acetate = 5/1), and 1.42 g of the above-mentioned compound was obtained as a white solid.
MS(FAB,Pos.):m/z=491[M+1]⁺
¹H-NMR(500MHz,CDCl₃):δ=1.40(9H,s),1.63(3H,d,J=6.8Hz),1.84-1.91 (1H,m),2.01-2.10(1H,m),2.32-2.36(1H,m),2.45-2.51(1H,m),4.05-4 .15(1H,m),5.07(2H,s),5.22-5.30(1H,m),5.90(1H,t,J=7.3Hz),6.42-6.50(1H,m),7.23-7.53(9H,m),7.78(1H,d,J=8.1Hz),7.84(1H,d,J=7.8 Hz),8.04(1H,d, J=8.5Hz).

### Synthesis Example 55-2: Synthesis of N^{α}-(4-(N-Boc-aminomethyl) naphthoyl)-L-glutamate (1'S)-(1'-(1-naphthyl) ethyl) amide γ-methylester (XL-1)

1.01 g of the compound obtained in Synthesis Example 55-1 was dissolved in 15.1 ml of methanol, and then 15.1 ml of a 10% hydrochloric acid/methanol solution was added to the solution and the whole was allowed to stand for 1 day at room temperature. The termination of the reaction was confirmed using TLC. After that, the reaction system was concentrated without modification under reduced pressure and dried under vacuum. The resultant product was dissolved in 12.9 ml of DMF, and then 0.620 g of the compound obtained in Synthesis Example 25-2, 0.592 g of WSCI hydrochloride and 0.752 g of DMAP were added to the solution and the whole was left standing for 1 day at room temperature. The termination of a reaction was confirmed using TLC. After that, the reaction system was concentrated without modification under reduced pressure, and then a saturated sodiumbicarbonate solution was added to the residue, followed by separatory extraction with chloroform. The resulting organic phase was dried with anhydrous sodium sulfate and concentrated under reduced pressure. A residue was purified by means of silica gel column chromatography (50 g, chloroform/methanol = 40/1), and 1.12 g of the above-mentioned compound was obtained as a white solid product.
MS(FAB,Pos.):m/z=598[M+1]⁺
¹H-NMR(500MHz,CDCl₃):δ=1.47(9H,s),1.69(3H,d,J=6.6Hz),2.01-2.08 (1H,m) ,2.15-2.22(1H,m),2.35-2.42(1H,m),2.56-2.63(1H,m),3.62(3 H,s),4.74-4.87(3H,m),5.95(1H,quintet,J=7.0Hz),6.82-6.90(1H,m) 6.96-7.02(1H,m),7.40-7.60(9H,m),7.81(1H,d,J==8.3Hz),7.88(1H,d ,J=7.8Hz),8.04-8.10(2H,m),8.29(1H,d,J=8.1Hz).

### Synthesis Example 55-3: Synthesis of (S)-2-(4-(N-Boc-aminomethyl) naphthoylamino)-4-formyl-lactate (1'S)-(1'-(1-naphthyl) ethyl) amide (XLI-1)

0.213 g of lithium aluminum hydride was suspended in 11.2 ml of anhydrous tetrahydrofuran, and then a solution prepared by dissolving 1.12 g of the compound obtained in Synthesis Example 55-2 in 22.4 ml of anhydrous tetrahydrofuran was added to the suspension and the whole was stirred for 0.5 hour at room temperature. The termination of a reaction was confirmed using TLC. After that, ethyl acetate, methanol, and a 10% sodiumpotass ium tartrate aqueous solution were added to the reaction solution in order and the whole was stirred for an additional 1 hour, followed by separatory extraction with chloroform. The resulting organic phase was dried with anhydrous sodium sulfate and concentrated under reduced pressure. A residue was purified by means of silica gel column chromatography (80 g, chloroform/methanol = 15/1) , and 1.44 g of intermediate alcohol was obtained as a white solid product. To 20.1 ml of methylene chloride, 0.151 ml of oxalyl chloride and 0.251 ml of dimethyl sulfoxide were added while being stirred at -78°C. After the mixture was stirred for 0.5 hour, a solution prepared by dissolving 0.670 g of the intermediate alcohol obtained in advance in 6.70 ml of methylene chloride was added thereto. After the mixture was stirred for additional 0.5 hour, 0.738 ml of triethylamine was added thereto and the whole was stirred for 1.5 hours under ice-cold condition. Water was added to the reaction solution, followed by separatory extraction with chloroform. The resulting organic phase was dried with anhydrous sodium sulfate and concentrated under reduced pressure. A residue was purified by means of silica gel column chromatography (50 g, chloroform/methanol = 30/1), and 0.450 g of the above-mentioned compound was obtained as a light-orange solid product.
MS(FAB,Pos.):m/z=568[M+1]⁺
¹H-NMR(500MHz,CDCl₃):δ=1.47(9H,s),1.73(3H,d,J=6.8Hz),1.82-1.99 (2H,m),2.28-2.40(1H,m),3.56-3.62(2H,m),4.62-4.92(4H,m),5.98(1 H,t,J=7.3Hz),7.08-7.20(2H,m),7.36-7.60(BH,m),7.79(1H,d,J=8.1H z),7.84-7.90(1H,m),8.04-8.16(3H,m).

### Synthesis Example 55-4: Synthesis of (S)-2-(4-(N-Boc-aminomethyl) naphthoylamino)-5-(2-picolylamino) valerate (1'S)-(1'-(1-naphthyl) ethyl) amide (XXI-5)

200.0 mg of the compound obtained in Synthesis Example 55-3 was dissolved in 6.0 ml of methanol, and then 44.3 mg of 2-aminomethylpyridine, and 44.3 mg of sodium cyanoborohydride were added to the solution at room temperature and the pH thereof was adjusted to pH = 4 to 5 with acetic acid, followed by stirring for 2 days. After disappearance of raw materials was confirmed with TLC, the reaction solution was concentrated without modification under reduced pressure and then water was added, followed by separatory extraction with chloroform. The organic phase thus obtained was dried with anhydrous sodium sulfate and concentrated under reduced pressure. A residue was purified by means of silica gel chromatography (10 g, chloroform/methanol = 15/1), and 91.5 mg of the above-mentioned compound was obtained as a light-yellow solid product.
MS(FAB,Pos.):m/z=660[M+1]⁺
¹H-NMR(500MHz,CDCl₃):δ=1.47(9H,s),1.67(3H,d,J=6.8Hz),1.70-1.90 (2H,m), 1.90-2.04 (2H,m), 2.88-3.00 (2H,m), 3.87 (2H,s), 4.62-4.75 (2 H,m), 4.82-4.88 (2H,m), 5.90-6.00 (1H,m), 7.00-7.10 (2H,m), 7.34 (1H, d,J=7.1Hz), 7.40-7.58 (7H,m), 7.77 (1H,m,J=8.1Hz), 7.87 (1H,d,J=7.6 Hz), 7.90-8.02 (2H,m), 8.12 (1H,d,J=8.5Hz), 8.20-8.32 (2H,m).

### Synthesis Example 55-5: Synthesis of (S)-2-(4-(imidazol-2-yl methyl) aminomethyl) naphthoylamino)-5-(2-picolylamino)valerate (1'S)-(1'-(1-naphthyl) ethyl) amide [Compound No. 56]

91.5 mg of the compound obtained in Synthesis Example 55-4 was dissolved in 2 ml of methanol, and then 2 ml of a 4 mol/l hydrochloric acid/dioxane was added to the solution and the whole was stirred for 1 hour. On completion of the reaction, the solvent was distilled off and a residue dried under vacuum. The resultant was dissolved in anhydrous methanol, and then 58.0 ml of triethylamine, and 16.0 mg of 2-imidazole carboaldehyde were added to the solution and the whole was stirred for 5 hours at room temperature. On completion of the reaction, the resultant was concentrated and dried under reduced pressure, followed by dissolving in 2 ml of anhydrous methanol and cooling to 0°C. Then, 10.5 mg of sodium borohydride was added to the solution and the whole was stirred for 0.5 hours. On completion of the reaction, the solution was concentrated. The residue was purified by means of silica gel column chromatography (5 g, chloroform/methanol/water = 7/3/0.5), followed by treating with a 1 mol/l hydrochloric acid. Consequently, 63. 5 mg of hydrochloride of the above-mentioned compound was obtained as a white solid product.
MS(FAB,Pos.):m/z=640[M+1]⁺
¹H-NMR (500MHz ,DMSO-d6) : δ=1.56 (3H ,d ,J=6.8Hz) ,1.66-1.90 (4H ,m) ,2 .96-3.08(2H,m),4.26-4.32(2H,m),4.62-4.78(2H,m),4.82-4.94(2H,m ),5.74-5.82(1H,m),7.40-7.96(14H,m),8.14(1H,d,J=8.5Hz),8.28(1H ,d,J=8.5Hz),8.30-8.36(1H,m),8.63(1H,d,J=7.1Hz),8.83(1H,d,J=7. 6Hz) ,8.80-8.84 (1H,m).

### Synthesis Example 56: Production of (S)-2-(4-(imidazol-2-yl methyl) aminomethyl) naphthoylamino)-5-(5,6,7,8-tetrahydro quinolin-8-ylamino) valerate(1'S)-(1'-(1-naphthyl) ethyl) amide [Compound No. 57]

### Synthesis Example 56-1: Synthesis of (S)-2-(4-(N-Boc-aminomethyl) naphthoylamino)-5-(5,6,7,8-tetrahydroquinolin-8-ylamino) valerate (1'S)-(1'-(1-naphthyl) ethyl) amide (XXI-6)

200.0 mg of the compound obtained in Synthesis Example 55-3 was dissolved in 6.00 ml of methanol, and then 126.1 mg of amine obtained in Synthesis Example 54-1 and 44.3 mg of sodium cyanoborohydride were added to the solution at room temperature and the pH thereof was adjusted to pH = 4 to 5 with acetic acid, followed by stirring for 23 hours. After disappearance of raw materials was confirmed with TLC, the reaction solution was concentrated without modification under reduced pressure and then water was added thereto, followed by separatory extraction with chloroform. The organic phase thus obtained was dried with anhydrous sodium sulfate and concentrated under reduced pressure. A residue was purified by means of silica gel chromatography (10 g, chloroform/methanol = 15/1), and 162.9 mg of the above-mentioned compound was obtained as a light-yellow solid product.
MS(FAB,Pos.):m/z=700[M+1]⁺
¹H-NMR(500MHz,CDCl₃): δ=:1.47(9H,s),1.68(3H,d,J=6.6Hz),1.76-2.1 0(8H,m),2.20-2.40(2H,m), 2.60-2.78(2H,m),3.02-3.38(4H,m),4.62-4.96(4H,m),5.84-5.96(1H,m),7.00-7.34.(8H,m),7.73-7.90(2H,m),7 .94-8.40(4H,m).

### Synthesis Example 56-2: Synthesis of (S)-2-(4-(imidazol-2-yl methyl) aminomethyl) naphthoylamino)-5-(5,6,7,8-tetrahydro quinolin-8-ylamino) valerate (1'S)-(1'-(1-naphthyl) ethyl) amide [Compound No. 57]

162.9 mg of the compound obtained in Synthesis Example 56-1 was dissolved in 2 ml of methanol, and then 2 ml of a 4 mol/l hydrochloric acid/dioxane was added to the solution and the whole was stirred for 1 hour at room temperature. On completion of the reaction, the solvent was distilled off and a residue was dried under vacuum. The resultant was dissolved in anhydrous methanol, and then 97.3 µl of triethylamine, and 26.8 mg of 2-imidazolecarboaldehyde were added to the solution and the whole was stirred for 5 hours at room temperature. On completion of the reaction, the resultant was concentrated and dried under reduced pressure, followed by dissolving in 2 ml of anhydrous methanol and cooling to 0°C. Then, 17.6 mg of sodium borohydride was added to the solution and the whole was stirred for 0.5 hours. On completion of the reaction, the solution was concentrated. A residue was purified by means of silica gel column chromatography (5 g, chloroform/methanol/water = 7/3/0. 5) , followed by treating with a 1 mol/l hydrochloric acid. Consequently, 102.0 mg of hydrochloride of the above-mentioned compound was obtained as a white solid product.
MS(FAB,Pos.):m/z=680[M+1]⁺
¹H-NMR(500MHz.DMSO-d₆) : δ=1.56(3H,d,J=6.8Hz),1.70-1.94 (6H,m),2. 24-2.36(2H,m),2.78-2.82(1H,m),4.60-4.76(2H,m),4.82-4.90(2H,m) , 5.76-5.80 (1H,m), 7.36-7.40 (1H,m), 7.44-7.66 (12H,m), 7.84 (1H,d,J =7.8Hz), 7.96 (1H,d,J=7.6Hz), 8.14 (1H,d,J=8.5Hz), 8.24-8.36 (2H,m) ,8.42-8.46(1H,m),8.68-8.82(2H,m).

### Synthesis Example 57: Production of (S)-2-(4-(imidazol-2-yl methyl)aminomethyl)naphthoylamino)-5-(2-picolylamino)valerate (1'R)-(1'-(1-naphthyl) ethyl) amide [Compound No. 58]

### Synthesis Example 57-1: Synthesis of N^{α}-Boc-L-glutamate(1'R)-(1'-(1-naphthyl) ethyl) amide γ-benzylester (Compound XXXV-3)

1.12g of commercially available N^{α}-Boc-L-glutamate γ-benzylester was dissolved in 21.0 ml of DMF, and then 0.853 g of commercially available (R)-1-(1-naphthyl) ethylamine, 0.955 g of WSCI hydrochloride and 0.673 g of HOBt were added to the solution and the whole was left standing for 1 day at room temperature. The termination of a reaction was confirmed using TLC. After that, the reaction system was concentrated without modification under reduced pressure, and then a 1N hydrochloric acid was added to a residue, followed by separatory extraction with chloroform. The resulting organic phase was washed with a saturated sodium bicarbonate solution and was then dried with anhydrous sodium sulfate and concentrated under reduced pressure. A residue was subjected to silica gel column chromatography (50 g, chloroform/ethyl acetate = 5/1) , and 1.63 g of the above-mentioned compound was obtained as a white solid.
MS(FAB,Pos.):m/z=491[M+1]⁺
¹H-NMR(500MHz,CDCl₃): δ=1.33(9H,s),1.63(3H,d,J=6.8Hz),1.88-1.98 (1H,m),2.12-2.22(1H,m),2.45(1H,dt,J=6.8,16.6Hz),2.50-2.58(1H, m),4.08-4.20(1H,m),5.11(2H,s),5.04-5.09(1H,m),5.88(1H,t,J=7.1 Hz),6.48-6.62(1H,m),7.23-7.53(9H,m),7.78(1H,d,J=7.8Hz),7.84(1 H,d, J=7.8Hz), 8.04 (1H,d, J=8.3Hz).

### Synthesis Example 57-2: Synthesis of N^{α}-(4-(N-Boc-aminomethyl) naphthoyl)-L-glutamate (1'R)-(1'-(1-naphthyl) ethyl) amide γ-methylester (XL-2)

1.15 g of the compound obtained in Synthesis Example 57-1 was dissolved in 17.3 ml of methanol, and then 17.3 ml of a 10% hydrochloric acid/methanol solution was added to the solution and the whole was left standing for 1 day at room temperature. The termination of the reaction was confirmed using TLC. After that, the reaction system was concentrated without modification under reduced pressure and dried under vacuum, and the above-mentioned compound was obtained as a white solid product. The product was dissolved in 14.7 ml of DMF, and then 0.706 g of the compound obtained in Synthesis Example 25-2, 0.674 g of WSCI hydrochloride and 0. 859 g of DMAP were added to the solution and the whole was left standing for 1 day at room temperature. The termination of the reaction was confirmed using TLC. After that, the reaction system was concentrated without modification under reduced pressure, and then a saturated sodium bicarbonate solution was added to the residue, followed by separatory extraction with chloroform. The resulting organic phase was dried with anhydrous sodium sulfate and concentrated under reduced pressure. A residue was purified by means of silica gel column chromatography (50 g, chloroform/methanol = 40/1) , and 1.03 g of the above-mentioned compound was obtained as a light-yellow solid product.
MS(FAB,Pos.):m/z=598[M+1]⁺
¹H-NMR(500MHz,CDCl₃):δ=1.47(9H,s),1.69(d,3H,J=6.8Hz),2.11-2.19 (1H,m),2.28-2.35(1H,m),2.50-2.56(1H,m),2.66-2.72(1H,m),3.67(3 H,s),4.70-4.84(3H,m),5.95(1H,quintet,J=7.0Hz),6.82-6.92(1H,d, J=7.8Hz),6.99(1H,d,J=8.3Hz),7.30-7.57(9H,m),7.77(1H,d,J=8.1Hz ),7.85(1H,d,J=7.8Hz),8.03(1H,d,J=8.3Hz),8.09(1H,d,J=8.3Hz),8. 15(1H,d, J=8.5Hz).

### Synthesis Example 57-3: Synthesis of (S)-2-(4-(N-Boc-aminomethyl) naphthoylamino)-4-formyl-lactate (1'R)-(1'-(1-naphthyl) ethyl) amide (XLI-2)

0.196 g of lithium aluminum hydride was suspended in 10.3 ml of anhydrous tetrahydrofuran, and then a solution prepared by dissolving 1.03 g of the compound obtained in Synthesis Example 57-2 in 20.6 ml of anhydrous tetrahydrofuran was added to the suspension and the whole was stirred for 0.5 hours at room temperature. The termination of the reaction was confirmed using TLC. After that, ethyl acetate, methanol, and a 10% sodium potassium tartrate aqueous solution were added to the reaction solution in order and the whole was stirred for an additional 1 hour, followed by separatory extraction with chloroform. The resulting organic phase was dried with anhydrous sodium sulfate and concentrated under reduced pressure. A residue was purified by means of silica gel column chromatography (80 g, chloroform/methanol = 15/1), and 0.785 g of intermediate alcohol was obtained as a white solid product. To 23.6 ml of methylene chloride, 0.177 ml of oxalyl chloride and 0.294 ml of dimethyl sulfoxide were added while being stirred at -78°C. After the mixture was stirred for 0.5 hours, a solution prepared by dissolving 0.670 g of the intermediate alcohol obtained in advance in 6.70 ml of methylene chloride was added thereto. After the mixture was stirred for an additional 0.5 hours, 0.864 ml of triethylamine was added thereto and the whole was stirred for 1.5 hours under ice-cold condition. Water was added to the reaction solution, followed by separatory extraction with chloroform. The resulting organic phase was dried with anhydrous sodium sulfate and concentrated under reduced pressure. A residue was purified by means of silica gel column chromatography (50 g, chloroform/methanol = 30/1), and 0.446 g of the above-mentioned compound was obtained as a light-orange solid product.
MS(FAB,Pos.):m/z=568[M+1]⁺
¹H-NMR(500MHz,CDCl₃): δ=1.45(9H,s),1.63(3H,d,J=6.8Hz),1.80-2.42 (4H,m),3.37(2H,m),4.40-5.04(4H,m),5.90-5.98(1H,m),7.05(1H,d,J =7.1Hz),7.12-7.60(8H,m),7.64-8.16(5H,m).

### Synthesis Example 57-4 Synthesisof (S)-2-(4-(N-Boc-aminomethyl) naphthoylamino)-5-(2-picolylamino) valerate (1'R)-(1'-(1-naphthyl) ethyl) amide (XXI-7)

200.0 mg of the compound obtained in Synthesis Example 57-3 was dissolved in 6.00 ml of methanol, and then 44.3 mg of 2-aminomethylpyridine, and 44.3 mg of sodium cyanoborohydride were added to the solution at room temperature and the pH thereof was adjusted to pH = 4 to 5 with acetic acid, followed by stirring for 3 days. After disappearance of raw materials was confirmed with TLC, the reaction solution was concentrated without modification under reduced pressure and then water was added thereto, followed by separatory extraction with chloroform. The organic phase thus obtained was dried with anhydrous sodium sulfate and concentrated under reduced pressure. A residue was purified by means of silica gel column chromatography (10 g, chloroform/methanol = 10/1) , and 66.6 mg of the above-mentioned compound was obtained as a light-yellow solid product.
MS(FAB,Pos.):m/z=660[M+1]⁺
¹H-NMR(500MHz,CDCl₃) : δ=1.47 (9H,s), 1.68 (3H,d,J=6.8Hz), 1.90-2.20 (4H,m), 3.00-3.16 (2H,m), 3.86-4.00 (2H,m), 4.64-4.88 (4H,m), 5.90-6 .00 (1H,m), 7.00-7.18 (2H,m), 7.28-7.60 (8H,m), 7.73 (1H,d,J=8.1Hz), 7.83 (1H,d,J=8.1Hz), 7.90-8.02 (2H,m), 8.14 (1H,d,J=8.3Hz), 8.22 (2H ,d,J=8.3Hz).

### Synthesis Example 57-5: Synthesis of (S)-2-(4-(imidazol-2-yl methyl) aminomethyl) naphthoylamino)-5-(2-picolylamino)valerate (1'R)-(1'-(1-naphthyl) ethyl) amide [Compound No. 58]

66.6 mg of the compound obtained in Synthesis Example 57-4 was dissolved in 2 ml of methanol, and then 2 ml of a 4 mol/l hydrochloric acid/dioxane was added to the solution and the whole was stirred for 1 hour at room temperature. On completion of the reaction, the solvent was distilled off and a residue was dried under vacuum. The resultant was dissolved in anhydrous methanol, and then 42.2 µl of triethylamine, and 11.6 mg of 2-imidazolecarboaldehyde were added to the solution and the whole was stirred for 5 hours at room temperature. On completion of the reaction, the resultant solution was concentrated and dried under reducedpressure, followedbydissolving in 2 ml of anhydrous ethanol and cooling to 0°C. Then, 7.6 mg of sodium borohydride was added to the solution and the whole was stirred for 0.5 hours at room temperature. On completion of the reaction, the solution was concentrated. The residue was purified by means of silica gel column chromatography (5g, chloroform/methanol/water = 7/3/0.5), followedby treating with a 1 mol/l hydrochloric acid. Consequently, 27.6 mg of hydrochloride of the above-mentioned compound was obtained as a white solid product.
MS(FAB,Pos.) :m/z=640[M+1]⁺ ¹H-NMR(500MHz,DMSO-d₆) :δ=1.55(3H,d,J=6.8Hz),1.78-1.94(4H,m), 3.00-3.08(2H,m),4.34-4.38(2H,m),4.58-4.76(2H,m),4.80-4.96(2H,m) ,5.74(1H,t,J=7.0Hz),7.40-7.96(14H,m),8.14(1H,d,J=8.5Hz),8.20 (1H,d,J=8.5Hz),8.31(1H,d,J=9.0Hz),8.65(1H,d,J=7.1Hz),8.78-8.84 (2H,m).

### Synthesis Example 58: Production of (S)-2-(4-(imidazol-2-yl methyl) aminomethyl) naphthoylamino)-5-(5,6,7,8-tetrahydro quinolin-8-ylamino) valerate (1'R)-(1'-(1-naphthyl) ethyl) amide [Compound No. 59]

### Synthesis Example 58-1: Synthesis of (S)-2-(4-(N-Boc-aminomethyl) naphthoylamino)-5-(5,6,7,8-tetrahydroquinolin-8-ylamino) valerate (1'R)-(1'-(1-naphthyl) ethyl) amide (XXI-8)

200.0 mg of the compound obtained in Synthesis Example 57-3 was dissolved in 6.00 ml of methanol, and then 126.1 mg of amine obtained in Synthesis Example 54-1 and 44.3 mg of sodium cyanoborohydride were added to the solution at room temperature and the pH thereof was adjusted to pH = 4 to 5 with acetic acid, followed by stirring for 3 days. After disappearance of raw materials was confirmed with using TLC, the reaction solution was concentrated without modification under a reduced pressure and then water was added thereto, followed by separatory extraction with chloroform. The organic phase thus obtained was dried with anhydrous sodium sulfate and concentrated under a reduced pressure. A residue was purified by means of silica gel chromatography (10 g, chloroform/methanol = 15/1) , and 123.3 mg of the above-mentioned compound was obtained as a light-yellow solid product.
MS(FAB,Pos.):m/z=700(M+1]⁺
¹H-NMR(500MHz,CDCl₃):δ-1.47(9H,s),1.68(3H,d,J-6.8Hz),1.76-2.40 (8H,m),2.60-2.78(2H,m),3.04-3.40(4H,m),4.62-4.90(4H,m),5.86-5 .96(1H,m),7.04-7.12(1H,m),7.22-7.58(7H,m),7.73-7.84(3H,m),7.9 0-8.04(4H,m),8.08-8.20(1H,m),8.24-8.36,(1H,m).

### Synthesis Example 58-2: Synthesis of (S)-2-(4-(imidazol-2-yl methyl) aminomethyl) naphthoylamino)-5-(5,6,7,8-tetrahydro guinolin-8-ylamino) valerate (1'R)-(1'-(1-naphthyl) ethyl) amide [Compound No. 59]

123.3 mg of the compound obtained in Synthesis Example 58-1 was dissolved in 2 ml of methanol, and then 2 ml of a 4 mol/l hydrochloric acid/dioxane was added to the solution and the whole was stirred for 1 hour at room temperature. On completion of the reaction, the solvent was distilled off and a residue was dried under vacuum. The resultant was dissolved in 3.17 ml of anhydrous methanol, and then 73.7 µl of triethylamine, and 20.3 mg of 2-imidazolecarboaldehyde were added to the solution and the whole was stirred for 5 hours at room temperature. On completion of the reaction, the resultant was concentrated and dried under reduced pressure, followed by dissolving in 2 ml of anhydrous methanol and cooling to 0°C. Then, 13.3 mg of sodium borohydride was added to the solution and the whole was stirred for 0.5 hours at room temperature. On completion of the reaction, the solution was concentrated. The residue was purified by means of silica gel column chromatography (5 g, chloroform/methanol/water = 7/3/0.5), followedby treating with a 1 mol/l hydrochloric acid. Consequently, 75.5 mg of hydrochloride of the above-mentioned compound was obtained as a white solid product.
MS(FAB,Pos.):m/z=680[M+1]⁺
¹H-NMR(500MHz,DMSO-d₆): δ=1.55(3H,d,J=6.8Hz),1.70-2.04(6H,m),2. 32-2.40 (2H,m), 2.78-2.82 (1H,m), 4.40-4.64 (2H,m), 4.68-4.76 (2H,m) , 4.82-4.94 (2H,m), 5.74 (1H,t,J=7.0Hz), 7.38-7.42 (1H,m), 7.46-7.78 (12H,m), 7.80-7.88 (2H,m), 7.96 (1H,d,J=7.0Hz), 8.14 (1H,d,J=7.5Hz) , 8.20 (2H,d,J=8.3Hz), 8.31 (1H,d,J=8.1Hz), 8.50 (1H,d,J=4.6Hz), 8.8 0-8.88(2H,m).

### Synthesis Example 59: Production of (S)-2-(4-(N-2-picolylamino methyl) benzoylamino)-5-(N-2-picolylamino) valerate 1-naphthalenemethylamide [Compound No. 60]

48.8 mg of the compound obtained in Synthesis Example 19-3 was dissolved in 1.0 ml of methanol, and then 30.0 µl of triethylamine and 17.5 µl of 2-pyridinealdehyde were added to the solution, followed by stirring for 30 minutes at room temperature. On completion of the reaction, the solvent was distilled off. Subsequently, 1.0 ml of anhydrous methanol was added thereto and the whole was cooled to 0°C. Then, 26.6 mg of sodium borohydride was added to the solution and the whole was stirred for 40 minutes while being gradually returned to room temperature. On completion of the reaction, the solvent was distilled off and a residue was purified by means of silica gel column chromatography (2 g, chloroform/methanol/water = 7/3/0.5). After the resultant compound was dissolved in a 1 mol/l hydrochloric acid, and water was distilled off. Consequently, 24.8 mg of hydrochloride of the above-mentioned compound was obtained as a white solid product.
MS(FAB,Pos.):m/z=587[M+1]⁺
¹H-NMR(500MHz, DMSO-d₆): δ=1.75-1.90 (4H,m) ,2.90-3.10 (2H,m) ,4.29 and 4.30(6H,s),4.54-4.58(1H,m),4.76(2H,d,J=5.6Hz),7.40-7.49 (4H,m),7.52-7.61(4H,m),7.67(2H,d,J=8.1Hz),7.84-7.86(1H,m),7.9 1-7.96 (3H,m) ,7.99 (2H,d,J=8.1Hz), 8.05-8.08 (1H,m) ,8.62 (1H,d,J=4 .9Hz),8.66(1H,d,J=4.9Hz),8.69-8.73(2H,m),9.37(2H,brs),9.95(2H ,brs).

### Synthesis Example 60: Production of (S)-2-(4-(N-2-picolylamino methyl) methylbenzoylamino)-4-(N-2-picolylamino) lactate 1-naphthalenemethylamide [Compound No. 61]

### Synthesis Example 60-1: Synthesis of (S)-2-(N-Fmoc amino)-4-(N-Boc amino) lactate 1-naphthalenemethylamide (Compound X-3)

201.9 mg of commercially available (S)-2-(N-Fmoc amino) lactate was dissolved in 2.0 ml of DMF, and then 106.1 mg of WSCI hydrochloride and 71.0 mg of HOBt were added and dissolved in the solution. Then, 67 ml of 1-naphthalene methylamine was added to the solution and the whole was stirred for 16 hours at room temperature. On completion of the reaction, the solvent was distilled off. The residue was purified by means of silica gel column chromatography (10 g, chloroform/methanol=30/1), and 231. 8 mg of the above-mentioned compound was obtained as a white solid product.
MS(FAB,Pos.):m/z=580(M+1]⁺
¹H-NMR(500MHz,DMSO-d₆) : δ=1.37(9H,s),1.65-1.73(1H,m),1.78-1.83 (1H,m),2.97-3.03(2H,m),4.05-4.10(1H,m),4.20-4.28(3H,m),4.75(2H ,brs),6.75(1H,brs),7.30-7.37(2H,m),7.42(2H,t,J=7.4Hz),7.43-7. 49(2H,m),7.50-7.57(2H,m),7.64(1H,d,J=8.3Hz),7.74(1H,t,J=6.7Hz ),7.84(1H,d,J=7.3Hz),7.90(2H,d,J=7.4Hz),7.93(1H,m),8.02(1H,d, J=6.3Hz),8.42(1H,t,J=5.6Hz).

### Synthesis Example 60-2: Synthesis of (S)-2-(4-(N-Boc aminomethyl) benzoylamino)-4-(N-Boc amino) lactate 1-naphthalenemethyl amide (Compound XVIII-8)

152.2 mg of the compound obtained in Synthesis Example 60-1 was dissolved in 3.0 ml of DMF, and then 0.3 ml of diethylamine was added to the solution and the whole was stirred for 50 minutes at room temperature. On completion of the reaction, the solvent was distilled off and a residue was dried by vacuum pump. The resulting compound was used in a subsequent reaction without purification. 141.7 mg of the resulting mixture was dissolved in 3 ml of DMF, and then 75.6 mg of WSCI hydrochloride, 35.5 mg of HOBt and 79.3 mg of the compound obtained in Synthesis Example 19-1 were added to the solution and the whole was stirred for 22 hours at room temperature. On completion of the reaction, the solvent was distilled off. A residue was dissolved in chloroform, and washed with a 1 mol/l hydrochloric acid, a 1 mol/l sodium hydroxide aqueous solution and a saturated salt solution. An organic layer was dried with anhydrous sodium sulfate, followed by distilling the solvent off. The residue was purified by means of silica gel column chromatography (7.5 g, chloroform/methanol = 30/1, and 109.1 mg of the above-mentioned compound was obtained as a white solid product.
MS(FAB,Pos.):m/z=591[M+1]⁺
¹H-NMR(500MHz,DMSO-d₆) : δ=1.35(9H,s) ,1.39(9H,s) ,1.83-1.87(1H,m) ,1.91-1.95(1H,m),2.98-3.03(2H,m),4.17(2H,d,J=6.3Hz),4.48-4.52 (1H,m),4.75(2H,d,J=5.6Hz),6.78(1H,t,J=5.3Hz),7.31(2H,d,J=8.1H z),7.44-7.49(3H,m),7.52-7.56(2H,m),7.83-7.86(3H,m),7.93-7.96 (1H,m),8.04-8.06 (1H,m), 8.47-8.49(2H,m).

### Synthesis Example 60-3: Synthesis of (S)-2-(4-aminomethyl benzoylamino)-4-aminolactate 1-naphthalenemethylamide (Compound XIX-2)

49.7 mg of the compound obtained in Synthesis Example 60-2 was dissolved in 0.5 ml of methanol, and then 0.5 ml of a 4 mol/l hydrochloric acid/dioxane was added to the solution and the whole was stirred for 1.5 hours at room temperature. On completion of the reaction, the solvent was distilled off and a residue was then dried by a vacuum pump, and 52.4 mg of the above-mentioned compound was obtained as a white solid product.
MS(FAB,Pos.):m/z=391 M+1]⁺

### Synthesis Example 60-4: Synthesis of (S)-2-(4-(N-2-picolylamino) methylbenzoylamino)-4-(N-2-picolylamino) lactate 1-naphthalene methylamide [Compound No. 61]

45.1 mg of the compound obtained in Synthesis Example 60-3 was dissolved in 0.9 ml of methanol and then 24.4 µl of triethylamine and 14.4 µl of 2-pyridinealdehyde were added to the solution, followed by stirring for 70 minutes at room temperature. On completion of the reaction, the solvent was distilled off. Subsequently, 1.0 ml of anhydrous methanol was added thereto and the whole was cooled to 0°C. Then, 23.4 mg of sodium borohydride was added to the solution and the whole was stirred for 35 minutes while being gradually returned to room temperature. On completion of the reaction, the solvent was distilled off and a residue was purified by means of silica gel column chromatography (chloroform/methanol/water = 7/3/0.5). After the resultant compound was dissolved in a 1 mol/l hydrochloric acid, water was distilled off. Consequently, 28.4 mg of hydrochloride of the above-mentioned compound was obtained as a white solid product.
MS(FAB,Pos.):m/z=573[M+1]⁺
¹H-NMR(500MHz,DMSO-d₆):δ2.20-2.37(4H,m) ,3.0-3.15(2H,m) ,4.22-4. 37(6H,m),4.61-4.67(1H,m),4.74(1H,dd,J=15.6,5.9Hz),4.79(1H,dd, J=15.6,5.9Hz),7.44-7.49(4H,m),7.52-7.58(3H,m),7.61(1H,d,J=7.8 Hz),7.67(2H,d,J=8.5Hz),7.86(1H,dd,J=7.3,1.9Hz),7.89-7.96(3H,m ),7.99(2H,d,3=8.5Hz),8.06-8.09(1H,m),8.62(1H,dd,J=4.9,1.7Hz), 8.67(1H,dd,J=4.9,1.7Hz),8.74(1H,t,J=5.6Hz),8.86(1H,d,J=7.8Hz) ,9.46(2H,brs),9.96(2H,brs).

### Synthesis Example 61: Production of (S)-2-(4-(N-2-picolylamino) methylbenzoylamino)-3-(N-2-picolylamino) propionate 1-naphthalenemethylamide [Compound No. 62]

### Synthesis Example 61-1: Synthesis of (S)-2-(N-Fmoc amino)-3-(N-Boc amino) propionate 1-naphthalenemethylamide (Compound X-4)

201.6 mg of commercially available (S)-2-(N-Fmoc amino)-4-(N-Boc amino) propionate was dissolved in 2.0 ml of DMF, and then 110.0 mg of WSCI hydrochloride and 75.6 mg of HOBt were added and dissolved therein. Then, 29 µl of 1-naphthalene methylamine was added to the solution and the whole was stirred for 16 hours at room temperature. On completion of the reaction, the solvent was distilled off. The residue was purified by means of silica gel column chromatography (10 g, chloroform/methanol = 30/1), and 226.3 mg of the above-mentioned compound was obtained as a white solid product.
MS(FAB,Pos.):m/z=566[M+1]⁺
¹H-NMR(500MHz,DMSO-d₆):δ=1.37(9H,s) ,3.26-3.29(2H,m) ,4.10-4.25 (2H,m),4.27-4.29(2H,m),4.76(2H,d,J=5.6Hz),6.80(1H,t,J=5.6Hz),7 .30-7.37(2H,m),7.43(2H,t,J=7.3Hz),7.44(2H,d,J=5.8Hz),7.71(2H, d,J=7.7Hz),7.88(1H,t,J=4.3Hz),7.90(2H,d,J=7.6Hz),7.93(1H,m),8 .03(1H,d,J=6.8Hz),8.51(1H,t,J=5.6Hz).

### Synthesis Example 61-2: Synthesis of (S)-2-(4-N-Boc aminomethyl) benzoylamino)-3-(N-Boc amino) propionate 1-naphthalenemethyl amide (Compound XVIII-9)

151.6 mg of the compound obtained in Synthesis Example 61-1 was dissolved in 3.0 ml of DMF, and then 0.3 ml of diethylamine was added to the solution and the whole was stirred for 60 minutes at room temperature. On completion of the reaction, the solvent was distilled off and the residue was dried by vacuum pump. The resulting compound was used in a subsequent reaction without purification. 136.0 mg of the resulting mixture was dissolved in 3 ml of DMF, and then 76.6 mg of WSCI hydrochloride, 35.3 mg of HOBt and 79.7 mg of the compound obtained in Synthesis Example 19-1 were added to the solution and the whole was stirred for 23 hours at room temperature. On completion of the reaction, the solvent was distilled off. The residue was purified by means of silica gel column chromatography (7.5 g, chloroform/methanol = 30/1), and 46.2 mg of the above-mentioned compound was obtained as a white solid product.
MS(FAB,Pos.):m/z=577[M+1]⁺
¹H-NMR(500MHz,DMSO-d₆):δ=1.35(9H,s),1.39 (9H,s),3.34-3.72 (2H,m) ,4.16(2H,d,J=6.3Hz),4.54-4.57(1H,m),4.74(1H,dd,J=11.7,5.6Hz), 4.78(1H,dd,J=11.7,5.6Hz),7.05(1H,t,J=5.6Hz),7.31(2H,d,J=8.3Hz ),7.44-7.49(3H,m),7.51-7.57(2H,m),7.81-7.85(3H,m),7.93-7.95(1 H,m),8.02-8.05(1H,m),8.33(1H,d,J=7.8Hz),8.54(1H,t,J=5.6Hz).

### Synthesis Example 61-3: Synthesis of (S)-2-(4-aminomethylbenzoyl amino)-3-aminopropionate 1-naphthalenemethylamide (Compound XIX-3)

43.2 mg of the compound obtained in Synthesis Example 3-(2) was dissolved in 0.86 ml of methanol, and then 0.86 ml of a 4 mol/l hydrochloric acid/dioxane was added to the solution and the whole was stirred for 100 minutes at room temperature. On completion of the reaction, the solvent was distilled off and the residue was then dried by a vacuum pump, and 42 .1 mg of the above-mentioned compound was obtained as a white solid product.
MS(FAB,Pos.):m/z=377[M+1]⁺

### Synthesis Example 61-4: Synthesis of (S)-2-(4-(N-2-picolylamino) methylbenzoylamino)-3-(N-2-picolylamino) propionate 1-naphthalenemethylamide [Compound No. 62]

36.8 mg of the compound obtained in Synthesis Example 61-3 was dissolved in 0. 8 ml of methanol and then 22.1 µl of triethylamine and 13.0 µl of 2-pyridinealdehyde were added to the solution, followed by stirring for 70 minutes at room temperature. On completion of the reaction, the solvent was distilled off. Subsequently, 1 ml of anhydrous methanol was added thereto and the whole was cooled to 0°C. Then, 20.8 mg of sodium borohydride was added to the solution and the whole was stirred for 35 minutes while being gradually returned to room temperature. On completion of the reaction, the solvent was distilled off and the residue was purified by means of silica gel column chromatography (2 g, chloroform/methanol/water = 7/3/0.5). After the resultant compound was dissolved in a 1 mol/l hydrochloric acid, water was distilled off. Consequently, 25.9 mg of hydrochloride of the above-mentioned compound was obtained as a white solid product.
MS(FAB,Pos.):m/z=559[M+1]⁺
¹H-NMR(500MHz,DMSO-d₆):δ=3.4-3.7(4H,m),4.34(4H,brs),4.42(2H,s) ,4.77(1H,d,J=5.9Hz),5.00-5.05(1H,m),7.43-7.50(4H,m),7.52-7.56 (2H,m),7.58(1H,d,J=7.8Hz),7.62(1H,d,J=7.8Hz),7.69(2H,d,J=8.3H z),7.85(1H,dd,J=7.3,2.0Hz),7.90-7.96(3H,m),8.05(2H,d,J=8.3Hz) ,8.05-8.06(1H,m),8.62(1H,dd,J=4.9,1.7Hz),8.67(1H,dd,J=4.9,1.7 Hz),8.85(1H,t,J=5.6Hz),9.16(1H,d,J=7.8Hz),9.58(2H,brs),9.99(2 H,brs).

### Synthesis Example 62: Production of (S)-2-(4-(N-2-picolylamino methyl) benzoylamino)-5-(N-2-picolylamino) caprate 1-naphthalenemethylamide [Compound No. 63]

### Synthesis Example 62-1: Synthesis of N^{α}-4-(N-Fmoc aminomethyl) benzoyl-Nε-Boc-L-lysine 1-naphthalenemethylamide (Compound X-5)

510. 0 mg of commercially available N^{α}-Fmoc-N^{δ}-Boc-L-lithine was dissolved in 10 ml of DMF, and then 255.4 mg of WSCI hydrochloride and 177.6 mg of HOBt were added and dissolved therein. Then, 157 µl of 1-naphthalene methylamine was added to the solution and the whole was stirred for 2 days at room temperature. On completion of the reaction, the solvent was distilled off. Then, the residue was dissolved in chloroform and washed with a 1N hydrochloric acid and a saturated salt solution. An organic layer was dried with anhydrous sodium sulfate and then the solvent was distilled off. The residue was purifiedbymeans of silica gel column chromatography (25 g, chloroform/ethyl acetate = 2/1), and 455.9 mg of the above-mentioned compound was obtained as a white frothy product.
MS(FAB.Pos.):m/z=608[M+1]⁺
¹H-NMR(500MHz,DMSO-d₆) : δ=1.36 (9H,s), 1 .22-1.40 (4H,m), 1.55-1.65 (2H,m), 2.86 (2H,d,J=6.1,5.6Hz), 4.02 (1H,m), 4.20-4.30 (3H,m), 4.75 (2H,m), 6.78 (1H,t,J=5.6Hz), 7.31-7.35 (2H,m), 7.41 (2H,t,J=7.6Hz), 7 .43-7.47(2H,m),7.50-7.55(3H,m),7.73(2H,d,J=7.6Hz),7.84(1H,m), 7.90(2H,d,J=7.6Hz),7.94(1H,m),7.93(1H,m),8.03(1R,m),8.45(1H,t ,J=5.6Hz).

### Synthesis Example 62-2: Synthesis of N^{α}-4-(N-Boc aminomethyl) benzoyl-N^{ε}-Boc-L-lysine 1-naphthalenemethylamide (Compound XVIII-10)

301.0 mg of the compound obtained in Synthesis Example 62-1 was dissolved in 6 ml of DMF, and then 0.6 ml of diethylamine was added to the solution and the whole was stirred for 140 minutes at room temperature. On completion of the reaction, the solvent was distilled off and the residue was dried by vacuum pump. The resultant was dissolved in 6 ml of DMF, followed by adding 143.8 mg of WSCI hydrochloride, 75.4 mg of HOBt, and 137.0 mg of the compound obtained in Synthesis Example 19-1 and stirring for 13.5 hours at room temperature. On completion of the reaction, the solvent was distilled off and the residue was purified by means of silica gel column chromatography (15 g, chloroform/ethyl acetate = 1/1) , and 125.2 mg of the above-mentioned compound was obtained as a white solid product.
MS(FAB,Pos.):m/z=619[M+1]⁺
¹H-NMR(500MHz,DMSO-d₆):δ=1.35(9H,s),1.39(9H,s),1.20-1.45(4H,m) ,1.70-1.80(2H,m),2.86(2H,m),4.17(2H,d,J=6.1Hz),4.46(1H,m),4.7 5(2H,m),6.77(1H,t,J=5.6Hz),7.31(2H,d,J=8.3Hz),7.43-7.49(3H,m) ,7.51-7.56(2H,m),7.83-7.86(3H,m),7.93-7.96(1H,m),8.04-8.60(1H ,m),8.40(1H,d,J=7.6Hz), 8.52(1H,t,J=5.6Hz).

### Synthesis Example 62-3: Synthesis of N^{α}-(4-aminomethyl benzoyl) L-lysine 1-naphthalenemethylamide (Compound XIX-4)

53.7 mg of the compound obtained in Synthesis Example 62-2 was dissolved in 2.0 ml of methanol, and then 2.0 ml of a 4 mol/l hydrochloric acid/dioxane was added to the solution and the whole was stirred for 20 hours at room temperature. On completion of the reaction, the solvent was distilled off and the residue was then dried by a vacuum pump, and 51.9 mg of the above-mentioned compound was obtained as a white solid product.
MS(FAB,Pos.):m/z=419[M+1]⁺

### Synthesis Example 62-4: Synthesis of (S)-2-(4-(2-picolylamino methyl) benzoylamino)-5-(2-picolylamino) caprate 1-naphthalenemethylamide [Compound No. 63]

51.9 mg of the compound obtained in Synthesis Example 62-3 was dissolved in 1.0 ml of methanol and then 29.3 µl of triethylamine and 17.5 µl of 2-pyridinealdehyde were added to the solution, followed by stirring for 90 minutes at room temperature. On completion of the reaction, the solvent was distilled off. Subsequently, 1.0 ml of anhydrous methanol was added thereto and the whole was cooled to 0°C. Then, 26.6 mg of sodium borohydride was added to the solution and the whole was stirred for 60 minutes while being gradually returned to room temperature. On completion of the reaction, the solvent was distilled off and the residue was purified by means of silica gel column chromatography (2.5 g, chloroform/methanol/water = 7/3/0.5). After the resultant compound was dissolved in a 1N hydrochloric acid, water was distilled off. Consequently, 31.1 mg of hydrochloride of the above-mentioned compound was obtained as a white solid product.
MS(FAB,Pos.):m/z=601[M+1]⁺
¹H-NMR(500MHz,DMSO-d₆) : δ1.30-1.46(2H,m), 1.60-1.75(2H,m),1.80-1 .83(2H,m),2.80-3.00(2H,m),4.30(6H,brs),4.52(1H,m),4.73(1H,dd, J=15.3,5.6Hz),4.78(1H,dd,J=15.3,5.6Hz),7.44-7.49(3H,m),7.52-7 .55(2H,m),7.59(2H,t,J=7.8Hz),7.66(2H,d,J=8.5Hz),7.83-7.86(1H, m),7.91-7.96(3H,m),7.99(2H,d,J=8.5Hz),8.05-8.09(1H,m),8.64-8. 68(4H,m),9.36(2H,brs),9.95(2H,brs).

### Synthesis Example 63: Production of (2S)-2-(4-((5,6,7,8-tetra hydroquinolin-8-yl) aminomethyl) benzoylamino)-5-((5,6,7,8-tetrahydroquinolin-8-yl) amino) valerate 1-naphthalenemethyl amide [Compound No. 64]

25.0 mg of the compound obtained in Synthesis Example 19-3 was dissolved in 0. 5 ml of methanol and then 11 mg of triethylamine and 16.0 mg of 5, 6,7, 8-tetrahydroquinolin-8-one were added to the solution, followed by stirring for 120 minutes at room temperature. On completion of the reaction, the solvent was distilled off. Subsequently, 0.5 ml of anhydrous methanol was added thereto and the whole was cooled to 0°C. Then, 12.0 mg of sodium borohydride was added to the solution and the whole was stirred for 40 minutes while being gradually returned to room temperature. On completion of the reaction, the solvent was distilled off and the residue was purified by means of silica gel column chromatography (1 g, chloroform/methanol = 5/1). After the resultant compound was dissolved in a 1 mol/l hydrochloric acid, water was distilled off. Consequently, 10.2 mg of hydrochloride of the above-mentioned compound was obtained as a white solid product.
MS(FAB,Pos.):m/z=667[M+1]⁺
¹H-NMR(500MHz,DMSO-d₆):δ=1.76-1.83 (4H,m) ,1.85-1.91 (3H,m) ,1.98-2.05(3H,m),2.23-2.36(1H,m),2.36-2.41(1H,m),2.78-2.82(4H,m),2. 90-2.99(1H,m),3.04-3.17(1H,m),4.37(2H,m),4.41(2H,m),4.54-4.58 (1H,m),4.77(2H,m),7.36-7.41(2H,m),7.47(2H,m),7.55(2H,m),7.65-7.73(4H,m),7.84(1H,m),7.94(1H,m),8.00(2H,m),8.06(1H,m),8.45(1 H,m),8.53(1H,m),8.70(2H,m),9.12(2H,m).

### Synthesis Example 64: Production of (2S)-2-(4-(N-2-picolylamino methyl) benzoylamino)-5-((5,6,7,8 tetrahydrocrquinolin-8-yl) amino) valerate 1-naphthalenemethylamide [Compound No. 65]

### Synthesis Example 64-1: Synthesis of N^{α}-4-(N-Boc-N-2-picolyl aminomethyl) benzoyl-(N^{δ}-2-chlorobenzyloxycarbonyl)-L-ornithine 1-naphthalenemethylamide (Compound XI-10)

2.03 g of N^{α}-Fmoc-(N^{δ}-2-chlorobenzyloxycarbonyl)-L-ornithine, 1.10 g of WSCI hydrochloride and 0.79 g of HOBt were dissolved in 40 ml of DMF, followed by the addition of 0.63 ml of 1-naphthalene methylamine. After 17 hours, the reaction solution was concentrated and then a 1 mol/l hydrochloric acid was added thereto, followed by extraction with chloroform. A saturated sodium bicarbonate solution was added to an organic layer, followed by extraction with chloroform. Then, the organic layer was dried with anhydrous sodium sulfate and concentrated, and 2. 47 g of a crude product was obtained as a light-yellow solid product. 1.12 g of the resulting crude product was dissolved in 20 ml of DMF, and then 2 ml of diethylamine was added to the solution. After 1 hour, the reaction solution was concentrated and dried under reduced pressure, and syrup was obtained. Then the syrup, 0.49 g of WSCI hydrochloride, 0.31 g of DMAP and 0.58 g of the compound synthesized in Synthesis Example 1-2 were dissolved in 15 ml of DMF. After 15 hours, the reaction solution was concentrated, and then a 1 mol/l hydrochloric acid was added thereto, followed by extraction with chloroform. The organic layer was washed with a saturated salt solution, and was then dried with anhydrous sodium sulfate and concentrated. The resulting residue was purified by means of silica gel column chromatography (50 g, chloroform/methanol = 20/1), and 1.24 g of the target product was obtained as a yellow solid product.
MS(FAB,Pos.):m/z=765[M+H]⁺
¹H-NMR(500MHz,CDCl₃):δ=1.44 and 1.46(9H,2s),1.48-1.80(3H,m), 1.85-1.93(1H,m),3.08-3.15(1H,m),3.46-3.57(1H,m),4.45(1H,s),4. 50(1H,s),4.58(2H,s),4.72-4.80(2H,m),4.84-5.00(3H,m),7.10-7.52 (15H,m),7.65(1H,t,J=7.8Hz),7.73(3H,d,J=8.1Hz),7.97(1H,d,J=8.3 Hz),8.53(1H,d,J=4.2Hz).

### Synthesis Example 64-2: Synthesis of (2S)-2-4-(N-Boc-N-2-picolyl aminomethyl) benzoyl-5-((5,6,7,8-tetrahydroquinolin-8-yl) amino) valerate 1-naphthalenemethylamide (Compound XIII-15)

130 mg of the compound obtained in Synthesis Example 64-1 was dissolved in 6 ml of methanol, and then 130 mg of 10% pal ladium-carbon was added to the solution, and the whole was reacted under pressure of three atmospheres. On completion of the reaction, the palladium carbon was separated by filtration and then the solvent was distilled off, thus 0.15 g of a residue was obtained. Subsequently, the residue was dissolved in 2 ml of methanol, and 27.5 mg of 5,6,7,8-tetrahydroquinolin-8-one was added to the solution and the whole was stirred for 1 hour at room temperature. After the solvent was distilled off, the residue was dissolved in 2 ml of anhydrous methanol, and then 20.7 mg of sodium borohydride was added thereto at 0°C. The mixture was stirred for 30 minutes while the temperature thereof was gradually increased to room temperature. On completion of the reaction, the solvent was distilled off, and the residue was then purified by means of silica gel column chromatography (7 g, chloroform/methanol = 20/1) , and 18. 4 mg of the above-mentioned compound was obtained as a light-brown solid product.
MS(FAB,Pos.):m/z=727[M+1]⁺
¹H-NMR(500MHz,DMSO-d₆):δ=1.31 and 1.38(9H,s),1.48-1.68(3H,m), 1.85(3H,m),1.98(1H,m),2.35-2.72(5H,m),4.39(1H,brs),4.47(4H,br s),4.55(1H,brs),4.76(2H,brs),7.18-7.29(6H,m),7.42-7.54(5H,m), 7.77(1H,m),7.85(3H,m),7.94(1H,m),8.06(1H,m),8.33(1H,m),8.44-8 .63(3H,m).

### Synthesis Example 64-3: Synthesis of (2S)-2-(4-(N-2-picolylamino methyl) benzoylamino)-5-((5,6,7,8-tetrahydroquinolin-8-yl) amino) valerate 1-naphthalenemethylamide [Compound No. 65]

18.4 mg of the compound obtained in Synthesis Example 64-2 was dissolved in 0.4 ml of methanol. Subsequently, 0.6 ml of a 4 mol/l hydrochloric acid/dioxane was added to the solution while being stirred at room temperature, and the whole was then stirred for 2 hours. On completion of the reaction, the solvent was distilled off. Then, the residue was purified by means of silica gel column chromatography (1 g, chloroform/methanol = 1/1), and 13.1 mg of a product was obtained. The product was treated with a 1mol/l hydrochloric acid, followed bydistilling the hydrochloric acid off. Consequently, 14.0 mg of hydrochloride of the above-mentioned compound was obtained as a light-yellow solid product.
MS(FAB,Pos.):m/z=627[M+1]⁺
¹H-NMR(500MHz,DMSO-d₆):δ=1.84(6H,m),1.98(1H,m),2.39(1H,m),2.81 (1H,m),2.97(1H,m),3.09(1H,m),4.32(4H,brs),4.42(1H,m),4.56(1H, m),4.77(2H,d),7.37(1H,m),7.47(3H,m),7.51(1H,m),7.55(2H,M),7.6 6(3H,m),7.85-7.91(2H,m),7.94(3H,m),8.05(1H,m),8.46(1H,m),8.66 (1H,m),8.72(2H,m),9.18(2H,brs),9.98(2H,brs).

### Synthesis Example 65: Synthesis of (S)-2-(4-(3-picolylamino methyl) benzoylamino)-5-(3-picolylamino) valerate 1-naphthalene methylamide [Compound No. 66]

### Synthesis Example 65-1: Synthesis of (S)-2-(4-(3-picolylamino methyl) benzoylamino)-5-(3-picolylamino) valerate 1-naphthalene methylamide [Compound No. 66]

98.2 mg of the compound obtained in Synthesis Example 19-2 was dissolved in 1 ml of methanol, and then 1 ml of a 4 mol/l hydrochloric acid/dioxane was added to the solution and the whole was stirred for 2 hours at room temperature. On completion of the reaction, the solvent was distilled off and the residue was then dissolved in methanol. Subsequently, the solution was neutralized with Amberlite IRA-410 and the solvent was then distilled off. The residue was dissolved in 1 ml of anhydrous methanol, and then 34.2 µl of 3-pyridine aldehyde was added to the solution, and the whole was reacted for 4.5 hours at room temperature. The reaction solution was concentrated and dried under reduced pressure, and then the resultant was dissolved in 1.4 ml of methanol again, followed by adding 25 mg of sodium borohydride and stirring for 1 hour at room temperature. On completion of the reaction, the solvent was concentrated. Subsequently, a residue was dissolved in chloroform, followed by washing with a saturated salt solution. After the solution was dried with anhydrous sodium sulfate, the solvent was distilled off. Then, the residue thus obtained was purified by means of silica gel column chromatography (4 g, chloroform/methanol/water = 7/3/0.5) , and a 1 mol/l hydrochloric acid was then added to the resulting compound. The mixture was concentrated and azeotropically distilled, and 33.6 mg of hydrochloride of the above-mentioned compound was obtained as a white solid product.
MS(FAB,Pos.):m/z=587[M+H]⁺
¹H-NMR(500MHz,DMSO-d₆) : δ=1.72-1.95(4H,m) ,2.90-3.02(2H,m) ,4.28 (2H,s),4.33(2H,t,J=5.6Hz),4.41(2H,s)4.31(6H,s),4.55-4.61(1H,m) ,4.76(2H,d,J=5.9Hz),7.43-7.48(2H,m),7.51-7.57(2H,m),7.71(2H,d ,J=8.1Hz),7.82-7.87(1H,m),7.92-7.95(1H,m),7.99(2H,d,J=8.1Hz), 7.99-8.04(2H,m),8.06-8.09(1H,m),8.68-8.80(4H,m),8.90(1H,d,J=6 .1Hz),8.91(1H,d,J=5.9Hz),9.09(1H,s),9.12(1H,s),9.88(2H,brs),1 0.45 (2H,brs).

### Synthesis Example 66: Production of (S)-2-(4-(N-2-picolylamino methyl) benzoylamino)-5-(N-2-picolylamino) valerate 3-(n-buthoxy) propylamide [Compound No. 67]

### Synthesis Example 66-1: Synthesis of N^{α}-Fmoc-N^{δ}-Boc-L-ornithine methylester (XLII-1)

In 20 ml of methanol, 1.00 g of commercially available N^{α}-Fmoc-N^{δ}-Boc-L-ornithine, 0.633 g of WSCI hydrochloride and 0. 446 g of HOBt were reacted for 23 hours at room temperature. On completion of the reaction, methanol was removed and the residue was then extracted with chloroform. The extract was washed with water, a saturated sodium hydrogencarbonate aqueous solution, and a saturated salt solution, followed by removing water using anhydrous sodium sulfate. After the solvent was removed, the resulting syrupy material was separated and purified by means of silica gel column chromatography (50 g, chloroform/ethyl acetate = 5/1), and 1.06 g of the above-mentioned compound was obtained as a white solid product.
MS(FAB,Pos.):m/z=469[M+1]⁺

### Synthesis Example 66-2: Synthesis of N^{α}-(4- (N-Boc-N-(2-picolyl) aminomethyl) benzoyl)-N^{δ}-Boc-L-ornithinemethylester (Compound XLIV-1)

0.300 g of the compound obtained in Synthesis Example 66-1, 0.136 ml of diethylamine and 5 ml of DMF were reacted for 1 hour at room temperature, followed by distilling the solvent off and drying under vacuum. Then, 0.241 g of the compound obtained in Synthesis Example 1-2, 0.184 g of WSCI hydrochloride, 0.130 g of HOBt and 5 ml of DMF were added to the resultant and then the whole was reacted for 25 hours at room temperature. On completion of the reaction, the reaction solution was diluted with water and was then extracted with ethyl acetate. The extract was washed with a saturated sodium hydrogencarbonate aqueous solution, and a saturatedsaltsolution,followed byremoving water using anhydrous sodium sulfate. After the solvent was removed, the resulting syrupy material was separated and purified by means of silica gel column chromatography (50 g, chloroform/ethyl acetate = 1/1), and 0.337 g of the above-mentioned compound was obtained as a colorless oily product.
MS(FAB,Pos.):m/z=571[M+1]⁺
¹H-NMR(500MHz,DMSO-d₆) : δ=1.44 and 1.46 (18H,2s) ,1.56-1.60 (2H,m) , 1.78-1.85(1H,m),1.97-2.04(1H,m),3.17-3.18(2H,m),3.79(3H,s),4. 46 and 4.51(2H,2s),4.59(2H,brs),4.65(1H,br),4.80-4.84(1H,m), 6.93(1H,m),7.17-7.19(2H,m),7.29-7.30(1H,m),7.34-7.36(1H,m),7. 64-7.67(1H,m),7.78-7.79(2H,m),8.53(1H,d,J=4.2Hz).

### Synthesis Example 66-3: Synthesis of (S)-2-(4-(N-Boc-N-(2-picolyl) aminomethyl) benzoyl)-5-(N-Boc-2-picolylamino) valerate methylester (XLVI-1)

0.337 g of the compound obtained in Synthesis Example 66-2 was dissolved in 5 ml of methanol, and then 5 ml of a 4 mol/l hydrochloric acid dioxane solution was added to the solution, and the whole was reacted for 1.5 hours at room temperature. On completion of the reaction, the solvent was distilled off. The resultant was dissolved in 5 ml of methanol, and then 0.085 ml of 2-pyridine aldehyde and 0.247 ml of triethylamine were added thereto and the whole was reacted 2 hours at room temperature. Subsequently, the reaction solution was cooled in an ice water bath, and then 0.04 g of sodium borohydride was added to the solution when the solution temperature was cooled to approximately 4°C. Methanol was removed after the reaction was continued for 0.5 hour, and a crude product was obtained. The product was dissolved in 5 ml of DMF, and then 0.256 g of di-t-butyldicarbonate and 0.242 ml of triethylamine were added therein, and the whole was reacted for 1.5 hours at room temperature. On completion of the reaction, the reaction solution was diluted with a large amount of water, followed by extraction with ethyl acetate. The extract was washed with a saturated salt solution, followed by removing water using anhydrous sodium sulfate. An oily material obtained after removing the solvent was separated and purified by means of silica gel column chromatography (35 g, chloroform/MeOH = 40/1), and 0.236 g of the above-mentioned compound was obtained as a colorless oily product.
MS(FAB,Pos.):m/z=662[M+1]⁺
¹H-NMR(500MHz,DMSO-d₆) : δ=1.39,1.44 and 1.46 (18H,3s) ,1.61-1.95 (4H,m),3.26-3.42(2H,m),3.76(3H,s),4.46-4.59(6H,m),4.7.6-4.79(1 H,m),6.70(1H,brs),7.15-7.20(4H,m),7.23-7.35(2H,m),7.64-7.67(2 H,m),7.73-7.74(1H,m),7.81-7.82(1H,m),8.49(1H,m),8.53(1H,d,J=4 . 2Hz) .

### Synthesis Example 66-4: Synthesis of (S)-2-(4-(N-Boc-N-(2-picolyl) aminomethyl) benzoyl)-5-(N-Boc-2-picolylamino) valerate (Compound XLVII-1)

8.96 g of the compound obtained in Synthesis Example 66-3 was dissolved in 134.4 ml of methanol, and then 134.4 ml of a 1N sodium hydroxide aqueous solution was added to the solution and the whole was stirred for 1 day. The termination of the reaction was confirmed using TLC. After that, the reaction solution was concentrated under reduced pressure. A1N sodium hydroxide aqueous solution was added and dissolved in the residue, followed by washing the residue with diisopropyl ether. Then, a 1N hydrochloric acid was added to the resulting water layer to adjust the solution to pH = 2 to 3. The organic phase obtained by separatory extraction with ethyl acetate was driedwith anhydrous sodium sulfate, followed by concentrating under reduced pressure and drying under vacuum, and 8.04 g of the above-mentioned compound was obtained as a light-brown solid product.
MS(FAB,Pos.):m/z=648[M+1]⁺
¹H-NMR(500MHz,CDCl₃): δ=1.44(9H,s) ,1.46(9H,s) ,1.62-2.04(4H,m) ,3 .20-3.42(2H,m),4.38-4.84(7H,m),7.18-7.48(6H,m),7.64-7.82(4H,m ),8.42-8.56(2H,m).

### Synthesis Example 66-5: Synthesis of (S)-2-(4-(N-2-picolylamino methyl) benzoylamino)-5-(N-2-picolylamino) valerate 3-(n-butoxy) propylamide [Compound No. 67]

A DMF (10 ml) solution of 0.600 g of the compound obtained in Synthesis Example 66-4, a DMF (10 ml) solution of 0.2508 g of HOBt, and a DMF (50 ml) solution of 0.3552 g of WSCI hydrochloride were prepared, respectively. Then, 19.7 mg of 3-(n-butoxy)propylamine was measured and placed in a test tube, and then 0.83 ml of the previously-prepared DMF solution of the compound obtained in Synthesis Example 59-5 and 0.83 ml of a DMF solution of HOBt were added thereto, respectively. 4.2 ml of a previously-cooled DMF solution of WSCI was added thereto while stirring at -20°C, respectively, followed by leaving the mixture standing without taking off the cooling medium bath, and increasing to room temperature. Then, the mixture was stirred for 63.5 hours at room temperature. The reaction solution was concentrated by centrifugation without modification, and then 2 ml of chloroform was added to the residue. Subsequently, the resultant was washed with 2 ml of a 1N hydrochloric acid and the water phase thereof was then extractedwith 2 ml of chloroform. The resultant was washed with 2 ml of a saturated sodium hydrogencarbonate aqueous solution, followed by subjecting to a dehydration process using Chem-Elut (manufactured by Varian, Inc.). The organic phase thus obtained was concentrated, and then the residue thereof was purified using a solid phase extraction column (Sep-Pak Vac silica (manufactured by Nihon Waters K. K.), 2 g, chloroform/benzene/methanol). In the resulting condensation product, 1.5 ml of a 4 mol/l hydrochloric acid/dioxane solution and 1.5 ml of water were added and the whole was stirred for 2 hours. After that, the reaction solution was centrifuged without modification. Then, the resultant was azeotropically distilled with ethanol and dried under vacuum, and 90.5 mg of hydrochloride of the above-mentioned compound was obtained as a white solid product.
MS(FAB,Pos.):m/z=561[M+1]⁺
¹H-NMR(500MHz,DMSO-d₆) : δ=0.86 (3H,t,J=7.6Hz), 1.22-1.35 (2H,m), 1. 42-1.50 (2H,m), 1.60-1.70 (2H,m), 1.70-1.90 (4H,m), 2.94-3.04 (2H,m) , 3.08-3.16 (2H,m), 3.30-3.40 (4H,m), 4.30 (6H,s), 4.40-4.48 (1H,m),7 .47 (1H,t,J=7.8Hz), 7.48 (1H,t,J=7.6Hz), 7.60 (2H,t,J=8.3Hz), 7.67 (2H,d,J=8.1Hz), 7.90-7.95 (2H,m), 7.98 (2H,d,J=8.3Hz), 8.17 (1H,t,J= 5.4Hz), 8.60-8.68 (2H,m), 9.39 (2H,bs), 9.97 (2H,bs).

### Synthesis Example 67: Production of [Compound No. 68] to [Compound No. 82]

As described below, hydrochlorides of the above-mentioned compounds were obtained by the same operation as that of Synthesis Example 66-5, respectively.

### Synthesis of (S)-2-(4-(N-2-picolylaminomethyl) benzoylamino)-5-(N-2-picolylamino) valerate tetrahydrofurfurylamide [Compound No. 68]

Using 15.6 mg of tetrahydrofurfurylamine, 9.0 mg of hydrochloride of the above-mentioned compound was obtained by similar operation.
MS(FAB,Pos.):m/z=531[M+1]⁺
¹H-NMR(500MHz,DMSO-d₆):δ=1.45-1.55(1H,m),1.70-2.00(7H,m),2.90-3.08(2H,m),3.12-3.18(1H,m),3.50-3.56(1H,m),3.68-3.78(2H,m),3. 80-3.86(1H,m),4.30(6H,s),4.45-4.52(1H,m),7.42-7.48(2H,m),7.54 (2H,t,J=8.3Hz),7.66(2H,d,J=8.3Hz),7.88-7.94(2H,m),7.97(2H,d,J =8.3Hz),8.15(1H,t,J=6.1Hz),8.58-8.68(3H,m),9.24(2H,bs),9.82(2 H,bs).

### Synthesis of (S)-2-(4-(N-2-picolylaminomethyl) benzoylamino)-5-(N-2-picolylamino) valerate phenylhydrazide [Compound No. 69]

Using 16.7 mg of phenylhydrazine, 34.4 mg of hydrochloride of the above-mentioned compound was obtainedas awhite solidproduct by similar operation.
MS(FAB,Pos.) :m/z=538[M+1]⁺
¹H-NMR(500MHz,DMSO-d₆) : δ=1.74-2.00 (4H,m) ,3.00-3.10 (1H,m) ,4.31 (6H,s),4.40-4.46(1H,m),6.60-7.40(5H,m),7.44-7.50(2H,m),7.50-7. 60(2H,m),7.60-7.74(2H,m),7.88-8.02(4H,m),8.60-8.70(2H,m),9.41 (2H,bs), 9.99(2H,bs).

### Synthesis of (S)-2-(4-(N-2-picolylaminomethyl) benzoylamino)-5-(N-2-picolylamino) valerate 2-(3-indolyl) ethylamide [Compound No. 70]

Using 24.7 mg of tryptamine, 45.7 mg of hydrochloride of the above-mentioned compound was obtained as a white solid product by similar operation.
MS(FAB,Pos.):m/z=590[M+1]⁺
¹H-NMR(500MHz,DMSO-d₆) : δ=1.60-1.90 (4H,m) ,2.80-3.10(4H,m) ,3.20-3.40(2H,m),4.30(6H,s),4.40-4.55(1H,m),6.84-7.40(5H,m),7.40-7. 56(4H,m),7.56-7.70(4H,m),7.84-8.06(4H,m),8.58-8.72(2H,m),9.43 (2H,bs) 10.03 (2H,bs) .

### Synthesis of (S)-2-(4-(N-2-picolylaminomethyl) benzoylamino)-5-(N-2-picolylamino) valerate (1-benzylpiperazine-4-yl)amide [Compound No. 71]

Using 29.4 mg of 4-amino-1-benzylpiperazine, 40.5 mg of hydrochloride of the above-mentioned compound was obtained as a white solid product by similar operation.
MS(FAB,Pos.):m/z=620[M+1]⁺
¹H-NMR(500MHz,DMSO-d₆):δ=1.68-2.10(8H,m),2.94-3.08(4H,m),3.24-3.34(2H,m),3.40-3.50(1H,m),3.64-3.80(2H,m),4.20-4.33(6H,m),4. 41-4.48(1H,m),7.40-7.52(5H,m),7.58-7.70(6H,m),7.88-8.00(4H,m) ,8.45(1H,d,J=7.3Hz),8.60-8.70(2H,m),9.44(2H,bs),9.99(2H,bs).

### Synthesis of (2S)-2-(4-(N-2-picolylaminomethyl) benzoylamino) -5-(N-2-picolylamino) valerate (1'S)-1'-(2-naphthyl)amino carbonylphenethylamide [Compound No. 72]

Using 44.8 mg of phenylalanine 2-naphthylamide, 39.8 mg of hydrochloride of the above-mentioned compound was obtained as a white solid product by similar operation.
MS(FAB,Pos.):m/z=720[M+1]⁺
¹H-NMR(500MHz,DMSO-d₆):δ=1.60-1.95(4H,m),2.86-3.08(2H,m),3.45-3.55(1H,m),3.64-3.76(1H,m),4.48-4.55(1H,m),4.70-4.77(1H,m),7. 14-7.25(3H,m),7.24(2H,d,J=7.3Hz),7.40-7.56(6H,m),7.56-7.70(3H ,m),7.76-8.10(8H,m),8.46(1H,d,J=7.8Hz),8.60-8.72(4H,m),9.33(1 H,bs),9.93(1H,bs).

### Synthesis of (S)-2-(4-(N-2-picolylaminomethyl)benzoylamino)-5-(N-2-picolylamino) valerate 4-hexadecylaminobenzylamide [Compound No. 73]

Using 53.5 mg of 4-hexadecylaminobenzylamine, 19.0 mg of hydrochloride of the above-mentioned compound was obtained as a white solid product by similar operation.
MS(FAB,Pos.):m/z=777[M+1]⁺
¹H-NMR(500MHz,DMSO-d₆):δ=0.85(3H,t,J=6.8Hz),1.10-1.38(26H,m),1 .65(2H,quint.,J=7.8Hz),1.70-1.96(4H,m),2.95-3.05(2H,m),3.19(2 H,t,J=7.8Hz),8.30(8H,s),4.48-4.56(1H,m),7.38(2H,d,J=8.3Hz),7. 42-7.50(2H,m),7.59(2H,t,J=8.1Hz),7,68(2H,d,J=8.3Hz),7.86-7.94 (2H,m),8.01(2H,d,J=8.3Hz),8.60-8.68(2H,m),8.70-8.78(2H,m),9.3 9(2H,bs), 9.96(2H,bs) .

### Synthesis of (S)-2-(4-(N-2-picolylaminomethyl) benzoylamino)-5-(N-2-picolylamino) valerate 4-(N-(1,2,3,4-tetrahydro-1,4-dicarbonyl phthalazine-6-yl)-N-ethylamino)butylamide [Compound No. 74]

Using 42.7 mg of N-(4-aminobutyl)-N-ethylisoluminol, 40.7 mg of hydrochloride of the above-mentioned compound was obtained as a white solid product by similar operation.
MS(FAB,Pos.):m/z=706[M+1]⁺
¹H-NMR(500MHz,DMSO-d₆): δ=0.85(3H,t,J=6.8Hz),1.10-1.38(26H,m),1 .65(2H,quint.,J=7.8Hz),1.70-1.96(4H,m),2.95-3.05(2H,m),3.19(2 H,t,J=7.8Hz),8.30(8H,s),4.48-4.56(1H,m),7,38(2H,d,J=8.3Hz),7. 42-7,50(2H,m),7.59(2H,t,J=8.1Hz),7.68(2H,d,J=8.3Hz),7.86-7.94 (2H,m),8.01(2H,d,J=8.3Hz),8.60-8.68(2H,m),8.70-8.78(2H,m),9.3 9(2H,bs) 9.96(2H,bs).

### Synthesis of (S)-2-(4-(N-2-picolylaminomethyl) benzoylamino)-5-(N-2-picolylamino) valerate 2,4,6-trichlorophenylhydrazide [Compound No. 75]

Using 32.6 mg of 2,4,6-trichlorophenylhydrazine, 61.4 mg of hydrochloride of the above-mentioned compound was obtained as a white solid product by similar operation.
MS(FAB,Pos.):m/z=640,642,644[M+1]⁺
¹H-NMR(500MHz,DMSO-d₆):δ=1.68-1.98 (4H,m) ,2.92-3.08 (2H,m) ,4.31 (6H,bs),4.56-4.60(1H,m),4.72-4.52(4H,m),7.50(2H,s),7.59(2H,t,J =8.3Hz),7.66(2H,d,J=8.5Hz),7.88-7.98(4H,m),8.60-8.68(2H,m),9. 38(2H,bs),10.32(2H,bs).

### Synthesis of (S)-2-(4-(N-2-picolylaminomethyl) benzoylamino)-5-(N-2-picolylamino) valerate 2-picolyamide [Compound No. 76]

Using 16.7 mg of 2-picolylamine, 61.5 mg of hydrochloride of the above-mentioned compound was obtainedas awhite solidproduct by similar operation.
MS(FAB,Pos.):m/z=538[M+1]⁺
¹H-NMR(500MHz,DMSO-d₆) : δ=1.75-2.00(4H,m) ,2.95-3.06(2H,m) ,4.31 (6H,bs),4.50-4.58(1H,m),4.66(1H,dd,J=17.0,5.6Hz),4.72(1H,dd,J= 17.0,5.6Hz),7.46-7.56(2H,m),7.62-7.75(6H,m)-,7.88-8.00(6H,m),8 .05(2H,d,J=8.3Hz),8.53(1H,t,J=7.6Hz),8.65-8.70(2H,m),8.97(1H, d,J=7.8Hz), 9.13 (1H,t,J=6.1Hz), 9.56 (2H,bs), 10.14 (2H,bs).

### Synthesis of (S)-2-(4-(N-2-picolylaminomethyl) benzoylamino)-5-(N-2-picolylamino) valerate 2-(N,N-diethylamino)ethylamide [Compound No. 77]

Using 17.9 mg of N,N-diethylethylenediamine, 58.7 mg of hydrochloride of the above-mentioned compound was obtained as a white solid product by similar operation.
MS(FAB,Pos.):m/z=546[M+1]⁺
¹H-NMR (500MHz,DMSO-d₆) : δ=1.21(3H,t,J=4.6Hz), 1.22(3H,t,J=4.6Hz) ,1.70-1.90(4H,m),2.95-3.05(2H,m),3.05-3.20(2H,m),3.44(2H,q,J= 7.1Hz),2.46-2.56(6H,m),4.46(1H,q,J=4.4Hz),7.46-7.52(2H,m),7.6 3(2H,d,J=7.8Hz),7.66(2H,d,J=7.8Hz),7.69(2H,d,J=8.5Hz),7.90-7. 98(2H,m),8.04(2H,d,J=8.3Hz),8.55(1H,t,J=5.6Hz),8.64(1H,dt,J=3 .2,1.0Hz),8.67(1H,dt,J=3.9,1.0Hz),8.87(1H,d,J=8.1Hz),2.47(2H, bs),10.08(2H,bs),11.01(1H,bs).

### Synthesis of (S)-2-(4-(N-2-picolylaminomethyl) benzoylamino)-5-(N-2-picolylamino) valerate 3-(morpholin-1-yl)propylamide [Compound No. 78]

Using 22.3 mg of N-(3-aminopropyl)morpholine, 59.6 mg of hydrochloride of the above-mentioned compound was obtained as a white solid product by similar operation.
MS(FAB,Pos.):m/z=574[M+1]⁺
¹H-NMR (500MHz,DMSO-d₆):δ=1.70-1.95(6H,m) ,2.95-3.10 (6H,m) ,3.09 (2H,q,J=5.6Hz),3.15(2H,q,J=6.6Hz),3.38(2H,q,J=7.3Hz),3.82(2H,t d,J=12.2,3.2Hz),3.93(2H,d,J=12.5Hz),4.26-4.36(6H,m),4.42(1H,q ,J=5.1Hz),7.50(1H,t,J=5.1Hz),7.51(1H,t,J=4.9Hz),7.64-7.74(4H, m),7.97(2H,td,J=7.6,1.5Hz),8.01(2H,d,J=8.3Hz),8.43(1H,t,J=5.9 Hz),8.66(1H,d,J=4.9Hz),8.68(1H,d,J=4.6Hz),8.75(1H,d,J=8.1Hz), 9.56(2H,bs),10.10(2H,bs),11.22(1H,bs).

### Synthesis of (S)-2-(4-(N-2-picolylaminomethyl) benzoylamino)-5-(N-2-picolylamino) valerate 2-(N,N-methylamino)ethylamide [Compound No. 79]

Using 13.6 mg of N,N-dimethylethylenediamine, 53.8 mg of hydrochloride of the above-mentioned compound was obtained as a white solid product by similar operation.
MS(FAB,Pos.):m/z=518[M+1]⁺
¹H-NMR(500MHz,DMSO-d₆) : δ=1.70-2.00(4H,m) ,2.76(5H,d,J=4.9Hz), 2.81(1H,d,J=4.9Hz),2.92-3.06(2H,m),3.15(2H,q,J=5.8Hz),3.30-3.60 (2H,m),4.26-4.36(6H,m),4.50(1H,q,J=4.2Hz),7.49(1H,t,J=8.1Hz), 7.50(1H,t,J=7.8Hz),7.64(1H,d,J=8.1Hz),7.66(1H,d,J=8.5Hz),7.69 (2H,d,J=8.3Hz),7.95(1H,td,J=7.6,1.7Hz),7.97(1H,td,J=7.3,1.7Hz ),8.05(2H,d,J=8.3Hz),8.48(1H,t,J=5.6Hz),8.65(1H,d,J=4.2Hz),8. 68(1H,d,J=4.2Hz),8.90(1H,d,J=8.3Hz),9.48(2H,bs),10.11(2H,bs), 10.62(1H,bs).

### Synthesis of (S)-2-(4-(N-2-picolylaminomethyl) benzoylamino)-5-(N-2-picolylamino) valerate 4-(2,4-di-t-amylphenoxy)butylamide [Compound No. 80]

Using 47.2 mg of 4-(2,4-di-t-amylphenoxy)butylamine, 59.7 mg of hydrochloride of the above-mentioned compound was obtained as a white solid product by similar operation.
MS(FAB,Pos,):m/z=735[M+1]⁺
¹H-NMR(500MHz,DMSO-d₆):δ=0.53(3H,t,J=7.6Hz),0.60(3H,t,J=7.3Hz) ,1.20(6H,s),1.28(6H,s),1.53-1.63(4H,m),1.68-1.88(8H,m),2.82-3 .04(2H,m),3.10-3.20(2H,m),3.30(2H,t,J=6.1Hz),4.26-4.36(6H,m), 4.44-4.52(1H,m),6.83(1H,d,J=8.3Hz),7.06(1H,d,J=7.8Hz),7.07(1H ,s),7.44-7.50(2H,m),7.60(2H,t,J=8.5Hz),7.67(2H,d,J=8.3Hz),7.9 2(1H,td,J=7.6,1.7Hz),7.93(1H,td,J=7.6,1.7Hz),7.99(2H,d,J=8.3H z),8.25(1H,t,J=5.6Hz),8.63(1H,s),8.62-8.68(1H,m),8.67(1H,td,J =4.9,1.0 Hz),9.39(2H,bs),9.97(2H,bs).

### Synthesis of (S)-2-(4-(N-2-picolylaminomethyl) benzoylamino)-5-(N-2-picolylamino) valerate 3-aminopropylamide [Compound No. 81]

Using 26.9 mg of 3-(Boc amino)propylamine, 35.2 mg of hydrochloride of the above-mentioned compound was obtained as a white solid product by similar operation.
MS(FAB,Pos.):m/z=504[M+1]⁺
¹H-NMR(500MHz,DMSO-d₆):δ=1.65-1.95(2H,m),2.95-3.05(2H,m),3.15(. 2H,q,J=6.3Hz),4.25-4.35(6H,m),4.35-4.45(1H,m),7.44-7.54(2H,m) ,7.58-7.68(2H,m),7.68(2H,d,J=8.3Hz),7.90-8.00(2H,m),8.00(2H,d ,J=8.3Hz),8.05(3H,bs),8.42(1H,t,J=5.9Hz),8.64(1H,dd,J=4.2,0.7 Hz),8.67(1H,td,J=3.4,0.7Hz),8.73(1H,d,J=7.8Hz),9.50(2H,bs),10 .06(2H,bs).

### Synthesis of (S)-2-(4-(N-2-picolylaminomethyl) benzoylamino)-5-(N-2-picolylamino) valerate 5-indazolamide [Compound No. 82]

Using 20.6 mg of 5-aminoindazole, 59.0 mg of hydrochloride of the above-mentioned compound was obtained as a white solidproduct by similar operation.
MS(FAB,Pos.) :m/z=563[M+1]⁺
¹H-NMR(500MHz,DMSO-d₆):δ=1.75-1.95(4H,m) ,2.95-3.05(2H,m) ,4.31 (6H,bs),4.35-4.40(1H,m),7.36(1H,dd,J=8.8,2.0Hz),7.48(1H,t,J=6. 8Hz),7.49(1H,t,J=6.6Hz),7.62(1H,d,J=9.3Hz),7.64(1H,d,J=8.1Hz) ,7.65-7.70(3H,m),7.84(1H,dd,J=2,0.7Hz),7.92-7.96(2H,m),7.96(2 H,d,J=8.3Hz),8.19(1H,dd,J=8.3,1.0Hz),8.65(1H,dq,J=4.9,0.7Hz), 8.68(1H,dq,J=4.9,0.7Hz),8.80(1H,d,J=7.8Hz),9.43(2H,bs),10.01 (2H,bs),10.41(2H,bs).

### Synthesis Example 68: Production of (2S)-2-(2-(N-2-picolylamino methyl) pyridin-5-ylcarbonyl) amino-5-(5,6,7,8-tetrahydro guinolin-8-yl)aminovalerate (1'S)-1'-(1-naphthyl) ethylamide [Compound No. 83]

### Synthesis Example 68-1: Synthesis of N^{α}-Fmoc-N^{δ}-Boc-ornithine (1'S)-1'-(1-naphthyl) ethylamide (Compound IX-4)

3.00 g of commercially available N^{α}-Fmoc-N^{δ}-Boc-ornithine was dissolved in 60 ml of DMF, and then 1. 6784 g of WSCI hydrochloride and 0.9561 g of HOBt were added and dissolved in the solution. In this solution, 0.165 ml of (1S)-1-(1-naphthyl) ethylamine was added and the whole was stirred for 3 days at room temperature. On completion of the reaction, the solvent was distilled off. Subsequently, the residue was dissolved in chloroform and then washed with a 1 mol/l hydrochloric acid and a saturated salt solution. An organic layer was dried with anhydrous sodium sulfate and the solvent was then distilled off, followed by drying the resultant under vacuum. Consequently, 3.6698 g of the above-mentioned compound was obtained as a white solid product.
MS(FAB,Pos.):m/z=608[M+1]⁺

### Synthesis Example 68-2: Synthesis of N^{α}-(2-(N-Boc-N-2-picolyl aminomethyl) pyridin-5-ylcarbonyl)-N^{δ}-Boc-L-ornithine (1'S)-1'-(1-naphthyl) ethylamide (Compound XI-11)

502.5 mg of the compound obtained in Synthesis Example 68-1 was dissolved in 10 ml of DMF, and then 1.0 ml of diethylamine was added to the solution and the whole was stirred for 4 hours at room temperature. On completion of the reaction, the solvent was distilled off and the residue was dried under vacuum. The resultant was dissolved in 10 ml of DMF, and then 237.4 mg of WSCI hydrochloride, 124.8 mg of HOBt and 305.2 mg of the compound obtained in Synthesis Example 12-3 were added to the solution and the whole was stirred for 21 hours at room temperature. On completion of the reaction, the solvent was distilled off and a residue was then dissolved in chloroform, followed by washing with a 1 mol/l hydrochloric acid, a 1 mol/l sodium hydroxide aqueous solution, and a saturated salt solution. An organic layer was dried with anhydrous sodium sulfate, followed by distilling the solvent off. The residuewas purifiedbymeans of silica gel column chromatography (35g, chloroform/methanol=25/1) , and 490 mg of the above-mentioned compound was obtained as a white frothy product.
MS(FAB,Pos.) :m/z=711 [M+1]⁺
¹H-NMR(500MHz,DMSO-d₆):δ=1.27-1.58(2H,m),1.31(9H,s),1.36(9H,s) ,1.51(3H,d,J=7.1Hz),1.62-1.69(2H,m),2.83-2.95(2H,m),4.45-4.68 (5H,m),5.71(1H,quint.,J=7.1Hz),6.78(1H,t,J=5.6Hz),7.25-7.40(3 H,m),7.46-7.58(4H,m),7.78(1H,td,J=7.8,1.7Hz),7.93-7.95(1H,m), 8.10(1H,d,J=8.3Hz),8.20(1H,d,J=8.1Hz),8.50(1H,dd,J=4.9,1.7Hz) ,8.60-8.67(2H,m),8.97(1H,d,J=2.0Hz).

### Synthesis Example 68-3: Synthesis of (2S)-2-(2-(N-2-picolylamino methyl) pyridin-5-ylcarbonyl)) amino-5-(5,6,7,8-tetrahydro quinolin-8-yl) aminovalerate (1'S)-1'-(1-naphthyl)-ethylamide [Compound No. 83]

209.6 mg of the compound obtained in Synthesis Example 68-2 was dissolved in 2 ml of methanol , and then 2 ml of 4 mol/l hydrochloric acid/dioxane was added to the solution and the whole was stirred for 8.5 hours at room temperature. On completion of the reaction, the solvent was distilled off and the residue was dried under vacuum. The resultant was dissolved in 4 ml of methanol, and then 0.119 ml of triethylamine, 50.8 mg of 5,6,7,8-tetrahydroquinolin-8-one, 26.5 mg of sodium cyanoborohydride and 25 drops of acetic acid were added to the solution to adjust pH to approximately 5, followed by stirring for 22 hours at room temperature. On completion of the reaction, the solvent was distilled off and then the residue was purified by means of silica gel column chromatography (5 g, chloroform/methanol/water = 7/3/0.5), and 108.7 mg of the above-mentioned compound was obtained as a white solid product.
MS(FAB,Pos.) :m/z=642 [M+1]⁺
¹H-NMR(500MHz,DMSO-d₆) : δ=1.52(3H,d,J=6.8Hz) ,1.65-2.02 (7H,m) ,2. 24-2.35(1H,m),2.7S-2.85(2H,m),2.90-3.01(1H,m),3.02-3.13(1H,m) ,4.38-4.50(1H,m),4.44(2H,s),4.50(2H,s),4.58-4.65(1H,m),5.71(1 H,quint.,J=6.8Hz),7.35-7.41(1H,m),7.42-7.73(10H,m),7.81(1H,d, J=8.3Hz),7.92-8.00(2H,m),8.11(1H,d,J=8.5Hz),8.35(1H,d,J=8.1Hz ),8.45(1H,ddd,J=10.8,4.9,1.7Hz),8.68(1H,ddd,J=4.9,1.7,1.0Hz), 8.90-9.07(2H,m),9.13(1H,s),9.2(2H,br),9.9(2H,br).

### Synthesis Example 69: Production of (2S)-2-(2-(N-2-picolylamino methyl) pyridin-5-ylcarbonyl) amino-5-((1-methyl-imidazol-2-yl) methylamino) aminovalerate (1'S)-1'-(1-naphthyl)-ethylamide [Compound No. 84]

150.1 mg of the compound obtained in Synthesis Example 68-2 was dissolved in 1.5 ml of methanol, and then 1.5 ml of a 4 mol/l hydrochloric acid/dioxane was added to the solution and the whole was stirred for 2 hours at room temperature. On completion of the reaction, the solvent was distilled off and the residue was dried under vacuum. The resultant was dissolved in 3 ml of methanol, and then 0.119 ml of triethylamine, and 23.6 mg of 1-methyl-2-imidazole carboaldehyde were added to the solution and the whole was stirred for 12 hours at room temperature. On completion of the reaction, the solvent was distilled off and the residue was dried under vacuum. The resultant was dissolved in 3 ml of anhydrous methanol, and then 16 mg of sodium borohydride was added to the solution and the whole was stirred for 1.5 hours at room temperature. On completion of the reaction, the solvent was distilled off, and then the residue was purified by means of silica gel column chromatography (5 g, chloroform/methanol/water =7/3/0.5), and 110.7 mg of the above-mentioned compound was obtained as a white solid product.
MS(FAB,Pos.):m/z=605[M+1]⁺
¹H-NMR(500MHz,DMSO-d₆):δ=1.52(3H,d,J=6.8Hz),1.74-1.95(4H,m),3. 02-3.17(2H,m),3.98(3H,s),4.44(2H,s),4.49(2H,s),4.53(1H,s),4.5 9-4.68(1H,m),5.71(1H,quint.,J=6.8Hz),7.45-7.58(6H,m),7.65(1H, d,J=8.3Hz),7.76-7.78(2H,m),7.81(1H,d,J=8.1Hz),7.90-7.96(2H,m) ,8.11(1H,d,J=8.3Hz),8.39(1H,dd,J=8.3,2.2Hz),8.67(1H,ddd,J=4.9 ,1.7,1.0Hz),8.94(1H,d,J=8.3Hz),8.95(1H,d,J=7.8Hz),9.14(1H,d,J =2.2Hz), 9.88 (2H,br), 10.25 (2H,br).

### Synthesis Example 70: Production of (2S)-2-(4-(N-2-picolylamino methyl) naphthoyl) amino-5-(5,6,7.8-tetrahydroguinolin-8-yl) aminovalerate (1'S)-1'-(1-naphthyl)-ethylamide [Compound No. 85]

### Synthesis Example 70-1: Synthesis of N^{α}-(4-(N-Boc-N-2-picolyl aminomethyl) naphthoyl)-N^{δ}-Boc-L-ornithine (1'S)-1'-(1-naphthyl) ethylamide (Compound XI-12)

499.9 mg of the compound synthesized in Synthesis Example 68-1 was dissolved in 10 ml of DMF, and then 1.0 ml of diethylamine was added to the solution and the whole was stirred for 1 hour at room temperature. On completion of the reaction, the solvent was distilled off and the residue was dried under vacuum. The resultant was dissolved in 10 ml of DMF, and then 239.0 mg of WSCI hydrochloride, 123. 7 mg of HOBt and 340.1 mg of the compound obtained in Synthesis Example 43-2 were added to the solution and the whole was stirred for 24 hours at room temperature. On completion of the reaction, the solvent was distilled off and the residue was then dissolved in chloroform, followed by washing with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purifiedbymeans of silica gel column chromatography (30 g, chloroform/methanol = 25/1), and 349.1 mg of the above-mentioned compound was obtained as a white frothy product.
MS(FAB,Pos.):m/z=760 [M+1]⁺
¹H-NMR(500MHz,DMSO-d₆₎ : δ=1.33 and 1.37 (18H,2s) ,1.25-1.78 (4H,m), 1.55(3H,d,J=6.8Hz),2.92(2H,m),4.36 and 4.44(2H,2s),4.50-4.59 1H,m),4.98 and 5.02(2H,2s),5.75(1H,quint.,J=6.8Hz),6.80(1H,t, J=5.6Hz),7.19-7.40(4H,m),7.49(1H,t,J=7.8Hz),7.51-7.62(8H;m), 7.77(1H,td,J=7.8,1.7Hz),7.84(1H,d,J=8.1Hz),7.94-8.00(2H,m),8.0 8-8.02(2H,m),8.26(1H,d,J=7.8Hz),8.52(1H,brs),8.61(1H,d,J=8.1H z).

### Synthesis Example 70-2: Synthesis of (2S) -2- (4- (N-2-picolylamino methyl) naphthoyl) amino-5-(5,6,7.8-tetrahydroquinolin-8-yl) aminovalerate (1'S)-1'-(1-naphthyl)-ethylamide [Compound No. 85]

200.0 mg of the compound obtained in Synthesis Example 70-1 was dissolved in 2 ml of methanol, and then 2 ml of a 4 mol/l hydrochloric acid/dioxane was added to the solution and the whole was stirred for 2.5 hours at room temperature. On completion of the reaction, the solvent was distilled off and the residue was dried under vacuum. The resultant was dissolved in 4 ml of methanol, and then 51.6 mg of 5, 6,7, 8-tetrahydroquinolin-8-one and 27. 1 mg of sodium cyanoborohydride were added to the solution, and the pH thereof was adjusted to approximately 5 with addition of 35 drops of acetic acid, followed by stirring for 13 hours at room temperature. On completion of the reaction, the solvent was distilled off and then the residue was purified by means of silica gel column chromatography (5 g, chloroform/methanol/water = 7/3/0.5) , and 83.7 mg of the above-mentioned compound was obtained as a white solid product.
MS(FAB,Pos.):m/z=691 [M+1]⁺
¹H-NMR(500MHz,DMSO-d₆) : δ=1.56 (3H,d,J=6.8Hz), 1.64-2.02 (7H,m), 2. 28-2.35(1H,m),2.78-2.83(2H,m),2.92-3.01(1H,m),3.05-3.20(1H,m) ,4.35-4.47(1H,m),4.48(2H,s),4.65-4.70(1H,m),4.78(2H,s),5.77(1 H,quint.,J=6.8Hz),7.38(1H,dd,J=7.8,4.9Hz),7.41-7.75(10H,m),7. 80-7.87(2H,m),7.93-8.00(2H,m),8.15(1H,d,J=8.5Hz),8.28(1H,d,J= 8.5Hz),8.30(1H,d,J=8.3Hz),8.48(1H,dd,J=4.6,0.7Hz),8.72(1H,dd, J=4.9,1.0Hz),8.70(1H,d,J=8.3Hz),8.88(1H,t,d=6.8Hz),9.27(2H,br ),9.96(2H,br).

### Synthesis Example 71: Production of N^{α}-(4-(N-2-picolylamino methyl)) benzoyl)-L-arginine (1'S)-1'-(1-naphthyl) ethylamide [Compound No. 86]

### Synthesis Example 71-1: Synthesis of N^{α}-Fmoc-N^{G}-Pbf-L-4-arginine (1'S)-1'-(1-naphthyl) ethylamide (Compound XXVI-6)

2.6467 g of commercially available N^{α}-Fmoc-N^{G}-Pbf-L-arginine was dissolved in 53 ml of DMF, and then 1.1830 g of WSCI hydrochloride and 0.6609 g of HOBt were added and dissolved in the solution. In this solution, 762.5 mg of (1S)-1-(1-naphthyl) ethylamine was added and the whole was stirred for 2 days at room temperature. On completion of the reaction, the solvent was distilled off. Subsequently, the residue was dissolved in chloroform and then washed with a 1 mol/l hydrochloric acid and a saturated salt solution. An organic layer was dried with anhydrous sodium sulfate and the solvent was then distilled off, followed by drying the resultant under vacuum. Consequently, 3. 62 g of the above-mentioned compound was obtained as a white solid product.
MS(FAB,Pos.) :m/z=802 [M+1]⁺

### Synthesis Example 71-2: Synthesis of N^{α}-(4-(N-Boc-N-2-picolyl aminomethyl) benzoyl)-N^{G}-Pbf- arginine (1'S)-1'-(1-naphthyl) ethylamide (Compound XXIX-10)

624.7 mg of the compound obtained in Synthesis Example 71-1 was dissolved in 12.8 ml of DMF, and then 1.28 ml of diethylamine was added to the solution and the whole was stirred for 2 hours at room temperature. On completion of the reaction, the solvent was distilled off and the residue was dried under vacuum. The resultant was dissolved in 12 ml of DMF, and then 228.0 mg of WSCI hydrochloride, 116.3 mg of HOBt, and 284.5 mg of the compound obtained in Synthesis Example 1-2 were added to the solution and the whole was stirred for 12.5 hours at room temperature. On completion of the reaction, the solvent was distilled off and the residue was dissolved in chloroform, followed by washing with a 1 mol/1 hydrochloric acid, a 1 mol/l sodium hydroxide aqueous solution, and a saturated salt solution, and further washing with anhydrous sodium sulfate. After the resultant was concentrated under reduced pressure, the residue was purified by means of silica gel column chromatography (30 g, chloroform/methanol = 25/1), and 582.5 mg of the above-mentioned compound was obtained as a yellow viscous oily product.
MS(FAB,Pos.):m/z=904 [M+1]⁺
¹H-NMR(500MHz,DMSO-d₆): δ1.25-1.55(2H,m),1.31(6H,s),1.37(9H,s), 1.51(3H,d,J=7.1Hz),1.60-1.81(2H,m),1.91(3H,s)2.40(3H,s),2.45 (3H,s), 2.90 (2H,s), 2.98-3.05 (2H,m), 4.35-4.40 (5H,m), 5.70 (1H,qui nt,J=6.8Hz), 7.19-7.38 (4H,m), 7.47 (1H,t,J=7.8Hz), 7.49-7.58 (3H,m ), 7.78 (1H,td,J=7.8,1.7Hz), 7.81 (1H,d,J=8.1Hz), 7.86 (2H,d,J=8.3H z), 7.92-7.96 (2H,m), 8.08 (1H,d,J=8.1Hz), 8.36 (1H,d,J=8.1Hz), 8.52 (1H,d,J=4.9Hz), 8.65 (1H,d,J=7.8Hz).

### Synthesis Example 71-3: Synthesis of N^{α}-(4-(N-2-picolylamino methyl) benzoyl)-L-arginine (1'S)-1'-(1-naphthyl) ethylamide [Compound No. 86]

250.3 mg of the compound obtained in Synthesis Example 71-2 was dissolved in 2.5 ml of chloroform, and then 2.5 ml of trifluoroacetic acid was added to the solution and the whole was stirred for 3.5 hours at room temperature. On completion of the reaction, thesolventwasdistilledoff. Subsequently, theresidue was dried by a vacuum pump and purified by means of silica gel column chromatography (chloroform/methanol/water = 7/3/0.5), followed by concentrating a fraction. The resultant was dissolved in a 1 mol/l hydrochloric acid, and was the concentrated and azeotropically distilled with ethanol, and 59.5 mg of hydrochloride of the above-mentioned compound was obtained as a white solid product.
MS(FAB,Pos.):m/z=552 [M+1]⁺
¹H-NMR(500MHz,DMSO-d₆):δ=1.51(3H,d,J=7.1Hz),1.41-1.59(2H,m),1. 68-1.85(2H,m),3.02-3.15(2H,m),4.31(4H,s),4.54-4.59(1H,m),5.71 (1H,quint.,J=7.1Hz),7.45(1H,ddd,J=7.6,4.5,1.0Hz),7.46-7.59(5H ,m),7.64(2H,d,J=8.5Hz),7.75(1H,brs),7.82(1H,d,J=8.3Hz),7.90(1 H,td,J=7.8,1.7Hz),7.94(1H,d,J=7.8Hz),7.98(2H,d,J=8.5Hz),8.10 (1H,d,J=8.3Hz),8.57(1H,d,J=8.1Hz),8.66(1H,ddd,J=4.9,1.7,1.0Hz) ,8.81(1H,d,J=8.1Hz),9.76(2H,brs).

### Synthesis Example 72: Production of (2S)-2-(4-((1-methylimidazol -2-ylmethyl) aminomethyl) naphthoylamino-5-((1-methylimidazol -2-ylmethyl) amino) valerate (1'S)-1'-(1-naphthyl)-ethylamide [Compound No. 87]

### Synthesis Example 72-1: Synthesis of N^{α}-(4-(N-Boc-aminomethyl) naphthoyl)-N^{δ}-Boc-L-ornithine (1'S)-1'-(1-naphthyl) ethylamide (Compound XVIII-11)

503.1 mg of the compound obtained in Synthesis Example 68-1 was dissolved in 10 ml of DMF, and 1.0 ml of diethylamine was then added to the solution and the whole was stirred for 1 hour at room temperature. On completion of the reaction, the solvent was distilled off and the residue was dried under vacuum. The resultant was dissolved in 10 ml of DMF, and then 236.9 mg of WSCI hydrochloride, 132.1 mg of HOBt, and 268.6 mg of the compound obtained in Synthesis Example 25-2 were added to the solution and the whole was stirred for 2 days at room temperature. On completion of the reaction, the solvent was distilled off and then the residue was dissolved in chloroform, followed by washing with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purified by means of silica gel column chromatography (17 g, chloroform/ethyl acetate = 1/1), and 434.4 mg of the above-mentioned compound was obtained as a white frothy product.
MS(FAB,Pos.):m/z=669[M+1]⁺
¹H-NMR(500MHZ,DMSO-d₆) : δ=1.30-1.80 (4H,m),1.37 (9H,s),1.41(9H,s) ,1.54(3H,d,J=6.8Hz),2.85-3.00(2H,m),4.55-4.62(1H,m),4.60(2H,d ,J=6.1Hz),5.75(1H,quint,J=6.8Hz),6.80(1H,t,J=5.6Hz),7.40(1H,d ,J=7.3Hz),7.43-7.61(8H,m),7.84(1H,d,J=8.1Hz),7.94-7.98(1H,m), 8.13(1H,d,J=8.1Hz),8.15(1H,d,J=8.3Hz),8.24(1H,d,J=8.1Hz),8.51 (1H,d,J=8.1Hz), 8.60 (1H,d,J=7.6Hz).

### Synthesis Example 72-2: Synthesis of (2S)-2-(4-((1-methylimidazol -2-ylmethyl) aminomethyl) naphthoylamino-5-((1-methylimidazol -2-ylmethyl) amino) valerate (1'S)-1'-(1-naphthyl)-ethylamide [Compound No. 87]

204.3 mg of the compound obtained in Synthesis Example 72-1 was dissolved in 2 ml of methanol, and then 2 ml of a 4 mol/l hydrochloric acid/dioxane solution was added to the solution and the whole was stirred for 4 hours at room temperature. On completion of the reaction, the solution was concentrated and dried under reduced pressure. Then, 109.8 mg of the resultant crude product was dissolved in 2 ml of methanol, and then 51.5 ml of triethylamine and 72.2 mg of 1-methyl-2-imidazole carboaldehyde were added to the solution and the whole was stirred for 15 hours at room temperature. On completion of the reaction, the solution was concentrated and dried under reduced pressure. Subsequently, the resultant was dissolved in 2 ml of anhydrous methanol and the whole was cooled to 0°C. In this solution, 46.2 mg of sodium borohydride was added and the whole was stirred for 0.5 hours at room temperature. On completion of the reaction, the solution was concentrated, and then the residue was purified by means of silica gel column chromatography (10 g, chloroform/methanol/water = 7/3/0.5), followed by processing with a 1 mol/l hydrochloric acid. Consequently, 156.4 mg of hydrochloride of the above-mentioned compound was obtained as a white solid product.
MS(FAB,Pos.):m/z=657[M+1]⁺
¹H-NMR(500MHz,DMSO-d₆) : δ=1.55 (3H,d,J=6.8Hz), 1.70-1.97 (4H,m), 3. 07-3.20 (2H,m), 3.97 (3H,s), 3.99 (3H,s), 4.54(2H,s), 4.63-4.70 (1H,m ), 4.77 (2H,s), 4.90 (2H,s), 5.76 (1H,d,J=6.8Hz), 7.50-7.80 (11H,m), 7 .54-7.60 (3H,m), 7.83 (1H,d,J=8.3Hz), 7.86 (1H,d,J=7.6Hz), 7.95-7.9 7 (1H,m), 8.14 (1H,d,J=8.3Hz), 8.29 (1H,d,J=8.3Hz), 8.35 (1H,d,J=8.3 Hz), 8.77 (1H,d,J=8.3Hz), 8.89 (1H,d,J=7.8Hz), 10.19 (2H,br).

### Synthesis Example 73: Production of (2S)-2-(2-(N-2-picolylamino methyl) pyridin-5-ylcarbonyl) amino-5-(N-methylpyrrol-2-yl methyl) aminovalerate (1'S)-1'-(1-naphthyl)-ethylamide [Compound No. 88]

87.2 mg of the compound obtained in Synthesis Example 68-2 was dissolved in 1 ml of methanol, and then 1 ml of a 4 mol/l hydrochloric acid/dioxane solution was added to the solution and the whole was stirred for 2 hours at room temperature. On completion of the reaction, the solvent was distilled off and the residue was dried under vacuum. The resultant was dissolved in 2 ml of methanol, and then 0.069 ml of triethylamine and 0.0145 ml of N-methyl-2-pyrrole carboaldehyde were added to the solution and the whole was stirred for 14 hours at room temperature. After the solvent was distilled off, the residue was dried under reduced pressure and dissolved in 2 ml of anhydrous methanol. The reaction solution was cooled with ice, and then 9.1 mg of sodium borohydride was added thereto and the whole was reacted for 5 hours at room temperature. On completion of the reaction, the solvent was distilled off and the residue was then purified by means of silica gel column chromatography (5g, chloroform/methanol/water = 7/3/0.5), and 65.0 mg of the above-mentioned compound was obtained as a white solid product.
MS(FAB,Pos.):m/z=604[M+1]⁺
¹H-NMR(500MHz,DMSO-d₆) : δ=1.52(3H,d,J=6.8Hz),1.65-1.84(4H,m), 2.84-2.93(2H,m),4.07(2H,t,J=5.6Hz),4.44(2H,s),4.50(2H,s),4.58-4 .63(1H,m),5.71(1H,quint.,J=6.8Hz),5.97(1H,dd,J=3.6,2.7Hz),6.2 3(1H,dd,J=3.6,2.0Hz),6.78(1H,dd,J=2.7,2.0Hz),7.43-7.58(5H,m), 7.62(1H,d,J=7.8Hz),7.66(1H,d,8.1Hz),7.81(1H,d,J=8.3Hz),7.91-8 .99(2H,m),8.11(1H,d,J=8.5Hz),8.38(1H,dd,J=8.1,2.2Hz),8.68(1H, ddd,J=4.9,1.7,1.0Hz),8.97(1H,d,J=7.8Hz),9.14(1H,d,J=2.2Hz),9.20(2H,brs).

### Synthesis Example 74: Production of N^{α}-(4-(N-(1-methylimidazol-2-yl) methylaminomethyl)-1-naphthalenecarbonyl)-L-arginine 2-(1-naphthyl) isopropylamide [Compound No. 89]

### Synthesis Example 74-1: Synthesis of methyl 4-((1-methylimidazol -2-ylmethyl) aminomethyl) naphthalene-1-carboxylate (Compound V-6)

In 25 ml of methanol, 0.8568 g of the compound obtained in Synthesis Example 17-3 was dissolved. Then, 0.526 g of 1-methyl-2-imidazole carboxyaldehyde and 1.11 ml of triethylamine were added to the solution and the whole was stirred for 63 hours, and furthermore 0.329 g of sodium borohydride was added thereto and the whole was stirred for 1.5 hours. After the solvent was distilled off, 0.9489 g of the above-mentioned compound was obtained as a light-orange viscous liquid by the purification by means of silica gel column chromatography (5 g, 3% methanol/47% chloroform/50% benzene).
MS(FAB,Pos.):m/z=310[M+1]⁺
¹H-NMR(500MHz,CDCl₃) : δ=3.58 (3H,s), 3.94 (2H,s), 3.99 (3H,s), 4.32 (2 H,s), 6.83 (1H,d,J=1.2Hz), 6.95 (1H,d,J=1.2Hz), 7.53-7.63 (3H,m), 8. 11 (1H,d,J=7.6Hz), 8.16 (1H,dd,J=7.8,0.7Hz), 8.93 (1H,dd,J=8.10.7H z).

### Synthesis Example 74-2: Synthesis of methyl 4-(N-Boc-(1-methyl imidazol-2-ylmethyl) aminomethyl) naphthalene-1-carboxylate (Compound VI-10)

In 10 ml of DMF, 0. 9452 g of the compound obtained in Synthesis Example 74-1 was dissolved. Then, 0.767g of di-t-butyldicarbonate and 0.464 g of triethylamine were added to the solution and the whole was stirred for 22 hours. On completion of the reaction, water was added and then the solvent was distilled off. Chloroform and water were added in the residue and then separated. After the resultant was dried with anhydrous magnesium sulfate, the solvent was distilled off and the residue was purified by means of silica gel column chromatography (25 g, 50% ethyl acetate/hexane), and 1.0569 g of the above-mentioned compound was obtained as a yellow viscous liquid.
MS(FAB,Pos.) :m/z=410[M+1]⁺
¹H-NMR(500MHz,CDCl₃) : δ=1.42(9H,s),3.55 and 3.70(3H,bs),4.00(3H, s),4.45 and 4.59(2H,bs),6.82(1H,bs),6.93(1H,s),7.35(1H,bs),7.55 (1H,td,J=8.1,1.2Hz),7.62(1H,t,J=7.1Hz),8.07 and 8.21(1H,bs), 8.13(1H,d,J=7.3Hz),8.94(1H,d,J=8.1Hz).

### Synthesis Example 74-3: Synthesis of 4-(N-Boc-(1-methylimidazol -2-ylmethyl) aminomethyl) naphthalene-1-carboxylic acid (Compound VII-14)

In 10 ml of methanol, 1.0526 g of the compound obtained in Synthesis Example 74-2 was dissolved. Then, 10 ml of a 1N sodium hydroxide aqueous solution and 10 ml of THF were added to the solution and the whole was stirred for 2.5 hours. The solvent was partially distilled off while water was added to the reaction solution, and a water layer was then adjusted to pH = 6, followed by extraction with chloroform. The resultant was dried with anhydrous magnesium sulfate, and the solvent was distilled off, followed by drying under vacuum. Consequently, 0.8551 g of the above-mentioned compound was obtained as a light-yellow solid product.
MS(FAB,Pos.):m/z=396[M+1]⁺
¹H-NMR(500MHz,CDCl₃) : δ=1.51 and 1.58 (9H,s), 3.76 and 3.78(3H,s), 4.74 and 4.85 (4H,s), 6.95 (1H,s), 7.19 and 7.22 (1H,s), 7.33 (1H,d, J=7.3Hz), 7.39-7.48 (2H,m), 7.92 and 8.19 (1H,d,J=7.8Hz), 8.01 (1H, d,J=8.1Hz), 8.89 and 9.00 (1H,d,J=7.8Hz)

### Synthesis Example 74-4: Synthesis of 2-(1-naphthyl) propylamine

14.594 g of cerium chloride hydrate was dried for 2 hours at 150°C (0.5 mm) while being stirred, and nitrogen was gradually introduced thereinto. The resultant was cooled in an ice bath, and then 80 ml of THF was added thereto and the whole was stirred for 1.5 hours at 25°C. While the mixture was cooled to -50°C or less, 0.861 g of methyl lithium was added thereto over 30 minutes. After the mixture was stirred for 30 minutes, a THF (5 ml) solution of 2 g of 1-cyanonaphthalene was added thereto and the whole was stirred for 2 hours at room temperature. Concentrated ammonia water (25 ml) was added in the reaction solution, followed by filtrating with celite. After the filtrate was dried with magnesium sulfate, the solvent was distilled off and then a residue was dissolved in 30 ml of toluene and extracted with a 1 mol/l hydrochloric acid. A water layer was adjusted to alkaline with concentrated ammonia water, followed by extraction with chloroform. After the resultant was dried with magnesium sulfate, the solvent was distilled off. Consequently, by purifying on silica gel column chromatography (10 g, 5% methanol/chloroform), 0.2640 g of the above-mentioned compound was obtained as a light-brown liquid.
MS(FAB,Pos.):m/z=186[M+1]⁺
¹H-NMR(500MHz,DMSO-d₆):δ-1.78(6H,s),7.40(1H,dd,J=8.1,7.3Hz),7. 46(1H,ddd,J=8.1,6.8,1.2Hz),7.50(1H,ddd,J=8.8,7.1,1.7Hz),7.60 (1H,dd,J=7.3,1.2Hz), 7.75 (1H,d,8.1Hz), 7.86 (1H,dd,J=8.1,1.7Hz), 8.89 (1H,dd,J=8.1,1.0Hz)

### Synthesis Example 74-5: Synthesis of N^{α}-Fmoc-N^{G}-Pmc-L-arginine 2-(1-naphthyl) isopropylamide (Compound XXVII-7)

In 10 ml of DMF, 487.1 mg of commercially available N^{α}-Fmoc-N^{G}-Pmc-L-arginine was dissolved. Then, 0.2516 g of WSCI hydrochloride and 0.1578 mg (1.17 mmol) of HOBt were added and dissolved in the solution. In this solution, 119.8 mg of the compound obtained in Synthesis Example 74-4 was added and the whole was stirred for 13 hours at room temperature. On completion of the reaction, the solvent was distilled off. Subsequently, the residue was dissolved in chloroform and was then washed with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purified by means of silica gel column chromatography (25 g, chloroform/ethyl acetate = 1/1), and 348.8 mg of the above-mentioned compound was obtained as a white frothy product.
MS(FAB,Pos.):m/z=830[M+1]⁺
¹H-NMR(500MHz,DMSO-d₆) :δ=1.23(6H,s),1.20-1.40(3H,m),1.50-1.62 (1H,m),1.74(2H,t,J=6.8Hz),1.77(3H,s),1.78(3H,s),2.02(3H,s),2.5 5(2H,t,J=6.8Hz),2.89-3.01(2H,m),3.98-4.06(1H,m),4.18-4.21(1H, m),4.21-4.29(2H,m),6.30-6.80(3H,br),7.21(1H,d,J==8.5Hz),7.27-7 .31(2H,m),7.30-7.42(4H,m),7.52(1H,d,J=7.3Hz),7.69(2H,dd,J=7.3 ,4.6Hz),7.77(1H,d,J=8.1Hz),7.82-7.90(3H,m),8.40(1H,s),8.49(1H ,d,J=9.3Hz).

### Synthesis Example 74-6: Synthesis of N^{α}-(4-(N-Boc-N-(1-methyl imidazol-2-yl) methylaminomethyl)-1-naphthalenecarbonyl)-N^{G}-Pbf-L-arginine 2-(1-naphthyl) isopropylamide (Compound XXIX-11)

343.7 mg of the compound obtained in Synthesis Example 74-5 was dissolved in 7 ml of DMF, and then 0.7 ml of diethylamine was added to the solution and the whole was stirred for 2 hours at room temperature. On completion of the reaction, the solvent was distilled off and a residue was dried under vacuum. The resultant was dissolved in 7 ml of DMF, and then 126.4 mg of WSCI hydrochloride, 65.9 mg of HOBt, and 179.3 mg of the compound obtained in Synthesis Example 74-3 were added to the solution and the whole was stirred for 16.5 hours at room temperature. On completion of the reaction, the solvent was distilled off and the residue was dissolved in chloroform, followed by washing with a 1 mol/l hydrochloric acid, a 1 mol/l sodium hydroxide aqueous solution, and a saturated salt solution, and further washingwith anhydrous sodium sulfate. After the resultant was concentrated under reduced pressure, the residue was purified by means of silica gel column chromatography (20 g, chloroform/methanol = 30/1), and 311.5 mg of the above-mentioned compound was obtained as a yellow frothy product.
MS(FAB,Pos.):m/z=985 [M+1]⁺

### Synthesis Example 74-7: Synthesis of N^{α}-(4-(N-(1-methylimidazol -2-yl) methylaminomethyl)-1-naphthalenecarbonyl)-L-arginine 2-(1-naphthyl) isopropylamide [Compound No. 89]

In 3.1 ml of chloroform, 308.7 mg of the compound obtained in Synthesis Example 74-6 was dissolved. Then, 3.1 ml of trifluoroacetic acid was added to the solution and the whole was stirred for 5 hours at room temperature. On completion of the reaction, the solvent was distilled off. Subsequently,the residue was dried by a vacuum pump and then a 1 mol/l hydrochloric acid was added thereto. A water layer was washed with chloroform and then water was distilled off. The residue was dissolved in methanol and then acetone was added. A supernatant was decanted and the resulting residue was then treated with a 1 mol/l hydrochloric acid, and 49.2 mg of hydroxide of the above-mentioned compound was obtained as a white solid product.
MS(FAB,Pos.):m/z=619[M+1]⁺
¹H-NMR(500MHz,DMSO-d₆):δ=1.40-1.65 (3H,m),1.68-1.82(1H,m),1.85 (6H,s),3.05-3.18(2H,m),3.96(3H,s),4.59-4.64(1H,m),4.73(2-H,s),2 .96(2H,s),6.90(2H,br) 7.20(1H,br),7.40-7.55(4H,m),7.58-7.62 (2H,m),7.64-7.81(6H,m),7.85(1H,d,J=7.7Hz),8.11(1H,d,J=8.5Hz),8 .28(1H,d,J=8.3Hz),8.51(1H,d,J=8.3Hz),8.64(1H,d,J=8.5Hz),8.79 (1H,s).

### Synthesis Example 75: Production of N^{α}-(2-(N-2-picolylamino methyl) pyridin-5-ylcarbonyl)-L-arginine (1'S)-1'-(1-naphthyl) ethylamide [Compound No. 90]

### Synthesis Example 75-1: Synthesis of N^{α}-(2-(N-Boc-N-2-picolyl aminomethyl) pyridin-5-ylcarbonyl)-N^{G}-Pbf-L-arginine (1'S)-1'-(1-naphthyl) ethylamide (Compound XXIX-12)

504.2 mg of the compound obtained in Synthesis Example 71-1 was dissolved in 10 ml of DMF, and then 1 ml of diethylamine was added to the solution and the whole was stirred for 2 hours at room temperature. On completion of the reaction, the solvent was distilled off and a residue was dried under vacuum. The resultant was dissolved in 12 ml of DMF, and then 180.3 mg of WSCI hydrochloride, 96.0 mg of HOBt, and 225.6 mg of the compound obtained in Synthesis Example 12-3 were added to the solution and the whole was stirred for 12.5 hours at room temperature. On completion of the reaction, the solvent was distilled off and the residue was dissolved in chloroform, followed by washing with a 1 mol/l hydrochloric acid, a 1 mol/l sodium hydroxide aqueous solution, and a saturated salt solution, and further washing with anhydrous sodium sulfate. After the resultant was concentrated under reduced pressure, the residue was purified by means of silica gel column chromatography (30 g, chloroform/methanol = 25/1), and 404.6 mg of the above-mentioned compound was obtained as a yellow viscous oily product.
MS(FAB,Pos.) :m/z=906[M+1]⁺

### Synthesis Example 75-2: Synthesis of N^{α}-(2-(N-2-picolylamino methyl) pyridin-5-ylcarbonyl)-L-arginine (1'S)-1'-(1-naphthyl) ethylamide [Compound No. 90]

In 2 ml of chloroform, 200.0 mg of the compound obtained in Synthesis Example 75-1 was dissolved. Then, 2 ml of trifluoroacetic acid was added to the solution and the whole was stirred for 12.5 hours at room temperature. On completion of the reaction, the solvent was distilled off and the residue was dried by a vacuum pump, and then a 1 mol/l hydrochloric acid was added thereto. A water layer was washed with chloroform and then water was distilled off. The residue was dissolved in methanol and then acetone was added thereto, followed by decantation. The resulting residue was dissolved in a 1 mol/l hydrochloric acid and the whole was concentrated, and then the resultant was azeotropically distilled with methanol and dried. Consequently, 114.2 mg of hydrochloride of the above-mentioned compound was obtained as a white solid product.
MS(FAB,Pos.):m/z=553[M+1]⁺
¹H-NMR(500MHz,DMSO-d₆) : δ=1.52 (3H,d,J=7.1Hz), 1.41-1.60 (2H,m), 1. 66-1.87 (2H,m), 3.02-3.17 (2H,m), 4.44 (2H,s), 4.50 (2H,s), 4.56-4.60 (1H,m), 5.72 (1H,quint,J=7.1Hz), 6.9 (2H,br), 7.4 (1H,br), 7.40-7.61 (6H,m), 7.65 (1H,d,J=8.5Hz), 7.81 (1H,d,J=8.1Hz), 7.86 (1H,brs), 7.9 1-7.96 (2H,m), 8.11 (1H,d,J=8.3Hz), 8.38 (1H,dd,J=8.1,2.2Hz), 8.67 (1H,ddd,J=4.9,1.7,1.0Hz), 8.88 (1H,d,J=7.8Hz), 8.91 (1H,d,J=8.1Hz) ,9.14(1H,d,J=2.2Hz).

### Synthesis Example 76: Production of (2S)-2-(4-(N-2-picolylamino methyl)benzoyl)amino-5-(5,6,7,8-tetrahydroquinolin-8-yl)amino valerate (1'S)-1'-(1-naphthyl) ethylamide [Compound No. 91]

### Synthesis Example 76-1: Synthesis of N^{α}-(4-(N-Boc-N-2-picolyl aminomethyl) benzoyl)-N^{δ}-Boc-L-ornithine (1'S)-1'-(1-naphthyl) ethylamide (Compound XI-13)

In 20 ml of DMF, 997.9 mg of the compound obtained in Synthesis Example 68-1 was dissolved, and 2.0 ml of diethylamine was then added to the solution and the whole was stirred for 1.5 hours at room temperature. On completion of the reaction, the solvent was distilled off and the residue was dried under vacuum. The resultant was dissolved in 20 ml of DMF, and then 469.3 mg of WSCI hydrochloride, 245.0 mg of HOBt, and 598.9 mg of the compound obtained in Synthesis Example 1-2 were added to the solution and the whole was stirred for 21 hours at room temperature. On completion of the reaction, the solvent was distilled off and then the residue was dissolved in chloroform, followed by washing with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purified by means of silica gel column chromatography (50 g, chloroform/methanol = 30/1), consequently 603.4 mg of the above-mentioned compound was obtained as a white frothy product.
MS(FAB,Pos.):m/z=710[M+1]⁺
¹H-NMR(500MHz,DMSO-d₆):δ=1.22-1.55 (2H,m),1.31,1.36 and 1.38 (18H, s),1.51(3H,d,J=7.1Hz),1.60-1.72(2H,m),2.80-2.92(2H,m),4.39-4. 60(5H,m),5.70(1H,quint.,J=7.1Hz),6.78(1H,t,J=5.6Hz),7.18-7.37 (4H,m),7.46-7.57(4H,m),7.78(1H,td,J=7.8,1.7Hz),7.82(1H,d,J=7. 8Hz),7.85(1H,d,J=8.3Hz),8.09(1H,d,J=8.1Hz),8.34(1H,d,J=7.8Hz) , 8.52 (1H,d,J=4.8Hz), 8.61 (1H,d,J=7.6Hz).

### Synthesis Example 76-2: Synthesis of (2S)-2-(4-(N-2-picolylamino methyl) benzoyl) amino-5-(5,6,7,8-tetrahydroquinolin-8-yl) aminovalerate (1'S)-1'-(1-naphthyl) ethylamide [Compound No. 91]

In 4 ml of methanol, 408.9 mg of the compound obtained in Synthesis Example 76-1 was dissolved. Then, 4 ml of a 4 mol/l hydrochloric acid/dioxane was added to the solution and the whole was stirred for 3.5 hours at room temperature. On completion of the reaction, the solvent was distilled off and the residue was dried under vacuum. The resultant was dissolved in 4 ml of methanol, and then 124.8 mg of 5,6,7,8-tetrahydroquinolin-8-one and 73.2 mg of sodium cyanoborohydride were added to the solution, and furthermore 50 drops of acetic acid were added to the solution to adjust pH thereof to approximately pH = 4 to 5 and the whole was stirred for 12. 5 hours at room temperature. On completion of the reaction, the solvent was distilled off and then the residue was purified by means of silica gel column chromatography (chloroform/methanol/water = 7/3/0.5), consequently 211.5 mg of the above-mentioned compound was obtained as a white solid product.
MS(FAB,Pos.):m/z=641[M+1]⁺
¹H-NMR(500MHz,DMSO-d₆) : δ=1.51 (3H,d,J=6.8Hz), 1.68-2.02 (7H,m), 2. 22-2.33 (1H,m), 2.77-2.85 (2H,m), 2.90-3.00 (1H,m), 3.01-3.15 (1H,m) , 4.31 (2H,s), 4.322H,s), 4.38-4.47 (1H,m), 4.57-4.63 (1H,m), 5.70 (1H ,quint.,J=6.8Hz), 7.37-7.41 (1H,m), 7.43-7.61 (5H,m), 7.63-7.70 (2H ,m), 7.68 (2H,d,J=7.8Hz), 7.81 (1H,d,J=8.1Hz), 7.94 (1H,d,J=7.8Hz), 7.97 (1H,t,J=7.8Hz), 7.99 (2H,d,J=7.8Hz), 8.10 (1H,d,J=8.3Hz), 8.44 (1H,m), 8.64-8.67 (2H,m), 8.90 (1H,d,J=7.1Hz), 9.23 (2H,br), 10.07 (2 H,br).

### Synthesis Example 77: Production of (2S)-2-(4-(N-2-picolylamino methyl) naphthoyl) amino-5-(5,6,7,8-tetrahydroquinolin-8-yl) aminovalerate 2-(3-indolyl) ethylamide [Compound No. 92]

### Synthesis Example 77-1: Synthesis of N^{α}-(4-(N-Boc-N-2-picolyl aminomethyl) naphthoyl)-N^{δ}-Boc-L-ornithine 2-(3-indolyl) ethylamide (Compound XI-14)

In 20 ml of DMF, 1.006 g of the compound obtained in Synthesis Example 17-5 was dissolved, and 2.0 ml of diethylamine was then added thereto and the whole was stirred for 4 hours at room temperature. On completion of the reaction, the solvent was distilled off and the residue was dried under vacuum. The resultant was dissolved in 20 ml of DMF, and then 499.0 mg of WSCI hydrochloride, 255.2 mg of HOBt, and 696.7 mg of the compound obtained in Synthesis Example 43-2 were added to the solution and the whole was stirred for 16 hours at room temperature. On completion of the reaction, the solvent was distilled off and then the residue was dissolved in chloroform, followed by washing with a 1 mol/l hydrochloric acid, a 1 mol/l sodium hydroxide aqueous solution, and a saturated salt solution. An organic layer was dried with anhydrous sodium sulfate, followed by distilling the solvent off. The residue was purified by means of silica gel column chromatography (50 g, chloroform/methanol = 30/1), consequently 860.9 mg of the above-mentioned compound was obtained as a white frothy product.
MS(FAB,Pos.):m/z=749[M+1]⁺
¹H-NMR (500MHz,DMSO-d₆):δ=1.25-1.80 (4H,m),1.33,1.37 and 1.43 (18H, 2s),2.83-2.92(2H,m),2.92-3.02(2H,m),3.32-3.52(2H,m),4.34-4.52 (3H,m),4.95-5.08(2H,m),6.82(1H,t,J=5.6Hz),6.98(1H,td,J=7.0,1. 0Hz),7.05(1H,td,J-7.0,1.0Hz),7.19(1H,s),7.20-7.40(3H,m),7.34 (1H,d,J=8.1Hz),7.66-7.64(4H,m),7.77(1H,td,J=7.6,1.7Hz),8.09-8. 21(2H,m),8.24-8.31(1H,m),8.52-8.64(2H,m),10.83(1H,d,J=1.7Hz).

### Synthesis Example 77-2: Synthesis of (2S)-2-(4-(N-2-picolylamino methyl) naphthoyl) amino-5-(5,6,7,8-tetrahydroquinolin-8-yl) aminovalerate 2-(3-indolyl) ethylamide [Compound No. 92]

In 4.7 ml of methanol, 469.7 mg of the compound obtained in Synthesis Example 77-1 was dissolved. Then, 4.7 ml of a 4 mol/l hydrochloric acid/dioxane was added to the solution and the whole was stirred for 2 hours at room temperature. On completion of the reaction, the solvent was distilled off and the residue was dried under vacuum. The resultant was dissolved in 4 ml of methanol, and then 143.9 mg of 5,6,7,8-tetrahydroquinolin-8-one and 78.7 mg of sodium cyanoborohydride were added to the solution, and furthermore 55 drops of acetic acid were added to the solution to adjust pH thereof to approximately pH = 5 and the whole was stirred for 17 hours at room temperature. On completion of the reaction, the solvent was distilled off and the residue was then dissolved in methanol. Subsequently, the solution was neutralized by the addition of Amberlite IRA-410 and concentrated, then the residue was purified by means of silica gel column chromatography (15 g, chloroform/methanol = 10/2), consequently 191.8 mg of the above-mentioned compound was obtained as a white solid product.
MS(FAB,Pos.):m/z=680[M+1]⁺
¹H-NMR(500MHz,DMSO-d₆): δ=1.50-1.73(4H,m),1.73-1.91(4H,m),1.97-2.05(1H,m),2.61-2.78(4H,m),2.83-2.94(2H,m),3.37-3.52(2H,m),3. 89(2H,s),4.20(2H,s),4.47-4.53(1H,m),6.98(1H,t,J=7.0Hz),7.05(1 H,t,J=7.0Hz), 7.13-7.22 (2H,m), 7.24-7.28 (1H,m), 7.33 (1H,d,J=8.1H z), 7.43-7.61 (7H,m), 7.76 (1H,td,J=7.6,1.7Hz), 8.08-8.17 (1H,m), 8. 20-8.30 (2H,m), 8.34 (1H,d,J=4.4Hz), 8.52 (1H,ddd,J=4.9,1.7,1.0Hz) ,8.62-8.72(1H,m),10.85(1H,s).

### Synthesis Example 78: Production of N^{α}-4-(N-2-picolylaminomethyl) benzoyl-N^{G}-nitroarginine (1'S)-1'-(1-naphthyl) ethylamide [Compound No. 93]

### Synthesis Example 78-1: Synthesis of N^{α}-Boc-N^{G}-nitroarginine (1'S)-1'-(1-naphthyl) ethylamide (Compound XXVII-8)

3.00 g of commercially available N^{α}-Boc-N^{G}-nitroarginine was dissolved in dichloromethane, and then 3.96 ml of triethylamine and 1.69 g of (S)-1-(1-naphthyl) ethylamine were added to the solution. This solution was cooled with ice, and then 20 ml of a dichloromethane solution of 2.38 g of 2-chloro-1,3-dimethylimidazolium chloride (hereinafter, referred to as DMC) was dropped therein over 60 minutes. After the dropping was completed, the reaction solution was returned to room temperature and stirred for 70 minutes. On completion of the reaction, a 1 mol/l hydrochloric acid was added thereto, followed by extraction with chloroform. After being washed with a saturated salt solution, the resultant was dried with anhydrous sodium sulfate and the solvent was then distilled off. The residue was purified by means of silica gel column chromatography (chloroform/methanol = 25/1), consequently 4.10 g of the target product was obtained as a white solid product.
MS(FAB,Pos.):m/z=473[M+1]⁺

### Synthesis Example 78-2: Synthesis of N^{α}-4-(N-Boc-N-2-picolylamino methyl) benzoyl-N^{G}-nitro-L-arginine (1'S)-1'-(1-naphthyl) ethylamide (Compound XXIX-13)

1.0009 g of the compound obtained in Synthesis Example 78-1 was dissolved in methanol, and then a 4 mol/l hydrochloric acid/dioxane was added to the solution and the whole was stirred for 2 hours at room temperature. On completion of the reaction, the solution was concentrated and dried so as to be solidified, followed by drying under vacuum. Subsequently, 20 ml of dichloromethane was added and dissolved therein. Then, 1.25 ml of tritylamine and 721.4 mg of the compound obtained in Synthesis Example 1-2 were added thereto and the whole was cooled with ice. 5 ml of a dichloromethane solution of 439.1 mg of DMC was dropped therein over 20 minutes and the whole was stirred for 35 minutes under ice-cold condition. On completion of the reaction, a 1 mol/l hydrochloric acid was added thereto, followed by extraction with chloroform. After being washed with a saturated,salt solution, the resultantwas driedwith anhydrous sodium sulfate and the solvent was then distilled off. The residue was purified by means of silica gel column chromatography (50 g, chloroform/acetone = 2/1), consequently 745.3 mg of the target material was obtained as a white solid product.
MS(FAB,Pos.):m/z=697[M+1]⁺

### Synthesis Example 78-3: Synthesis of N^{α}-4-(N-2-picolylamino methyl) benzoyl-N^{G}-nitroarginine (1'S)-1'-(1-naphthyl) ethylamide [Compound No. 93]

In 1 ml of methanol, 204.1 mg of the compound obtained in Synthesis Example 78-2 was dissolved. Then, 1 ml of a 4 mol/l hydrochloric acid/dioxane was added to the solution and the whole was stirred for 2.5 hours at room temperature. On completion of the reaction, the solvent was distilled off, and the residue was azeotropically distilled with methanol, followed by drying under reduced pressure. Consequently, 196.1 mg of the above-mentioned compound was obtained as a white solid product.
MS(FAB,Pos.):m/z=597[M+1]⁺
¹H-NMR(500MHz,DMSO-d₆):δ=1.51(3H,d,J=6. 8Hz),1.45-1.60(2H,m),1. 62-1.81(2H,m),3.09-3.23(2H,m),4.30(2H,s),4.31(2H,s),4.51-4.58 (1H,m),5.70(1H,quint.,J=6.8Hz),7.42-7.58(6H,m),7.62(2H,d,J=8. 1Hz),7.82(1H,d,J=7.8Hz),7.90(1H,td,J=7.8,2.0Hz),7.94(1H,d,J=7 .8Hz),7.96(2H,d,J=8.1Hz),8.09(1H,d,J=8.5Hz),8.49(2H,brs),8.66 (1H,ddd,J=4.9,1.7,1.0Hz),8.73(1H,brs},9.60-9.78(1H,br).

### Synthesis Example 79: Production of (2R)-2-(4-(N-(imidazol-2-yl methyl) aminomethyl) benzoyl) amino-5-(5,6,7,8-tetrahydro quinolin-8-yl) aminovalerate (1'S)-1'-(1-naphthyl) ethylamide [Compound No. 94]

### Synthesis Example 79-1: Synthesis of 4-(N-Boc-(N-imidazol-2-yl methyl) aminomethyl) benzoic acid (Compound VII-12)

10.01 g of commercially available bromomethyl benzoate methylester was dissolved in 100 ml of DMF, and then 9.70 g of potassium phthalimide was added to the solution and the whole was stirred for 1.5 hours at room temperature. On completion of the reaction, the solution was concentrated and then water was added, followed by extraction with chloroform. Subsequently, the resultant was washed with a saturated salt solution. After the resultant was dried with sodium hydrogensulfate, the solvent was distilled off. Consequently, 12.91 g of a white solid product was obtained. 7.56 g of the product was dissolved in 100 ml of methanol, and then 6.25 ml of hydrazine monohydrate was added to the solution and the whole was stirred for 1.5 hours at 60°C. On completion of the reaction, a precipitated solid product was separated by filtration and then the solvent was distilled off. Water was added totheproduct, followedbyextractionwithchloroform. Theproduct was washed with a 0.3 mol/l sodium hydroxide aqueous solution and a saturated salt solution. The resultant was dried with anhydrous sodium sulfate and then a solvent was distilled off. In this, 120 ml of methanol and 2.35 g of 2-imidazole carboaldehyde were added and the whole was stirred for 2 days at room temperature. On completion of the reaction, a precipitated solid product was collected by filtration. A liquid layer was concentrated and dried so as to be solidified, followed by adding and washing with 30 ml of anhydrous methanol. A solid product was separated by filtration. Said solid product and the solid product previously-collected by filtration were suspended in 86 ml of methanol, and then 1.42 g of sodium borohydride was added to the suspension under ice cold condition. The mixture was stirred for 1 hour at room temperature and the solvent was distilled off. After water was added, the resultant was extracted with chloroform, and an organic layer was then washed with a saturated salt solution. After being dried with anhydrous sodium sulfate, the resultant was concentrated under reduced pressure and dried, and 4.32 g of a colorless viscous product was obtained. 4.28 g of the product was dissolved in 65 ml of DMF, and 8.9 ml of di-t-butyldicarbonate was added to the solution and the whole was stirred for 1 hour at room temperature. On completion of the reaction, the solvent was distilled off. Subsequently, the residue was dissolved in chloroform and washed with a saturated salt solution. After the resultant was dried with anhydrous sodium sulfate, the solvent was distilled off, and then 43 ml of THF, 43 ml of methanol, and 43 ml of a 1 mol/l sodium hydroxide aqueous solution were added thereto and the whole was stirred for 14 hours at room temperature. On completion of the reaction, the solvent was distilled off, 5 ml of water was added to the resultant, and a 1 mol/l hydrochloric acid was carefully added to the resultant. Then, an acidic-precipitated product was collected by filtration and dried, consequently 4.87 g of the above-mentioned compound was obtained as a white solid product.
MS(FAB,Pos.):m/z=332[M+1]⁺

### Synthesis Example 79-2: Synthesis of N^{α}-(4-(N-(imidazol-2-yl methyl) aminomethyl) naphthoyl)-N^{δ}-Boc-D-ornithine (1'S)-1'-(1-naphthyl) ethylamide (Compound XI-15)

414.6 mg of commercially available (S)-1-(1-naphthyl) ethylamine was dissolved in 10 ml of DMF, and then 1.0006 g of commercially available N^{α}-Fmoc-N^{δ}-Boc-D-ornithine, 414.6 mg of WSCI hydrochloride, and 375. 5 mg of HOBt were added to the solution and the whole was stirred for 3 hours at room temperature. On completion of the reaction, the solvent was distilled off and chloroform was added to the residue, followed by washing with a 1 mol/l hydrochloric acid, a 1 mol/l sodium hydroxide aqueous solution, and a saturated salt solution. The solution was dried with anhydrous sodium sulfate and the solvent was distilled off, and 1.38 g of a white solid product was obtained. The product was dissolved in 27 ml of DMF, and then 2.7 ml of diethylamine was added to the solution and the whole was stirred for 40 minutes at room temperature, followed by distilling the solvent off and drying the residue under reduced pressure. The resultant was dissolved in 27 ml of DMF again, and then 633.5 mg of WSCI hydrochloride, 335.9 mg of HOBt, and 734.7 mg of the compound obtained in Synthesis Example 79-1 were added to the solution and the whole was stirred for 12 hours at room temperature. On completion of the reaction, the solvent was distilled off and chloroform was added to the residue, followed by washing with distilled water, a 1 mol/l sodium hydroxide aqueous solution, and a saturated salt solution. The solution was dried with anhydrous sodium sulfate, and the solvent was then distilled off. The resulting residue was purified by means of silica gel column chromatography (55 g, chloroform/methanol = 25/1), consequently 906.2 mg of the above-mentioned compound was obtained as a white solid product.
MS(FAB,Pos.):m/z=699[M+1]⁺
¹H-NMR(500MHz,DMSO-d₆) : δ=1.35 and 1.38(9H,2s),1.35-1.60 (2H,m), 1.50(3H,d,J=6.8Hz),1.68-1.82(2H,m),2.90-3.00(2H,m),4.30-4.55 (5H,m),5.70(1H,quint,J=6.8Hz),6.84(1H,t,J=5.6Hz),6.95(2H,brs), 7.19-7.31(2H,m),7.44-7.59 (3H,m),7.60(1H,d,J=7.1Hz),7.82(1H,d, J=8.1Hz),7.84(2H,d,J=8.3Hz),7.93(1H,d,J=7.6Hz),8.09(1H,d,J=7. 8Hz),8.35(1H,d,J=7.8Hz),8.55(1H,d,J=7.8Hz).

### Synthesis Example 79-3: Synthesis of (2R)-2-(4-(N-(imidazol-2-yl methyl) aminomethyl) benzoyl) amino-5-(5,6,7,8-tetrahydro quinolin-8-yl) aminovalerate (1'S)-1'-(1-naphthoyl)-ethylamide [Compound No. 94]

In 2.5 ml of methanol, 504.9 mg of the compound obtained in Synthesis Example 79-3 was dissolved. Then, 2.5 ml of a 4 mol/l hydrochloric acid/dioxane solution was added to the solution and the whole was stirred for 1.5 hours at room temperature. On completion of the reaction, the solvent was distilled off, followed by dissolving the residue in methanol again. The solution was neutralized with Amberlite IRA-410 . The solvent was distilled off and the residue was dissolved in 50 ml of methanol again. Subsequently, 0.1928 g of 5,6,7,8-tetrahydroquinolin-8-one, 0.5 ml of acetic acid, and 0.0861 g of sodium cyanoborohydride were added to the solution and the whole was stirred for 3 days at room temperature. On completion of the reaction, the solvent was distilled off. The residue was suspended in chloroform and was then washed with a 0.5 mol/l sodium hydroxide aqueous solution and a saturated salt solution. An organic layer was dried with anhydrous sodium sulfate. The solution was concentrated and the residue was then purified by means of silica gel column chromatography (25 g, chloroform/methanol/water = 7/3/0.5), consequently 312.3 mg of the above-mentioned compound was obtained as a white solid product.
MS(FAB,Pos.):m/z=630[M+1]⁺
¹H-NMR(500MHz,DMSO-d₆) : δ=1.48 (3H,d,J=6.8Hz), 1.42-1.70 (4H,m), 1. 75-1.90 (2H,m), 1.80-1.93 (1H,m), 1.95-2.05 (1H,m), 2.65-2.75 (2H,m) , 2.70-2.80 (2H,m), 3.66 (2H,s), 3.71 (2H,s), 3.62-3.70 (1H,m), 4.49-4 .55 (1H,m), 5.69 (1H,quint,J=6.8Hz), 6.81 and 6.99 (2H,br), 7.15-7.19 (1H,m), 7.40 (1H,d,J=8.5Hz), 7.41 (1H,d,J=8.8Hz), 7.44-7.55 (2H,m), 7.59(1H,d,J=7.3Hz),7.79(1H,d,J=8.3Hz),7.81(2H,d,J=8.5Hz),7.92 (1H,d,J=7.6Hz),8.09(1H,d,J=8.1Hz),8.32-8.36(1H,m),8.38 and 8.45(1H,d,J= 8.1Hz),8.56 and 8.58(1H,d,J=8.1Hz).

### Synthesis Example 80: Production of N^{α}-4-(N-2-(imidazol-2-yl methyl) aminomethyl) benzoyl-L-arginine (1'S)-1'-(1-naphthyl) ethylamide [Compound No. 95]

### Synthesis Example 80-1: Synthesis of N^{α}-(4-(N-Boc-N-(imidazol-2-ylmethyl) aminomethyl) benzoyl-N^{G}-Pmc-L-arginine (1'S)-1'-(1-naphthyl) ethylamide (Compound XXIX-14)

In 15 ml of DMF, 750. 5 mg (0. 920 mmol) of the compound obtained in Synthesis Example 71-1 was dissolved. Subsequently, 1.5 ml of diethylamine was added to the solution and the whole was stirred for 30 minutes at room temperature, and then the solvent was distilled off and dried under reduced pressure. The resultant was dissolved in 15 ml of DMF again, and then 269.0 mg of WSCI hydrochloride, 140.4 mg of HOBt, and 326.4 mg of the compound obtained in Synthesis Example 79-1 were added to the solution and the whole was stirred for 2 days at room temperature. On completion of the reaction, the solvent was distilled off and then chloroform was added to the residue, followed by washing with a 0.5 mol/l sodium hydroxide aqueous solution and a saturated salt solution. The solution was dried with anhydrous sodium sulfate and then the solvent was distilled off. The resulting residue was purified by means of silica gel column chromatography (50 g, chloroform/methanol = 20/1), consequently 237.4 mg (28.5%) of the above-mentioned compound was obtained as a white solid product.
MS(FAB,Pos.):m/z=907 [M+1]⁺

### Synthesis Example 80-2: Synthesis of N^{α}-4-(N-2-(imidazol-2-yl methyl) aminomethyl) benzoyl-L-arginine (1'S)-1'-(1-naphthyl) ethylamide [Compound No. 95]

In 4.2 ml of chloroform, 210.1 mg of the compound obtained in Synthesis Example 80-1 was dissolved. Then, 0.45 ml of methanesulfonic acid was added to the solution and the whole was stirred for 1 day at room temperature. On completion of the reaction, the solution was concentrated and dried. The residue was washed with diethylether and then methanol was added thereto; followed by distilling the solvent off again. The residue was purified by means of silica gel column chromatography (5 g, chloroform/methanol/water = 7/3/0.5) and then a fraction was concentrated and dried under reduced pressure, consequently 99.6 mg of salt of the above-mentioned compound with methanesulfonic acid was obtained as a white solid product.
MS(FAB,Pos.):m/z=541[M+1]⁺
¹H-NMR(500MHz,DMSO-d₆):δ=1.41-1.61(2H,m),1.51(3H,d,J=6.8Hz),1. 65-1.82(2H,m),2.38(12H,s),3.02-3.12(2H,m),4.34(2H,s),4.48(2H, s),4.52-4.58(1H,m),5.71(1H,quint,J=6.8Hz),6.7-7.4(4H,br),7.46 -7.58(5H,m),7,.60(1H,d,J=8.3Hz),7.68(2H,s),7.83(1H,d,J=8.3Hz), 7.95(1H,d,J=7.1Hz),7.98(2H,d,J=8.3Hz),8.10(1H,d,J=8.1Hz),8.50 (1H,d,J=8.1Hz),8.70(1H,d,J=7.6Hz).

### Synthesis Example 81: Production of (2S)-2-((1-methylimidazol-2-ylmethyl) aminomethyl) benzoylamino-5-(5,6,7,8-tetrahydro quinolyl-8-yl)aminovalerate 1-naphthaleneamide [Compound No. 96]

### Synthesis Example 81-1: Synthesis of methyl 4-(N-Boc-N-(1-methyl imidazol-2-yl) methylamino-methyl) benzoate (Compound VI-9)

In 10 ml of methanol, 1.00 g of commercially available methyl aminomethyl benzoate and 0.68 g of 1-methyl-2-imidazole carboxylaldehyde were dissolved. While stirring the solution at 0°C, 1.95 g of triacetoxy sodium borohydride was added to the solution and the whole was stirred for 1 hour. Subsequently, it was returned to room temperature and further stirred for 45 minutes. The reaction solution was concentrated and then chloroform was added, followed by washing with a saturated sodium bicarbonate aqueous solution and further washing with distilled water and a saturated salt solution. After that, an organic layer was concentrated and dried under reduced pressure and was then dissolved in 30 ml of DMF . 0.61 g of triethylamine was dropped into the solution, and furthermore anhydrous di-t-butyldicarbonate was dropped at room temperature and stirred for 3.5 hours. The reaction solution was concentrated and dried under reduced pressure. Then, chloroform was added to the residue, followed by washing the residue with a saturated ammonium chloride solution, distilled water, and a saturated salt solution. An organic layer was concentrated and evaporated under reduced pressure. Subsequently, the residue was purified by means of silica gel column chromatography (100 g, chloroform/methanol = 50/1), consequently 1.33 g of the above-mentioned compound was obtained as a yellow-oily product.
MS(FAB,Pos.):m/z=360[M+1]⁺
¹H-NMR(500MHz,CDCl₃) :δ=1.47,1.53(9H,2s),3.49,3.61(3H,2s),3.91 1H,s),4.47(2H,s),4.50(brs,2H),6.80(1H,brs),6.93(1H,d,J=1.2Hz) ,7.22(1H,brs),7.95(1H,d,J=7.8Hz),8.02(1H,s).

### Synthesis Example 81-2: Synthesis of N-Boc-N-(1-methylimidazol -2-yl) aminomethyl benzoic acid (Compound VII-13)

In 10 ml of methanol, the compound obtained in Synthesis Example 81-1 was dissolved. Then, 10ml of a 1 mol/l sodiumhydroxide aqueous solution was dropped therein and the whole was stirred for 3.5 hours at room temperature. Ion exchange resin (CG 50) was added to the reaction solution and pH was adjusted to almost neutral, followed by leaving the solution as it was for 1 hour. After that, the resin was removed by a glass filter. The reaction solution was concentrated and evaporated to dryness under reduced pressure, and 1.39 g of a crude reaction product of yellow solid was obtained. It was purified by means of silica gel column chromatography (100 g, chloroform/methanol = 50/1), consequently 1.33 g of the above-mentioned compound was obtained as a yellow oily product.
MS(FAB,Pos.):m/z=346(M+1]⁺
¹H-NMR(500MHz,DMSO-d₆): δ=1.34,1.42(9H,2s),3.56(3H,s), 4.39-4.48 (4H,brs),6.80(1H,d,J=1.2Hz),7.08(1H,brs),7.24(1H,brs),7.89(2H ,s,J=8.3Hz).

### Synthesis Example 81-3: Synthesis of N^{α}(1-methylimidazol-2-yl methyl) aminomethylbenzoyl-N^{δ}-Boc-L-ornithine 1-naphthalene methyleneamide (Compound XI-16)

In 30 ml of DMF, 1. 50 g of the compound obtained in Synthesis Example 23-1 was dissolved. Then, 3.0 ml of diethylamine was dropped therein at room temperature. The whole was stirred for 2.5 hours without modification, and then the reaction solution was concentrated and dried under reducedpressure. Itwas dissolved in 30 ml of DMF, and then 0.73 g of WSCI hydrochloride and 0.46 g of DMAP were added. Furthermore, 0.87 g of the compound obtained in Synthesis Example 81-2 was added. After being stirred overnight at room temperature, the reaction solution was concentrated and evaporated to dryness under reduced pressure. The residue was dissolved in chloroform and was then washed with 1 mol/l hydrochloric acid, distilled water, and a saturated salt solution, followed by concentration under reduced pressure. Subsequently, the residue was purified by means of silica gel column chromatography (120 g, chloroform/methanol = 20/1), consequently 0.73 g of the above-mentioned compound was obtained as a yellow oily product.
MS(FAB,Pos.):m/z=699[M+1]
¹H-NMR(500MHz,DMSO-d₆): δ=1.36-1.42(9H,brs),1.46-1.51(2H,brs), 1.71-1.76(2H,brs),2.29(2H,m),3.57(3H,brs),4.36-4.50(5H,brs), 4.75(2H,d,J=5. 8Hz),6.82 (2H,brs),7.09(1H,brs),7.22(1H,brs),7.46 (2H,s),7.53(3H,m),7.85(3H,m),7.94(1H,m),8.05(1H,m),8.45(1H,brs ),8.51(1H,brs).

### Synthesis Example 81-4: Synthesis of (2S)-2-((1-methylimidazol-2-ylmethyl) aminomethyl) benzoylamino-5-(5,6,7,8-tetrahydro quinolyl-8-yl) aminovalerate 1-napthalenemethyleneamide [Compound No. 96]

In 4 ml of methanol, 0.19 g of the compound obtained in Synthesis Example 81-3 was dissolved. Then, 4 ml of a 4 mol/l hydrochloric acid/dioxane solution was dropped therein at room temperature and the whole was stirred for 2 hours without modification. The reaction solution was concentrated, and 0.20 g of a crude reaction product was obtained as a colorless oily product. This product and 0.05 g of 5,6,7,8-tetrahydroquinoline were dissolved in 4 ml of methanol, and then 0.08 g of triethylamine was dropped therein at room temperature. Acetic acid was added to the solution to adjust pH to about 4. Subsequently, 0.07 g of sodium cyanoborohydride was added to the solution and the whole was stirred overnight at room temperature. After that,thereaction solution was concentrated under reduced pressure. The residue was purified by means of silica gel column chromatography (9g, chloroform/methanol = 5/1) and was then treated with a 1 mol/l hydrochloric acid, consequently 0.07 g of hydrochloride of the above-mentioned compound was obtained as a white solid product.
MS(FAB,Pos.):m/z=630[M+1]⁺
¹H-NMR(500MHz,DMSO-d₆) : δ=1.73-1.84 (3H,brs),1.86-1.91 (3H,brs),1 .98 (1H,brs), 2.29 (1H,brs), 2.79 (2H,m), 2.95 (1H,brs), 3.08 (1H,m), 3 .97 (3H,s), 4.41 (3H,brs), 4.53-4.60 (3H,brs), 4.76 (2H,d), 7.37 (1H,m ), 7.46 (2H,d), 7.54 (2H,m), 7.67 (1H,d), 7.74 (2H,d), 7.76 (2H,d), 7.84 (1H,m), 7.94 (1H,m), 8.00 (2H,m), 8.07 (1H,m), 8.46 (1H,m), 8.69-8.74 (2H,m),9.14(1H,brs),10.73(1H,brs).

### Synthesis Example 82: Production of N^{α}-(4-((N-(1-methylimidazol -2-ylmethyl) amino) methyl) naphthalene-1-carbonyl)-L-arginine (1'S)-1'-(1-naphthyl) ethylamide [Compound No. 97]

### Synthesis Example 82-1: Synthesis of N^{α}-(4-((N-Boc-N-(1-methyl imidazol-2-ylmethyl) amino) methyl) naphthalene-1-carbonyl amino)-N^{G}- Pmc-L-arginine (1'S)-1'-(1-naphthyl) ethylamide (Compound XXIX-15)

In 7 ml of DMF, 0. 3131 g of the compound obtained in Synthesis Example 48-1 was dissolved. Then, 0.056 g of diethylamine was added to the solution and the whole was stirred for 1.5 hours, followed by distilling the solvent off. The residue was dissolved in 6 ml of DMF, and then 0.167 g of the compound obtained in Synthesis Example 74-3, 0.0778 g of HOBt, and 0.110 g of WSCI hydrochloride were added to the solution and the whole was stirred for 19 hours. Subsequently, water was added and the solvent was distilled off. Then, chloroform was added to the residue. The residue was washed with a saturated sodium bicarbonate aqueous solution and dried with anhydrous magnesium sulfate, followed by distilling the solvent off and purifying the reside through silica gel column chromatography (15 g, 5% methanol/chloroform). Consequently, 0.3137 g of the above-mentioned compound was obtained as a white solid product.
MS(FAB,Pos.):m/z=971[M+1]⁺
¹H-NMR(500MHz,CDCl₃) : δ=1.28 (6H,s), 1.38-1.70 (4H,m), 1.43 (9H,s), 1.60 (3H,d,J=7.1Hz), 1.78 (2H,t,J=6.8Hz), 2.07 (3H,s), 2.49 (3H,s), 2.58 (2H,t,J=6.8Hz), 3.18-3.30 (2H,m), 3.54 and 3.68 (3H,s), 4.39 and 4.53 (2H,s), 4.74-4.84 (1H,m), 4.96 (2H,s), 5.85 (1H,quint.,J=6.8Hz), 6.03 and 6.16 (1H,bs), 6.80 (1H,s), 6.83 (1H,s), 7.24 (1H,d,J=8.5Hz), 7.39 (1H,t,J=7.8Hz), 7.44-7.55 (5H,m), 7.59 (1H,d,J=7.3Hz), 7.74 (1H, d,J=8.3Hz),7.83(1H,d,J=7.3Hz),8.01(2H,bs),8.23(1H,d,J=6.1Hz).

### Synthesis Example 82-2: Synthesis of N^{α}-(4-((N-(1-methylimidazol -2-ylmethyl) amino) methyl) naphthalene-1-carbonyl)-L-arginine (1'S)-1'-(1-naphthyl) ethylamide [Compound No. 97]

In 3.2 ml of chloroform, 0.3099 g of the compound obtained in Synthesis Example 82-4 was dissolved. This solution was cooled with ice, and then 3.2 ml of TFA was added to the solution and the whole was stirred for 3.5 hours at room temperature, followed by distilling the solvent off. On completion of the reaction, 1 mol/l hydrochloric acid and chloroform were added to the solution and mixed together, and an aqueous phase was separated and washed with chloroform, followed by distilling the solvent off. The resulting residue was dissolved in 1 mol/l hydrochloric acid and the solvent was then distilled off. Furthermore, the resulting residue was reprecipitated from water-acetone. A precipitate was collected by filtration and was then dried, consequently 0.2088 g of hydrochloride of the above-mentioned compound was obtained as a white solid product.
MS(FAB,Pos.):m/z=[M+1]⁺
¹H-NMR(500MHz,DMSO-d₆) : δ=1.50-1.62 (2H,m),1.56 (3H,d,J=7.1Hz), 1. 62-1.75 (1H,m), 1.76-1.84 (1H,m), 3.08-3.15 (2H,m), 3.89 (3H,s), 4.55 -4.68 (3H,m), 4.80 (2H,s), 5.78 (1H,quint.,J=7.1Hz), 7.49-7.73 (7H,m ), 7.78 (1H,d,J=7.3Hz), 7.84 (1H,d,J=8.3Hz), 7.96 (1H,d,J=7.8Hz), 8. 14 (1H,d,J=8.3Hz), 8.26 (1H,d,J=8.3Hz), 8.30 (1H,d,J=8.3Hz), 8.70 (1 H,d,J=8.1Hz),8.79(1H,d,J=7.8Hz).

### Synthesis Example 83: Production of N^{α}-(4-((imidazol-2-ylmethyl) amino) methyl) naphthalene-1-carbonyl)-L-arginine (1'S)-N-methyl-N-(1'-(1-naphthyl) ethyl) amide [Compound No. 98]

### Synthesis Example 83-1: Synthesis of N^{α}-Fmoc-N^{G}-Pmc-L-arginine (1'S)-N-methyl-(1'-(1-naphthyl) ethyl) amide (Compound XXVII-9)

In 20 ml of DMF, 1.000 g of commercially available N^{α}-Fmoc-N^{G}-Pmc-arginine was dissolved. Then, 0.224 g of HOBt was added to the solution and the whole was cooled with ice, and also 0.318 g of WSCI hydrochloride was added to the solution and the whole was stirred for 15 hours at room temperature. The reaction solution was concentrated and then chloroform was added to the solution, followed by washing with a saturated sodium bicarbonate aqueous solution. A water layer was extracted with chloroform and an organic layer was dried with magnesium sulfate. After that, the solvent was distilled off and the residue was then purified by means of silica gel column chromatography (40 g, 2% methanol/chloroform), consequently 0.5749g of the above-mentioned compound was obtained as a light-yellow viscous product.
MS(FAB,Pos.):m/z=830[M+1]⁺
¹H-NMR(500MHz,CDCl₃):δ=1.26(3H,s),1.27(3H,s),1.38-1.60(4H,m),1 .62(3H,d,J=7.1Hz),1.77(2H,t,J=7.1Hz),2.04(3H,s),2.45(3H,s),2. 49(6H,s),2.56 (2H,t,J=7.1Hz),2.95-3.05(1H,m),3.16-3.26(1H,m),4 .21(1H,t,J=7.1Hz),4.37(1H,dd,J=10.5,7.1Hz),4.43(1H,dd,J=10.5, 7.1Hz),4.57(1H,t,J=8.3Hz),5.87(2H,bs),6.09(1H,d,J=8.3Hz),6.52 (1H,q,J=6.6Hz),7.31(1H,t,J=7.1Hz),7.33(1H,t,J=7.1Hz),7.38-7.5 1(6H,m),7.54(1H,d,J=7.1Hz),7.60(2H,t,J=6.8Hz),7.76-7.90(5H,m)

### Synthesis Example 83-2: Synthesis of N^{α}-(4-((N-Boc-N-(imidazol -2-ylmethyl) aminomethyl) naphthalene-1-carbonyl)-N^{G}-Pmc-L-arginine (1'S)-N-methyl-(1'-(1-naphthyl) ethyl) amide (Compound XXIX-16)

In 5 ml of DMF, 0.2356 g of the compound obtained in Synthesis Example 83-1 was dissolved. Then, 0.042 g of diethylamine was added to the solution and the whole was stirred for 1.5 hours, followed by distilling the solvent off. The residue was dissolved in 5 ml of DMF, and then 0.108 g of the compound obtained in Synthesis Example 17-4, 0.0581 g of HOBt, and 0.164 g of WSCI hydrochloride were added to the solution and the whole was stirred for 6 days. Subsequently, water was added to the reaction solution and the solvent was distilled off. Subsequently, chloroform was added to the residue, and then the residue was washed with a saturated sodium bicarbonate aqueous solution and a 1 mol/l hydrochloric acid solution, and was then dried with anhydrous magnesium sulfate, followed by distilling the solvent off and purifying the residue through silica gel column chromatography (20 g, 5% methanol/chloroform). Consequently, 0.1814 g of the above-mentioned compound was obtained as a light-yellow viscous liquid product.
MS(FAB,Pos.):m/z=971[M+1]⁺
¹H-NMR(500MHz,CDCl₃):δ=1.27 and 1.28 (9H,s),1.40-1.60(4H,m),1.50 (9H,s),1.67(3H,d,J=6.8Hz),1.76(2H,t,J=6.6Hz),2.04(3H,s),2.50 (6H,s),2.56(3H,s),2.57(2H,t,J=6.7Hz),3.04-3.26(1H,m),3.34-3.42 (1H,m),4.36(2H,s),4.96(2H,s),5.14(1H,t,J=6.1Hz),6.07 and 6.31 (2H,bs),6.54(1H,q,J=7.1Hz),6.96(2H,s),7.33(2H,d,J=6.6Hz),7.43 -7.60(6H,m),7.68(2H,d,J=7.3Hz),7.83(2H,d,J=8.3Hz),7.85(2H,d,J =8.1Hz),7.88(2H,d,J=7.8Hz),8.04(2H,d,J=7.3Hz),8.35(2H,d,J=8.3 Hz).

### Synthesis Example 83-3: Synthesis of N^{α}-(4-((imidazol-2-ylmethyl) amino) methyl) naphthalene-1-carbonyl) L-arginine (1'S)-N-methyl-N-(1'-(1-naphthyl) ethyl) amide [Compound 98]

In 1.8 ml of chloroform, 0.1814 g of the compound obtained in Synthesis Example 83-1 was dissolved. Then, 1.8 ml of TFA was added to the solution and the whole was stirred for 5 hours at room temperature, followed by distilling the solvent off. Then, 1 mol/l hydrochloric acid and chloroform were added to the residue. A water layer was separated and washed with chloroform, followed by distilling the solvent off. The residue was passed through silica gel column chromatography (2 g, chloroform/methanol/32% AcOH = 7/3/0.5) and dissolved in 2 ml of 1 mol/l hydrochloric acid, followed by distilling the solvent off. Subsequently, it was dissolved in water and then acetone was added. The resulting oily product was precipitated by a centrifugal separator and then a supernatant was removed. The remaining solvent was distilled off and the residue was dissolved in a small amount of methanol again, followed by the addition of ethyl acetate to precipitate a solid material. Subsequently, the solvent was distilled off, consequently 0.0566 g of hydrochloride of the above-mentioned compound was obtained as a white solid product.
MS(FAB,Pos.):m/z=605[M+1]⁺
¹H-NMR(500MHz,DMSO-d₆) : δ=1.50-1.76 (4H,m), 1.61 (3H,d,J=7.1Hz), 2. 61 (3H,s), 3.00-3.15 (2H,m), 4.65 (2H,s), 4.82-4.92 (1H,m), 4.85 (2H,s ), 6.41 (1H,q,J=6.8Hz), 7.50-7.62 (4H,m), 7.64-7.75 (9H,m), 7.83 (1H, d,J=7.3Hz), 7.91 (1H,d,J=8.1Hz), 7.94 (1H,d,J=8.3Hz), 7.98 (1H,d,J= 7.8Hz), 8.30 (1H,dd,J=7.1,1.2Hz), 8.33 (1H,d,J=7.6Hz), 9.01 (1H,d,J =7.3Hz).

### Synthesis Example 84: Production of N^{α}-(4-(N-2-picolylamino methyl) naphthoyl)-L-arginine (1'S)-1'-(1-naphthyl) ethylamide [Compound No. 99]

### Synthesis Example 84-1: Synthesis of N^{α}-(4-((N-Boc-N-2-picolyl amino) methyl) naphthoyl)-N^{G}-Pmc-L-arginine (1'S)-1'-(1-naphthyl) ethylamide (Compound XXIX-17)

In 4 ml of DMF, 0.200 g of the compound obtained in Synthesis Example 48-1 was dissolved. Then, 0.054 g of diethylamine was added to the solution and the whole was stirred for 2 hours, and then the solvent was distilled off, and a viscous liquid product was obtained. In the viscous product, 0.0962 g of the compound obtained in Synthesis Example 43-2 and 0.0497 g of HOBt were added. Subsequently, the viscous product was dissolved in 5 ml of DMF, and 0.071 g of WSCI hydrochloride was then added to the solution and the whole was stirred for 16 hours. Water was added to the reaction solution and the mixture solution was concentrated, followed by the addition of chloroform. It was subjected to a solid-phase extraction column: Chem-Elut (CE1003) permeated with a saturated sodium bicarbonate aqueous solution, and then the solvent was distilled off. The resulting mixture was purified by means of silica gel column chromatography (20 g, 4% methanol/chloroform), consequently 0.1504g of the above-mentioned compound was obtained as a white frothy material.
MS(FAB,Pos.):m/z=968[M+1]⁺
¹H-NMR(500MHz,CDCl₃):δ=1.29 (6H,s),1.44 and 1.48(9H,s),1.40-1.70 (4H,m),1.59(3H,d,J=5.4Hz),1.78(2H,t,J=6.6Hz),2.08(3H,s),2.50 (3H,s),2.51(3H,s),2.58(3H,t,J=6.3Hz),3.20-3.30(2H,m),4.38 and 4.53(2H,s),4.78-4.86(1H,m) 4.99 and 5.03(2H,s), 5.80-5.88(1H,m), 5.92-6.00 and 6.10-6.22(2H,bs),7.05-7.20(3H,m),7.34-7.60(8H,m), 7.73(1H,d,J=8.3Hz),7.83(1H,d,J=7.8Hz),7.94-8.06(2H,m),8.18-8. 30 (2H,m), 8.49 (1H,s).

### Synthesis Example 84-2: Synthesis of N^{α}-(4-(N-2-picolylamino methyl) naphthoyl)-L-arginine (1'S)-1'-(1-naphthyl) ethylamide [Compound No. 99]

In 1.5 ml of chloroform, 0.1448 g of the compound obtained in Synthesis Example 84-1 was dissolved. The solution was cooled with ice, and 1.44 ml of TFA was then added to the solution and the whole was stirred for 4 hours at room temperature, followed by distilling the solvent off. It was dissolved in methanol and was then neutralized with ion exchange resin (Amberlite, IRA-410), followed by filtrating and distilling the solvent off. Then, 1 mol/l hydrochloric acid was added and the solvent was distilled off. A mixture, which was obtained by drying under vacuum after azeotropically distilling with methanol and dehydrating, was purified by means of silica gel column chromatography (3 g chloroform/methanol/water = 7/3/0.5). Subsequently, 1 mol/l hydrochloric acid was added to the resulting solid material and then the solvent was distilled off, followed by azeotropically distilling with methanol. After that, it was dissolved in methanol and reprecipitated with acetone, followed by distilling the solvent off. Consequently, 0.0981 g of hydrochloride of the above-mentioned compound was obtained as a white solid product.
MS(FAB,Pos.):m/z=602[M+1]+
¹H-NMR(500MHz,DMSO-d₆):δ=1.50-1.64(2H,m),1.56(3H,d,J=6.8Hz), 1.64-1.76(2H,m),1.80-1.90(2H,m),3.08-3.20(2H,m),4.60-4.68(1H,m) ,4.77(2H,s),5.778(1H,quint.,J=7.1Hz),7.20-7.40(2H,bs),7.48-7. 72(9H,m),7.81(1H,d,J=7.3Hz),7.84(1H,d,J=8.3Hz),7.90-8.00(3H,m ),8.15(1H,d,J=8.1Hz),8.26(1H,d,J=8.5Hz);8.29(1H,d,J=8.3Hz),8. 71(1H,d,J=4.2Hz),8.73(1H,d,J=8.1Hz),8.86(1H,d,J=7.6Hz),9.91(2H,bs).

### Synthesis Example 85: Production of N^{α}-(4-(N-2-picolylamino methyl) naphthalene-1-carbonyl)-L-arginine-D-3-(1-naphthyl) alaninemethylester [Compound No. 100]

### Synthesis Example 85-1: Synthesis of D-3-(1-naphthyl) alanine methylester

1 ml of methanol was cooled to-10°C, and 0.091 g of thionyl chloride was gradually added while stirring it. After 10 minutes, 0.04569 g of commercially available D-3-(1-naphthyl)alanine was added to the solution and the whole was stirred for 21 hours at room temperature, followed by concentrating it under reduced pressure. Then, 12 ml of methanol was added and the solvent was distilled off,these procedureswererepeated twice. The resulting mixture was distributed into a saturated sodiumbicarbonate aqueous solution and chloroform, followed by liquidly separating the mixture. A water layer was extracted with chloroform. An organic layer was dried with anhydrous sodium sulfate and then the solvent was distilled off, consequently 40.3 mg of the above-mentioned compound was obtained as a colorless liquid.
MS(FAB,Pos.) :m/z=230[M+1]⁺
¹H-NMR(500MHz,CDCl₃):δ=3.14(1H,dd,J=13.9,8.8Hz),3.69(1H,dd,J=1 3.9,4.9Hz),3.91(1H,dd,J=8.8,4.9Hz),7.35(1H,d,6.1Hz),7.42(1H,d d,7.1,1.0Hz),7.48-7.57(2H,m),7.78(1H,d,8.3Hz),7.87(1H,dd,J=8. 1,1.2Hz), 8.09 (1H,d,J=8.5Hz).

### Synthesis Example 85-2: Synthesis of N^{α}-Fmoc-N^{G}-Pmc-L-arginine -D-3-(1-naphthyl) alanine methylester (Compound XXVII-10)

In 2 ml of DMF, 0.1258 g of commercially available N^{α}-Fmoc-N^{G}-Pmc-arginine was dissolved. Then, a reaction solution, which was prepared by dissolving 0.0233 g of HOBt, 0.0473 g of WSCI hydrochloride, and 0.0377 g of the compound obtained in Synthesis Example 85-1 in 2 ml of DMF, was added to the solution and the whole was stirred for 3 days. Water was added to the reaction solution and then the solvent was distilled off. The residue was dissolved in chloroform and washed sequentially with a 1 mol/l hydrochloric acid and a saturated sodium bicarbonate aqueous solution, followed by drying with anhydrous sodium sulfate. The crude product obtained by distilling the solvent off was purified by means of silica gel column chromatography (7 g, 5% methanol/chloroform),consequently 0.1497 g of the above-mentioned compound was obtained as a white solid product.
MS(FAB,Pos.) :m/z=874[M+1]⁺
¹H-NMR(500MHz,DMSO-d₆) : δ=1.25(3H,s), 1.26(3H,s), 1.28-1.46(3H,m) ,1.54-1.66(1H,m),1.73(2H,t,6.8Hz),2.08(3H,s),2.56(3H,s),2.57 (2H,t,6.8Hz),2.59(3H,s),3.02-3.22(2H,m),3.41(1H,dd,J=14.2,8.8H z),3.63(3H,s),3.666(1H,dd,J=14.2,5.6Hz),4.09(1H,t,J=7.1Hz),4. 14-4.20(1H,m),4.24-4.33(2H,m),4,90-4.96(1H,m),5.75(1H,d,J=7.3 Hz),6.01(1H,bs),7.20-7.30(4H,m),7.36(2H,t,J=7.6Hz),7.44-7.53 (4H,m),7.67-7.69(1H,m),7.73(2H,d,J=7.6Hz),7.80(1H,d,J=7.8Hz),8 .052(1H,d,J=8.5Hz).

### Synthesis Example 85-3: Synthesis of N^{α}-(4-((N-Boc-N-2-picolyl amino) methyl) naphthalene-1-carbonyl)-N^{G}-Pmc-L-arginine-D-3-(1-naphthyl) alanine methylester (Compound XXIX-18)

In 5 ml of DMF, 0.142 g of the compound obtained in Synthesis Example 85-2 was dissolved. Then, 0.036 g of diethylamine was added to the solution and the whole was stirred for 1.5 hours and the solvent was then distilled off. Subsequently, 0.0701 g of the compound obtained in Synthesis Example 43-2 and 0.0329 g of HOBt were added to the resulting residue, and then the residue was dissolved in 5 ml of DMF, followed by adding 0.0467 g of WSCI and stirring for 38 hours. Water was added to the reaction solution and the mixture solution was concentrated, and then it was dissolved in chloroform andwashedwith a saturated sodiumbicarbonate aqueous solution, followed by drying with anhydrous magnesium sulfate. The solvent was distilled off and the residue was purified by means of silica gel column chromatography (10 g, 4% methanol/chloroform), consequently 0.1612 g of the above-mentioned compound was obtained as a white solid product.
MS(FAB,Pos.):m/z=1026[M+1]⁺
¹H-NMR(500MHz,CDCl₃) : δ=1.28 (6H,s), 1.38-1.56 (3H,m), 1. 43 and 1.47 (9H,s), 1.64-1.76 (1H,m), 1.77 (2H,t,J=6.8Hz), 2.08 (3H,s), 2.50-2.6 2(8H,m),3.04-3.22(2H,m),3.40(1H,dd,J=13.9,9.3Hz),3.64-3.72(1H ,m),3.65(3H,s),4.37 and 4.51(2H,s),4.73(1H,dd,J=13.7,8.3Hz), 4.94-5.04(3H,m),6.08(2H,bs),6.94-7.02(1H,m),7.05-7.15(2H,m),7 .20-7.36(6H,m),7.42-7.62(7H,m),7.69(1H,d,J=8.10Hz),7.80(1H,d,J =.1Hz),8.06(1H,d,J=8.5Hz),8.20(1H,d,J=7.6Hz),8.48(1H,bs).

### Synthesis Example 85-4: Synthesis of N^{α}-(4-(N-2-picolylamino methyl) naphthalene-1-carbonyl)-L-arginine-D-3-(1-naphthyl) alanine methylester [Compound No. 100]

In 0.4 ml of chloroform, 0.04066 g of the compound obtained in Synthesis Example 85-3 was dissolved. Then, 0.20 ml of trifluoroacetic acid was added to the solution and the whole was stirred for 8 hours, followed by distilling the solvent off. It was azeotropically distilled with methanol twice and then purified by means of silica gel column chromatography (3 g, chloroform/methanol/water = 7/3/0.5). Subsequently, it was treated with a hydrochloric acid/methanol solution and the solvent was then distilled off, consequently 0.0307 g of hydrochloride of the above-mentioned compound was obtained as a white solid product.
MS(FAB,Pos.):m/z=660[M+1]⁺
¹H-NMR(500MHz,DMSO-d₆) : δ=1.33-1.41 (2H,m), 1.45-1.53 (1H,m), 1.60-1.67 (1H,m), 3.02-3.09 (2H,m), 3.35 (1H,dd,J=14.2,10Hz), 3.66-3.70 (1H,m), 3.67 (3H,s), 4.47 (2H,s), 4.56 (1H,dd,J=8.3,5.9Hz), 4.67-4.72 (1H,m), 4.77 (2H,s), 7.40 (1H,dd,J=8.3,7.1Hz), 7.46-7.49 (2H,m), 7.5 2-7.71 (7H,m), 7.77 (1H,d,J=7.3Hz), 7.82 (1H,d,J=8.3Hz), 8.12 (1H,d, J=8.3Hz), 8.22 (1H,dd,J=8.3,1Hz), 8.29 (1H,d,J=8.5Hz), 8.66 (1H,d,J =8.1Hz), 8.70 (1H,d,J=4.9Hz), 8.80 (1H,d,J=8.1Hz), 9.75 (2H,bs).

### Synthesis Example 86: Production of N^{α}-(4-(N-2-picolylamino methyl) naphthalene-1-carbonyl)-L-arqinine-D-3-(1-naphthyl) alanine [Compound No. 101]

0.0727 g of the compound obtained in Synthesis Example 85-3 was suspended in 0.5 ml of methanol and 1 ml of THF, followed by adding 0.08 ml of a 1 mol/l sodium hydrate aqueous solution and stirring the whole for 1 hour at room temperature. After distilling the solvent off, the mixture was dissolved in methanol and was then treated with Amberlite CG-50. The solvent was distilled off, and then it was dissolved in 0.7 ml of chloroform. Then, 0.7 ml of trifluoroacetic acid was added to the solution and the whole was stirred for 10 hours, followed by distilling the solvent off. It was washed with dichloromethane twice after adding 1 mol/l hydrochloric acid, and then the solvent in a water layer was distilled off, followed by treatment with Amberlite CG-50. After distilling the solvent off, it was purified by means of silica gel column chromatography (500 mg, chloroform/methanol/water = 7/3/0.5), consequently 0.0507 g of the above-mentioned compound was obtained as a light-yellow solid product.
MS(FAB,Pos.):m/z=646[M+1]⁺
¹H-NMR(500MHz,DMSO-d₆) : δ=1.32-1.42 (2H,m), 1.43-1.49 (1H,m),1.59-1.65 (1H,m), 3.00-3.11 (2H,m), 3.29 (1H,dd,J=14.2,10.3Hz), 3.67-3.7 2 (1H,m), 4.465 (2H,s), 4.56-4.67 (2H,m), 4.77 (2H,s), 7.40 (1H,dd,J=8 . 1,6.8Hz), 7.46-7.55 (3H,m), 7.58-7.64 (4H,m), 7.68 (1H,ddd,J=8.3,6 . 8,1.2Hz), 7.75-7.82 (3H,m), 7.90-7.95 (2H,m), 8.16 (1H,d,J=8.3Hz), 8.22 (1H,dd,J=8.5,1.0Hz), 8.28 (1H,d,J=8.5Hz), 8.63 (1H,d,J=8.3Hz) ,8.67(1H,d,J=8.5Hz),8.70(1H,d,J=4.9Hz),9.85(2H,bs).

### Synthesis Example 87: Production of (2S)-2-(8-2-picolylamino methylquinoline-5-carbonyl) amino-5-(5,6,7,8-tetrahydro quinolin-8-yl) aminovalerate (1'S)-1'-(1-naphthyl) ethylamide [Compound No. 102]

### Synthesis Example 87-1: Synthesis of 5-iodine-8-methylquinoline (Compound II-1)

In 50 ml of concentrated sulfuric acid, 5 . 002 g of commercially available 8-methylquinoline and 5.446 g of silver sulfate were dissolved. Then, 6 ml of water was added to the solution and the whole was heated at 80°C. In this mixture, 9.753 g of iodine was added in several times and the whole was stirred for 5 hours. Then, 0.5446 g of silver sulfate was added and the whole was stirred for 0.5 hours. Subsequently, 0.9753 g of iodine was added and the whole was stirred for 1 hour. The reaction solution was cooled to roomtemperature and then diluted with water, followedby removing an excess amount of iodine with sodium sulfite. A solid material was separated by filtration through a glass filter and adjusted to strong alkalinity with a sodium hydroxide aqueous solution. The solid material thus generated was removed by a glass filter, and then a water layer was extracted with chloroform. After drying with anhydrous magnesium sulfate, the solvent was distilled off. The residue was purified bymeans of silica gel column chromatography (210 g, 30% chloroform/35% benzene/35% hexane), consequently 8.2066 g of the above-mentioned compound was obtained as a light-yellow solid product.
MS(FAB,Pos.):m/z=270[M+1]⁺
¹H-NMR(500MHz,CDCl₃) : δ=2.78 (3H,s), 7.30 (1H,d,J=7.6Hz), 7.48 (1H,d d,J=8.5,4.2Hz), 8.01 (1H,d,J=7.6Hz), 8.38 (1H,dd,J=8.5,1.7Hz), 8.9 1 (1H,dd,J=4.2,1.7Hz)

### Synthesis Example 87-2: Synthesis of 8-methylquinoline-5-carboxlic acid (Compound II-2)

Under nitrogen atmosphere, 4 g of the compound obtained in Synthesis Example 87-1 was dissolved in 100 ml of diethylether and the whole was cooled to -55°C. In this solution, 21.16 ml of an n-butyllithium/15% hexane solution was dropped at -50°C or less and the whole was stirred for 20 minutes. It was heated to room temperature, and then water was added and a low polarity material was extracted with chloroform. Then, 1 mol/l hydrochloric acid was added to a water layer, causing acidic precipitation. Subsequently, a solid material was collected by filtration and washed with dilute hydrochloric acid, followed by drying under vacuum. Consequently, 1.8869 g of the above-mentioned compound was obtained as a white solid product.
MS(FAB,Pos.) :m/z=188[M+1]⁺
¹H-NMR(500MHz,CDCl₃) :δ=2.78(3H,d,J=0.6Hz),7.66(1H,dd,J=8.7,4Hz ),7.71(1H,dd,J=7.5,0.6Hz),8.18(1H,d,7.5Hz),8.99(1H,dd,J=4.1,1 .8Hz),9.34(1H,dd,J=8.7,1.8Hz),13.24(1H,bs).

### Synthesis Example 87-3: Synthesis of methyl 8-methylquinoline-5-carbonate (Compound III-3)

1 g of the compound obtained in Synthesis Example 87-2 was dissolved in 25 ml of methanol and stirred for 15 hours while using hydrochloric acid gas, and then the solvent was distilled off. The resulting residue was dissolved in water and then a 1 mol/l sodium hydroxide aqueous solution was added to obtain a precipitated material. The precipitated materials were collected by filtration, and furthermore a filtrate was extracted with chloroform and dried with anhydrous sodium sulfate, followed by distilling the solvent off to thereby obtain the solid materials. Those solid materials were collected and purified together by means of silica gel column chromatography (20 g, chloroform), and 0.7337 g of the above-mentioned compound was obtained as a white solid product.
MS(FAB,Pos.):m/z=202[M+1]⁺
¹H-NMR(500MHz,CDCl₃) : δ=2.87 (3H,s), 3.99 (3H,s), 7.52 (1H,dd,J=8.8, 3.9Hz), 7.60 (1H,d,J=7.6Hz), 8.20 (1H,d,J=7.6Hz), 8.98 (1H,dd,J=3.9 , 1.7Hz), 9.39 (1H,dd,J=8.8,1.7Hz).

### Synthesis Example 87-4: Synthesis of methyl 8-(N-Boc-N-2-picolyl amino) methylquinoline-5-carbonate (Compound VI-11)

In 15 ml of carbon tetrachloride, 0.726 g of the compound obtained in Synthesis Example 87-3 was dissolved. 0.676 g of N-bromosuccinimide and 0.059 g of azobisisobutyronitrile were added thereto and the whole was stirred for 3 hours at 70°C. After terminating the reaction, a solidproduct in the solution was removed by filtration and washed with a 1 mol/l sodium hydroxide solution and a saturated salt solution. After drying with anhydrous sodium sulfate, the solvent was distilled off. It was dissolved in 10 ml of DMF, and then 1.173 g of 2-picolylamine and 0. 51 g of potassium carbonate were added to the solution and the whole was stirred for 17 hours at room temperature. On completion of the reaction, the solvent was distilled off and the residue was then dissolved in chloroform, followed by washing with water and drying with anhydrous magnesium sulfate. The solvent was distilled off. Subsequently, the residue was dissolved in 10 ml of DMF, and then 1.098 g of triethylamine and 2.49 ml of di-t-butyldicarbonate were added to the solution and the whole was stirred for 1 hour at room temperature. On completion of the reaction, the solvent was distilled off, and the residue was then dissolved in chloroform and washed with a saturated sodium bicarbonate aqueous solution. After drying with anhydrous magnesium sulfate, the solvent was distilled off and the residue was purified by means of silica gel column chromatography (60 g, ethyl acetate/hexane = 2/3), consequently 0.7372 g of the above-mentioned compound was obtained as a light-red solid product.
MS(FAB,Pos.):m/z=408[M+1]⁺
¹H-NMR (500MHz,CDCl₃) : δ =1.40 and 1.43 (9H,2s), 4.00 and 4.01 (3H,2s), 4.70 and 4.77 (2H,s), 5.22 and 5.31 (2H,2s), 7.15 (1H,t,J=6.1Hz), 7.24 and 7.36 (1H,d,J=7.8Hz), 7.47-7.54 (2H,m), 7.60 and 7.73 (1H,d,J= 7.6Hz),7.65 and 7.66 (1H,t,J=7.6Hz),8.29 and 8.50 (1H,d,J=4.2Hz), 8.88(1H, d, J=3.9Hz), 9.35 and 9.38(1H, d, J=8.5Hz).

### Synthesis Example 87-5: Synthesis of 8-(N-Boc-N-2-picolylamino methyl) quinoline-5-carboxylic acid (Compound VII-15)

In 7 ml of THF and 7 ml of methanol, 0.7308 g of the compound obtained in Synthesis Example 87-5 were dissolved, and 7 ml of a 1 mol/l sodium hydroxide aqueous solution was added to the solution and the whole was stirred for 19 hours at room temperature. On completion of the reaction, the solvent was distilled off. The residue was dissolved in distilled water and precipitated with acidity of hydrochloric acid. Then, a solid product was collected by filtration and dried under reduced pressure, consequently 0.619 g of the above-mentioned compound was obtained as a light-pink solid product.
MS(FAB,Pos.):m/z=394[M+1]⁺
¹H-NMR(500MHz,CDCl₃):δ=1.43 and 1.53 (9H,2s),4.80 and 4.91 (2H,s), 5.14 and 5.25(2H,2s),7.22-7.36(2H,m),7.47 and 7.49 (1H,d,J=6.3Hz), 7.56 and 7.61(1H,d,J=7.8Hz),7.83 and 7.85(1H,t,J=7.8Hz),8.06 and 8.08 (1H,d,J=7.6Hz), 8.58 (1H,d,J=4.6Hz), 8.67 (1H,d,J=3.9Hz), 9.02 and 9.10 (1H,d,J=8.1Hz).

### Synthesis Example 87-6: Synthesis of N^{α}(8-(N-Boc-N-2-picolylamino methyl) quinoline-5-carbonyl)-N-Boc-L-ornithine (1'S)-1'-(1-naphthyl) ethylamide (Compound XI-17)

The compound obtained in Synthesis Example 68-1 was dissolved in 10 ml of DMF, and then 1 ml of diethylamine was added to the solution and the whole was stirred for 3.5 hours at room temperature. On completion of the reaction, the solvent was distilled off. The residue was dissolved in 10 ml of DMF, and then 0.237 g of WSCI hydrochloride, 0.166 g of HOBt, and 0.356 g of the compound obtained in Synthesis Example 87-5 were added to the solution and the whole was stirred for 19 hours at room temperature. On completion of the reaction, the solvent was distilled off, and then it was dissolved in chloroform and washed with a saturated sodium bicarbonate aqueous solution and a saturated salt solution, followed by drying with anhydrous magnesium sulfate. The solvent was distilled off and the residue was then purified by means of silica gel column chromatography (45 g, chloroform/methanol = 30/1), consequently 0.660 g of the above-mentioned compound was obtained as a light-pink solid product.
MS(FAB,Pos.):m/z=761[M+1]⁺

### Synthesis Example 87-7: Synthesis of (2S)-2-(8-2-picolylamino methylquinoline-5-carbonyl) amino-5-(5,6,7,8-tetrahydro quinolin-8-yl) aminovalerate (1'S)-1'-(1-naphthyl) ethylamide [Compound No.102]

In 13 ml of methanol, 0.651 g of the compound obtained in Synthesis Example 87-6 was dissolved. Then, 13 ml of 4 mol/l hydrochloric acid/dioxane was added to the solution and the whole was stirred for 2 hours at room temperature. On completion of the reaction, the solvent was distilled off. After dissolving the mixture in methanol again, it was neutralized using Amberlite IRA-410. The solvent was distilled off and the residue was dissolved in methanol, and then 0.151 g of 5,6,7,8-tetrahydroquinolin-8-one, 0.108 g of sodium cyanoborohydride, and 10 drops of acetic acid were added to the solution and the whole was stirred for 16 hours at room temperature. On completion of the reaction, the solvent was distilled off and the residue was purified bymeans of silica gel column chromatography (chloroform/methanol/water = 7/3/0.5), followed by treatment with hydrochloric acid. Consequently, 0.1512 g of the above-mentioned compound was obtained as a light-yellow solid product.
MS(FAB,Pos.):m/z=692[M+1]⁺
¹H-NMR(500MHz,DMSO-d₆) : δ=1.55(3H,d,J=7.1Hz), 1.64-2.05(7H,m),2. 28-2.36(1H,m),2.76-2.84(2H,m),2.90-3.04(1H,m),4.36-4.50(1H,m) ,4.43(2H,s),4.60-4.70(1H,m),4.86(2H,s),5.77(1H,quint,J=7.1Hz) ,7.37-7.40(1H,m),7.46-7.63(6H,m),7.68-7.73(2H,m),7.92-7.97(2H ,m), 8.03 (1H,d,J=7.6Hz), 8.14 (1H,d,J=8.5Hz), 8.47 (1H,d,J=4.4Hz), 8.66 (1H,d,J=4.9Hz), 8.77 (1H,dt,J=8.5,1.5Hz), 8.89-8.94 (2H,m), 9. 04 (1H,dd,J=4.2,1.5Hz).

### Synthesis Example 88: Production of N^{α}-(4- ((imidazol-2-ylmethyl) amino) methyl) naphthalene-1-carbonyl)-L-arginine N-methyl-1-naphthylmethylamide [Compound No. 103]

### Synthesis Example 88-1: Synthesis of N^{α}-Fmoc-N^{G}-Pmc-L-arginine N-methyl-1-naphthylmethylamide (Compound XXVII-11)

In 20 ml of DMF, 1.0 g of commercially available N^{α}-Fmoc-N^{G}-Pmc-arginine was dissolved and 0.43 g of WSCI hydrochloride, 0.31 g of HOBt, and 0.34 g of N-methyl-1-naphthylmethylamine were stirred for 1 day at room temperature. On completion of the reaction, the solvent was distilled off and then 1 mol/l hydrochloric acid was added to the residue, followed by separating an insoluble fractionby filtration using a glass filter. Furthermore, the resulting solid product was washed with a 1 mol/l sodium hydroxide aqueous solution and water in order, consequently 1.03 g of the target product was obtained as a white solid product.
MS(FAB,Pos.):m/z=816[M+1]⁺
¹H-NMR(500MHz,CDCl₃): δ=1.22 and 1.23(2s,6H),1.41-1.79(6H,m), 2.00 and 2.01(2s,3H),2.45 and 2.46(2s,6H),2.50-2.60(2H,m), 2.95-3.10(4H,m),3.32(3H,s),4.03-20(3H,m), 7.24-8.06(15H,m).

### Synthesis Example 88-2: Synthesis of N^{α}-(4-((N-Boc-N-(imidazol -2-ylmethyl) aminomethyl) naphthalene-1-carbonyl)-N^{G}- Pmc-L-arginine N-methyl-1-naphthylmethylamide (Compound XXIX-19)

In 8.5 ml of DMF, 428 mg of the compound obtained in Synthesis Example 88-1 was dissolved and then 0.85 ml of diethylamine was added. After the reaction was continued for 1 hour, the reaction solution was concentrated. Then, the resulting residue was dissolved in 6.2 ml of DMF, and 200 mg of the compound obtained in Synthesis Example 17-4, 151 mg of WSCI, and 96 mg of DMAP were added. After the reaction was continued for 15.5 hours at room temperature, the reaction solution was concentrated and 1 mol/l hydrochloric acid was added, followed by extracting with chloroform. An organic layer was washed with a saturated salt solution and was then dried with anhydrous sodium sulfate and concentrated. The residue thus obtained was purified by means of silica gel column chromatography (15 g, chloroform/methanol = 10/1), consequently 530 mg of the above-mentioned compound was obtained as a colorless viscous liquid product.
MS(FAB,Pos.) :m/z=957[M+1]⁺

### Synthesis Example 88-3: Synthesis of N^{α}-(4-((imidazol-2-ylmethyl) amino) methyl) naphthalene-1-carbonyl) L-arginine N-methyl-1-naphthylmethylamide [Compound No. 103]

In 5.3 ml of chloroform, 530 mg of the compound obtained in Synthesis Example 88-2 was dissolved. Then, 5.3 ml of trifluoroacetic acid was added to the solution, followedby reaction for 15. 5 hours. After that, the reaction solution was concentrated and azeotropically distilled with chloroform. The resulting residue was purified by means of silica gel column chromatography (15g, chloroform/methanol/water=7/3/0.5), followed by treatment with hydrochloric acid. Consequently, 108 mg of the above-mentioned compound was obtained as hydrochloride of a white solid product.
MS(FAB,Pos.):m/z=687[M+1]⁺
¹H-NMR(500MHz,DMSO-d₆) : δ=1.50-1.90 (4H,m), 3.14(3H,s), 3.10-3.25 (2H,m), 4.94-5.50 (6H,m), 7.20-8.20 (16H,m), 8.29-8.33 (1H,m), 8.92-9 .00 (1H,m).

### Synthesis Example 89: Production of (2S)-2-(4-(2-pyridyl) aminomethylnaphthalene-1-carbonyl) anmino-5-(5,6,7,8-tetra hydroquinolin-8-yl) aminovalerate-1-naphthylmethylamide [Compound No. 104]

### Synthesis Example 89-1: Synthesis of 4-(2-pyridyl) aminomethyl naphthalene-1-carboxylic acid (Compound VII-16)

In 33 ml of DMF, 1.6728 g of the compound obtained in Synthesis Example 17-2 was dissolved. Then, 1.66 g of potassium carbonate and 0.68 g of 2-aminopyridine were added to the solution and the whole was stirred for 15 hours at room temperature. On completion of the reaction, the solvent was distilled off and then it was dissolved in chloroform. After washing with a 1 mol/l hydrochloric acid and a saturated salt solution, an organic layer was dried with anhydrous sodium sulfate and the solvent was distilled off. The resulting residue was dissolved in 44 ml of DMF, and then 1.58 ml of triethylamine and 2.60 ml of di-t-butyldicarbonate were added to the solution and the whole was stirred for 6 hours at room temperature. On completion of the reaction, the solvent was distilled off. Subsequently, it was dissolved in ethyl acetate and washed with 0.5 mol/l hydrochloric acid and a saturated salt solution. After drying with anhydrous sodium sulfate, the solvent was distilled off. The resulting residue was dissolved in 6 ml of methanol and then 6 ml of a 1 mol/l sodium hydroxide aqueous solution was added. After the reaction was continued for 1 day, the reaction solution was concentrated and dissolved in methanol again, followed by neutralization with an ion exchange resin CG50. The resin was separated by filtration. Then, the residue obtained by concentrating the filtrate was purified by means of silica gel column chromatography (15 g, chloroform/methanol = 5/1), consequently 159 mg of the above-mentioned compound was obtained as a colorless viscous liquid product.
MS(FAB,Pos.):m/z=379[M+1]⁺

### Synthesis Example 89-2: Synthesis of (2S)-2-(4-(2-pyridyl) aminomethylnaphthalene-1-carbonyl) amino-5-(5,6,7,8-tetrahydro quinolin-8-yl) aminovalerate 1-naphthylmethylamide [Compound No. 104]

In 2.4 ml of DMF, 240 g of the compound obtained in Synthesis Example 23-1 was dissolved. Then, 0.24 ml of diethylamine was added to the solution and the whole was stirred for 2 hours at room temperature. On completion of the reaction, the solvent was distilled off and dried under reduced pressure, followed by dissolving the resultant in 1.6 ml of DMF. 159 mg of the compound obtained in Synthesis Example 89-1, 121 mg of WSCI hydrochloride, and 77 mg of DMAP were added thereto and reacted for 1 day at room temperature. The reaction solution was concentrated, and then the resulting residue was added with 1 mol/l hydrochloric acid and extracted with chloroform. An organic layer was washed with a saturated salt solution. Then, it was dried with anhydrous sodium sulfate and concentrated. The residue thus obtained was roughly purified by means of silica gel column chromatography (20 g, chloroform/methanol = 20/1). The resultant was dissolved in 4.5 ml of methanol and then 4.5 ml of a 4 mol/l hydrochloric acid/dioxane solution was added. After the reaction was continued for 1 hour, the reaction solution was concentrated and the resulting residue was dissolved in 2.2 ml of methanol, followed by the addition of 0.12 ml of triethylamine, 40 mg of 5,6,7,8-tetrahydroquinolin-8-one, 0.55 ml of acetic acid, and 39 mg of sodium cyanoborohydride. After the reaction was continued for 3 days, the reaction solution was concentrated. The resulting residue was purified by means of silica gel column chromatography (80 g, chloroform/methanol = 20/1), followed by treating the target product thus obtained with hydrochloric acid. Consequently, 20.7 mg of hydrochloride of the above-mentioned compound was obtained as a white solid product.
MS(FAB,Pos.):m/z=663[M+1]⁺
¹H-NMR(500MHz,DMSO-d₆) : δ=1.70-2.02 (7H,m),2.28-2.34 (1H,m),2.76 -2.83(2H,m),2.93-3.03(1H,m),3.04-3.16(1H,m),4.57-4.64(1H,m),4 .82 (2H,t,J=5.5Hz), 5.15 (2H,d,J=4.9Hz), 6.93 (1H,t,J=6.7Hz), 7.20 (1H,d,J=8.8Hz), 7.38 (1H,dd,J=4.7,2.9Hz), 7.48 (1H,dd,J=7.1,8.1Hz) , 7.52-7.70 (8H,m), 7.87 (1H,d,J=7.6Hz), 7.92-8.02 (3H,m), 8.10 (1H,d ,J=6.8Hz),8.17(1H,d,J=8.3Hz),9.13(2H,brs)

### Synthesis Example 90: Production of (2S)-2-(4-(N-2-picolylamino methyl) naphthalene-1-carbonyl) amino-5-((8S)-5,6,7,8-tetra hydroquinolin-8-yl) aminovalerate 1-naphthylmethylamide [Compound No. 105]

### Synthesis Example 90-1: Synthesis of (S)-8-amino-5,6,7,8-tetra hydroquinoline

In 160 ml of benzene, 16.586 g of 5,6,7,8-tetrahydroquinoline-8-ol synthesized by the method described in Journal of Medicinal Chemistry, vol. 20, No. 10, pp 1351-1354 (1997) was dissolved. Then, 31.7 ml of phosphorus tribromide was dropped at 0°C. The whole was returned to room temperature and stirred overnight, followed by adjusting pH to 10 through the addition of a sodium hydroxide aqueous solution under ice-cold condition. The extraction was carried out with chloroform and then an organic layer was washed with a saturated salt solution, followed by drying with anhydrous sodium sulfate. The solvent was distilled off and the residue was dissolved in 300 ml of DMF. 14.5 g of potassium phthalimide was added thereto and the whole was stirred for 6.5 hours at room temperature. On completion of the reaction, the solvent was distilled off. The resultant was dissolved in chloroform and was then washed with distilled water and a saturated salt solution, followed by drying with anhydrous sodium sulfate. The solvent was distilled off and the residue was recrystallized with methanol, and 9.884 g of a light-brown solid product was obtained. 2.98 g of the product was dissolved in 29 ml of methanol and 2. 55 ml of hydrazine monohydrate was added to the solution and the whole was stirred for 2 hours at room temperature. On completion of the reaction, the solvent was distilled off and water was added. Subsequently, it was extracted with chloroform and dried with anhydrous sodium sulfate, followed by distilling the solvent off. The residue was dissolved in 8 ml of methanol and then 1.58 g of D-tartaric acid was added. It was reprecipitated by the addition of 160 ml of chloroform, consequently a white solid product was obtained as (RS)-8-amino-5,6,7,8-tetrahydroquinoline-D-tartrate. By recrystallizing 1 g thereof from methanol three times, 126.8 mg of the above-mentioned compound was obtained as a white needle-shaped crystal.
[α]₂₅=+26.5°

### Synthesis Example 90-2: Synthesis of (2S)-2-(4-(N-Boc-N-2-picolyl amino) methylnaphthalene-1-carbonyl) amino-5-((8S)-5,6,7,8-tetrahydroquinolin-8-yl) aminovalerate 1-naphthylmethylamide (Compound XIII-16)

In 0.7 ml of methanol, 34 mg of the compound obtained in Synthesis Example 53-2 was dissolved. Then, 24 mg of the compound obtained in Synthesis Example 90-1, 0.15 ml of acetic acid, and 10 mg of sodium cyanoborohydride were added to the solution and the whole was stirred for 3 days at room temperature. On completion of the reaction, the solvent was distilled off and the residue was purified by means of silica gel column chromatography (1.5 g, chloroform/methanol = 10/1), consequently 20 mg of the above-mentioned compound was obtained as a light-yellow solid product.
MS(FAB,Pos.):m/z=777[M+1]⁺
¹H-NMR(500MHz,CDCl₃) : δ=1.45 and 1.49 (9H,2s), 1.70-2.12 (6H,m), 2.14-2.25 (1H,m), 2.33-2.43 (1H,m), 2.70-2.81 (2H,m), 3.17-3.24 (1H, m), 3.46-3.53 (1H,m), 4.19-4.57 (3H,m), 4.77-5.08 (5H,m), 7.09-7.18 (3H,m), 7.38-7.63 (10H,m), 7.79 (1H,d,J=8.3Hz), 7.86 (1H,d,J=8.3Hz), 7.99 (1H,d,J=8.3Hz), 8.08-8.19 (2H,m), 8.32 (1H,brd,J=12.3Hz), 8.51 (1H,s).

### Synthesis Example 90-3: Synthesis of (2S)-2-(4-(N-2-picolyl aminomethyl) naphthalene-1-carbonyl) amino-5-((8S)-5,6,7,8-tetrahydroquinolin-8-yl) aminovalerate 1-naphthylmethylamide [Compound No. 105]

In 0.3 ml of methanol, 14.2 mg of the compound obtained in Synthesis Example 90-2 was dissolved. Then, 0.3 ml of a 4 mol/l hydrochloric acid/dioxane solution was added to the solution and the whole was reacted for 3.5 hours. After that, the reaction solution was concentrated. The resulting residue was purified by means of silica gel column chromatography (0.5 g, chloroform/methanol/water=7/3/0.5), followed by treating with hydrochloric acid. Consequently, 8.1 mg of hydrochloride of the above-mentioned compound was obtained as a white solid product.
MS(FAB,Pos.) :m/z=677[M+1]⁺
¹H-NMR(500MHz,DMSO-d₆):δ=1.60-1.95(6H,m), 1.95-2.03(1H,m),2.28-2.37(1H,m),2.81(2H,t,J=6.1Hz),2.96-3.06(1H,m),3.07-3.16(1H,m) ,4.60-4.67(1H,m),4.76-4.88(5H,m),7.39(1H,dd,J=7.8,4.9Hz),7.47 - 7.73(9H,m),7.81(1H,d,J=7.6Hz),7.88(1H,d,J=8.1Hz),7.93(1H,dt, J=1.8,5.9Hz),7.95(1H,d,J=8.5Hz),7.97(1H,d,J=9.5Hz),8.11(1H,d, J=9.5Hz),8.26-8.33(3H,m),8.49(1H,d,J=3.7H),8.71(1H,d,J=4.6Hz) ,8.74(1H,t,J=5.6Hz),8.87(1H,d,J=7.8Hz),9.14(2H,br),9.84(2H,,b r)

### Synthesis Example 91: Production of (2S)-2-(4-((N-imidazol-2-yl methyl) aminomethyl) benzoylamino-5-(5,6,7,8-tetrahydroquinolin -8-yl) aminovalerate (1'S)-1'-(1-naphthyl) ethylamide [Compound No. 106]

### Synthesis Example 91-1: Synthesis of N-(4-(N-Boc-(N-imidazol-2-ylmethyl) amino) methyl)-N-Boc-L-ornithine (1'S)-1'-(1-naphthyl) ethylamide (Compound XI-18)

In 10 ml of DMF, 500 mg of the compound obtained in Synthesis Example 68-1 was dissolved. Then, 1 ml of diethylamine was added to the solution and the whole was stirred for 0.5 hours at room temperature. On completion of the reaction, the solvent was distilled off. The residue was dissolved again in 15 ml of DMF, and then 312.4 mg of the compound obtained in Synthesis Example 81-2, 246 mg of WSCI hydrochloride, and 174 mg of HOBt were added to the solution and the whole was stirred for 15 hours at room temperature. On completion of the reaction, the solvent was distilled off and the residue was dissolved in chloroform, followed by washing with a saturated sodium bicarbonate aqueous solution and a saturated salt solution. After drying with anhydrous sodium sulfate, the solvent was distilled off. The residue was purified by means of silica gel column chromatography (15 g, ethyl acetate), consequently 490.4 mg of the above-mentioned compound was obtained as a colorless viscous liquid product.
MS(FAB,Pos.):m/z=699[M+1]⁺
¹H-NMR(500MHz,CDCl₃):δ=1.32(9H,s),1.45(9H,s),1.40-1.72(6H,m),1 .83-1.91(1H,m),2.95-3.06(1H,m),3.34-3.42(1H,m),4.39(2H,s),4.4 9(2H,s),5.88-5.93(1H,m),7.23(1H,d,J=7.5Hz),7.42-7.56(4H,m),7. 75-7.82(3H,m),7.87(1H,d,J=7.9Hz),8.02(2H,s),8.08(1H,d,J=8.4Hz ).

### Synthesis Example 91-2: Synthesis of (2S)-2-(4-((N-imidazol-2-yl methyl) aminomethyl) benzolylamino-5-(5,6,7,8-tetrahydro quinolin-8-yl) aminovalerate (1'S)-1'-(1-naphthyl) ethylamide [Compound No. 106]

In 4.8 ml of methanol, 478 mg of the compound obtained in Synthesis Example 91-1 was dissolved. Then, 4.8 ml of a 4 mol/l hydrochloric acid/dioxane was added to the solution and the whole was stirred for 2 hours at room temperature. The reaction solution was concentrated and then neutralized with an ion exchange resin, followed by dissolving the residue in 10 ml of methanol. 151 mg of 5,6,7,8-tetrahydroquinolin-8-one, 2.5 ml of acetic acid, and 129 mg of sodium cyanoborohydride were added thereto and reacted for 3 days at room temperature. The solvent was distilled off and the residue was then purified by means of silica gel column chromatography (chloroform/methanol = 5/1), followed by treating with hydrochloric acid. Consequently, 233.5 mg of hydrochloride of the above-mentioned compound was obtained as a light-yellow solid product.
MS(FAB,Pos.):m/z=630[M+1]⁺
¹H-NMR(500MHz,DMSO-d₆):δ=1.51 (3H,d,J=6.8Hz),1.70-1.91 (6H,m),1. 91-2.01(1H,m),2.22-2.34(1H,m),2.76-2.84(2H,m),2.88-3.00(1H,m) ,3.02-3.14(1H,m),4.35-4.45(1H,m),4.42(2H,s),4.45-4.64(1H,m),4 .59(2H,s),5.71(1H,quint.,J=6.8Hz),7.35-7.40(1H,m),7.46-7.58(4 H,m),7.67(1H,d,J=7.8Hz),7.71(2H,d,J=8.3Hz),7.77(2H,s),7.82(1H ,d,J=8.3Hz),7.94(1H,d,J=8.1Hz),7.99(2H,d,J=8.3Hz),8.10(1H,d,J =8.5Hz),8.42-8.48(1H,m),8.62-8.67(1H,m),8.83-8.92(1H,m),9.12 (2H,brs),10.70(2H,br).

### Synthesis Example 92: Production of (2S)-2-(4-((N-1-methyl imidazol-2-ylmethyl) aminomethyl) benzoylamino-5-(5,6,7,8-tetrahydroquinolin-8-yl) aminovalerate (1'S)-1'-(1-naphthyl) ethylamide [Compound No. 107]

### Synthesis Example 92-1: Synthesis of N-(4-((N-1-methylimidazol -2-ylmethyl) aminomethyl)-N-Boc-L-ornithine (1'S)-1'-(1-naphthyl) ethylamide (Compound XI-19)

In 12.9 ml of DMF, 646.7 mg of the compound obtained in Synthesis Example 68-1 was dissolved. Then, 1.29 ml of diethylamine was added to the solution and the whole was stirred for 1 hour at room temperature. The reaction solution was concentrated under reduced pressure and dried under vacuum. The resulting crude product was dissolved in 12.5 ml of DMF again. Subsequently, 367.5 mg of the compound obtained in Synthesis Example 81-2, 306.0 mg of WSCI hydrochloride, and 215.7 mg of HOBt were added to the solution and the whole was stirred for 1 day at room temperature. On completion of the reaction, the solution was concentrated under reduced pressure. A saturated sodium bicarbonate solution was added to the residue and the whole was subjected to separatory extraction with chloroform, followed by drying with anhydrous sodium sulfate and concentrating under reduced pressure. The residue was purified by means of silica gel column chromatography (30 g, chloroform/methanol = 20/1), consequently 758.6 mg of the above-mentioned compound was obtained as a white solid product.
MS(FAB,Pos.):m/z=713[M+1]⁺
¹H-NMR(500MHz,DMSO-d₆) : δ=1.32 (9H,s), 1.45 (9H,s), 1.40-1.60 (2H,m) , 1.66 (3H,d,J=6.8Hz), 1.62-1.72 (1H,m), 1.82-1.91 (1H,m), 2.93-3.01 (2H,m), 3.61 (3H,s), 4.46 (2H,s), 4.49-4.65 (3H,m), 4.91 (1H,m), 5.91 (1H,quint.,J=6.8Hz) ,6.80 (1H ,s) ,6.93 (1H,s) ,7.18-7.29 (4H ,m) ,7.43 - 7.57 (4H,m), 7.69-7.76 (2H,m), 7.78 (1H,d,J=7.3Hz), 7.86 (1H,d,J=8. 1Hz),8.08(1H,d,J=8.3Hz).

### Synthesis Example 92-2: Synthesis of (2S)-2-(4-((N-1-methyl imidazol-2-ylmethyl) aminomethyl) benzoylamino-5-(5,6,7,8-tetrahydroquinolin-8-yl) aminovalerate (1'S)-1'-(1-naphthyl) ethylamide [Compound No. 107]

In 15.2 ml of methanol, 758.6 mg of the compound obtained in Synthesis Example 92-1 was dissolved, and 15.2 ml of a 4 mol/l hydrochloric acid/dioxane solution was added to the solution and the whole was stirred for 1 hour at room temperature. On completion of the reaction, a crude product obtained by concentrating the solution under reduced pressure and drying under vacuum was dissolved in 16.4 ml of methanol again. Subsequently, 187.9 mg of 5,6,7,8-tetrahydroquinolin-8-one, 133.7 mg of sodium cyanoborohydride, and 0.445 ml of triethylamine were added to the solution at room temperature and the pH thereof was adjusted to 4 to 5 with acetic acid, followed by stirring for 3 days. On completion of the reaction, the solution was concentrated under reduced pressure. The residue was purified by means of silica gel column chromatography (20g,chloroform/methanol/water=7/3/0.5) and was then treated with hydrochloric acid, consequently 357.6 mg of hydrochloride of the above-mentioned compound was obtained as a white solid product.
MS(FAB,Pos.):m/z=644[M+1]⁺
¹H-NMR(500MHz,DMSO-d₆) : δ=1.51(3H,d,J=6.8Hz), 1.70-2.01 (7H,m), 2. 22-2.33 (1H,m), 2.75-2.82 (2H,m), 2.87-2.29 (1H,m), 3.02-3.14 (1H,m) , 3.98 (3H,s), 4.35-4.45 (1H,m), 4.42 (2H,s), 4.53-4.68 (1H,m), 4.61 (2 H,s), 5.71 (1H,quint.,J=6.8Hz), 7.35-7.40 (1H,m), 7.46-7.58 (4H,m), 7.67 (1H,d,J=7.8Hz), 7.73 (2H,d,J=8.1Hz), 7.77 (2H,s), 7.81 (1H,d,J= 8.1Hz), 7.94 (1H,dd,J=7.8,1.0Hz), 7.98 (2H,d,J=8.3Hz), 8.11 (1H,d,J =8.3Hz), 8.42-8.47 (1H,m), 8.67 (1H,d,J=7.6Hz), 8.88 (1H,d,J=5.6Hz) ,9.20(2H,brs),10.29(1H,br),10.79(1H,br).

### Synthesis Example 93: Production of (2S)-2-(4-(2-picolylamino methyl) benzoyl-5-(imidazol-2-yl) aminovalerate (1'S)-1'-(1-naphthyl) ethylamide [Compound No. 108]

### Synthesis Example 93-1: Synthesis of N^{α}-(4-(N-Boc-N-2-picolyl aminomethyl) benzoyl)-O-methyl-L-glutamate(1'S)-1'-(1-naphthyl) ethylamide (Compound XXXVII-2)

In 30 ml of anhydrous methanol, 3.0726 g of the compound obtained in Synthesis Example 55-1 was dissolved. In this solution, 15 ml of a 4 mol/l hydrochloric acid/dioxane was added and the whole was stirred for 4 hours at room temperature. On completion of the reaction, the solvent was distilled off under reduced pressure. The resulting residue was dissolved in 30 ml of chloroform together with 2.2554 g of the compound obtained in Synthesis Example 1-2 and 1.1681 gof DMAP. Inthis solution, a chloroform (10 ml) solution of 2.0902 g of DCC was gradually added and the whole was stirred for 16 hours at room temperature. A precipitate was filtrated and then a 1 mol/l hydrochloric acid was added to the filtrate to make the filtrate acidic, followed by extracting with chloroform. After being washed with a saturated sodium hydrogencarbonate aqueous solution and a saturated salt solution, the resultant was dried withmagnesiumsulfate. The solvent was distilled off under reduced pressure and then the residue was purified by means of silica gel column chromatography (157.8 g, hexane/ethyl acetate = 1/2), consequently 3.4691 g of the above-mentioned compound was obtained as a white solid product.
MS(FAB,Pos.):m/z=639[M+1]⁺
¹H-NMR(500MHz,CDCl₃):δ=1.45(9H,brs),1.66(3H,d,J=6.8Hz),2.02-2. 08(1H,m),2.10-2.18(1H,m),2.31-2.37(1H,m),2.55-2.60(1H,m),3.62 (3H,s),4.49(2H,brs),4.60(2H,m),4.63-4.67(1H,m),5.93(1H,quint, J=6.8Hz),6.90(1H,d,J=8.3Hz),7.17-7.19(1H,m),7.31(1H,d,J=7.3Hz ),7.35(1H,d,J=7.6Hz),7.46-7.56(4H,m),7.64-7.67(1H,dt,J=1.7,6. 0Hz),7.76(2H,d,J=8.3Hz),7.80(1H,d,J=8.3Hz).7.88(1H,d,J=8.1Hz) ,8.09(1H,d,J=8.6Hz),8.53(1H,d,J=4.2Hz).

### Synthesis Example 93-2: Synthesis of (2S) -2- (4- (N-Boc-N-2-picolyl aminomethyl) benzoylamino)-5-hydroxyvalerate(1'S)-1'-(1-naphthyl) ethylamide (Compound XXXVIII-1)

In a mixture solvent of 30 ml of THF and 40 ml of ethanol, 3.4477 g of the compound obtained in Synthesis Example 93-1, 821.4 mg of sodium borohydride, and 1.2154 g of calcium chloride were dissolved and the whole was stirred for 2 hours at room temperature. On completion of the reaction, a 1 mol/1 citric acid aqueous solution was added to the solution. Then, the solution was extracted with ethyl acetate and washed with a saturated sodium hydrogencarbonate aqueous solution, and was then dried with magnesium sulfate, followed by distilling the solvent off under reduced pressure. The resulting residue was purified by means of silica gel column chromatography (170 g, ethyl acetate), consequently 2.6203 g of the above-mentioned compound was obtained as a white solid product.
MS(FAB,Pos.):m/z=611[M+1]⁺
¹H-NMR(500MHz,CDCl₃) : δ=1.45 (9H,br), 1.52-1.61 (2H,m), 1.65 (3H,d,J =7.3Hz), 1.69-1.87 (1H,m), 1.88-1.95 (1H,m), 3.55-3.59 (1H,m), 3.63-3.67 (1H,m), 4.49 (2H,br), 4.59 (2H,br), 4.78 (1H,q,J=7.1Hz), 5.93 (1H ,quint.,J=7.3Hz), 6.95 (1H,d,J=8.1Hz), 7.17-7.19 (1H,m), 7.30 (1H,d ,J=7.8Hz), 7.34 (1H,d,J=7.8Hz), 7.45-7.56(4H,m), 7.66 (1H,dt,J=7.6 ,1.7Hz), 7.74-7.76 (2H,m), 7.80 (1H,d,J=8.1Hz), 7.88 (1H,d,J=7.8Hz) , 8.10 (1H,d,J=8.5Hz), 8.53 (1H,d,J=4.2Hz).

### Synthesis Example 93-3: Synthesis of (2S)-2-(4-(N-Boc-N-2-picol ylaminomethyl) benzoyl-5-(imidazol-2-yl) aminovalerate (1'S) -1'-(1-naphthyl) ethylamide (Compound XIII-17)

In 10 ml of chloroform, 0.700 g of the compound obtained in Synthesis Example 93-2 was dissolved. Then, 31.9 mg of tetrabutylammonium chloride, 17.9 mg of 2,2,6,6-tetramethyl-1-piperidyloxide, and 195 mg of N-chlorosuccinimide were added to the solution, and furthermore 10 ml of a 0.5 mol/l sodium hydrogencarbonate aqueous solution and 10 ml of a 0.05 mol/l potassium carbonate aqueous solution were added to the solution and stirred violently for 5 hours at room temperature. On completion of the reaction, liquid separation was performed and a water layer was extracted with chloroform and washed with a saturated salt solution together with an organic layer. The resultant was dried with anhydrous sodium sulfate and then solventwas distilled off. The resulting residue was dissolved in 14ml of methanol, and then 0.17 gof 2-aminoimidazole 0.5 sulfate, and molecular sieves 3A were added, followed by adjusting the pH of the reaction solution to 8 with triethylamine and stirring the solution overnight. Subsequently, pH was adjusted to 6 to 7 with acetic acid and 0.22 g of sodium cyanoborohydride was added to the solution and the whole was stirred for 4 days. An insoluble fraction was filtrated by a glass filter and concentrated under reduced pressure. The residue was purified by means of silica gel column chromatography (70 g, chloroform/methanol = 10/1), consequently a 0.15 g of the above-mentioned compound was obtained as a white solid product.
MS(FAB,Pos.):m/z=676[M+1]⁺
¹H-NMR(500MHz,DMSO-d₆) : δ=1.31 and 1.38(9H,2s),1.51(3H,d,J= 6.8Hz),1.69-1.78(2H,m),1.69-1.78(2H,m),3.15-3.30(2H,m),4.41 (1H,brs),4.50(2H,brs),4.55(2H,brs),5.71(1H,m),6.84(1H,s),7.23 (1H,m),7.28-7.36(4H,brs),7.45(1H,m),7.49-7.55(3H,m),7.76-7.82 (2H,m),7.93(1H,m),8.10(1H,m),8.32(1H,brs),8.53(01H,brs),8.66 (1H,brs).

### Synthesis Example 93-4: Synthesis of (2S)-2-(4-(2-picolylamino methyl) benzoyl-5-(imidazol-2-yl) aminovalerate (1'S)-1'-(1-naphthyl) ethylamide [Compound No. 108]

In 4.0 ml of methanol, 0.18 g (0.27 mmol) of the compound obtained in Synthesis Example 93-3 was dissolved. Then, 4.0 ml of a 4 mol/l hydrochloric acid/dioxane solution was dropped in the solution at room temperature and the solution was stirred for 2 hours without modification. The reaction solution was concentrated and the residue was purified by means of silica gel column chromatography (7g, chloroform/methanol/water= 7/3/0.5), followed by treating with a 1 mol/l hydrochloric acid solution. Consequently, 0.09 g of hydrochloride of the above-mentioned compound was obtained as a light-yellow frothy compound.
MS(FAB,Pos.):m/z=576[M+1]⁺
¹H-NMR(500MHz,DMSO-d₆):δ=1.51(3H,d,m,J=6.8Hz),1.61(1H,m),1.75-1.82(2H,m),3.24(2H,d),4.30(4H,brs),4.57(2H,m),5.71(1H,m),6.93 (2H,s),7.46(2H,m),7.49-7.59(4H,brs),7.65(2H,d,J=8.5Hz),7.80(1 H,d,J=8.1Hz),7.90-7.94(2H,m),8.00(2H,d,J=8.3Hz),8.10(2H,m,J=8 .3Hz),8.61(1H,d,J=7.8Hz),8.66(1H,m),8.86(2H,d,J=7.7Hz),9.91(2 H.brs).

### Synthesis Example 94: Production of (2S)-2-(4-2-picolylamino methyl) benzoyl-5-(pyridin-2-yl) aminovalerate (1'S)-1'-(1-naphthyl) ethylamide [Compound No. 109]

### Synthesis Example 94-1: Synthesis of (2S)-2-(4-(N-Boc-N-2-picolyl aminomethyl) benzoyl-5-(pyridin-2-yl) aminovalerate (1'S)-1'-(1-naphthyl) ethylamide (Compound XIII-18)

In 4 ml of chloroform, 180 mg of the compound obtained in Synthesis Example 93-2 was dissolved. 8.2 mg of tetrabutylammonium chloride, 4.6 mg of 2,2,6,6-tetramethyl-1-piperidiloxide, and 51 mg of N-chlorosuccinimide were added to the solution, and furthermore 4 ml of a 0.5 mol/l sodium hydrogencarbonate aqueous solution and 4 ml of a 0.05 mol/l potassium carbonate aqueous solution were added to the solution and the whole was vigorously stirred for 5 hours at room temperature. On completion of the reaction, the solution was liquidly separated and then a water layer was extracted with chloroform and washed with a saturated salt solution together with an organic layer. The resultant was dried with anhydrous sodium sulfate and the solvent was distilled off. The resulting residue was dissolved in 4 ml of methanol and then 0.03 g of 2-aminopyridine was added thereto. The reaction solution was adjusted to pH = 9 with triethylamine and stirred overnight. Then, the solution was adjusted to pH = 4 with acetic acid, and then 0.05 g of sodium cyanoborohydride was added thereto and the whole was stirred overnight at room temperature. The reaction solution was concentrated under reduced pressure. The residue was purified by means of silica gel column chromatography (15 g, chloroform/methanol 20/1), consequently 0.04 g of the above-mentioned compound was obtained as a white solid product.
MS(FAB,Pos.):m/z=687[M+1]⁺
¹H-NMR(500MHz,DMSO-d₆) : δ=1.31, 1.38 (9H,2s), 1.51 (3H,d,J=6.8Hz), 1. .56 (2H,m), 1.75 (2H,m), 3.18 (2H,m), 4.40 (1H,brs), 4.48 (2H,brs), 4.5 6 (2H,brs), 5.69 (1H,m),6.42 (1H,s), 6.51 (1H,m), 7.23 (1H,m), 7.26-7. 35 (4H,m), 7.46 (1H,m), 7.54 (3H,m), 7,91 (2H,m), 8.09 (1H,d,J=7.5Hz), 8.39(1H,d,J=7.5Hz),8.51(2H,brs),8.65(1H,brs).

### Synthesis Example 94-2: Synthesis of (2S)-2-(4-2-picolylamino methyl) benzoyl-5-(pyridin-2-yl) aminovalerate (1'S)-1'-(1-naphthyl) ethylamide [Compound No. 109]

In 0.5 ml of thanol, 0.03 g of Synthesis Example was dissolved. Then, 0.5 ml of a 4 mol/l hydrochloric acid/dioxane solution was dropped in the solution at room temperature and the whole was stirred for 2 hours without modification. The reaction solution was concentrated and the residue was purified by means of silica gel column chromatography (7g, chloroform/methanol = 5/1), followed by treating with a 1 mol/l hydrochloric acid solution. Consequently, 0.03 g of hydrochloride of the above-mentioned compound was obtained as a light-yellow frothy compound.
MS(FAB,Pos.) :m/z=576[M+1]⁺
¹H-NMR(500MHz,DMSO-d₆) :δ=1.51(3H,d,J=6.8Hz),1.56(1H,m),1.61(1H ,m),1.67-1.85(2H,m),4.30(4H,brs),4.60(1H,m),5.70(1H,m),6.84(1 H,s),7.05(1H,brs),7.46(2H,brs),7.50-7.68(5H,m),7.80(2H,d,J=8. 3Hz),7.91(5H,m),8.10(1H,d,J=8.5Hz),8.60(1H,d,J=7.1Hz),8.66(1H ,d,J=7.5Hz), 8.82 (1H,brs).

### Synthesis Example 95: Production of (S)-2-(4-((N-imidazol-2-yl methyl) aminomethyl) benzoyl) amino-5-(4,5-dihydroimidazol-2-yl) aminovalerate (1'S)-1'-(1-naphthyl ethylamide [Compound No. 110]

### Synthesis Example 95-1: Synthesis of (S)-5-phthalimide-2-Boc-aminovalerate (Compound VIII-1)

In 135 ml of water, 13.35 g of commercially available ornithine hydrochloride was dissolved. Then, while being stirred in 100°C oil bath, 10.4 g of basic copper (II) carbonate was gradually added to the solution and the whole was stirred for 10 minutes while heating. The reaction suspension was filtrated. Water was added to and diluted the filtrate until the volume thereof was reached to 270 ml. Then, 13.2g of sodium carbonate and 19.1g of carboethoxy phthalimide were added to the filtrate and the whole was stirred for 2 hours at room temperature. The reaction suspension was cooled to 2°C and left standing overnight. A light-blue precipitate was collected by filtration and dried under reduced pressure. The precipitate was added to a mixture solution of 80 ml of a 4 mol/l hydrochloric acid and 80 ml of methanol and was dissolved therein. After the water layer was washed with ether, the solution was cooled to 2°C and left standing overnight. The resultingwhite precipitate was collected by filtration and dried under reduced pressure. The white precipitate was dissolved in 100 ml of DMF, and then 23.7 ml of triethylamine and 18.8 ml of di-t-butyldicarbonate were added tothesolution. Afterbeing reacted overnight at room temperature, the reaction solution was concentrated and diluted with chloroform. Then, the solution was washed twice with a saturated salt solution, and was then dried with anhydrous sodium sulfate. The solvent was distilled off. The resulting residue was purified by means of silica gel column chromatography (200 g, chloroform/methanol = 10/1), consequently 26.93 g of the above-mentioned compound was obtained as a colorless viscous liquid product.
MS(FAB,Pos.):m/z=363[M+1]⁺

### Synthesis Example 95-2: Synthesis of (S)-5-phthalimide-2-Boc-aminovalerate (1'S)-1'-(1-naphthyl)ethylamide (Compound IX-5)

In 194 ml of DMF, 19.4 g of the compound obtained in Synthesis Example 95-1 was dissolved. Then, 9.17 g of (S)-1-(1-naphthyl) ethylamine, 15.4 g of WSCI hydrochloride, and 10.9 g of HOBt were added to the solution. After being reacted all day and night, the reaction solution was concentrated. The resulting residue was diluted with chloroform and then a saturated sodium hydrogencarbonate aqueous solution was added thereto, followed by extracting with chloroform. An organic layer was washed with a saturated salt solution, and then the resultant was dried with anhydrous sodium sulfate and concentrated. The residue thus obtained was recrystallized from ethyl acetate, consequently 20.96 g of the above-mentioned compound was obtained.
MS(FAB,Pos,) :m/z=516[M+1]⁺
¹H-NMR(500MHz,CDCl₃) : δ=1.44(9H,s),1.50-1.70(4H,m),1.71(3H,d,J= 6Hz),3.66-3.71(1H,m),3.81-3.90(1H,m),4.37-4.43(1H,m),5.29(1H, d,J=8.3Hz),5.85(1H,dq,J=6.8,7.1Hz),7.32(1H,t,J=7.1Hz),7.41(2H ,t,J=7.3Hz),7.52(1H,d,J=7.3Hz),7.56-7.62(2H,m),7.63-7.70(2H,m ),7.71(1H,d,J=7.8Hz),7.74(1H,d,J=8.3Hz)7.96(1H,d,J=8.5Hz).

### Synthesis Example 95-3: Synthesis of (S)-2-(4-((N-Boc-N-imidazol -2-ylmethyl) aminomethyl) benzoyl) amino-5-phthalimide valerate (1'S)-1'-(1-naphthyl)ethylamide (Compound XI-20)

In 100 ml of methanol, 19.3 g of the compound obtained in Synthesis Example 95-2 was dissolved. Then, 100 ml of dioxane and 20 ml of concentrated hydrochloric acid were added to the solution and the whole was stirred for 4 hours at 45°C. On completion of the reaction, the solvent was distilled off and the residue was dissolved in 200 ml of DMF. 13.64 g of the compound obtained in Synthesis Example 79-1, 18.8 g of WSCI hydrochloride, 9.40 g of DMAP, and 7.60 g of HOBt were added to the solution and the whole was stirred for 24 hours at room temperature. On completion of the reaction, the solvent was distilled off and then chloroform was added to the residue. The residue was washed with a saturated sodium hydrogencarbonate aqueous solution and a saturated salt solution, and was then dried with anhydrous sodium sulfate. After that, the solvent was distilled off and the residue was then purif ied by means of silica gel column chromatography (chloroform/methanol = 20/1), consequently 26.24 g of the above-mentioned compound was obtained as a white solid product.
MS(FAB,Pos.) :m/z=729[M+1]⁺
¹H-NMR(500MHz,CDCl₃) : δ=1.50-1.82(16H,m),3.69-3.74(1H,m),3.93-4 .02(1H,m)4.01(2H,s),4.50(2H,s).5.00-5.07(1H,m),5.83-5.90(1H,m ),6.94(1H,d,J=8.3Hz),6.98(2H,s),7.12(1H,d,J=8.3Hz),7.24(1H,d, J=8.5Hz),7.37-7.43(2H,m),7.52-7.58(3H,m),7.64-7.69(2H,m),7.72 (1H,d,J=8.1Hz),7.75(1H,d,J=8.3Hz),7.79(2H,d,J=8.3Hz),7.98(1H, d, J=8.3Hz), 8.02(1H,s).

### Synthesis Example 95-4: Synthesis of (S)-2-(4-((N-Boc-N-imidazol -2-ylmethyl) aminomethyl) benzoyl) amino-5-aminovalerate (1'S) -1'-(1-naphthyl)ethylamide (Compound XII-3)

26.13 g of the compound obtained in Synthesis Example 95-3 was dissolved in a 40% methylamine/methanol solution and the whole was stirred for 24 hours at room temperature. On completion of the reaction, the solution was concentrated under reducedpressure. The residue was dissolved in chloroform and washed with distilled water and a saturated salt solution. Subsequently, the solution was dried with anhydrous sodium sulfate, and then the solvent was distilled off. The residue was purified by means of silica gel column chromatography (chloroform/methanol/water = 7/3/0.5), consequently 12.6 g of the above-mentioned compound was obtained as a white solid product.
MS(FAB,Pos.):m/z=599[M+1]⁺
¹H-NMR(500MHz,DMSO-d₆) : δ=1.40(9H,brs),1 .30-1.45(2H,m),1.51(3H, s,J=6.8Hz),1.62-1.78(2H,m),2.45-2.55(2H,m),4.33(2H,brs),4.43 (2H,s),4.40-4.52(1H,m),5.71(1H,quint.,J=6.8Hz),6.84(1H,s),7.05 (1H,s),7.20-7.32(2H,m),7.47-7.57(4H,m),7.82(1H,d,J=8.1Hz),7.8 4(2H,d,J=8.1Hz),7.94(1H,d,J=7.6Hz),8.10(1H,d,J=8.1Hz),8.48(1H ,d,J=7.8Hz),8.64(1H,d,J=7.8Hz),11.8-12.0(1H,br).

### Synthesis Example 95-5: Synthesis of (S)-2-(4-((N-imidazol-2-yl methyl) aminomethyl) benzoyl) amino-5-(4,5-dihydroimidazol-2-yl) aminovalerate (1'S)-1'-(1-naphthyl)ethylamide [Compound No. 110]

In 1 ml of DMF, 212.1 mg of the compound obtained in Synthesis Example 95-4, 91.1 mg of 2-(3,5-dimethylpyrazolyl)-4,5-dihydro imidazole hydrobromate, and 0.061 ml of diisopropylamine were dissolved and the whole was stirred for 24 hours at 80°C. On completion of the reaction, the solvent was distilled off under reduced pressure and then water was added to the residue, followed by extracting with chloroform. After the resultant was dried with magnesium sulfate, the solvent was distilled off under reduced pressure. The residue was purified by means of silica gel column chromatography (10 g, chloroform/methanol/water = 7/3/0.5) and was then dissolved in methanol. Then, 0.13 ml of a 4 mol/l hydrochloric acid/dioxane was added to the solution and the whole was stirred for 7 hours at room temperature. On completion of the reaction, the solvent was distilled off under reduced pressure. Consequently, 6.7 mg of the above-mentioned compound was obtained as a white solid product.
MS (FAB,Pos.) :m/z=577[M+1]⁺
¹H-NMR(500MHz,DMSO-d₆): δ=1.47-1.56(2H,m),1.52(3H,d,J=6.8Hz),1. 68-1.76(2H,m),3.11(2H,dd,J=7.1,12.7Hz),4.26(4H,br),4.56(1H,dd ,J=8.1,13.9Hz),5.71(1H,dd,J=6.8,14.4Hz),7.50-7.57(6H,m),7.62 (2H, d, J=8.1Hz), 7.84(1H, d, J=7.3Hz), 7.94-7.97(3H,m), 8.11(1H, d, J= 7.3Hz),8.30-8.32(1H,m),8.55(1H,d,J=8.8),8.78(1H,d,J=7.6Hz).

### Synthesis Example 96: Production of (S)-2-(4-((N-imidazol-2-yl methyl) aminomethyl) benzoyl) amino-5-(pyrimidin-2-yl) amino valerate (1'S)-1'-(1-naphthyl) ethylamide [Compound No. 111]

202.7 mg of the compound obtained in Synthesis Example 95-4, 57.0 mg of 2-bromopyrimidine, and 0.061 ml of diisopropylamine were dissolved in 2.4 ml of DMF and the whole was stirred for 45 hours at 80°C. On completion of the reaction, the solvent was distilled off under reduced pressure and the residue was dissolved in chloroform, followed by washing with a saturated salt solution. The resultant was dried with magnesium sulfate, and then the solvent was distilled off under reduced pressure. The residue was then purified by means of silica gel column chromatography (18 g, chloroform/methanol = 9/1) and was then dissolved in 1.2 ml of methanol. 1.2 ml of a 4 mol/l hydrochloric acid-dioxane was added to the solution and the whole was stirred for 7 hours at room temperature. After the reaction, the solvent was distilled off under reduced pressure, consequently a red brown crystal of the above-mentioned compound was obtained.
MS(FAB,Pos.) :m/z=577[M+1]⁺
¹H-NMR(500MHz,DMSO-d₆) : δ=1.51(3H,d,J=6.8Hz),1.53-1.62(2H,m),1. 71-1.79(2H,m),4.38(2H,brs),4.51(2H,brs),4.54-4.59(1H,m),5.67-5.72(1H,quint.,J=6.8Hz),6.69(1H,t,J=4.9Hz),7.46(1H,d,J=8.1Hz) ,7.49-7.55(3H,m),7.67(2H,d,J=8.3Hz),7.71(2H,s),7.81(1H,d,J=8. 1Hz),7.92-7.96(1H,m),7.96(2H,d,J=8.5Hz),8.09(1H,dd,J=2.2,8.2H z),8.36(2H,d,J=5.1Hz),8.54(1H,d,J=8.1Hz),8.74(1H,d,J=7.8Hz).

### Synthesis Example 97: Production of (S)-2-(4-((N-imidazol-2-yl methyl) aminomethyl) benzoyl) amino-5-(3,4,5,6-tetrahydro pyrimidin-2-yl) aminovalerate (1'S)-1'-(1-naphthyl) ethylamide [Compound No. 112]

In 8 ml of acetic acid, 97.3 mg of the compound obtained in Synthesis Example 96 was dissolved. Then, 0.9 ml of concentrated hydrochloric acid was added to the solution. 7 ml of an acetic acid solution of 67.2 mg of 10% palladium-carbon was added to the solution, followed by reacting the whole for 2 hours under 2.9 kg/cm² of filled hydrogen. Subsequently, the catalyst was removed, and then the solvent was distilled off under reduced pressure and azeotropically distilled with toluene. The residue was purified by means of silica gel column chromatography (3 g, chloroform/methanol/water = 7/3/0.5), consequently 82.7 mg of hydrochloride of the above-mentioned compound was obtained as a white solid product.
MS(FAB,Pos.): m/z=581[M+1]⁺
¹H-NMR(500MHz,DMSO-d₆) : δ=1. 42-1. 60 (2H,m), 1.52 (3H, d, J=6. 8Hz), 1. 68-1.85 (4H,m), 3.01-3.11(2H,m), 3.18-3.25(4H,m), 4.32(2H,s), 4.41 (2H,s), 4.52-4. 58 (1H,m), 5.72 (1H, quint., J=6.8Hz), 7.46-7.60(6H,m ),7.65 (2H, d, J=8. 3Hz), 7.76 (1H,brs), 7.82 (1H, d, J=8.1Hz), 7.95 (1H, d, J=7.3Hz), 7.98 (2H, d, J=8.3Hz), 8.10 (1H, d, J=8.1Hz), 8.58(1H, d, J= 8.1Hz),8.80(1H,d,J=7.8Hz).

### Synthesis Example 98: Production of (S)-2-(4-(N-2-picolylamino methyl) benzoyl) amino-5-(4,5-dihydroimidazol-2-yl) amino valerate (1'S)-1'-(1-naphthyl) ethylamide [Compound No. 113]

### Synthesis Example 98-1: Synthesis of (S)-2-(4-(N-Boc-N-2-picolyl aminomethyl) benzoyl) amino -5-aminovalerate (1'S)-1'-(1-naphthyl) ethylamide (Compound XII-4)

In 93 ml of DMF, 9.2827 g of the compound obtained in Synthesis Example 95-1 was dissolved, and 4.940 g of (1S)-1-(1-naphthyl) ethylamine, 7.4057 g of WSCI hydrochloride, and 5.336 g of HOBt were added to the solution and the whole was stirred for 16 hours at room temperature. On completion of the reaction, the solvent wad distilled off under reduced pressure, and then a saturated sodium hydrogencarbonate aqueous solution and chloroform were added to the residue. After being extracted with chloroform, a water layer was combined together with an organic layer and the whole was then washed with a saturated salt solution and dried with magnesium sulfate. Subsequently, the solvent was distilled off under reduced pressure. The residue was dissolved in 80 ml of dioxane, and then 80 ml of methanol and 8 ml of concentrated hydrochloric acid were added to the solution and the whole was stirred for 2 hours at 45°C. The solvent was distilled off under reduced pressure, and then the residue was suspended in chloroform and washed with a 1 mol/l sodium hydroxide aqueous solution. After the resultant was dried with magnesium sulfate, the solvent was distilled off under reduced pressure, the resultant residue was dissolved in 110 ml of DMF. Then, 9.6129 g of the compound obtained in Synthesis Example 1-2, 7.4937 gof WSCI hydrochloride, and 5.2943 g of HOBt were added to the solution and the whole was stirred for 16 hours at room temperature. On completion of the reaction, the solvent was distilled off under reduced pressure, and then a saturated sodium hydrogencarbonate aqueous solution and chloroform were added to the residue. A water layer which was extracted with chloroform was combined with the previously-separated organic layer and the whole was then washed with a saturated salt solution. The resultant was dried with magnesium sulfate and the solvent was distilled off under reduced pressure. Subsequently, theresiduewaspurifiedbymeansofsilica gel column chromatography (294 g, chloroform/ethyl acetate = 2/1). Then, 100 ml of a 40%-methylamine/methanol solution was added to the resulting compound, followed by stirring for 40 hours at room temperature. On completion of the reaction, the solvent was distilled off under reduced pressure and the residue was then purified by means of silica gel column chromatography (572 g, chloroform/methanol/water = 7/3/0.5), consequently 6.8058 g of the above-mentioned compound was obtained as a white solid product.
MS(FAB,Pos.):m/z=610[M+1]⁺
¹H-NMR(500MHz,CDCl₃):δ=1.34-1.42(2H,m), 1.35(9H,br),1.51(3H,d,J =7.1Hz),1.65-1.73(2H,m),3.16-3.44(2H,m),4.40(1H,brs),4.49-4.5 6(4H,m),5.68-5.72(1H,quint.,J=7.1Hz),7.20-7.34(4H,m),7.42-7.5 7(4H,m),7.76-7.80(1H,m),7.82(1H,d,J=7.8Hz),7.86(2H,d,J=8.3Hz) ,7.94(1H,d,J=7.6Hz),8.10(1H,d,J=8.1Hz),8.52(1H,d,J=4.9Hz).

### Synthesis Example 98-2: Synthesis of (S)-2-(4-(N-2-picolylamino methyl) benzoyl) amino-5-(4,5-dihyroimidazol-2-yl) amino valerate (1'S)-1'-(1-naphthyl) ethylamide [Compound No. 113]

In 1. 4ml of DMF, 279.0mg of the compound obtained in Synthesis Example 98-1, 178.1 mg of 2-(3,5-dimethylpirazolyl)-4,5-dihydroimidazole hydrobromate, and 0.127 ml of diisopropylamine were dissolved and the whole was stirred for 3.5 hours at 80°C. On completion of the reaction, the solvent was distilled off under reduced pressure and the residue was then purified by means of silica gel column chromatography (9g, chloroform/methanol = 10/1). The purified residue was dissolved in 5 ml of chloroform and then 0.5 ml of methanol and 0.256 ml of mesylic acid were added to the solution and the whole was stirred for 24 hours at room temperature. On completion of the reaction, the solvent was dissolved under reduced pressure and the residue was purified by means of silica gel column chromatography (8g, chloroform/methanol/water = 7/3/0.5), consequently 218.9 mg of mesylate of the above-mentioned compound was obtained as a white solid product.
MS(FAB, Pos.):m/z=578[M+1]⁺
¹H-NMR(500MHz,DMSO-d₆) : δ=1.42-1.57 (2H,m), 1.52 (3H, d, J=6.8Hz), 1. 64-1.78(2H,m),2.32(9H,s),3.10(2H,q,J=6.8Hz),3.56(4H,brs),4.31 (2H,brs), 4.33 (2H,brs), 4.55 (1H,dd, J=8.5, 14.2Hz), 5.72 (1H,quint. ,J=6.8Hz), 7.45-7.57 (6H,m), 7.62(2H, d, J=8.5Hz), 7.84(1H, d, J=8.1H z),7.91(1H,dt,J=1.7,7.8Hz),7.94(1H,d,J=2.44Hz),7.97(1H,d,J=8. 3Hz), 8.10(1H, d, J=7.1Hz), 8.22(1H, q, J=5.4Hz), 8.52(1H, J=8.1Hz), 8 .66-8.68(1H,m),8.70(1H,d,J=7.8Hz),9.56(2H,brs)

### Synthesis Example 99: Production of (S)-2-(4-(N-2-picolyl) aminomethyl) benzoyl) amino-5-(pyridin-2-yl) aminovalerate (1'S)-1'-(1-naphthyl) ethylamide [Compound No. 114]

In 1.6ml of DMF, 311.0 mg of the compound obtained in Synthesis Example 98-2, 97.1 mg of 2-bromopyrimidine, and 0.106 ml of diisopropylamine were dissolved and the whole was stirred for 23 hours at 80°C. On completion of the reaction, the solvent was distilled off under reduced pressure and the residue was dissolved in ethyl acetate and washed with a saturated salt solution. After the resultant was dried with magnesium sulfate, the solvent was distilled under reduced pressure, and the residue was purified by means of silica gel column chromatography (18 g, chloroform/methanol = 13/1). The purified residue was dissolved in 2.8 ml of methanol, and then 2.8 ml of a 4 mol/l hydrochloric acid/dioxane was added to the solution and the whole was stirred for 2 hours at room temperature. On completion of the reaction, the solvent was distilled off under reduced pressure, consequently 276.3 mg of hydrochloride of the above-mentioned compound was obtained as a white solid material.
MS(FAB,Pos.):m/z=588[M+1]⁺
¹H-NMR(500MHz,DMSO-d₆) : δ=1.51(3H,d,J=6.8Hz), 1.54-1.65(2H,m),1. 72-1.81(2H,m),3.30-3.38(2H,m),4.31(4H,brs),4.55-4.59(1H,m),5. 67-5.73(1H,quint.,J=6.8Hz),6.79(1H,brs),7.44-7.56(6H,m),7.64 (2H,d,J=8.5Hz),7.81(1H,d,J=8.1Hz),7.90-7.94(2H,m),7.97(2H,d,J= 8.1Hz),8.09(1H,d,J=7.6Hz),8.45(1H,d,J=4.2Hz),8.55(1H,d,J=8.1H z),8.66(1H,dd,J=1.0,3.9Hz),8.77(1H,d,J=7.6Hz),9.80-9.86(2H,br ).

### Synthesis Example 100: Production of (S)-2-(4-(N-2-picolylamino methyl) benzoyl) amino-5-(3,4,5,6-tetrahydropyrimidin-2-yl) aminovalerate (1'S)-1'-(1-naphthyl) ethylamide [Compound No. 115]

In 10 ml of acetic acid, 202.0 mg of the compound obtained in Synthesis Example 99 was dissolved. Then, 1.8 ml of concentrated hydrochloric acid was added to the solution. Subsequently, 10 ml of an acetic acid suspension of 109.2 mg of 10% palladium-carbon was added to the solution and the whole was reacted for 2 hours under 2.85 kg/cm² of filled hydrogen. On completion of the reaction, the catalyst was removed, and then the solvent was distilled off under reduced pressure and azeotropically distilled with toluene. After that, the residue was purified by means of silica gel column chromatography (6 g, chloroform/methanol/water = 7/3/0.5), consequently 189.5 mg of hydrochloride of the above-mentioned compound was obtained as a white solid product.
MS(FAB,Pos.):m/z=592(M+1]⁺
¹H-NMR(500MHz,DMSO-d₆):δ=1.40-1.60(2H,m),1.52(3H,d,J=6.8Hz),1. 65-1.84(4H,m),3.00-3.10(2H,m),3.18-3.22(4H,m),4.30(2H,s),4.31 (2H,s),4.53-4.58(1H,m),5.72(1H,quint.,J=6.8Hz),7.44-7.57(6H,m ),7.64(2H,d,J=8.3Hz),7.79(1H,br s) (1H,brs),7.82(1H,d,J=8.3Hz), 7.90(1H,td,J=7.8,1.7Hz),7.94(1H,d,J=7.6Hz),7.99(2H,d,J=8.3Hz) ,8.11(1H, d, J=8.1Hz), 8.59 (1H, d, J=8.1Hz), 8.66 (1H, ddd, J=4.9,1.7, 1.0Hz), 8.82 (1H,d,J=7.8Hz),9.70-9.90(2H,br).

### Synthesis Example 101: Production of [Compound No. 116] to [Compound No. 130]

As described below, hydrochlorides of the respective above-mentioned compounds were obtained by the same operation as that of Synthesis Example 66-5.

### Synthesis of (S)-2-(4-(2-picolylaminomethyl)benzoyl)-5-(2-picolylamino) valerate 1'-(1-naphthyl) ethylamide [Compound No. 116]

Using 26.4 mg of 1'-(1-naphthyl) ethylamine, 46.6 mg of hydrochloride of the above-mentioned compound was obtained as a white solid product by similar operation.
MS(FAB,Pos.):m/z=601[M+1]⁺
¹H-NMR(500MHz,DMSO-d₆):δ=1.50(1.5H,d,J=6.8Hz),1.51(1.5H,d,J=6. 8Hz),1.68-1.94(4H,m),2.92-3.04(2H,m),4.24-4.36(6H,m),4.52-4.6 0(1H,m),5.62-5.72(1H,m),7.44-7.68(10H,m),7.80-7.84(1H,m),7.88 - 8.02(5H,m),8.06-8.12(1H,m),8.60-8.70(3H,m),8.83(0.5H,d,J=7.8 Hz),8.88(0.5H,d,J=7.6Hz),9.40(2H,bs),9.96(2H,bs).

### Synthesis of (S)-2-(4-(2-picolylaminomethyl)benzoyl)-5-(2-picolylamino) valerate n-dodecylamide [Compound No. 117]

Using 28.6 mg of n-dodecylamine, 58.0 mg of hydrochloride of the above-mentioned compound was obtained as a white solid product by similar operation.
MS(FAB,Pos.) :m/z=615[M+1]⁺
¹H-NMR(500MHz,DMSO-d₆):δ=0.85(3H,t,J=6.8Hz),1.16-1.32(18H,m),1 .32-1.44(2H,m),1.68-1.88(4H,m),2.94-3.08(4H,m),4.28-4.38(6H,m ),4.40-4.48(1H,m),7.44-7.50(2H,m),7.58-7.62(2H,m),7.66(2H,d,J =8.3Hz),7.90-7.96(2H,m),7.980(2H,d,J=8.3Hz),8.14(1H,t,J=5.6Hz ),8.60-8.70(3H,m),9.36-9.44(2H,bs),9.90-10.02(2H,bs).

### Synthesis of (S)-2-(4-(2-picolylaminomethyl)benzoyl)-5-(2-picolylamino) valerate 3,5-ditrifluoromethylbenzylamide [Compound No. 118]

Using 37.5 mg of 3,5-ditrifluoromethylbenzylamine, 57.8 mg of hydrochloride of the above-mentioned compound was obtained as a white solid product by similar operation.
MS (FAB,Pos.) :m/z=673[M+1]⁺
¹H-NMR (500MHz, DMSO-d₆) : δ=1.70-1.96 (4H,m), 2. 96-3.06 (2H,m), 4.30-4.36(6H,m),4.40-4.58(3H,m),7.42-7.50(2H,m),7.56-7.60(2H,m),7. 678(2H, d, J=8.3Hz),7.90-7.96(2H,m), 7.96-8.06 (5H,m), 8.63 (1H, d, J =4.9Hz), 8.66 (1H, d, J=4.9Hz), 8.80(1H, d, J=7, 6Hz), 8.94(1H, t, J=6, 1 Hz),9.30-9.40(2H,bs),9.88-10.00(2H,bs).

### Synthesis of (S)-2-(4-(2-picolylaminomethyl)benzoyl)-5-(2-picolylamino) valerate (+)-dehydroabietylamide [Compound No. 119]

Using 44.1 mg of (+)-dehydroabietylamine, 65.9 mg of hydrochloride of the above-mentioned compound was obtained as a white solid product by similar operation.
MS(FAB,Pos.):m/z=715[M+1]⁺
¹H-NMR(500MHz,DMSO-d₆) : δ=0.70-1.90(24H,m),2.18-2.30(1H,m),2.66 - 2.84(4H,m),2.86-3.04(2H,m),3.06-3.14(1H,m),4.24-4.36(6H,m),4 .50-4.56(1H,m),6.77(0.5H,s),6.86(0.5H,s),6.90-6.98(1H,m),7.10 - 7.18(1H,m),7.44-7.52(2H,m),7.56-7.70(4H,m),7.82-8.04(5H,m),8 .58-8.70(3H,m),9.36-9.50(2H,bs),9.90-10.08 (2H,bs).

### Synthesis of (S)-2-(4-(2-picolylaminomethyl)benzoyl)-5-(2-picolylamino) valerate2,3-dichlorobenzylamide[Compound No.120]

Using 27.0 mg of 2,3-dichlorobenzylamine, 53.0 mg of hydrochloride of the above-mentioned compound was obtained as a white solid product by similar operation.
MS(FAB,Pos.):m/z=606,608,610[M+1]⁺
¹H-NMR(500MHz,DMSO-d₆):δ=1.76-1.98 (4H,m),2.98-3.06(2H,m),4.30-4.36(6H,m),4.38(2H,d,J=5.9Hz),4.51-4.61(1H,m),7.34-7.36(2H,m) ,7.43-7.50(2H,m),7.53-7.62(3H,m),7.68(2H,d,J=8.3Hz),7.90-7.98 (2H,m),8.01(2H,d,J=8.3Hz),8.63-8.67(2H,m),8.77(1H,d,J=8.1Hz), 8.81(1H,t,J=5.9Hz),9.40(1H,bs),9.97(1H,bs).

### Synthesis of (S)-2-(4-(2-picolylaminomethyl)benzoyl)-5-(2-picolylamino) valerate 2-octylamide [Compound No. 121]

Using 20.0 mg of 2-octylamine, 45.1 mg of hydrochloride of the above-mentioned compound was obtained as a white solid product by similar operation.
MS(FAB,Pos.):m/z=559[M+1]⁺
¹H-NMR(500MHz,DMSO-d₆) : δ=0.82(1.5H,d,J=8.1Hz), 0.83 (1.5H,d,J=8. 1Hz),1.02(3H,t,J=6.8Hz),1.10-1.30(8H,m),1.30-1.44(2H,m),1.68-1.86(4H,m),2.94-3.06(2H,m),3.64-3.78(1H,m),4.24-4.38(6H,m),4. 40-4.48(1H,m),7.44-7.54(2H,m),7.58-7.66(2H,m),7.673(2H,d,J=8. 3Hz), 7.90-8.02 (5H,m), 8.56-8.62 (1H,m), 8.62-8.68 (2H,m), 9.40-9.5 0(2H,bs),9.96-10.06(2H,bs).

### Synthesis of (S)-2-(4-(2-picolylaminomethyl)benzoyl)-5-(2-picolylamino) valerate 3-(3-indolyl)-2-propylamide [Compound No. 122]

Using 27.0 mg of 3-(3-indolyl)-2-propylamine, 57.0 mg of hydrochloride of the above-mentioned compound was obtained as a white solid product by similar operation.
MS (FAB, Pos.):m/z=604[M+1]⁺
¹H-NMR (500MHz, DMSO-d₆) : δ=1.03 and 1.07 (3H, d, J=6.6Hz), 1.50-1, 82 (4H,m), 2.64-3.10 (4H,m), 4.04 (1H,quint, J=6.6Hz), 4.20-4.40(6H,m) ,4.32-4.54(1H,m), 6.82-7.36 (4H,m), 7.40-7.50 (2H,m), 7.50-7.70 (5H ,m),7.89-8.20(5H,m),8.54-8.67(3H,m),9.34-0.52(2H,bs),9.90-10. 10(2H,brs).

### Synthesis of (S)-2-(4-(2-picolylaminomethyl)benzoyl)-5-(2-picolylamino) valerate 2,2-diphenylethylamide [Compound No. 123]

Using 30.5 mg of 2,2-diphenylethylamine, 55.0 mg of hydrochloride of the above-mentioned compound was obtained as a white solid product by similar operation.
MS(FAB,Pos.):m/z=627[M+1]⁺
¹H-NMR(500MHz,DMSO-d₆):δ=1.48-1.72(4H,m),2.80-2.92(2H,m),3.62-3.70(1H,m),3.76-3.84(1H,m),4.20(1H,t,J=7.8Hz),4.24-4.34(6H,m) ,4.36-4.40(1H,m),7.14-7.20(2H,m),7.22-7.30(8H,m),7.46-7.50(2H ,m),7.58(1H,d,J=7.8Hz),7.61(1H,d,J=7.8Hz),7.66(2H,d,J=8.3Hz), 7.90-7.98(4H,m),8.13(1H,t,J=5.4Hz),8.52(1H,d,J=8.3Hz),8.62-8. 64(1H,m),8.66-8.68(1H,m),9.30-9.40(2H,bs),9.92-10.02(2H,bs).

### Synthesis of (S)-2-(4-(2-picolylaminomethyl)benzoyl)-5-(2-picolylamino) valerate 4-t-butylcyclohexylamide [Compound No. 124]

Using 24.0 mg of 4-t-butylcyclohexylamine, 49.8 mg of hydrochloride of the above-mentioned compound was obtained as a white solid product by similar operation.
MS(FAB,Pos.):m/z=555[M+1]⁺
¹H-NMR(500MHz,DMSO-d₆) : δ=0.80 (3H,s), 0.82 (6H,s), 0.88-1.34 (4H,m) , 1.36-1.50 (1H,m), 1.66-1.86 (8H,m), 2.94-3.04 (2H,m), 3.38-3.48 (1H ,m), 4.26-4.36 (6H,m), 4.46-4.56 (0.5H,m), 4.58-4.62 (0.5H,m), 7.44-7.50 (2H,m), 7.58-7.64 (2H,m), 7.66 (2H,d,J=8.3Hz), 7.90-8.00 (4.5H, m), 8.06 (0.5H,d,J=7.8Hz), 8.57 (0.5H,d,J=8.1Hz), 8.60-8.70 (2.5H,m ),9.36-9.46(2H,bs),9.94-10.00(2H,bs).

### Synthesis of (S)-2-(4-(2-picolylaminomethyl)benzoyl)-5-(2-picolylamino) valerate 2,4-dichlorobenzylamide [Compound No. 125]

Using 27.2 mg of 2,4-dichlorobenzylamine, 60.1 mg of hydrochloride of the above-mentioned compound was obtained as a white solid product by similar operation.
MS(FAB,Pos.) :m/z=606,608,610[M+1]⁺
¹H-NMR(500MHz,DMSO-d₆) :δ=1.72-1.96(4H,m),2.96-3.06(2H,m),4.26-4.38(8H,m),4.50-4.58(1H,m),7.38-7.44(2H,m),7.46-7.52(2H,m),7. 58-7.66(3H,m),7.68(2H,d,J=8.3Hz),7.92-7.98(2H,m),8.00(2H,d,J= 8.3Hz),8.64-8.68(2H,m),8.76-8.82(2H,m),9.40-9.50(2H,bs),9.98-10.08(2H,bs).

### Synthesis of (S)-2-(4-(2-picolylaminomethyl)benzoyl)-5-(2-picolylamino) valerate benzhydrylamide [Compound No. 126]

Using 28.3 mg of benzhydrylamine, 56.8 mg of hydrochloride of the above-mentioned compound was obtained as a white solid product by similar operation.
MS (FAB,Pos.):m/z=613[M+1]⁺
¹H-NMR(500MHz,DMSO-d₆):δ=1.70-1.96(4H,m),2.94-3.06(2H,m),4.26-4.36(6H,m),4.64-4.70(1H,m),6.11(1H,d,8.5Hz),7.20-7.28(2H,m),7 .30-7.40(6H,m),7.40-7.50(3H,m),7.53(1H,d,J=7.3Hz),7.58(2H,t,J =8.1Hz), 7.667(2H, d, J=8.3Hz), 7.88-7.96 (2H,m), 7.98 (2H,d,J=8.3Hz ),8.62(1H,d,J=4.2Hz),8.66(1H,d,J=4.4Hz),8.71(1H,d,J=8.3Hz),8. 81(1H,d,J=8.5Hz),9.36-9.42(2H,bs),9.88-9.96(2H,bs).

### Synthesis of (S)-2-(4-(2-picolylaminomethyl)benzoyl)-5-(2-picolylamino) valerate 3-chlorobenzylamide [Compound No. 127]

Using 21.9 mg of 3-chlorobenzylamine, 54.9 mg of hydrochloride of the above-mentioned compound was obtained as a white solid product by similar operation.
MS(FAB,Pos.):m/z=571,573[M+1]⁺
¹H-NMR(500MHz,DMSO-d₆):δ=1.70-1.96(4H,m),2.96-3.06(2H,m),4.24-4.38(8H,m),4.48-4.54(1H,m),7.23(1H,d,J=7.8Hz),7.26-7.36(3H,m) ,7.44-7.52(2H,m),7.58-7.66(2H,m),7.68(2H,d,J=8.3Hz),7.90-8.00 (2H,m),8.00(2H,d,J=8.3Hz),8.62-8.68(2H,m),8.84-8.90(2H,m),9.3 8-9.50 (2H,bs), 9.96-10.06 (2H,bs).

### Synthesis of (S)-2-(4-(2-picolylaminomethyl)benzoyl)-5-(2-picolylamino) valerate 2-(4-methoxyphenyl)ethylamide [Compound No. 128]

Using 23. 3 mg of 2- (4-methoxyphenyl) ethylamine, 46. 7 mg of hydrochloride of the above-mentioned compound was obtained as a white solid product by similar operation.
MS(FAB,Pos.):m/z=581[M+1]⁺
¹H-NMR(500MHz,DMSO-d₆) δ=1.66-1.84 (4H,m), 2.649 (2H,t,J=7.3Hz), 2 .90-3.02 (2H,m), 3.18-3.30 (2H,m), 3.68 (3H,s), 4.22-4.38 (6H,m), 4.4 0-4.46(1H,m),6.804(2H,d,J=8.5Hz),7.11(2H,d,J=8.5Hz),7.44-7.50 (2H,m),7.58-7.64(2H,m),7.68(2H,d,J=8.3Hz),7.90-7.96(2H,m),7.9 9(2H,d,J=8.3Hz),8.21(1H,t,J=5.6Hz),8.60-8.68(3H,m),9.36-9.46 (2H,bs),9.96-10.06(2H,bs).

### Synthesis of (S)-2-(4-(2-picolylaminomethyl)benzoyl)-5-(2-picolylamino) valerate (4-(4-methylphenyl)oxy)phenylamide [Compound No. 129]

Using 38.0 mg of (4- (4-methylphenyl) oxy) phenylamine, 58.0 mg of hydrochloride of the above-mentioned compound was obtained as a white solid product by similar operation.
MS(FAB,Pos.):m/z=629[M+1]⁺
¹H-NMR(500MHz,DMSO-d₆) : δ=1.76-1.98 (4H,m), 2.30 (3H,s), 2.96-3.06 (2H,m), 4.26-4.38 (6H,m), 4.38-4.48 (1H,m), 6.94-6.98 (2H,m), 7.04-7. 08 (2H,m), 7.20-7.26 (2H,m), 7.38-7.40 (2H,m), 7.44-7.50 (2H,m), 7.58-7.64 (2H,m), 7.66-7.72 (2H,m), 7.90-8.00 (4H,m), 8.62-8.68 (2H,m), 8 .80 (0.5H,d,J=7.8Hz), 8.96 (0.5H,d,J=7.6Hz), 9.38-9.48 (2H,bs), 9.9 6-10.06 (2H,bs) .

### Synthesis of (S)-2-(4-(2-picolylaminomethyl)benzoyl)-5-(2-picolylamino) valerate 1-(1,2,3,4-tetrahydronaphthyl)amide [Compound No. 130]

Using 22.8 mg of 1-(1,2,3,4-tetrahydronaphthyl)amine, 52.2 mg of hydrochloride of the above-mentioned compound was obtained as a white solid product by similar operation.
MS(FAB,Pos.):m/z=577[M+1]⁺
¹H-NMR(500MHz,DMSO-d₆):δ=1.64-1.94(8H,m),2.64-2.80(2H,m),2.92-3.06(2H,m),4.24-4.36(6H,m),4.46-4.56(1H,m),4.92-5.00(1H,m),7. 06-7.22(4H,m),7.44-7.522H,m),7.56-7.82(4H,m),7.88-8.02(4H,m), 8.43(0.5H,d,J=8.8Hz),8.50(0.5H,d,J=8.5Hz),8.58-8.70(2H,m),9.3 4-9.52(2H,bs),9.88-10.10(2H,bs).

Next, the present invention will be described more concretely by representing Examples.

### Example 1

### CXCR4-binding inhibition activity

MT-4 cells (5 x 10⁶/0.2 ml/well) were cultured on a 24-well microtiter plate. After the cells were incubated for 24 hours at 37°C in a carbon dioxide gas incubator, a culture medium was replaced with a buffer solution (RPMI-1640 containing 0.1%BSA). Together with a ligand ¹²⁵I-SDF-1α (specific activity: 2,2000 Ci/mmol; DAIICHI PURE CHEMICALS CO.,LTD. (Tokyo)), various concentrations of test materials were subjected to a binding reaction for 2 hours under ice cold condition. Ligands that did not bind in cold PBS were washed out, and then the radioactivities of bound ligands were measured by Scintillation Counter (Nihon Packard K.K (Tokyo)) and calculated a rate (a binding-inhibition % at 0.1 µM) that a test material inhibits the binding between radio-active ligands and receptors CXCR4.

The results are shown in Table 1 below.

**Table 1**

| Compound No. | inhibition rate | Compound No. | inhibition rate |
|---|---|---|---|
| 5 | 26.4 | 59 | 86.0 |
| 16 | 75.3 | 60 | 16.8 |
| 19 | 73.8 | 61 | 32.9 |
| 21 | 12.8 | 64 | 51.6 |
| 26 | 78.6 | 65 | 75.1 |
| 27 | 82.1 | 83 | 78.0 |
| 28 | 78.1 | 85 | 90.7 |
| 29 | 56.8 | 86 | 91.4 |
| 30 | 60.9 | 87 | 57.9 |
| 38 | 90.0 | 91 | 94.0 |
| 40 | 85.3 | 95 | 100.0 |
| 41 | 97.9 | 96 | 96.8 |
| 43 | 43.6 | 97 | 100.0 |
| 44 | 88.1 | 99 | 76.4 |
| 45 | 95.8 | 104 | 40.6 |
| 49 | 97.8 | 105 | 91.9 |
| 53 | 71.5 | 106 | 100.0 |
| 55 | 94.6 | 107 | 96.5 |
| 56 | 69.9 | 110 | 75.7 |
| 57 | 97.8 | 112 | 84.2 |
| 58 | 52.0 | AMD3100 | 10.0 |

### Example 2

### Acute toxicity

7-week-old ICR mice (male) (obtained from Charls River Japan Inc) were divided into three-animals for each groups. After domestication-breeding for 1 week, the compound of the corresponding Synthesis Example was dissolved in a physiological salt solution or distilled water and the solution was then intraperitoneally dosed twice a day for 4 days (50 mg/kg in dosage). The number of dead mice after 5 days was determined and the results were shown in Table 2.

As shown in Table 2, it was confirmed that the administration of any compound did not cause death at all and did not show acute toxicity.

**Table 2**

| Compound No. | Number of dead mice | Compound No. | Number of dead mice |
|---|---|---|---|
| 39 | 0/3 | 92 | 0/3 |
| 41 | 0/3 | 96 | 0/3 |
| 49 | 0/3 | 97 | 0/3 |
| 57 | 0/3 | 99 | 0/3 |
| 65 | 0/3 | 106 | 0/3 |
| 86 | 0/3 | 107 | 0/3 |

### Example 3

### Drug efficacy test of the compound No. 86 on type-II collagen-induced arthritis mouse

Using the type-II collagen-induced mouse arthritis (CIA) model, which was a model of articular rheumatism (RA), the compound No. 86 was tested for drug efficacy.

24 of 6-week-old male DBA/1 mice (obtained from Charles River Japan, Inc.) were used as test animal and were divided in groups of 12 animals, that is, a test group of and a control group, such that the average weights of the respective groups were almost equal to each other.

An initial-sensitizing antigen solution used was a liquid of adjusted emulsion prepared by dissolving chicken type-II collagen (obtained from Collagen Gijyutu Kensyuukai) in 0.01N acetic acid (4mg/mL) and then mixing with an equal amount of Freund's complete adjuvant (manufactured by Difco BRL), and an additional-sensitizing antigen solution used was a liquid of emulsion prepared by dissolving chicken type-II collagen in 0.01N acetic acid (8 mg/mL), and then mixing with an equal amount of Freund's complete adjuvant.

An initial sensitization was performed by endodermic injection of 50 µl of the initial-sensitizing antigen solution (containing 100 µg of chicken type-II collagen) into the tail of the mouse. The additional sensitization was performed after 21 days from the initial sensitization by endodermic injection of 50 µl of the additional-sensitizing antigen solution (containing 200 µg of chicken type-II collagen) into the tail of the mouse.

The compound No. 86, which was provided as a test material, was provided as one at a concentration of 5 mg/mL prepared by dissolving ditartrate thereof in a physiological saline solution. The test material was administered such that the test material was interperitoneally administered once a day for consecutive 17 days from 2 days before the additional sensitization (an amount of administered liquid was 10 mL/kg). In addition, for the control group, the test material was administered by dissolving ditartrate in a physiological saline solution so as to make the concentration of tartaric acid of 1.7 mg/mL because a dosage solvent is ditartrate for the test material compound No. 86.

Amacroscopic observation on extremities after the additional sensitization was performed once a day. A scoring of the macroscopic observation was according to the following criteria and judged on each of extremities with respect to edema of foot. The total of the scores of extremities was provided as an index of the onset of symptoms and pharmacometrics with respect to arthritis. In addition, the total of the numbers of fingers of each extremity on which edema was observed was also used as an index of pharmacometrics.

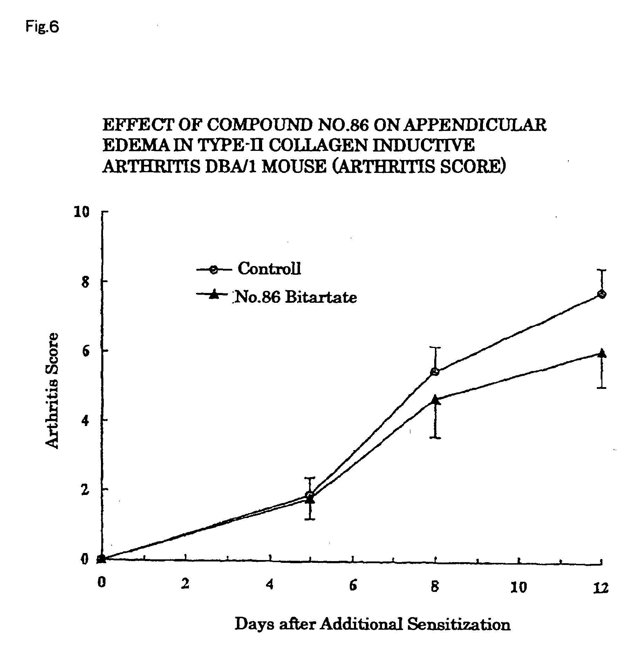

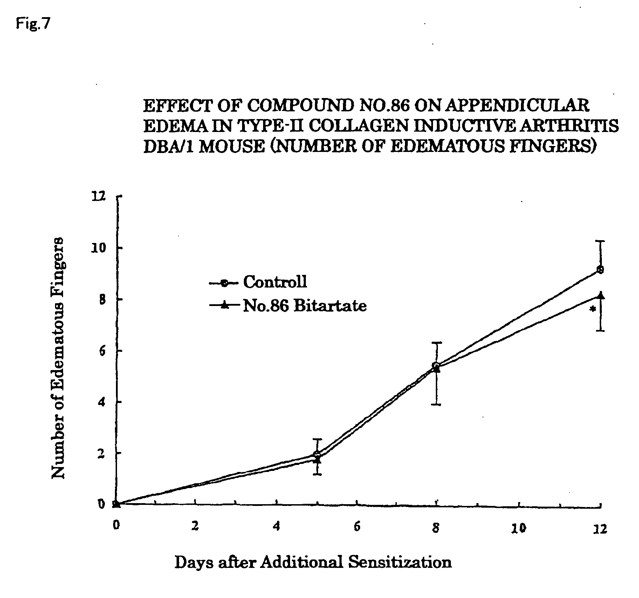

The results of the observation for 12 days after the additional sensitization were shown in Fig. 6 and Fig. 7. In addition, the scores in Fig. 6 were defined as follows:

| Score | Symptom |
|---|---|
| 0 | No edema |
| 1 | Edema on one finger |
| 2 | Edema on two or more fingers or dorsum pedis |
| 3 | Edema on the whole foot |

As is clear from Fig. 6 and Fig. 7, after the additional sensitization, edema on the finger, edema on the dorsum pedis, or edema on the whole foot was found in all animals of the control group, and the lesion score of extremities and the number of edematous fingers were increased with the passage of time. On the other hand, the compound No. 86 tartrate group tended to inhibit a chronological increase in lesion score of extremities or the number of edematous fingers. After 12 days from the additional sensitization, an increase in number of edematous fingers was significantly inhibited. Furthermore, no influence was found on change in animal body weights.

From those findings, it was presumed that the compound No. 86 tartrate was able to inhibit the onset of mouse CIA.

### Example 4

34.6% of the compound No. 86, 34.6% of lactose (Japanese Pharmacopoeia, hereinafter referred to as JP), 17.3% of corn starch (JP), 7.3% of hydroxypropylcellulose (JP), and 6.2% of low-substitution hydroxypropylcellulose (JP) were sieved and then mixed well in a vinyl bag. The same amount of purified water (JP) as that of those compounds was added thereto and then a wet cake was obtained by kneading the mixture for 20 minutes by a biaxial kneader. The wet cake was granulated using an extrusive granulating machine (diameter of cylindrical hole:1mm), and then the granulated product was dried using a fluid bed dryer (40°C, 30 minutes). The dried granules were sieved. Subsequently, magnesium stearate was added to the sieved product in the proportions of 1% magnesium stearate to 99% sieved product and then the whole was mixed well, followed by making tablets therefrom using a tableting machine. Tablets having an average weight of 292 mg were obtained.

In addition, an undercoat solution was prepared by dissolving 8% of hydroxypropyl methylcellulose 2910 (JP) and 1.6% of macrogol 6000 (JP) in purified water (JP) so as to be 100% in total. An under coat tablet was prepared by spraying the undercoat solution using a hicoater in a ratio of 5% with respect to the weight of the tablet which was previously made and subjecting the sprayed tablet to drying for 20 minutes after the spraying.

Furthermore, an enteric coating solution was prepared by dissolving 10% of hydroxypropyl methylcellulose acetate succinate (Pharmaceutical additive specification), 3% of triethyl citrate (JP), 2% of titanium oxide (JP), and 0.05% of hydroxypropyl cellulose (JP) in purified water (JP) so as to be 100% in total. The enteric coating solution was sprayed using a hicoater in a ratio of 10% with respect to the weight of the tablet. The enteric tablet was prepared by drying the tablet for 30 minutes, after the spraying. This enteric tablet had properties of not allowing a main component to be eluted within 2 hours in one liquid (JP), and allowing 80% or more of the main component to be eluted within 30 minutes in 2 liquids (JP).

### Industrial Applicability

Novel nitrogen-containing compounds and salts thereof of the present invention are able to provide novel CXCR4 antagonists. The novel CXCR4 antagonist of the present invention has a novel CXCR4 antagonism and shows excellent effects as a therapeutic or preventive agent for disease, such as rheumatism, cancer metastases, and so on.

## Claims

1. A CXCR4 antagonist comprising a nitrogen-containing compound represented by the following general formula (I) or a pharmaceutically acceptable salt thereof as an active ingredient: 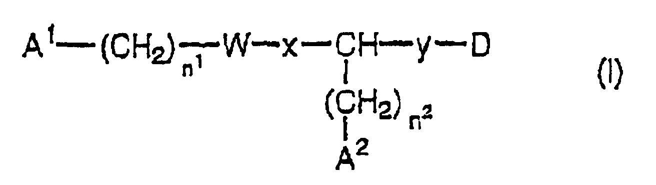 wherein n¹ represents an integer of 0 to 3, and n² represents an integer of 0 to 4, and
each of A¹ and A² independently represents (a) a guanidino group, which may be substituted with a nitro group, a cyano group, an alkyl group having 1 to 6 carbon atoms, or an alkylene group having 2 or 3 carbon atoms, (b) an amidino group, which may be substituted with a nitro group, a cyano group, an alkyl group having 1 to 6 carbon atoms, or an alkylene group having 2 or 3 carbon atoms, or (c) a group represented by the following formula (i): 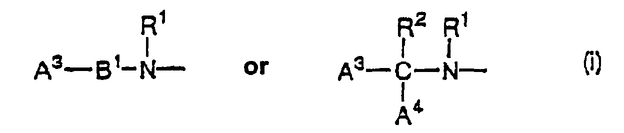 wherein each of A³ and A⁴ independently represents a 5 to 12-membered monocyclic or polycyclic heterocyclic aromatic ring, which contains 1 to 4 nitrogen atoms and may contain 1 or 2 other hetero atoms wherein a hydrogen atom on said nitrogen atoms may be substituted, or a 5 to 12-membered monocyclic or polycyclic heterocyclic aromatic ring, which contains 1 to 3 nitrogen atoms and may contain 1 or 2 other hetero atoms,
wherein a hydrogen atom on said nitrogen atoms may be substituted and said heterocyclic aromatic ring may be partially saturated, and
B¹ represents a single bond or a group represented by the following formula (ii): 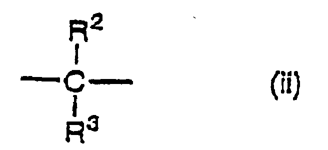 wherein each of R¹, R², an R³ independently represents a hydrogen atom, an alkyl group having 1 to 6 carbon atoms, which may be substituted, an alkenyl group having 2 to 6 carbon atoms, which may be substituted, and an alkynyl group having 2 to 6 carbon atoms, which may be substituted, and R² may bind to R¹ or R² to form a ring,
W represents an alkylene group having 1 to 7 carbon atoms, which may be substituted, an alkenylene group having 2 to 7 carbon atoms, which may be substituted, an alkynylene group having 2 to 7 carbon atoms, which may be substituted, or a monocyclic or polycyclic cyclic alkylene group having 3 to 10 carbon atoms, which may be substituted, or a 6 to 15-membered monocyclic or polycyclic aromatic ring which may be substituted, or a partially-saturated 6 to 15-membered monocyclic or polycyclic aromatic ring which may be substituted, or a 5 to 15-membered monocyclic or polycyclic heterocyclic aromatic ring, which may contain 1 to 3 oxygen atoms, 1 to 3 sulfur atoms, and 1 to 3 nitrogen atoms and may be substituted, or a partially-saturated 5 to 15-membered monocyclic or polycyclic heterocyclic aromatic ring, which may contain 1 to 3 oxygen atoms, 1 to 3 sulfur atoms, and 1 to 3 nitrogen atoms and may be substituted, or a 3 to 15-membered monocyclic or polycyclic saturated heterocyclic ring, which may contain 1 to 3 oxygen atoms, 1 to 3 sulfur atoms, and 1 to 3 nitrogen atoms and may be substituted, and
D represents a functional group represented by the following formula (iii) :
**-W**^{**1**}**-G**^{**1**}**-G**^{**2**}**-W**^{**2**}**-G**^{**3**} **(iii)**
wherein W¹ represents an oxygen atom, a sulfur atom, or a functional group represented by the following formula (iv): 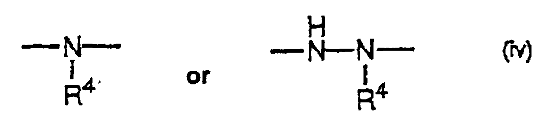 wherein R⁴ represents a hydrogen atom, or -G^{1'}-G^{2'}-W^{2'}-G^{3'}-, and
each of G¹ and G^{1'} independently represents a single bond, or a straight- or branched-chain alkylene group having 1 to 10 carbon atoms, which may be substituted, a straight- or branched-chain alkenylene group having 2 to 10 carbon atoms and 1 or 2 double bonds, which may be substituted, a straight- or branched-chain alkynylene group having 2 to 10 carbon atoms and 1 or 2 triple bonds, which may be substituted, or a functional group represented by the following formula (v): 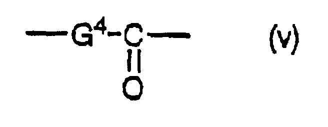 wherein G⁴ represents an alkylene group having 1 to 3 carbon atoms, which may be substituted,
each of G² and G^{2'} independently represents a single bond, or a monocyclic or polycyclic cyclic alkylene group having 3 to 10 carbon atoms, which may be substituted, a 6 to 15-membered monocyclic or polycyclic aromatic ring, which may be substituted, or a partially-saturated 6 to 15-membered monocyclic or polycyclic aromatic ring, which may be substituted, or a 5 to 15-membered monocyclic or polycyclic heterocyclic aromatic ring, which may have 1 to 3 oxygen atoms, 1 to 3 sulfur atoms, and 1 to 3 nitrogen atoms and may be substituted, or a partially-saturated 5 to 15-membered monocyclic or polycyclic heterocyclic aromatic ring, which may have 1 to 3 oxygen atoms, 1 to 3 sulfur atoms, and 1 to 3 nitrogen atoms and may be substituted, or a 3 to 15-memered saturated heterocyclic ring which may contain 1 to 3 oxygen atoms, 1 to 3 sulfur atoms, and 1 to 3 nitrogen atoms and may be substituted, and
each of W² and W^{2'} independently represents a single bond, or an oxygen atom, a sulfur atom, or a functional group represented by the following formula (vi): 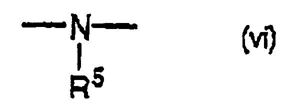 wherein R⁵ represents a hydrogen atom, a straight- or branched-chain alkyl group having 1 to 10 carbon atoms, which may be substituted, or G³", which may form a ring with G¹ or G² when R⁵ represents the alkyl group,
each of G³, G^{3'}, and G^{3"} independently represents a hydrogen atom, a straight- or branched-chain alkyl group having 1 to 6 carbon atoms, which may be substituted, a straight- or branched-chain alkenyl group having 2 to 6 carbon atoms and 1 or 2 double bonds, which may be substituted, a straight- or branched-chain alkynyl group having 2 to 6 carbon atoms and 1 or 2 triple bonds, which may be substituted, or a monocyclic or polycyclic cyclic alkylene group having 3 to 10 carbon atoms, which may be substituted, or an aralkyl group having 7 to 15 carbon atoms, whichmaybe substituted, or a 6 to 15-membered monocyclic or polycyclic aromatic ring, which may be substituted, or a partially-saturated 6 to 15-membered monocyclic or polycyclic aromatic ring which may be substituted, or a 5 to 15-membered monocyclic orpolycyclic heterocyclic aromatic ring, which may contain 1 to 3 oxygen atoms, 1 to 3 sulfur atoms, and 1 to 3 nitrogen atoms and may be substituted, or a partially-saturated 5 to 15-membered monocyclic or polycyclic heterocyclic aromatic ring, which may contain 1 to 3 oxygen atoms, 1 to 3 sulfur atoms, and 1 to 3 nitrogen atoms and may be substituted, or a 3 to 15-membered saturated heterocyclic ring, which may contain 1 to 3 oxygen atoms, 1 to 3 sulfur atoms, and 1 to 3 nitrogen atoms and may be substituted, and
x represents a functional group represented by the following formula (vii): 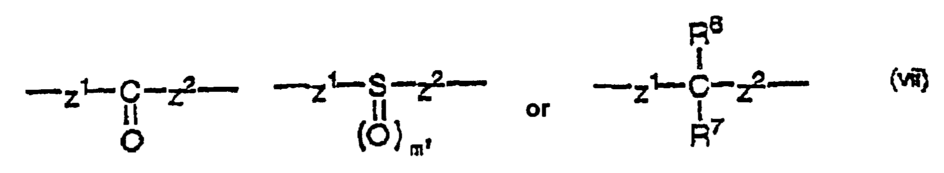 wherein each of z¹ and z² independently represents a single bond, a methylene group, an oxygen atom, a sulfur atom, or a substituent represented by the following formula (viii): 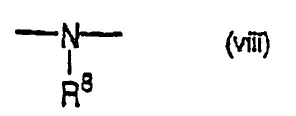 wherein each of R⁶, R⁷, and R⁸ is a hydrogen atom, or an alkyl group having 1 to 3 carbon atoms, which may be substituted, and m¹ represents an integer of 0 to 2, and
y represents a functional group represented by the following formula (ix): 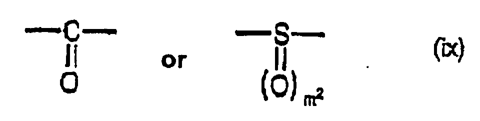 wherein m² represents an integer of 0 to 2,
wherein, when the compounds include asymmetric points, each of absolute configurations thereof includes R, S, or a mixture thereof.

2. A CXCR4 antagonist comprising the compound or the salt thereof according to Claim 1 as an active ingredient, wherein n¹ represents 1 or 2 and n² represents 2 or 3 in the general formula (I).

3. A CXCR4 antagonist comprising the compound or the salt thereof according to Claim 1 or 2 as an active ingredient, wherein, in the general formula (vii), z¹ represents a single bond and z² represents the following formula (viii'): 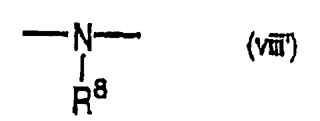 (wherein R⁸ represents a hydrogen atom or an alkyl group having 1 to 3 carbon atoms, which may be substituted).

4. A CXCR4 antagonist comprising the compound or the salt thereof according to any one of Claims 1 to 3 as an active ingredient, wherein, in the general formula (I), y represents the following general formula (ix').

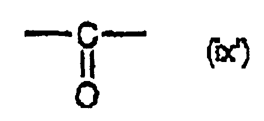

5. A CXCR4 antagonist comprising the compound or the salt thereof according to any one of Claims 1 to 4 as an active ingredient, wherein, in the general formula (iii), W¹ represents the following formula (iv').

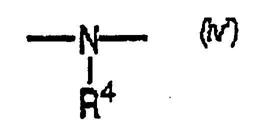

6. A CXCR4 antagonist comprising the compound or the salt thereof according to any one of Claims 1 to 5 as an active ingredient, wherein, in the general formula (I), each of A¹ and A² represents a guanidino group or is represented by the following formula (ia), A³ represents a monocyclic heterocyclic aromatic ring having 1 or 2 hetero atoms wherein a hydrogen atom on said nitrogen atoms may be substituted, or a bicyclic heterocyclic aromatic ring having 1 or 2 hetero atoms wherein a hydrogen atom on said nitrogen atoms may be substituted and said heterocyclic aromatic ring may be partially saturated, W represents an alkylene group having 2 to 3 carbon atoms, a cyclic alkylene group having 5 to 10 carbon atoms, a monocyclic or bicyclic aromatic ring having 6 to 10 carbon atoms, or a monocyclic or bicyclic heterocyclic aromatic ring having 5 to 10 carbon atoms, y represents -C(=O)-, x represents (CH₂)ₙ₃- (C=O)-NH-, n³ represents 0 or 1, and n¹, n², and D are the same as defined in the previous claims:

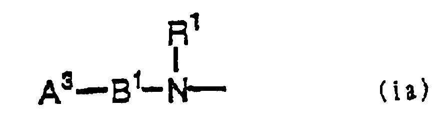

7. A CXCR4 antagonist comprising the compound or the salt thereof according to any one of Claims 1 to 6 as an active ingredient, wherein, in the general formula (I), W¹ represents -NR⁴-, R⁴ represents a hydrogen atom or a straight- or branched-chain alkyl group having 1 to 5 carbon atoms, G¹ represents a straight- or branched-chain alkylene group having 1 to 5 carbon atoms, G² represents a single bond, W² represents a single bond, or an oxygen atom or a sulfur atom, G³ represents a monocyclic or polycyclic aromatic ring having 6 to 15 carbon atoms, which may be substituted, or a 3 to 15-memberedmonocyclic or polycyclic heterocyclic aromatic ring, which may contain 1 to 3 oxygen atoms, 1 to 3 sulfur atoms, and 1 to 3 nitrogen atoms and may be substituted.

8. A CXCR4 antagonist according to any one of Claims 1 to 7, which is an associated-disease improving agent based on a CXCR4 antagonism.

9. A CXCR4 antagonist according to any one of Claims 1 to 7, which is an agent for improving rheumatism.

10. A CXCR4 antagonist according to any one of Claims 1 to 7, which is an agent for improving cancer metastasis.
